# EUROPEAN PATENT APPLICATION

(11) **EP 3 028 707 A1**
(43) Date of publication of application: **08.06.2016**
(21) Application number: 16150606.8
(22) Date of filing: 28.04.2010
(51) Int. Cl.: A61K 31/7088, A61K 39/395, C07K 14/00, C07K 16/22, C12N 15/115

(54) **METHODS FOR TREATING OR PREVENTING OPHTHALMOLOGICAL DISEASES**

(30) Priority: 01.05.2009 US 174746 P; 13.05.2009 US 178010 P; 25.09.2009 US 245784 P
(62) Divisional of application: 10770279.7
(71) Applicant: Ophthotech Corporation, New York, NY 10119 (US)
(72) Inventor: Patel, Samir, New York, NY 10119 (US); Masonson, Harvey, New York, NY 10119 (US)
(74) Representative: Maiwald Patentanwalts GmbH

(57) **Abstract**

This invention relates to methods and compositions useful for the treatment or prevention of an ophthalmological disease, comprising administration of an effective amount of a PDGF antagonist and a VEGF antagonist to a mammal in need thereof.

## Description

### 1. RELATED APPLICATIONS

This application claims the benefit of U.S. Provisional Application No. 61/174,746, filed May 1,2009, U.S. Provisional Application No. 61/178,010, filed May 13, 2009, and U.S. Provisional Application No. 61/245,784, filed September 25, 2009, each of which is incorporated by reference herein in its entirety.

### 2. FIELD OF THE INVENTION

This invention relates to methods and compositions useful for the treatment or prevention of an ophthalmological disease, comprising administration of an effective amount of (a) ARC-127, Antagonist A, Antagonist B, Antagonist C, Antagonist D, 1B3 antibody, CDP860, IMC-3G3, imatinib, 162.62 antibody, 163.31 antibody, 169.14 antibody, 169.31 antibody, αR1 antibody, 2A1E2 antibody, M4TS.11 antibody, M4TS.22 antibody, A10, brefeldin A, sunitinib, Hyb 120.1.2.1.2 antibody, Hyb 121.6.1.1.1 antibody, Hyb 127.5.7.3.1 antibody, Hyb 127.8.2.2.2 antibody, Hyb 1.6.1 antibody, Hyb 1.11.1 antibody, Hyb 1.17.1 antibody, Hyb 1.18.1 antibody, Hyb 1.19.1 antibody, Hyb 1.23.1 antibody, Hyb 1.24 antibody, Hyb 1.25 antibody, Hyb 1.29 antibody, Hyb 1.33 antibody, Hyb 1.38 antibody, Hyb 1.39 antibody, Hyb 1.40 antibody, Hyb 1.45 antibody, Hyb 1.46 antibody, Hyb 1.48 antibody, Hyb 1.49 antibody, Hyb 1.51 antibody, Hyb 6.4.1 antibody, F3 antibody, Humanized F3 antibody, C1 antibody, Humanized C1 antibody, 6.4 antibody, anti-mPDGF-C goat IgG antibody, C3.1 antibody, 5-methyl-7-diethylamino-s-triazolo (1,5-a) pyrimidine, interferon, protamine, PDGFR-B1 monoclonal antibody, PDGFR-B2 monoclonal antibody, 6D11 monoclonal antibody, Sis 1 monoclonal antibody, PR7212 monoclonal antibody, PR292 monoclonal antibody, HYB 9610 monoclonal antibody, HYB 9611 monoclonal antibody, HYB 9612 monoclonal antibody, HYB 9613 monoclonal antibody, 4-(2-(N-(-2-carboxamidoindole) aminoethyl)-benzenesulfonamide, 4-(2-(N-(-2-carboxamidoindole)aminoethyl)-sulfonylurea, CGP 53716, human antibody g 162, pyrazolo[3,4-g]quinoxaline, 6-[2-(methylcarbamoyl)phenylsulphanyl]-3-E-[2-(pyridiue-2-yl)ethenyl]-indazole, 1-{2-[5-(2-methoxy-ethoxy)-benzoimidazole-1-yl]-quinoline-8-yl}-piperidine-4-ylamine, 4-[4-[N-(4-nitrophenyl)carbamoyl]-1-piperazinyl]-6,7-dimethoxyquinazoline, 4-amino-5-fluoro-3-(6-(4-methyl-piperazine-1-yl)-1H-benzimidazole2-yl)-1H-quinoline-2-one, (4-tert-butylphenyl){4-[(6,7-dimethoxy-4-quinolyl)oxy]phenyl}methaneone, 5-methyl-N-[4-(trifluoromethyl)phenyl]-4-isoxazolecarboxamide, trans-4-[(6,7-dimethoxyquinoxaline-2-yl)amino]cyclohexanol, (Z)-3-[(2,4-dimethyl-5-(2-oxo-1,2-dihydroindole-3-ylidenemetethyl)-1H-pyrrole-3-yl)-propionic acid, 5-(5-fluoro-2-oxo-1,2-dihydroindole-3-ylidenemethyl)-2,4-dimethyl-1H-pyrrole-3-carboxylic acid, 1-(4-chloroanilino)-4-(4-pyridylmethyl)phthalazine, N-[4-(3-amino-1H-indazole-4-yl)phenyl-N'-(2-fluoro-5-methylphenyl)urea, 1,2-dimethyl-7- (2-thiophene) imidazolo [5, 4-g] quinoxaline, 1, 2-dimethyl-6-phenyl imidazolo [5, 4-g] quinoxaline, 1, 2-dimethyl-6- (2-thiophene) imidazolo [5, 4-g] quinoxaline, AG1295, AG1296, 3-arylquinoline, 4-pyridyl-2-arylpyrimidine, sorafenib, MLN518, PKC412, AMN107, suramin, or neomycin, or a pharmaceutically acceptable salt thereof and (b) ranibizumab, bevacizumab, aflibercept, KH902 VEGF receptor-Fc fusion protein, 2C3 antibody, ORA102, pegaptanib, bevasiranib, SIRNA-027, decursin, decursinol, picropodophyllin, guggulsterone, PLG101, eicosanoid LXA4, PTK787, pazopanib, axitinib, CDDO-Me, CDDO-Imm, shikonin, beta-hydroxyisovalerylshikonin, ganglioside GM3, DC101 antibody, Mab25 antibody, Mab73 antibody, 4A5 antibody, 4E10 antibody, 5F12 antibody, VA01 antibody, BL2 antibody, VEGF-related protein, sFLT01, sFLT02, Peptide B3, TG100801, sorafenib, or G6-31 antibody, or a pharmaceutically acceptable salt thereof, to a mammal in need thereof.

### 3. BACKGROUND OF THE INVENTION

Various disorders of the eye are characterized, caused by, or result in choroidal, retinal or iris neovascularization or retinal edema. One of these disorders is macular degeneration. Age-related macular degeneration (AMD) is a disease that affects approximately one in ten Americans over the age of 65. One type of AMD, "wet-AMD" accounts for only 10% of age-related macular degeneration cases but results in 90% of cases of legal blindness from macular degeneration in the elderly. Another disorder of the eye is diabetic retinopathy. Diabetic retinopathy can affect up to 80% of all patients having diabetes for 10 years or more and is the third leading cause of adult blindness, accounting for almost 7% of blindness in the USA. Other disorders include hypertensive retinopathy, central serous chorioretinopathy, cystoid macular edema, Coats disease and ocular or adnexal neoplasms such as choroidal hemangioma, retinal pigment epithelial carcinoma and intraocular lymphoma.

Therefore, although advances in the understanding of the molecular events accompanying neovascularization have been made, there exists a need to utilize this understanding to develop improved methods for treating or preventing neovascular diseases disorders, including ocular neovascular diseases and disorders such as the neovascularization that occurs with AMD and diabetic retinopathy.

### 4. SUMMARY OF THE INVENTION

In one aspect the invention provides methods for treating or preventing an ophthalmological disease, comprising administering to a mammal in need thereof an effective amount of (a) ARC-127 or imatinib, or a pharmaceutically acceptable salt thereof; and (b) ranibizumab, bevacizumab, aflibercept, KH902 VEGF receptor-Fc fusion protein, ORA102, bevasiranib, SIRNA-027, decursin, decursinol, picropodophyllin, guggulsterone, PLG101, eicosanoid LXA4, PTK787, pazopanib, axitinib, CDDO-Me, CDDO-Imm, shikonin, beta-hydroxyisovalerylshikonin, or ganglioside GM3, DC101 antibody, Mab25 antibody, Mab73 antibody, 4A5 antibody, 4E10 antibody, 5F12 antibody, VA01 antibody, BL2 antibody, VEGF-related protein, sFLT01, sFLT02, Peptide B3, TG100801, sorafenib, or G6-31 antibody, or a pharmaceutically acceptable salt thereof.

In another aspect the invention provides methods for treating or preventing an ophthalmological disease, comprising administering to a mammal in need thereof an effective amount of (a) 1B3 antibody, CDP860, TMC-3G3, 162.62 antibody, 163.31 antibody, 169.14 antibody, 169.31 antibody, αR1 antibody, 2A1E2 antibody, M4TS.11 antibody, M4TS.22 antibody, A10, brefeldin A, sunitinib, Hyb 120.1.2.1.2 antibody, Hyb 121.6.1.1.1 antibody, Hyb 127.5.7.3.1 antibody, Hyb 127.8.2.2.2 antibody, Hyb 1.6.1 antibody, Hyb 1.11.1 antibody, Hyb 1.17.1 antibody, Hyb 1.18.1 antibody, Hyb 1.19.1 antibody, Hyb 1.23.1 antibody, Hyb 1.24 antibody, Hyb 1.25 antibody, Hyb 1.29 antibody, Hyb 1.33 antibody, Hyb 1.38 antibody, Hyb 1.39 antibody, Hyb 1.40 antibody, Hyb 1.45 antibody, Hyb 1.46 antibody, Hyb 1.48 antibody, Hyb 1.49 antibody, Hyb 1.51 antibody, Hyb 6.4.1 antibody, F3 antibody, Humanized F3 antibody, C1 antibody, Humanized C1 antibody, 6.4 antibody, anti-mPDGF-C goat IgG antibody, C3.1 antibody, 5-methyl-7-diethylamino-s-triazolo (1,5-a) pyrimidine, interferon, protamine, PDGFR-B1 monoclonal antibody, PDGFR-B2 monoclonal antibody, 6D11 monoclonal antibody, Sis 1 monoclonal antibody, PR7212 monoclonal antibody, PR292 monoclonal antibody, HYB 9610 monoclonal antibody, HYB 9611 monoclonal antibody, HYB 9612 monoclonal antibody, HYB 9613 monoclonal antibody, 4-(2-(N-(-2-carboxamidoindole)aminoethyl)-benzenesulfonamide,4-(2-(N-(-2-carboxamidoindole)aminoethyl)-sulfonylurea, CGP 53716, human antibody g162, pyrazolo[3,4-g]quinoxaline, 6-[2-(methylcarbamoyl)phenylsulphanyl]-3-E-[2-(pyridine-2-yl)ethenyl]-indazole, 1-{2-[5-(2-methoxy-ethoxy)-benzoimidazole-1-yl]-quinoline-8-yl}-piperidine-4-ylamine, 4-[4-[N-(4-nitrophenyl)carbamoyl]-1-piperazinyl]-6,7-dimethoxyquinazoline, 4-amino-5-fluoro-3-(6-(4-methyl-piperazine-1-yl)-1H-benzimidazole-2-yl)-1H-quinoline-2-one, (4-tert-butylphenyl){4-[(6,7-dimethoxy-4-quinolyl)oxy]phenyl}methaneone, 5-methyl-N-[4-(trifluoromethyl)phenyl]-4-isoxazolecarboxamide, trans-4-[(6,7-dimethoxyquinoxaline-2-yl)amino]cyclohexanol, (Z)-3-[(2,4-dimethyl-5-(2-oxo-1,2-dihydroindole-3-ylidenemethyl)-1H-pyrrole-3-yl)-propionic acid, 5-(5-fluoro-2-oxo-1,2-dihydroindole-3-ylidenemethyl)-2,4-dimethyl-1H-pyrrole-3-carboxylic acid, 1-(4-chloroanilino)-4-(4-pyridylmethyl)phthalazine, N-[4-(3-amino-1H-indazole-4-yl)phenyl-N'-(2-fluoro-5-methylphenyl)urea, 1, 2-dimethyl-7- (2-thiophene) imidazolo [5, 4-g] quinoxaline, 1, 2-dimethyl-6-phenyl imidazolo [5, 4-g] quinoxaline, 1, 2-dimethyl-6- (2-thiophene) imidazolo [5, 4-g] quinoxaline, AG1295, AG1296, 3-arylquinoline, 4-pyridyl-2-arylpyrimidine, sorafenib, MLN518, PKC412, AMN107, suramin, or neomycin, or a pharmaceutically acceptable salt thereof; and (b) ranibizumab, bevacizumab, aflibercept, KH902 VEGF receptor-Fc fusion protein, 2C3 antibody, ORA102, pegaptanib, bevasiranib, SIRNA-027, decursin, decursinol, picropodophyllin, guggulsterone, PLG101, eicosanoid LXA4, PTK787, pazopanib, axitinib, CDDO-Me, CDDO-Imm, shikonin, beta-hydroxyisovalerylshikonin, or ganglioside GM3, DC101 antibody, Mab25 antibody, Mab73 antibody, 4A5 antibody, 4E10 antibody, SF12 antibody, VA01 antibody, BL2 antibody, VEGF-related protein, sFLT01, sFLT02, Peptide B3, TG100801, sorafenib, or G6-31 antibody, or a pharmaceutically acceptable salt thereof.

In another aspect the invention provides methods for treating or preventing an ophthalmological disease, comprising administering to a mammal in need thereof an effective amount of (a) ARC-127 or imatinib, or a pharmaceutically acceptable salt thereof; and (b) 2C3 antibody or pegaptanib, or a pharmaceutically acceptable salt thereof, wherein the ophthalmological disease is choroidal vasculopathy, condition associated with choroidal neovascularization, hypertensive retinopathy, sickle cell retinopathy, condition associated with peripheral retinal neovascularization, retinopathy of prematurity, venous occlusive disease, arterial occlusive disease, central serous chorioretinopathy, cystoid macular edema, retinal telangiectasia, arterial macroaneurysm, retinal angiomatosis, radiation-induced retinopathy, or a neoplasm.

In another aspect the invention provides methods for treating or preventing an ophthalmological disease, comprising administering to a mammal in need thereof an effective amount of (a) Antagonist A, a compound of Formula A or a pharmaceutically acceptable salt thereof; and (b) ranibizumab, bevacizumab, aflibercept, KH902 VEGF receptor-Fc fusion protein, 2C3 antibody, ORA102, pegaptanib, bevasiranib, SIRNA-027, decursin, decursinol, picropodophyllin, guggulsterone, PLG101, eicosanoid LXA4, PTK787, pazopanib, axitinib, CDDO-Me, CDDO-Imm, shikonin, beta-hydroxyisovalerylshikonin, or ganglioside GM3, DC101 antibody, Mab25 antibody, Mab73 antibody, 4A5 antibody, 4E10 antibody, 5F12 antibody, VA01 antibody, BL2 antibody, VEGF-related protein, sFLT01, sFLT02, Peptide B3, TG100801, sorafenib, or G6-31 antibody, or a pharmaceutically acceptable salt thereof.

In another aspect the invention provides methods for treating or preventing an ophthalmological disease, comprising administering to a mammal in need thereof an effective amount of (a) Antagonist B, a compound of Formula B or a pharmaceutically acceptable salt thereof; and (b) ranibizumab, bevacizumab, aflibercept, KH902 VEGF receptor-Fc fusion protein, 2C3 antibody, ORA102, pegaptanib, bevasiranib, SIRNA-027, decursin, decursinol, picropodophyllin, guggulsterone, PLG101, eicosanoid LXA4, PTK787, pazopanib, axitinib, CDDO-Me, CDDO-Imm, shikonin, beta-hydroxyisovalerylshikonin, or ganglioside GM3, DC101 antibody, Mab25 antibody, Mab73 antibody, 4A5 antibody, 4E10 antibody, 5F12 antibody, VA01 antibody, BL2 antibody, VEGF-related protein, sFLT01, sFLT02, Peptide B3, TG100801, sorafenib, or G6-31 antibody, or a pharmaceutically acceptable salt thereof.

In another aspect the invention provides methods for treating or preventing an ophthalmological disease, comprising administering to a mammal in need thereof an effective amount of (a) Antagonist C, a compound of Formula C or a pharmaceutically acceptable salt thereof; and (b) ranibizumab, bevacizumab, aflibercept, KH902 VEGF receptor-Fc fusion protein, 2C3 antibody, ORA102, pegaptanib, bevasiranib, SIRNA-027, decursin, decursinol, picropodophyllin, guggulsterone, PLG101, eicosanoid LXA4, PTK787, pazopanib, axitinib, CDDO-Me, CDDO-Imm, shikonin, beta-hydroxyisovalcrylshikonin, or ganglioside GM3, DC101 antibody, Mab25 antibody, Mab73 antibody, 4A5 antibody, 4E10 antibody, 5F12 antibody, VA01 antibody, BL2 antibody, VEGF-related protein, sFLT01, sFLT02, Peptide B3, TG100801, sorafenib, or G6-31 antibody, or a pharmaceutically acceptable salt thereof.

In another aspect the invention provides methods for treating or preventing an ophthalmological disease, comprising administering to a mammal in need thereof an effective amount of (a) Antagonist D, a compound of Formula E or a pharmaceutically acceptable salt thereof; and (b) ranibizumab, bevacizumab, aflibercept, KH902 VEGF receptor-Fc fusion protein, 2C3 antibody, ORA102, pegaptanib, bevasiranib, SIRNA-027, decursin, decursinol, picropodophyllin, guggulsterone, PLG101, eicosanoid LXA4, PTK787, pazopanib, axitinib, CDDO-Me, CDDO-Imm, shikonin, beta-hydroxyisovalerylshikonin, or ganglioside GM3, DC101 antibody, Mab25 antibody, Mab73 antibody, 4A5 antibody, 4E10 antibody, 5F12 antibody, VA01 antibody, BL2 antibody, VEGF-related protein, sFLT01, sFLT02, Peptide B3, TG100801, sorafenib, or G6-31 antibody, or a pharmaceutically acceptable salt thereof.

In another aspect the invention provides methods for treating or preventing an ophthalmological disease, comprising administering to a mammal in need thereof an effective amount of (a) 1B3 antibody, CDP860, IMC-3G3, 162.62 antibody, 163.31 antibody, 169.14 antibody, 169.31 antibody, αR1 antibody, 2A1E2 antibody, M4TS.11 antibody, M4TS.22 antibody, Hyb 120.1.2.1.2 antibody, Hyb 121.6.1.1.1 antibody, Hyb 127.5.7.3.1 antibody, Hyb 127.8.2.2.2 antibody, Hyb 1.6.1 antibody, Hyb 1.11.1 antibody, Hyb 1.17.1 antibody, Hyb 1.18.1 antibody, Hyb 1.19.1 antibody, Hyb 1.23.1 antibody, Hyb 1.24 antibody, Hyb 1.25 antibody, Hyb 1.29 antibody, Hyb 1.33 antibody, Hyb 1.38 antibody, Hyb 1.39 antibody, Hyb 1.40 antibody, Hyb 1.45 antibody, Hyb 1.46 antibody, Hyb 1.48 antibody, Hyb 1.49 antibody, Hyb 1.51 antibody, Hyb 6.4.1 antibody, F3 antibody, Humanized F3 antibody, C1 antibody, Humanized C1 antibody, 6.4 antibody, anti-mPDGF-C goat IgG antibody, C3.1 antibody, PDGFR-B1 monoclonal antibody, PDGFR-B2 monoclonal antibody, 6D11 monoclonal antibody, Sis 1 monoclonal antibody, PR7212 monoclonal antibody, PR292 monoclonal antibody, HYB 9610 monoclonal antibody, HYB 9611 monoclonal antibody, HYB 9612 monoclonal antibody, or HYB 9613 monoclonal antibody, or a pharmaceutically acceptable salt thereof; and (b) ranibizumab, bevacizumab, aflibercept, KH902 VEGF receptor-Fc fusion protein, 2C3 antibody, ORA102, pegaptanib, bevasiranib, SIRNA-027, decursin, decursinol, picropodophyllin, guggulsterone, PLG101, eicosanoid LXA4, PTK787, pazopanib, axitinib, CDDO-Me, CDDO-Imm, shikonin, beta-hydroxyisovalerylshikonin, or ganglioside GM3, DC101 antibody, Mab25 antibody, Mab73 antibody, 4A5 antibody, 4E10 antibody, 5F12 antibody, VA01 antibody, BL2 antibody, VEGF-related protein, sFLT01, sFLT02, Peptide B3, TG100801, sorafenib, or G6-31 antibody, or a pharmaceutically acceptable salt thereof.

The invention provides compositions comprising an effective amount of (a) ARC-127, Antagonist A, Antagonist B, Antagonist C, Antagonist D, 1B3 antibody, CDP860, IMC-3G3, imatinib, 162.62 antibody, 163.31 antibody, 169.14 antibody, 169.31 antibody, αR1 antibody, 2A1E2 antibody, M4TS.11 antibody, M4TS.22 antibody, A10, brefeldin A, sunitinib, Hyb 120.1.2.1.2 antibody, Hyb 121.6.1.1.1 antibody, Hyb 127.5.7.3.1 antibody, Hyb 127.8.2.2.2 antibody, Hyb 1.6.1 antibody, Hyb 1.11.1 antibody, Hyb 1.17.1 antibody, Hyb 1.18.1 antibody, Hyb 1.19.1 antibody, Hyb 1.23.1 antibody, Hyb 1.24 antibody, Hyb 1.25 antibody, Hyb 1.29 antibody, Hyb 1.33 antibody, Hyb 1.38 antibody, Hyb 1.39 antibody, Hyb 1.40 antibody, Hyb 1.45 antibody, Hyb 1.46 antibody, Hyb 1.48 antibody, Hyb 1.49 antibody, Hyb 1.51 antibody, Hyb 6.4.1 antibody, F3 antibody, Humanized F3 antibody, C1 antibody, Humanized C1 antibody, 6.4 antibody, anti-mPDGF-C goat IgG antibody, C3.1 antibody, 5-methyl-7-diethylamino-s-triazolo (1,5-a) pyrimidine, interferon, protamine, PDGFR-B1 monoclonal antibody, PDGFR-B2 monoclonal antibody, 6D11 monoclonal antibody, Sis 1 monoclonal antibody, PR7212 monoclonal antibody, PR292 monoclonal antibody, IIYB 9610 monoclonal antibody, HYD 9611 monoclonal antibody, HYB 9612 monoclonal antibody, HYB 9613 monoclonal antibody, 4-(2-(N-(-2-carboxamidoindole)aminoethyl)-benzenesulfonamide, 4-(2-(N-(-2-carboxamidoindole)aminoethyl)-sulfonylurea, CGP 53716, human antibody g 162, pyrazolo[3,4-g]quinoxaline, 6-[2-(methylcarbamoyl)phenylsulphanyl]-3-E-[2-(pyridine-2-yl)ethenyl]-indazole, 1-{2-[5-(2-methoxy-ethoxy)-benzoimidazole-1-yl]-quinoline-8-yl}-piperidine-4-ylamine, 4-[4-[N-(4-nitrophenyl)carbamoyl]-1-piperazinyl]-6,7-dimethoxyquinazoline, 4-amino-5-fluoro-3-(6-(4-methyl-piperazine-1-yl)-1H-benzimidazole-2-yl)-1H-quinoline-2-one, (4-tert-butylphenyl){4-[(6,7-dimethoxy-4-quinolyl)oxy]phenyl}methaneone, 5-methyl-N-[4-(trifluoromethyl)phenyl]-4-isoxazolecarboxamide, trans-4-[(6,7-dimethoxyquinoxaline-2-yl)amino]cyclohexanol, (Z)-3-[(2,4-dimethyl-5-(2-oxo-1,2-dihydroindole-3-ylidenemethyl)-1H-pyrrole-3-yl)-propionic acid, 5-(5-fluoro-2-oxo-1,2-dihydroindole-3-ylidenemethyl)-2,4-dimethyl-1H-pyrrole-3-carboxylic acid, 1-(4-chloroanilino)-4-(4-pyridylmethyl)phthalazine, N-[4-(3-amino-1H-indazole-4-yl)phenyl-N'-(2-fluoro-5-methylphenyl)urea, 1, 2-dimethyl-7- (2-thiophene) imidazolo [5, 4-g] quinoxaline, 1, 2-dimethyl-6-phenyl imidazolo [5, 4-g] quinoxaline, 1, 2-dimethyl-6- (2-thiophene) imidazolo [5, 4-g] quinoxaline, AG1295, AG1296, 3-arylquinoline, 4-pyridyl-2-arylpyrimidine, sorafenib, MLN518, PKC412, AMN107, suramin, or neomycin, or a pharmaceutically acceptable salt thereof; (b) ranibizumab, bevacizumab, aflibercept, KH902 VEGF receptor-Fc fusion protein, 2C3 antibody, ORA102, pegaptanib, bevasiranib, SIRNA-027, decursin, decursinol, picropodophyllin, guggulsterone, PLG101, eicosanoid LXA4, PTK787, pazopanib, axitinib, CDDO-Me, CDDO-Imm, shikonin, beta-hydroxyisovalerylshikonin, or ganglioside GM3, DC101 antibody, Mab25 antibody, Mab73 antibody, 4A5 antibody, 4E10 antibody, 5F12 antibody, VA01 antibody, BL2 antibody, VEGF-related protein, sFLT01, sFLT02, Peptide B3, TG100801, sorafenib, or G6-31 antibody, or a pharmaceutically acceptable salt thereof; and (c) a pharmaceutically acceptable carrier or vehicle.

The invention provides compositions comprising an effective amount of (a) Antagonist A or a pharmaceutically acceptable salt thereof; (b) ranibizumab, bevacizumab, aflibercept, KH902 VEGF receptor-Fc fusion protein, 2C3 antibody, ORA102, pegaptanib, bevasiranib, SIRNA-027, decursin, decursinol, picropodophyllin, guggulsterone, PLG101, eicosanoid LXA4, PTK787, pazopanib, axitinib, CDDO-Me, CDDO-Imm, shikonin, beta-hydroxyisovalerylshikonin, or ganglioside GM3, DC101 antibody, Mab25 antibody, Mab73 antibody, 4A5 antibody, 4E10 antibody, 5F12 antibody, VA01 antibody, BL2 antibody, VEGF-related protein, sFLT01, sFLT02, Peptide B3, TG100801, sorafenib, or G6-31 antibody, or a pharmaceutically acceptable salt thereof; and (c) a pharmaceutically acceptable carrier or vehicle.

The invention provides compositions comprising an effective amount of (a) Antagonist B or a pharmaceutically acceptable salt thereof; (b) ranibizumab, bevacizumab, aflibercept, KH902 VEGF receptor-Fc fusion protein, 2C3 antibody, ORA102, pegaptanib, bevasiranib, SIRNA-027, decursin, decursinol, picropodophyllin, guggulsterone, PLG101, cicosanoid LXA4, PTK787, pazopanib, axitinib, CDDO-Mc, CDDO-Imm, shikonin, beta-hydroxyisovalerylshikonin, or ganglioside GM3, DC101 antibody, Mab25 antibody, Mab73 antibody, 4A5 antibody, 4E10 antibody, 5F12 antibody, VA01 antibody, BL2 antibody, VEGF-related protein, sFLT01, sFLT02, Peptide B3, TG100801, sorafenib, or G6-31 antibody, or a pharmaceutically acceptable salt thereof; and (c) a pharmaceutically acceptable carrier or vehicle.

The invention provides compositions comprising an effective amount of (a) Antagonist C or a pharmaceutically acceptable salt thereof; (b) ranibizumab, bevacizumab, aflibercept, KH902 VEGF receptor-Fc fusion protein, 2C3 antibody, ORA102, pegaptanib, bevasiranib, SIRNA-027, decursin, decursinol, picropodophyllin, guggulsterone. PLG101, eicosanoid LXA4, PTK787, pazopanib, axitinib, CDDO-Me, CDDO-Imm, shikonin, beta-hydroxyisovalerylshikonin, or ganglioside GM3, DC101 antibody, Mab25 antibody, Mab73 antibody, 4A5 antibody, 4E10 antibody, 5F12 antibody, VA01 antibody, BL2 antibody, VEGF-related protein, sFLT01, sFLT02, Peptide B3, TG100801, sorafenib, or G6-31 antibody, or a pharmaceutically acceptable salt thereof; and (c) a pharmaceutically acceptable carrier or vehicle.

The invention provides compositions comprising an effective amount of (a) Antagonist D or a pharmaceutically acceptable salt thereof; (b) ranibizumab, bevacizumab, aflibercept, KH902 VEGF receptor-Fc fusion protein, 2C3 antibody, ORA102, pegaptanib, bevasiranib, SIRNA-027, decursin, decursinol, picropodophyllin, guggulsterone, PLG101, eicosanoid LXA4, PTK787, pazopanib, axitinib, CDDO-Me, CDDO-Imm, shikonin, beta-hydroxyisovalerylshikonin, or ganglioside GM3, DC101 antibody, Mab25 antibody, Mab73 antibody, 4A5 antibody, 4E10 antibody, 5F12 antibody, VA01 antibody, BL2 antibody, VEGF-related protein, sFLT01, sFLT02, Peptide B3, TG100801, sorafenib, or G6-31 antibody, or a pharmaceutically acceptable salt thereof; and (c) a pharmaceutically acceptable carrier or vehicle.

In another aspect the invention provides Antagonist A or a pharmaceutically acceptable salt thereof.

In another aspect the invention provides compositions comprising Antagonist A or a pharmaceutically acceptable salt thereof.

In another aspect the invention provides compositions comprising: (a) an effective amount of Antagonist A or a pharmaceutically acceptable salt thereof; and (b) a pharmaceutically acceptable carrier or vehicle.

In another aspect the invention provides compounds of Formula B and a pharmaceutically acceptable salt thereof.

In another aspect the invention provides compositions comprising a compound of Formula B or a pharmaceutically acceptable salt thereof.

In another aspect the invention provides compositions comprising: (a) an effective amount of a compound of Formula B or a pharmaceutically acceptable salt thereof; and (b) a pharmaceutically acceptable carrier or vehicle.

In another aspect the invention provides a compound of Formula C or a pharmaceutically acceptable salt thereof.

In another aspect the invention provides compositions comprising a compound of Formula C or a pharmaceutically acceptable salt thereof.

In another aspect the invention provides compositions comprising: (a) an effective amount of a compound of Formula C or a pharmaceutically acceptable salt thereof; and (b) a pharmaceutically acceptable carrier or vehicle.

In another aspect the invention provides methods and compositions as described above, wherein Antagonist A, Antagonist B, Antagonist C or Antagonist D is linked with one or more nonphysiologically active groups, lipophilic groups or high-molecular weight compounds.

### 5. BRIEF DESCRIPTION OF THE DRAWINGS

Reference is made to the following detailed description, which sets forth illustrative embodiments and the accompanying drawings of which:
Figure 1 (A) is a schematic representation of the nucleic acid sequence of a human PDGF-B (GenBank Accession No. X02811) (SEQ ID NO: 1).
Figure 1 (B) is a schematic representation of the amino acid sequence of a human PDGF-B (GenBank Accession No. CAA26579) (SEQ ID NO: 2).
Figure 1 (C) is a schematic representation of the nucleic acid sequence of a human PDGF-A (GenBank Accession No. X06374) (SEQ ID NO: 11).
Figure 1 (D) is a schematic representation of the polypeptide sequence of a human PDGF-A (GenBank Accession No. CAA29677) (SEQ ID NO: 12).
Figure 1 (E) is a schematic representation of the nucleic acid sequence of a human PDGF-C (GenBank Accession No. NM_016205) (SEQ ID NO: 17).
Figure 1 (F) is a schematic representation of the polypeptide sequence of a human PDGF-C (GenBank Accession No. NP_057289) (SEQ ID NO: 18).
Figure 1 (G) is a schematic representation of the nucleic acid sequence of a human PDGF-D, variant 1 (GenBank Accession No. NM_025208) (SEQ ID NO: 19).
Figure 1 (H) is a schematic representation of the polypeptide sequence of a human PDGF-D, variant 1 (GenBank Accession No. NP_079484) (SEQ ID NO: 20).
Figure 1 (I) is a schematic representation of the nucleic acid sequence of a human PDGF-D, variant 2 (GenBank Accession No. NM_033135) (SEQ ID NO: 21).
Figure 1 (J) is a schematic representation of the polypeptide sequence of a human PDGF-D, variant 2 (GenBank Accession No. NP_149126) (SEQ ID NO: 22).
Figure 2 (A) is a schematic representation of the nucleic acid sequence of a human VEGF (GenBank Accession No: NM_003376) (SEQ ID NO: 3).
Figure 2 (B) is a schematic representation of the amino acid sequence of a human VEGF polypeptide (GenBank Accession No. NP_003367) (SEQ ID NO: 4).
Figure 3 (A) is a schematic representation of the nucleic acid sequence of a human PDGFR-B (GenBank Accession No. NM_002609) (SEQ ID NO: 5).
Figure 3 (B) is a schematic representation of the polypeptide sequence of a human PDGFR-B (GenBank Accession No. NP_002600) (SEQ ID NO: 6).
Figure 3 (C) is a schematic representation of the nucleic acid sequence of a human PDGFR-A (GenBank Accession No. NM_006206) (SEQ ID NO: 13).
Figure 3 (D) is a schematic representation of the polypeptide sequence of a human PDGFR-A (GenBank Accession No. NP_006197) (SEQ ID NO: 14).
Figure 4 (A) is a schematic representation of the nucleic acid sequence of a human VEGFR-1 (Flt-1)
(GenBank Accession No. AF063657) (SEQ ID NO: 7).
Figure 4 (B) is schematic a representation of the polypeptide sequence of a human VEGFR-1 (Flt-1) (GenBank Accession No.) (SEQ ID NO: 8).
Figure 4 (C) is a schematic representation of the nucleic acid sequence of a human VEGFR-2 (KDR/Flk-1) (GenBank Accession No. AF035121) (SEQ ID NO: 9).
Figure 4 (D) is a schematic representation of the polypeptide sequence of a human VEGFR-2 (KDR/Flk-1) (GenBank Accession No. AAB88005) (SEQ ID NO: 10).
Figure 5 is a graph of change in mean foveal thickness from a baseline over a 12 week period when treated with Antagonist A and ranibizumab (as the commercially available composition Lucentis^{®}). The square symbol represents foveal thickness in the central subfield and diamond symbol represents foveal thickness in the central point.
Figure 6 shows Formula A, wherein w is an integer from 2 to 12.
Figure 7 shows the chemical structure of Antagonist A.
Figure 8 shows Formula B, wherein w is an integer from 2 to 12.
Figure 9 shows the chemical structure of Antagonist B.
Figure 10 shows Formula C, wherein w is an integer from 2 to 12.
Figure 11 shows the chemical structure of Antagonist C.
Figure 12 shows the chemical structure of Antagonist D.
Figure 13 shows Formula E, wherein L is a linker, Y is 0 or 1, R is a nonphysiologically active group, lipophilic group or High Molecular Weight Compound, and X is an integer ranging from 1 to 4

### 6. DETAILED DESCRIPTION OF THE INVENTION

### 5.1 Definitions and Abbreviations

As used herein, the following terms and phrases shall have the meanings set forth below. Unless defined otherwise, all technical and scientific terms used herein have the same meaning as commonly understood to one of skill in the art to which this invention belongs.

The term "about" a referenced numeric indication means the referenced numeric indication plus or minus up to 10% of that referenced numeric indication. For example, "about 100" means from 90 to 110.

The term "antagonist" refers to an agent that inhibits, either partially or fully, the activity or production of a target molecule. In particular, the term "antagonist," as applied selectively herein, means an agent capable of decreasing levels of gene expression, mRNA levels, protein levels or protein activity of the target molecule. Illustrative forms of antagonists include, for example, proteins, polypeptides, peptides (such as cyclic peptides), antibodies or antibody fragments, peptide mimetics, nucleic acid molecules, antisense molecules, ribozymes, aptamers, RNAi molecules, and small organic molecules. Illustrative non-limiting mechanisms of antagonist inhibition include repression of ligand synthesis and/or stability (*e.g*., using, antisense, ribozymes or RNAi compositions targeting the ligand gene/nucleic acid), blocking of binding of the ligand to its cognate receptor (*e.g.,* using anti-ligand aptamers, antibodies or a soluble, decoy cognate receptor), repression of receptor synthesis and/or stability (*e.g.,* using, antisense, ribozymes or RNAi compositions targeting the ligand receptor gene/nucleic acid), blocking of the binding of the receptor to its cognate receptor (*e.g.,* using receptor antibodies) and blocking of the activation of the receptor by its cognate ligand (*e.g*., using receptor tyrosine kinase inhibitors). In addition, the antagonist may directly or indirectly inhibit the target molecule.

The term "antibody fragment" includes a portion of an antibody that is an antigen binding fragment or single chains thereof. An antibody fragment can be a synthetically or genetically engineered polypeptide. Examples of binding fragments encompassed within the term "antigen-binding portion" of an antibody include (i) a Fab fragment, a monovalent fragment consisting of the V_{L}, V_{H}, C_{L} and C_{Hl} domains; (ii) a F(ab')₂ fragment, a bivalent fragment comprising two Fab fragments linked by a disulfide bridge at the hinge region; (iii) a Fd fragment consisting of the V_{H} and C_{Hl} domains; (iv) a Fv fragment consisting of the V_{L} and V_{H} domains of a single arm of an antibody, (v) a dAb fragment (Ward et al., (1989) Nature 341:544-546), which consists of a V_{H} domain; and (vi) an isolated complementarity determining region (CDR). Furthermore, although the two domains of the Fv fragment, V_{L} and V_{H}, are coded for by separate genes, they can be joined, using recombinant methods, by a synthetic linker that enables them to be made as a single protein chain in which the V_{L} and V_{H} regions pair to form monovalent molecules (known as single chain Fv (scFv); see e.g., Bird et al. (1988) Science 242:423-426; and Huston et al. (1988) Proc. Natl. Acad. Sci. USA 85:5879-5883). Such single chain antibodies are also intended to be encompassed within the term "antigen-binding fragment" of an antibody. These antibody fragments are obtained using conventional techniques known to those in the art, and the fragments can be screened for utility in the same manner as whole antibodies.

The term "aptamer" refers to a peptide or nucleic acid that has an inhibitory effect on a target. Inhibition of the target by the aptamer can occur by binding of the target, by catalytically altering the target, by reacting with the target in a way which modifies the target or the functional activity of the target, by ionically or covalently attaching to the target as in a suicide inhibitor or by facilitating the reaction between the target and another molecule. Aptamers can be peptides, ribonucleotides, deoxyribonucleotides, other nucleic acids or a mixture of the different types of nucleic acids. Aptamers can comprise one or more modified amino acid, bases, sugars, polyethylene glycol spacers or phosphate backbone units as described in further detail herein.

A nucleotide sequence is "complementary" to another nucleotide sequence if each of the bases of the two sequences matches, i.e., are capable of forming Watson Crick base pairs. The complement of a nucleic acid strand can be the complement of a coding strand or the complement of a non-coding strand.

The phrase "conserved residue" refers to an amino acid of a group of amino acids having particular common properties. A functional way to define common properties among individual amino acids is to analyze the normalized frequencies of amino acid changes among corresponding proteins of homologous organisms. According to such analyses, groups of amino acids may be characterized where amino acids within a group exchange preferentially with each other, and therefore resemble each other most in their impact on the overall protein structure (Schulz, G. E. and R. H. Schirmer, Principles of Protein Structure, Springer-Verlag). Examples of amino acid groups defined in this manner include:
(i) a charged group, consisting of Glu and Asp, Lys, Arg and His,
(ii) a positively-charged group, consisting of Lys, Arg and His,
(iii) a negatively-charged group, consisting of Glu and Asp,
(iv) an aromatic group, consisting of Phe, Tyr and Trp,
(v) a nitrogen ring group, consisting of His and Trp,
(vi) a large aliphatic nonpolar group, consisting of Val, Leu and Ile,
(vii) a slightly-polar group, consisting of Met and Cys,
(viii) a small-residue group, consisting of Ser, Thr, Asp, Asn, Gly, Ala, Glu, Gln and Pro,
(ix) an aliphatic group consisting of Val, Leu, Ile, Met and Cys, and
(x) a small hydroxyl group consisting of Ser and Thr.

Members of each of the above groups are conserved residues.

The term "label" includes, but is not limited to, a radioactive isotope, a fluorophore, a chemiluminescent moiety, an enzyme, an enzyme substrate, an enzyme cofactor, an enzyme inhibitor, a dye, a metal ion, a ligand (*e.g.,* biotin or a hapten) and the like. Examples of fluorophore labels include fluorescein, rhodamine, dansyl, umbelliferone, Texas red, luminol, NADPH, alpha-beta-galactosidase and horseradish peroxidase.

The term "nucleic acid" refers to a polynucleotide such as deoxyribonucleic acid (DNA) or ribonucleic acid (RNA). The term also includes analogs of RNA or DNA made from nucleotide analogs, and, as applicable to the embodiment being described, single (sense or antisense) and double-stranded polynucleotides, ESTs, chromosomes, cDNAs, mRNAs, and rRNAs.

The terms product interference," "RNAi," "miRNA," and "siRNA" refer to any method by which expression of a gene or gene product is decreased by introducing into a target cell one or more double-stranded RNAs, which are homologous to a gene of interest (particularly to the messenger RNA of the gene of interest, e.g., PDGF or VEGF).

The term "neovascularization" refers to new blood vessel formation in abnormal tissue or in abnormal positions.

The term "angiogenesis" refers to formation of new blood vessels in normal or in abnormal tissue or positions.

The term "ophthalmological disease" includes diseases of the eye and the ocular adnexa.

The term "ocular neovascular disorder" refers to an ocular disorder characterized by neovascularization. In one embodiment, the ocular neovascular disorder is a disorder other than cancer. Examples of ocular neovascular disorders include diabetic retinopathy and age-related macular degeneration.

The term "mammal" includes a human, monkey, cow, hog, sheep, horse, dog, and cat.

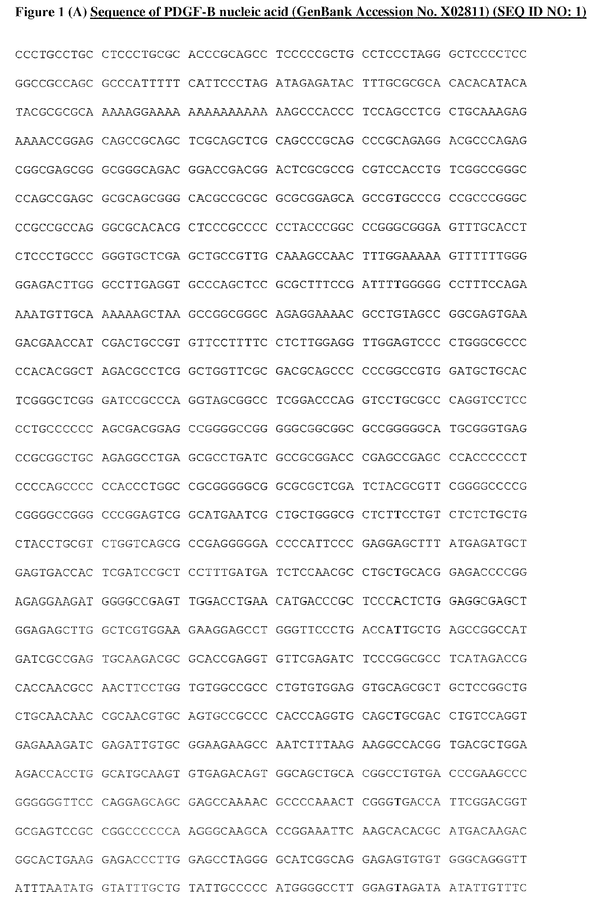

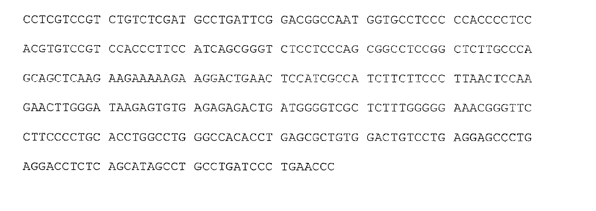

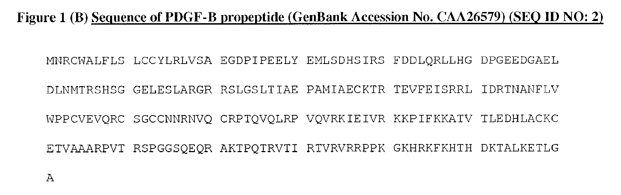

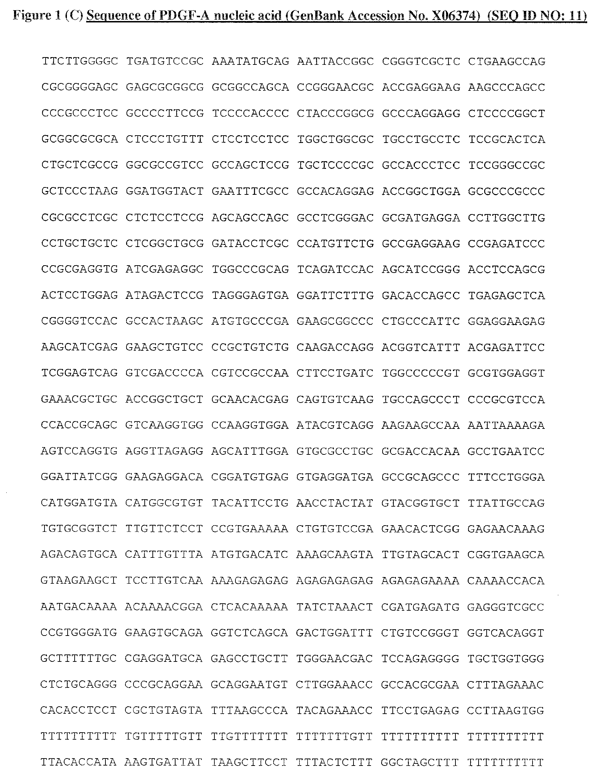

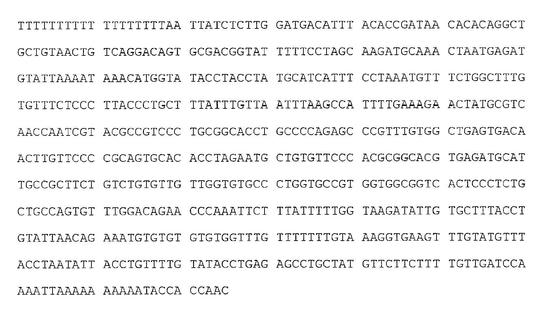

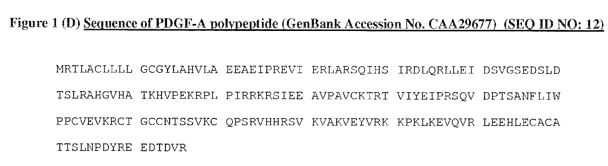

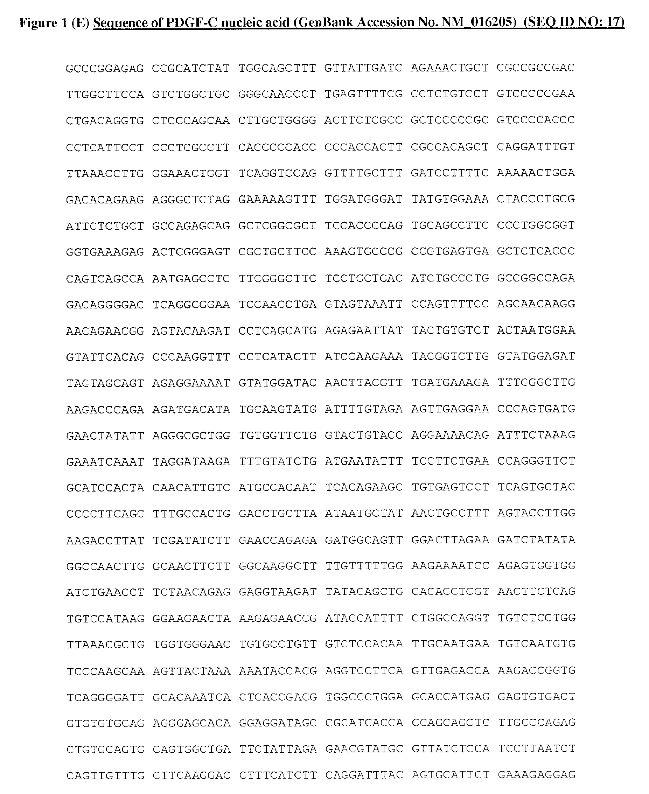

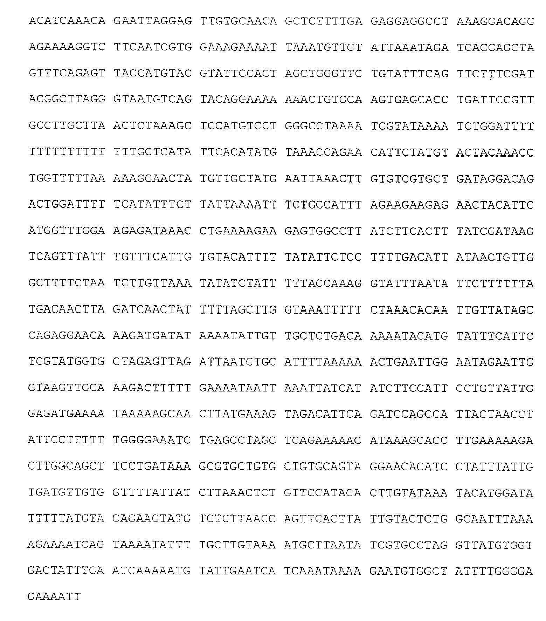

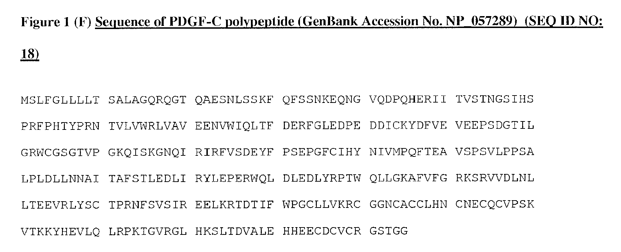

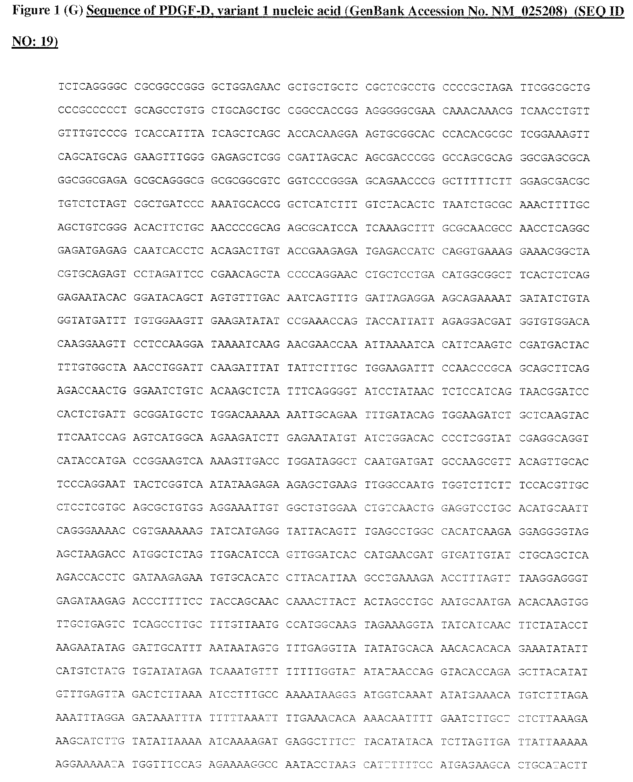

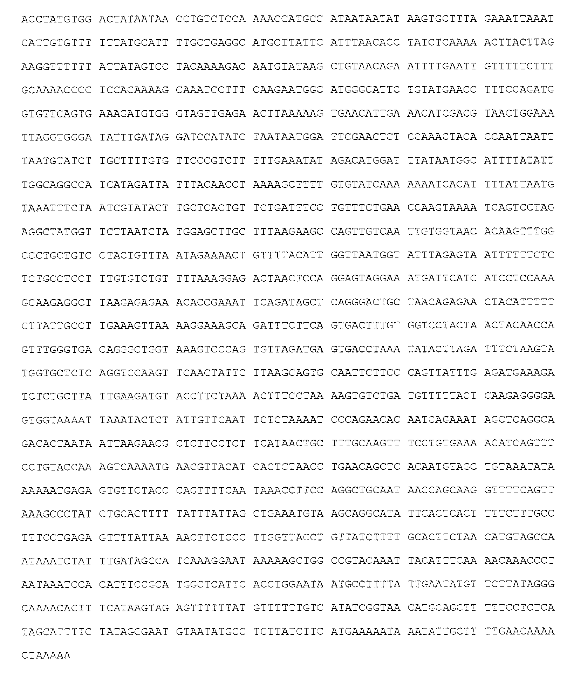

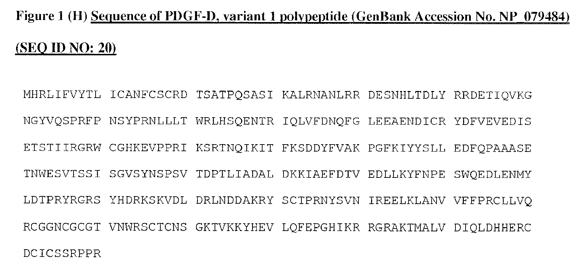

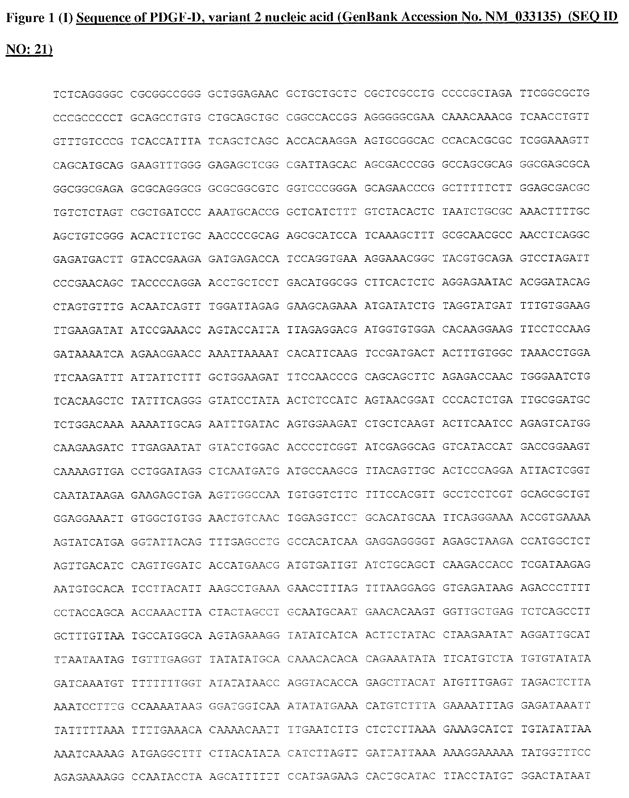

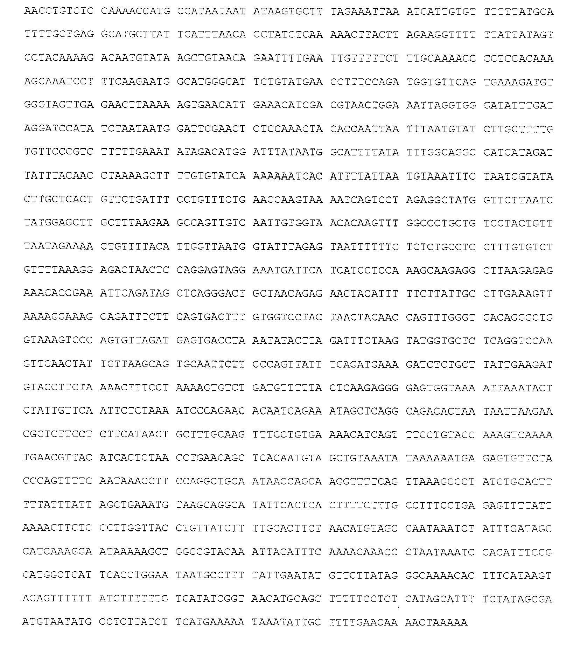

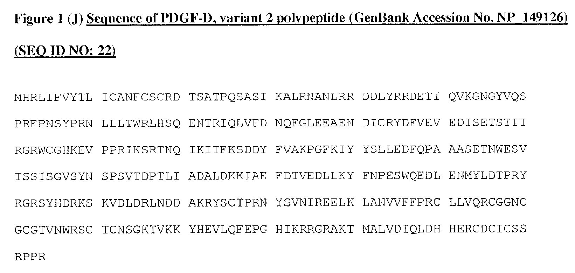

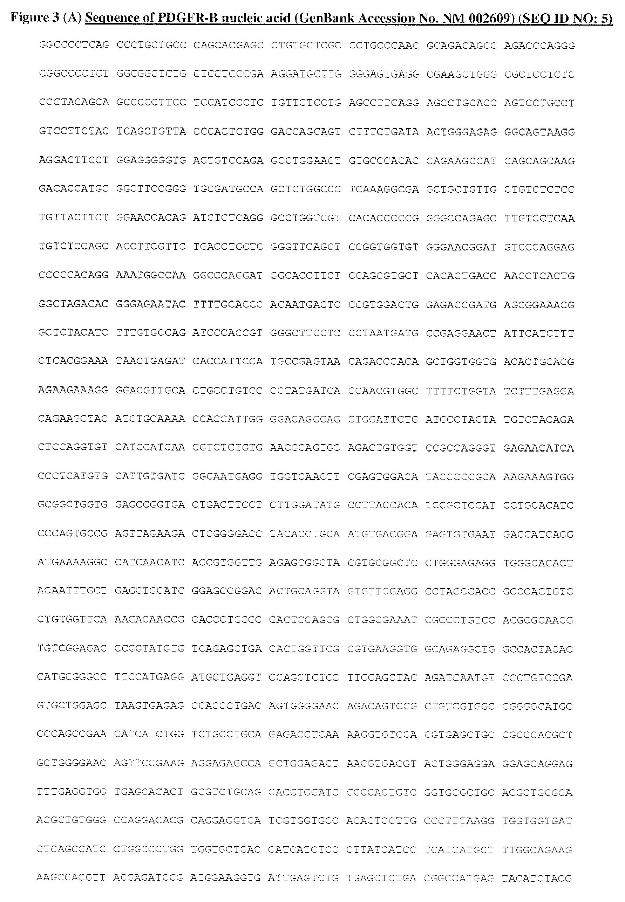

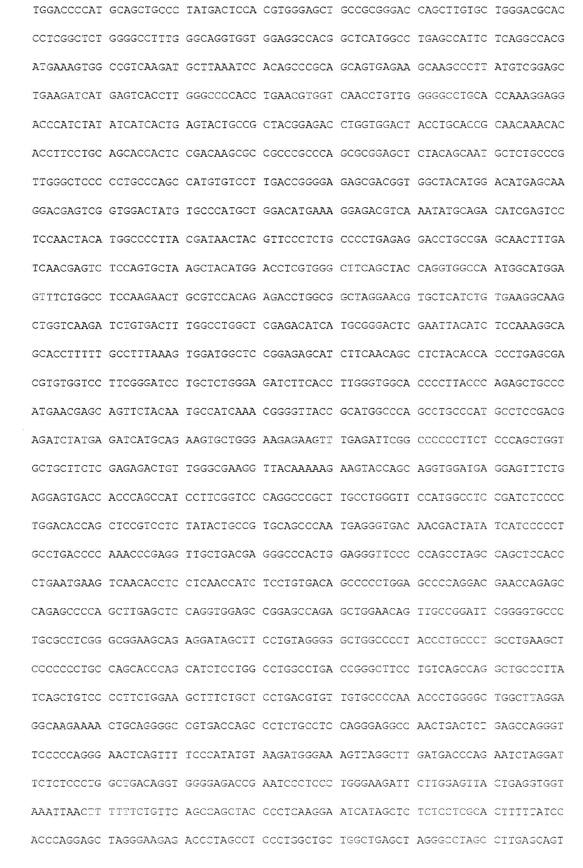

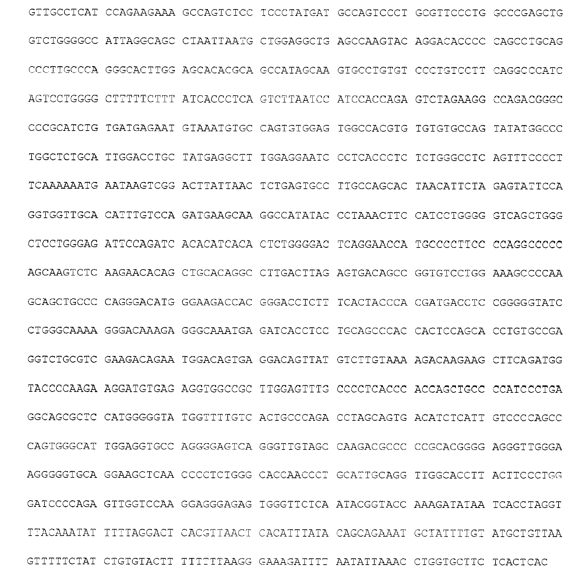

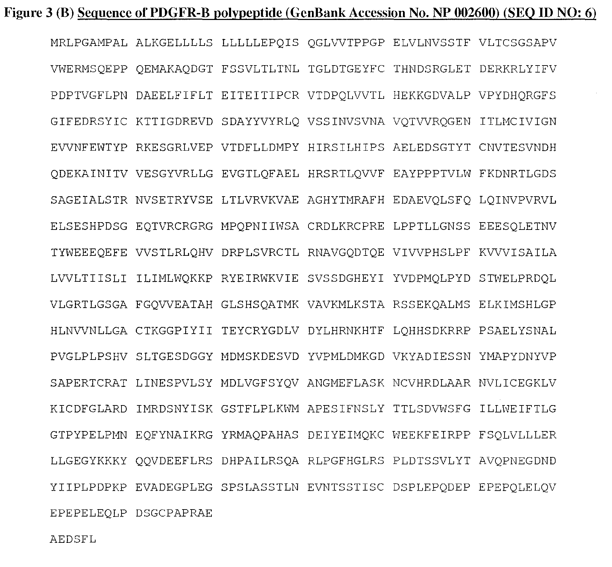

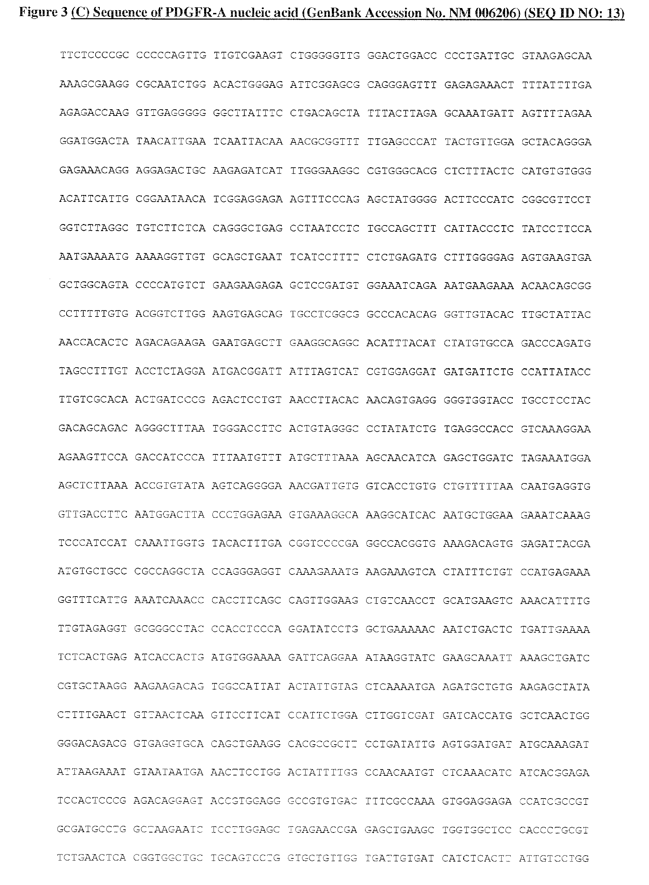

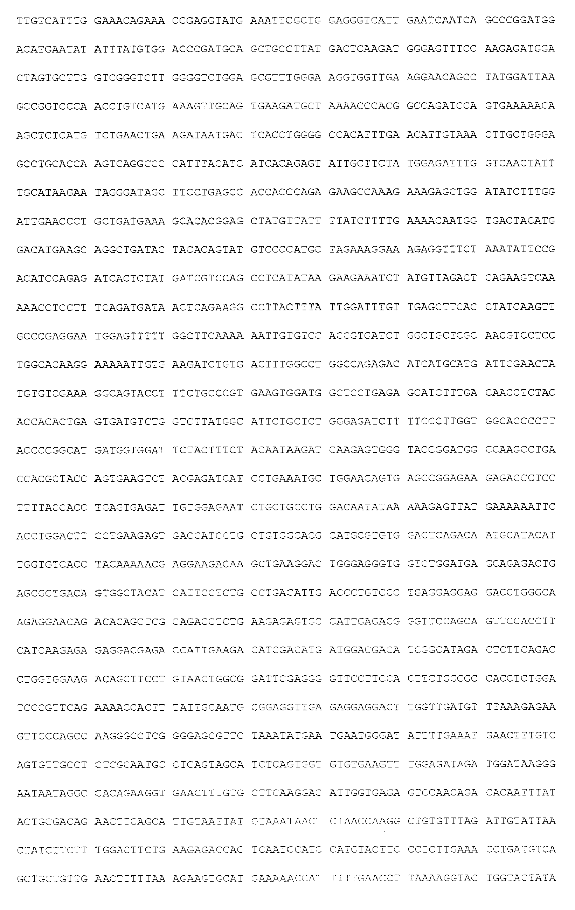

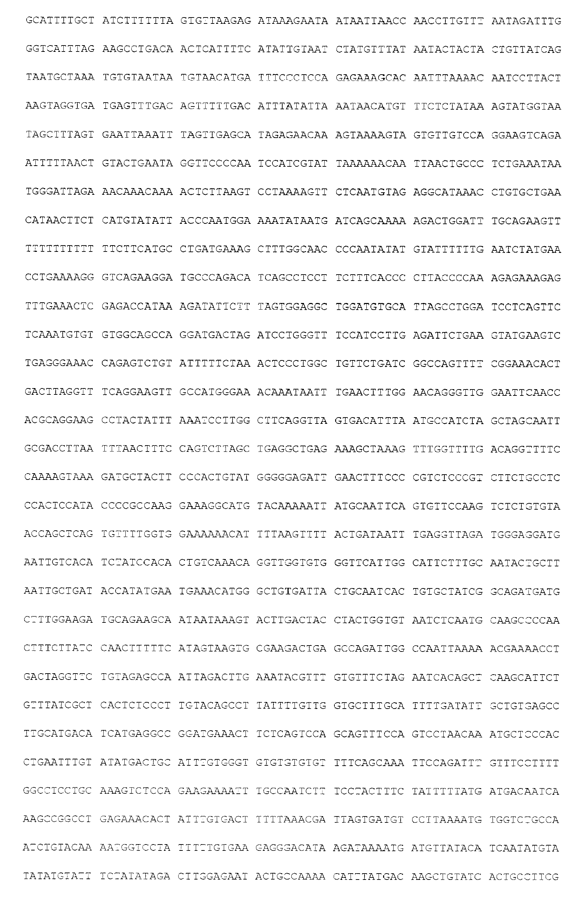

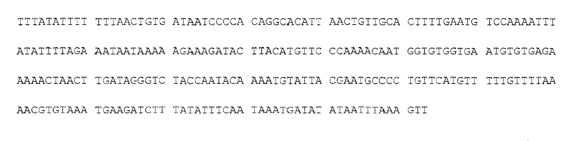

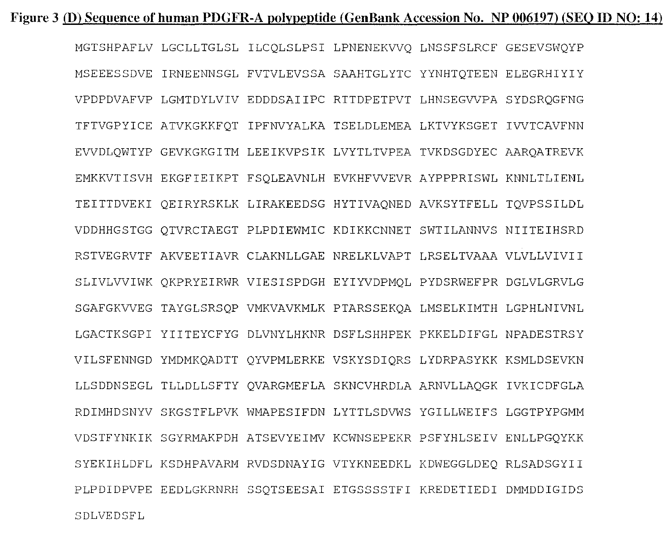

The term "PDGF" refers to a platelet-derived growth factor that regulates cell growth or division. As used herein, the term "PDGF" includes the various subtypes of PDGF including PDGF-B (see Figure 1(A) and (B)), PDGF-A (see Figure 1(C) and (D)), PDGF-C (see Figure 1(E) and (F)), PDGF-D, variants 1 and 2 (see Figure 1(G), (H), (I) and (J)), and dimerized forms thereof, including PDGF-AA, PDGF-AB, PDGF-BB, PDGF-CC, and PDGF-DD. Platelet derived growth factors includes homo- or heterodimers of A-chain (PDGF-A) and B-chain (PDGF-B) that exert their action via binding to and dimerization of two related receptor tyrosine kinase platelet-derived growth factor cell surface receptors (*i*.*e*., PDGFRs), PDGFR-α (see Figure 3 (C) and (D)) and PDGFR-β (see Figure 3 (A) and (B)). In addition, PDGF-C and PDGF-D, two additional protease-activated ligands for the PDGFR complexes, have been identified (Li et al., (2000) Nat. Cell. Biol. 2: 302-9; Bergsten et al., (2001) Nat. Cell. Biol. 3: 512-6; and Uutele et al., (2001) Circulation 103: 2242-47). Due to the different ligand binding specificities of the PDGFRs, it is known that PDGFR-α/α binds PDGF-AA, PDGF-BB, PDGF-AB, and PDGF-CC; PDGFR-β/β. binds PDGF-BB and PDGF-DD; whereas PDGFR-α/β binds PDGF-AB, PDGF-BB, PDGF-CC, and PDGF-DD (Betsholtz et al., (2001) BioEssays 23: 494-507). As used herein, the term "PDGF" also refers to those members of the class of growth factors that induce DNA synthesis and mitogenesis through the binding and activation of a PDGFR on a responsive cell type. PDGFs can effect, for example: directed cell migration (chemotaxis) and cell activation; phospholipase activation; increased phosphatidylinositol turnover and prostaglandin metabolism; stimulation of both collagen and collagenase synthesis by responsive cells; alteration of cellular metabolic activities, including matrix synthesis, cytokine production, and lipoprotein uptake; induction, indirectly, of a proliferative response in cells lacking PDGF. receptors; and potent vasoconstrictor activity. The term "PDGF" can be used to refer to a "PDGF" polypeptide, a "PDGF" encoding gene or nucleic acid, or a dimerized form thereof.

The term "PDGF-A" refers to an A chain polypeptide of PDGF or its corresponding encoding gene or nucleic acid.

The term "PDGF-B" refers to a B chain polypeptide of PDGF or its corresponding encoding gene or nucleic acid.

The term "PDGF-C" refers to a C chain polypeptide of PDGF or its corresponding encoding gene or nucleic acid.

The term "PDGF-D" refers to a D chain polypeptide of PDGF or its corresponding encoding gene or nucleic acid, including variants 1 and 2 of the D chain polypeptide of PDGF.

The term "PDGF-AA" refers to a dimer having two PDGF-A chain polypeptides.

The term "PDGF-AB" refers to a dimer having one PDGF-A chain polypeptide and one PDGF-B chain polypeptide.

The term "PDGF-BB" refers to a dimer having two PDGF-B chain polypeptides.

The term "PDGF-CC" refers to a dimer having two PDGF-C chain polypeptides.

The term "PDGF-DD" refers to a dimer having two PDGF-D chain polypeptides.

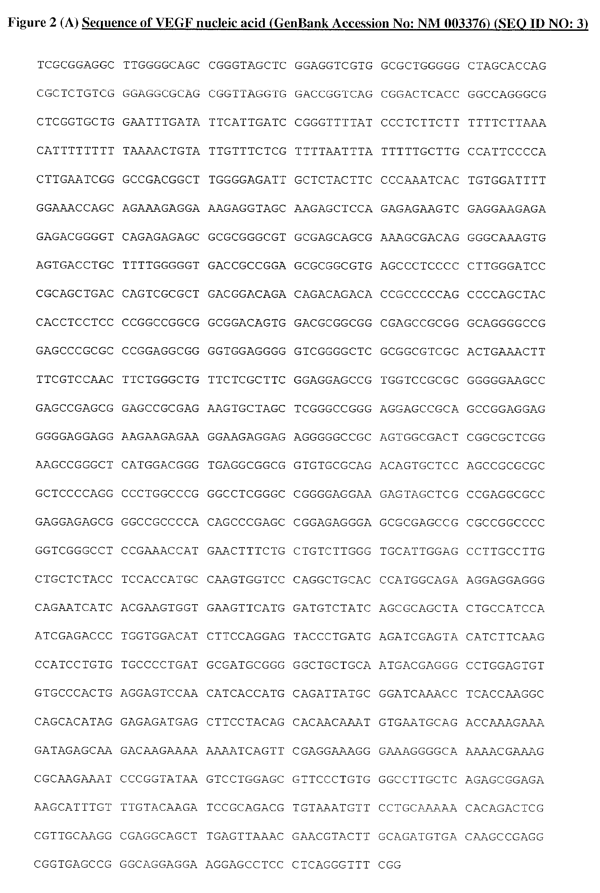

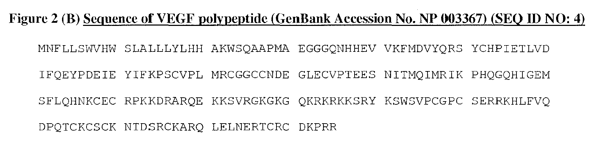

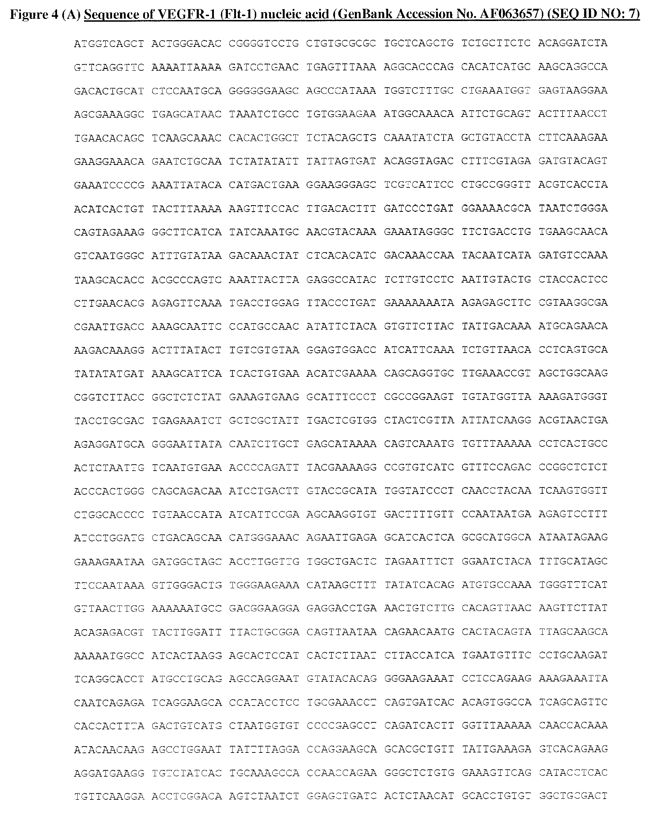

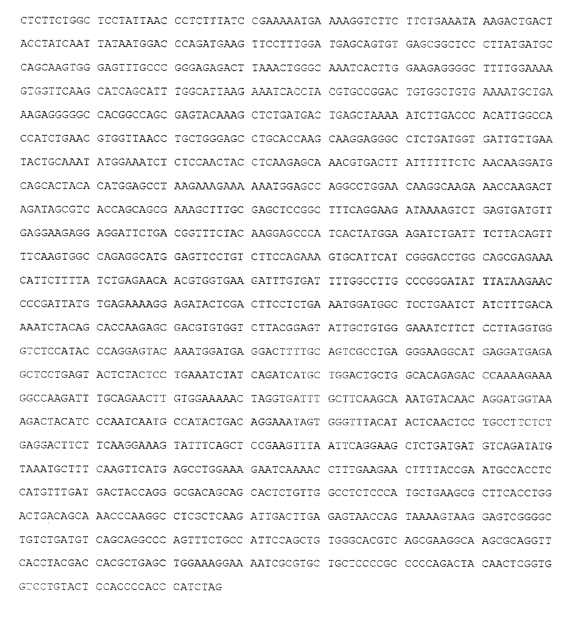

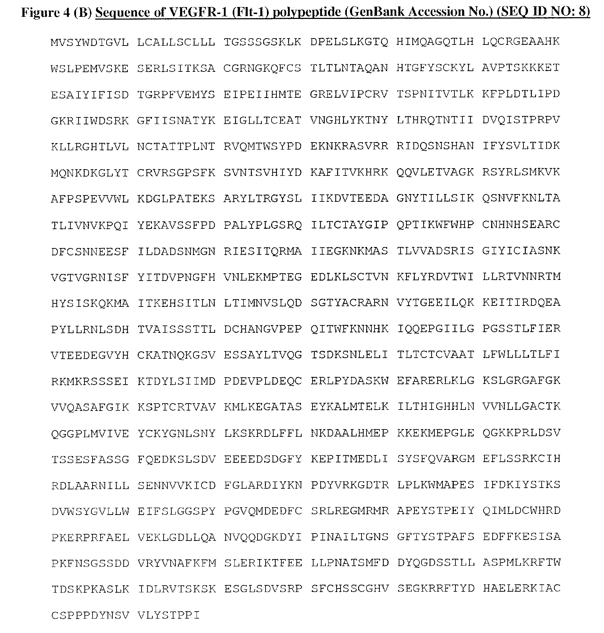

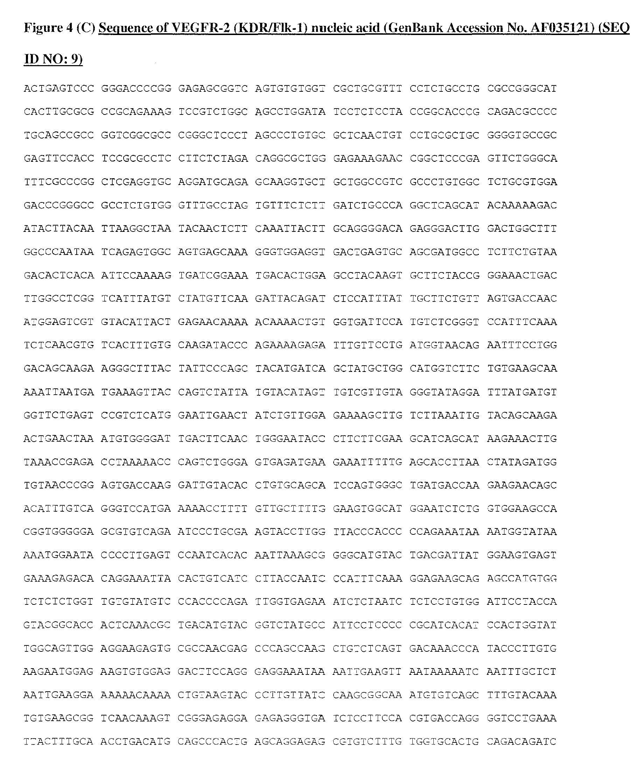

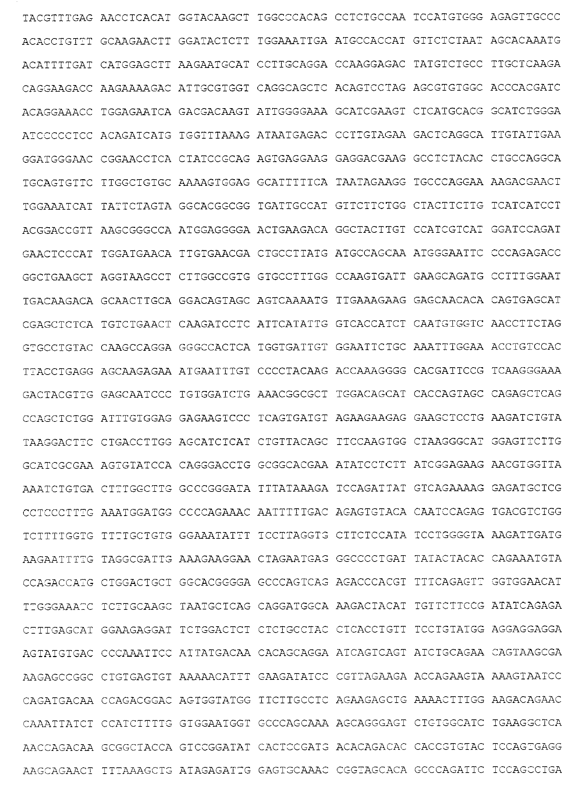

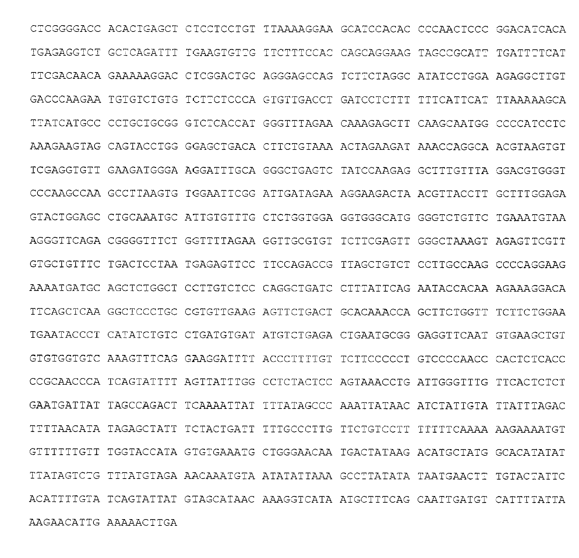

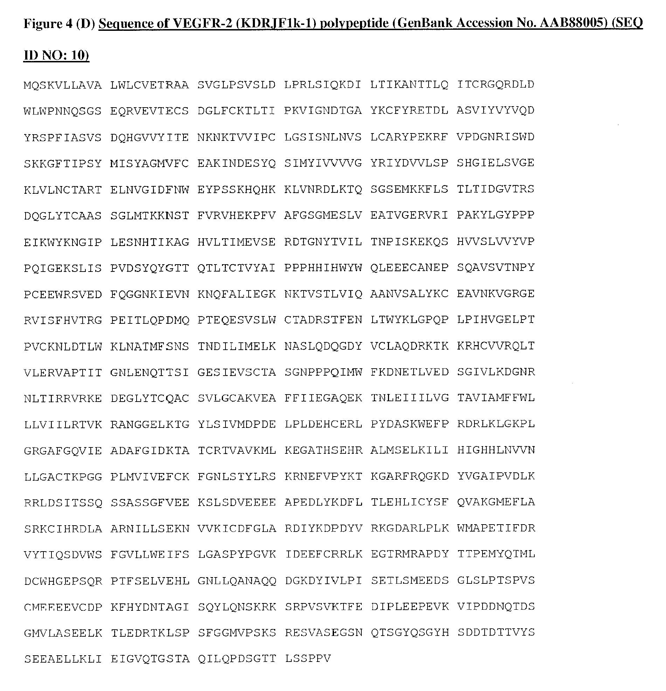

The term "VEGF" refers to a vascular endothelial growth factor that induces angiogenesis or an angiogenic process. As used herein, the term "VEGF" includes the various subtypes of VEGF (also known as vascular permeability factor (VPF) and VEGF-A) (see Figure 2(A) and (B)) that arise by, *e*.*g*., alternative splicing of the VEGF-A/VPF gene including VEGF₁₂₁, VEGF₁₆₅ and VEGF₁₈₉. Further, as used herein, the term "VEGF" includes VEGF-related angiogenic factors such as PIGF (placenta growth factor), VEGF-B, VEGF-C, VEGF-D) and VEGF-E, which act through a cognate VEFG receptor (*i.e.,* VEGFR) to induce angiogenesis or an angiogenic process. The term "VEGF" includes any member of the class of growth factors that binds to a VEGF receptor such as VEGFR-1 (Plt-1) (see Figure 4(A) and (B)), VEGFR-2 (KDR/Flk-1) (see Figure 4(C) and (D)), or VEGFR-3 (FLT-4). The term "VEGF" can be used to refer to a "VEGF" polypeptide or a "VEGF" encoding gene or nucleic acid.

The term "PDGF antagonist" refers to an agent that reduces, or inhibits, either partially or fully, the activity or production of a PDGF. A PDGF antagonist can directly or indirectly reduce or inhibit the activity or production of a specific PDGF such as PDGF-B. Furthermore, "PDGF antagonists" consistent with the above definition of "antagonist," include agents that act on a PDGF ligand or its cognate receptor so as to reduce or inhibit a PDGF-associated receptor signal. Examples of "PDGF antagonists" include antisense molecules, ribozymes or RNAi that target a PDGF nucleic acid; anti-PDGF aptamers, anti-PDGF antibodies to PDGF itself or its receptor, or soluble PDGF receptor decoys that prevent binding of a PDGF to its cognate receptor; antisense molecules, ribozymes or RNAi that target a cognate PDGF receptor (PDGFR) nucleic acid; anti-PDGFR aptamers or anti-PDGFR antibodies that bind to a cognate PDGFR receptor; and PDGFR tyrosine kinase inhibitors.

The term "VEGF antagonist" refers to an agent that reduces, or inhibits, either partially or fully, the activity or production of a VEGF. A VEGF antagonist can directly or indirectly reduce or inhibit the activity or production of a specific VEGF such as VEGF₁₆₅. Furthermore, "VEGF antagonists" consistent with the above definition of "antagonist," include agents that act on either a VEGF ligand or its cognate receptor so as to reduce or inhibit a VEGF-associated receptor signal. Examples of "VEGF antagonists" include antisense molecules, ribozymes or RNAi that target a VEGF nucleic acid; anti-VEGF aptamers, anti-VEGF antibodies to VEGF itself or its receptor, or soluble VEGF receptor decoys that prevent binding of a VEGF to its cognate receptor; antisense molecules, ribozymes, or RNAi that target a cognate VEGF receptor (VEGFR) nucleic acid; anti-VEGFR aptamers or anti-VEGFR antibodies that bind to a cognate VEGFR receptor; and VEGFR tyrosine kinase inhibitors.

The term "effective amount," when used in connection with an ophthalmological disease, refers to an amount of a PDGF antagonist of Table 1 or Table (below) and a VEGF antagonist of Table 1 or Table 2 that is useful to treat or prevent an ophthalmological disease. The "effective amount" can vary depending upon the mode of administration, specific locus of the ophthalmological disease, the age, body weight, and general health of the mammal. The administration of the PDGF antagonist of Table 1 or Table 2 can occur prior to, subsequent to or concurrently with administration of the VEGF antagonist of Table 1 or Table 2. In one embodiment, the PDGF antagonist of Table 1 or Table 2 and VEGF antagonist of Table 1 or Table 2 are administered as components of the same composition. The effective amount is the total amount of the PDGF antagonist and the VEGF antagonist that is useful for treating or preventing an ophthalmological disease, even if the amount of the PDGF antagonist without the VEGF antagonist, or the VEGF antagonist without the PDGF antagonist, is ineffective to treat or prevent the ophthalmological disease.

A "variant" of polypeptide X refers to a polypeptide having the amino acid sequence of polypeptide X in which is altered in one or more amino acid residues. The variant can have "conservative" changes, wherein a substituted amino acid has similar structural or chemical properties (e.g., replacement of leucine with isoleucine). More rarely, a variant can have "nonconservative" changes (*e*.*g*., replacement of glycine with tryptophan). Analogous minor variations may also include amino acid deletions or insertions, or both. Guidance in determining which amino acid residues may be substituted, inserted, or deleted without eliminating biological or immunological activity can be determined using computer programs well known in the art, for example, LASERGENE software (DNASTAR).

The term "variant," when used in the context of a polynucleotide sequence, can encompass a polynucleotide sequence related to that of gene or the coding sequence thereof. This definition also includes, for example, "allelic," "splice," "species," or "polymorphic" variants. A splice variant can have significant identity to a reference molecule, but will generally have a greater or lesser number of polynucleotides due to alternative splicing of exons during mRNA processing. The corresponding polypeptide can possess additional functional domains or an absence of domains. Species variants are polynucleotide sequences that vary from one species to another. The resulting polypeptides generally will have significant amino acid identity relative to each other. A polymorphic variant is a variation in the polynucleotide sequence of a particular gene between individuals of a given species.

### 5.2 Methods for Treating or Preventing an Ophthalmological disease

Accordingly, the invention provides methods and compositions useful for treating or preventing an ophthalmological disease. In several embodiments of the present invention, the methods for treating or preventing an ophthalmological disease comprise administration of an effective amount of (a) ARC-127, Antagonist A, Antagonist B, Antagonist C, Antagonist D, 1B3 antibody, CDP860, IMC-3G3, imatinib, 162.62 antibody, 163.31 antibody, 169.14 antibody, 169.31 antibody, αR1 antibody, 2A1E2 antibody, M4TS.11 antibody, M4TS.22 antibody, A10, brefeldin A, sunitinib, Hyb 120.1.2.1.2 antibody, Hyb 121.6.1.1.1 antibody, Hyb 127.5.7.3.1 antibody, Hyb 127.8.2.2.2 antibody, Hyb 1.6.1 antibody, Hyb 1.11.1 antibody, Hyb 1.17.1 antibody, Hyb 1.18.1 antibody, Hyb 1.19.1 antibody, Hyb 1.23.1 antibody, Hyb 1.24 antibody, Hyb 1.25 antibody, Hyb 1.29 antibody, Hyb 1.33 antibody, Hyb 1.38 antibody, Hyb 1.39 antibody, Hyb 1.40 antibody, Hyb 1.45 antibody, Hyb 1.46 antibody, Hyb 1.48 antibody, Hyb 1.49 antibody, Hyb 1.51 antibody, Hyb 6.4.1 antibody, F3 antibody, Humanized F3 antibody, C1 antibody, Humanized C1 antibody, 6.4 antibody, anti-mPDGF-C goat IgG antibody, C3.1 antibody, 5-methyl-7-diethylamino-s-triazolo (1,5-a) pyrimidine, interferon, protamine, PDGFR-B1 monoclonal antibody, PDGFR-B2 monoclonal antibody, 6D11 monoclonal antibody, Sis 1 monoclonal antibody, PR7212 monoclonal antibody, PR292 monoclonal antibody, HYB 9610 monoclonal antibody, HYB 9611 monoclonal antibody, HYB 9612 monoclonal antibody, HYB 9613 monoclonal antibody, 4-(2-(N-(-2-carboxamidoindole) aminoethyl)-benzenesulfonamide, 4-(2-(N-(-2-carboxamidoindole)aminoethyl)-sulfonylurea, CGP 53716, human antibody g162, pyrazolo[3,4-g]quinoxaline, 6-[2-(methylcarbamoyl)phenylsulphanyl]-3-E-[2-(pyridine-2-yl)ethenyl]-indazole, 1-{2-[5-(2-methoxy-ethoxy)-benzoimidazole-1-yl]-quinoline-8-yl}-piperidine-4-ylamine, 4-[4-[N-(4-nitrophenyl)carbamoyl]-1-piperazinyl]-6,7-dimethoxyquinazoline, 4-amino-5-fluoro-3-(6-(4-methyl-piperazine-1-yl)-1H-benzimidazole-2-yl)-1H-quinoline-2-one, (4-tert-butylphenyl){4-[(6,7-dimethoxy-4-quinolyl)oxy]phenyl}methaneone, 5-methyl-N-[4-(trifluoromethyl)phenyl]-4-isoxazolecarboxamide, trans-4-[(6,7-dimethoxyquinoxaline-2-yl)amino]cyclohexanol, (Z)-3-[(2,4-dimethyl-5-(2-oxo-1,2-dihydroindole-3-ylidenemethyl)-1H-pyrole-3-yl)-propionic acid, 5-(5-fluoro-2-oxo-1,2-dihydroindole-3-ylidenemethyl)-2,4-dimethyl-1H-pyrrole-3-carboxylic acid, 1-(4-chloroanilino)-4-(4-pyridylmethyl)phthalazine, N-[4-(3-amino-1H-indazole-4-yl)phenyl-N'-(2-fluoro-5-methylphenyl)urea, 1, 2-dimethyl-7- (2-thiophene) imidazolo [5, 4-g] quinoxaline, 1, 2-dimethyl-6-phenyl imidazolo [5, 4-g] quinoxaline, 1, 2-dimethyl-6- (2-thiophene) imidazolo [5, 4-g] quinoxaline, AG1295, AG1296, 3-arylquinoline, 4-pyridyl-2-arylpyrimidine, sorafenib, MLN518, PKC412, AMN107, suramin, or neomycin, or a pharmaceutically acceptable salt thereof; and (b) ranibizumab, bevacizumab, aflibercept, KH902 VEGF receptor-Fc fusion protein, 2C3 antibody, ORA102, pegaptanib, bevasiranib, SIRNA-027, decursin, decursinol, picropodophyllin, guggulsterone, PLG101, eicosanoid LXA4, PTK787, pazopanib, axitinib, CDDO-Me, CDDO-Imm, shikonin, beta-hydroxyisovalerylshikonin, or ganglioside GM3, DC101 antibody, Mab25 antibody, Mab73 antibody, 4A5 antibody, 4E10 antibody, 5F12 antibody, VA01 antibody, BL2 antibody, VEGF-related protein, sFLT01 sFLT02, Peptide B3, TG100801, sorafenib, or G6-31 antibody, or a pharmaceutically acceptable salt thereof (see Table 1). ARC-127, Antagonist A, Antagonist B, Antagonist C, Antagonist D, 1B3 antibody, CDP860, IMC-3G3, imatinib, 162.62 antibody, 163.31 antibody, 169.14 antibody, 169.31 antibody, αR1 antibody, 2A1E2 antibody, M4TS.11 antibody, M4TS22 antibody, A10, brefeldin A, sunitinib, Hyb 120.1.2.1.2 antibody, Hyb 121.6.1.1.1 antibody, Hyb 127.5.7.3.1 antibody, Hyb 127.8.2.2.2 antibody, Hyb 1.6.1 antibody, Hyb 1.11.1 antibody, Hyb 1.17.1 antibody, Hyb 1.18.1 antibody, Hyb 1.19.1 antibody, Hyb 1.23.1 antibody, Hyb 1.24 antibody, Hyb 1.25 antibody, Hyb 1.29 antibody, Hyb 1.33 antibody, Hyb 1.38 antibody, Hyb 1.39 antibody, Hyb 1.40 antibody, Hyb 1.45 antibody, Hyb 1.46 antibody, Hyb 1.48 antibody, Hyb 1.49 antibody, Hyb 1.51 antibody, Hyb 6.4.1 antibody, F3 antibody, Humanized F3 antibody, C1 antibody, Humanized C1 antibody, 6.4 antibody, anti-mPDGF-D goat IgG antibody, C3.1 antibody, 5-methyl-7-diethylamino-s-triazolo (1,5-a) pyrimidine, interferon, protamine, PDGFR-B1 monoclonal antibody, PDGFR-B2 monoclonal antibody, 6D11 monoclonal antibody, Sis 1 monoclonal antibody, PR7212 monoclonal antibody, PR292 monoclonal antibody, HYB 9610 monoclonal antibody, HYB 9611 monoclonal antibody, HYB 9612 monoclonal antibody, HYB 9613 monoclonal antibody, 4-(2-(N-(-2-carboxamidoindole) aminoethyl)-benzenesulfonamide, 4-(2-(N-(-2-carboxamidoindole)aminoethyl)-sulfonylurea, CGP 53716, human antibody g162, pyrazolo[3,4-g]quinoxaline, 6-[2-(methylcarbamoyl)phenylsulphanyl]-3-E-[2-(pyridine-2-yl)ethenyl]-indazole, 1-{2-[5-(2-methoxy-ethoxy)-benzoimidazole-1-yl]-quinoline-8-yl}-piperidine-4-ylamine, 4-[4-[N-(4-nitrophenyl)carbamoyl]-1-piperazinyl]-6,7-dimethoxyquinazoline, 4-amino-5-fluoro-3-(6-(4-methyl-piperazine-1-yl)-1H-benzimidazole-2-yl)-1H-quinoline-2-one, (4-tert-butylphenyl){4-[(6,7-dimethoxy-4-quinolyl)oxy]phenyl}methaneone, 5-methyl-N-[4-(trifluoromethyl)phenyl]-4-isoxazolecarboxamide, trans-4-[(6,7-dimethoxyquinoxaline-2-yl)amino]cyclohexanol, (Z)-3-[(2,4-dimethyl-5-(2-oxo-1,2-dihydroindole-3-ylidenemethyl)-1H-pyrrole-3-yl)-propionic acid, 5-(5-fluoro-2-oxo-1,2-dihydroindole-3-ylidenemethyl)-2,4-dimethyl-1H-pyrrole-3-carboxylic acid, 1-(4-chloroanilino)-4-(4-pyridylmethyl)phthalazine, N-[4-(3-amino-1H-indazole-4-yl)phenyl-N'-(2-fluoro-5-methylphenyl)urea, 1, 2-dimethyl-7- (2-thiophene) imidazolo [5, 4-g] quinoxaline, 1, 2-dimethyl-6-phenyl imidazolo [5, 4-g] quinoxaline, 1, 2-dimethyl-6- (2-thiophene) imidazolo [5, 4-g] quinoxaline, AG1295, AG1296, 3-arylquinoline, 4-pyridyl-2-arylpyrimidine, sorafenib, MLN518, PKC412, AMN107, suramin, and neomycin, and their pharmaceutically acceptable salts are agents that inhibit platelet-derived growth factor (PDGF). Ranibizumab, bevacizumab, aflibercept, KH902 VEGF receptor-Fc fusion protein, 2C3 antibody, ORA102, pegaptanib, bevasiranib, SIRNA-027, decursin, decursinol, picropodophyllin, guggulsterone, PLG101, eicosanoid LXA4, PTK787, pazopanib, axitinib, CDDO-Me, CDDO-Imm, shikonin, beta-hydroxyisovalerylshikonin, or ganglioside GM3, DC101 antibody, Mab25 antibody, Mab73 antibody, 4A5 antibody, 4E10 antibody, 5F12 antibody, VA01 antibody, BL2 antibody, VEGF-related protein, sFLT01, sFLT02, Peptide B3, TG100801, sorafenib, and G6-31 antibody, and their pharmaceutically acceptable salts are agents that inhibit vascular endothelial growth factor (VEGF). Specific PDGF antagonist-VEGF antagonist pairs useful in the present methods or compositions are set forth in Table 2 (pairs A-EID). The PDGF antagonist or VEGF antagonist of Tables 1 and 2 can be in the form of a pharmaceutically acceptable salt. In the present methods, the PDGF antagonist of any of pairs A-EID can be administered prior to, subsequently to or concurrently with administration of the VEGF antagonist of any of pairs A-EID. In a particular embodiment, the PDGF antagonist is Antagonist A or a pharmaceutically acceptable salt thereof. In another particular embodiment, the PDGF antagonist is Antagonist B or a pharmaceutically acceptable salt thereof. In another particular embodiment, the PDGF antagonist is Antagonist C or a pharmaceutically acceptable salt thereof. In another particular embodiment, the PDGF antagonist is Antagonist D or a pharmaceutically acceptable salt thereof. In another embodiment, the VEGF antagonist is ranibizumab, bevacizumab or aflibercept, or a pharmaceutically acceptable salt thereof. In further embodiments, the methods can further comprise administering another agent that is useful for treating or preventing an ophthalmological disease, such as volociximab.

**Table 1. List of (a) PDGF antagonists and (b) VEGF antagonists**

| (a) PDGF Antagonists | (b) VEGF Antagonists |
|---|---|
| ARC-127 | ranibizumab |
| A compound of Formula A | bevacizumab |
| Antagonist A | aflibercept |
| A compound of Formula B | KH902 VEGF receptor-Fc fusion protein |
| Antagonist B | 2C3 antibody |
| A compound of Formula C | ORA102 |
| Antagonists C | pegaptanib |
| Antagonist D | bevasiranib |
| A compound of Formula E | SIRNA-027 |
| 1B3 antibody | decursin |
| CDP860 | decursinol |
| IMC-3G3 | picropodophyllin |
| Imatinib | guggulsterone |
| 162.62 antibody | PLG101 |
| 163.31 antibody | eicosanoid LXA4 |
| 169.14 antibody | PTK787 |
| 169.31 antibody | pazopanib |
| αR1 antibody | axitinib |
| 2A1E2 antibody | CDDO-Me |
| M4TS.11 antibody | CDDO-Imm |
| M4TS.22 antibody | shikonin |
| A10 | beta-hydroxyisovalerylshikonin |
| brefeldin A | ganglioside GM3 |
| Sunitinib | DC101 antibody |
| Hyb 120.1.2.1.2 antibody | Mab25 antibody |
| Hyb 121.6.1.1.1 antibody | Mab73 antibody |
| Hyb 127.5.7.3.1 antibody | 4A5 antibody |
| Hyb 127.8.2.2.2 antibody | 4E10 antibody |
| Hyb 1.6.1 antibody | 5F12 antibody |
| Hyb 1.11.1 antibody | VA01 antibody |
| Hyb 1.17.1 antibody | BL2 antibody |
| Hyb 1.18.1 antibody | VEGF-related protein |
| Hyb 1.19.1 antibody | sFLT01 |
| Hyb 1.23.1 antibody | sFLT02 |
| Hyb 1.24 antibody | Peptide B3 |
| Hyb 1.25 antibody | TG100801 |
| Hyb 1.29 antibody | sorafenib |
| Hyb 1.33 antibody | G6-31 antibody |
| Hyb 1.38 antibody | A fusion antibody substance that specifically binds to one or more of a human vascular endothelial growth factor-A (VEGF-A), human vascular endothelial growth factor-B (VEGF-B), human vascular endothelial growth factor-C (VEGF-C), human vascular endothelial growth factor-D (VEGF-D), or human vascular endothelial growth factor-E (VEGF-E) |
| Hyb 1.39 antibody | An antibody that binds to an epitope of VEGF |
| Hyb 1.40 antibody | |
| Hyb 1.45 antibody | |
| Hyb 1.46 antibody | |
| Hyb 1.48 antibody | |
| Hyb 1.49 antibody | |
| Hyb 1.51 antibody | |
| Hyb 6.4.1 antibody | |
| F3 antibody | |
| Humanized F3 antibody | |
| C1 antibody | |
| Humanized C1 antibody | |
| 6.4 antibody | |
| anti-mPDGF-C goat IgG antibody | |
| C3.1 antibody | |
| 5-methyl-7-diethylamino-s-triazolo (1,5-a) pyrimidine | |
| Interferon | |
| Protamine | |
| PDGFR-B 1 monoclonal antibody | |
| PDGFR-B2 monoclonal antibody | |
| 6D11 monoclonal antibody | |
| Sis 1 monoclonal antibody | |
| PR7212 monoclonal antibody | |
| PR292 monoclonal antibody | |
| IIYB 9610 monoclonal antibody | |
| HYB 9611 monoclonal antibody | |
| HYB 9612 monoclonal antibody | |
| HYB 9613 monoclonal antibody | |
| 4-(2-(N-(-2-carboxamidoindole) aminoethyl)-benzenesulfonamide | |
| 4-(2-(N-(-2-carboxamidoindole)aminoethyl)-sulfonylurea | |
| CGP 53716 small molecule | |
| human antibody g162 | |
| pyrazolo[3,4-g]quinoxaline | |
| 6-[2-(methylcarbamoyl)phenylsulphanyl]-3-E-[2-(pyridine-2-yl)ethenyl]-indazole | |
| 1-{2-[5-(2-methoxy-ethoxy)-benzoimidazole-1-yl]-quinoline-8-yl}-piperidine-4-ylamine | |
| 4-[4-[N-(4-nitrophenyl)carbamoyl]-1-piperazinyl]-6,7-dimethoxyquinazoline | |
| 4-amino-5-fluoro-3-(6-(4-methyl-piperazine-1-yl)-1H-benzimidazole-2-yl)-1H-quinoline-2-one | |
| (4-tert-butylphenyl){4-[(6,7-dimethoxy-4-quinolyl)oxy]phenyl} methaneone | |
| 5-methyl-N-[4-(trifluoromethyl)phenyl]-4-isoxazolecarboxamide | |
| trans-4-[(6,7-dimethoxyquinoxaline-2-yl)amino]cyclohexanol | |
| (Z)-3-[(2,4-dimethyl-5-(2-oxo-1,2-dihydroindole-3-ylidenemethyl)-1H-pyrrole-3-yl)-propionic acid | |
| 5-(5-fluoro-2-oxo-1,2-dihydroindole-3-ylidenemethyl)-2,4-dimethyl-1H-pyrrole-3-carboxylic acid | |
| 1-(4-chloroanilino)-4-(4-pyridylmethyl)phthalazine | |
| N-[4-(3-amino-1H-indazole-4-yl)phenyl-N'-(2-fluoro-5-methylphenyl)urea | |
| 1, 2-dimethyl-7- (2-thiophene) imidazolo [5, 4-g] quinoxaline | |
| 1, 2-dimethyl-6-phenyl imidazolo [5, 4-g] quinoxaline | |
| 1, 2-dimethyl-6- (2-thiophene) imidazolo [5, 4-g] quinoxaline | |
| AG1295 | |
| AG1296 | |
| 3-arylquinoline | |
| 4-pyridyl-2-arylpyrimidine | |
| Sorafenib | |
| MLN518 | |
| PKC412 | |
| AMN107 | |
| Suramin | |
| Neomycin | |
| A fusion antibody substance that specifically binds to one or more of a human platelet-derived growth factor-A (PDGF-A), human | |
| platelet-derived growth factor-B (PDGF-B), human platelet-derived growth factor-C (PDGF-C), or human platelet-derived growth factor-D (PDGF-D) | |
| An antibody that binds to an epitope of PDGF | |

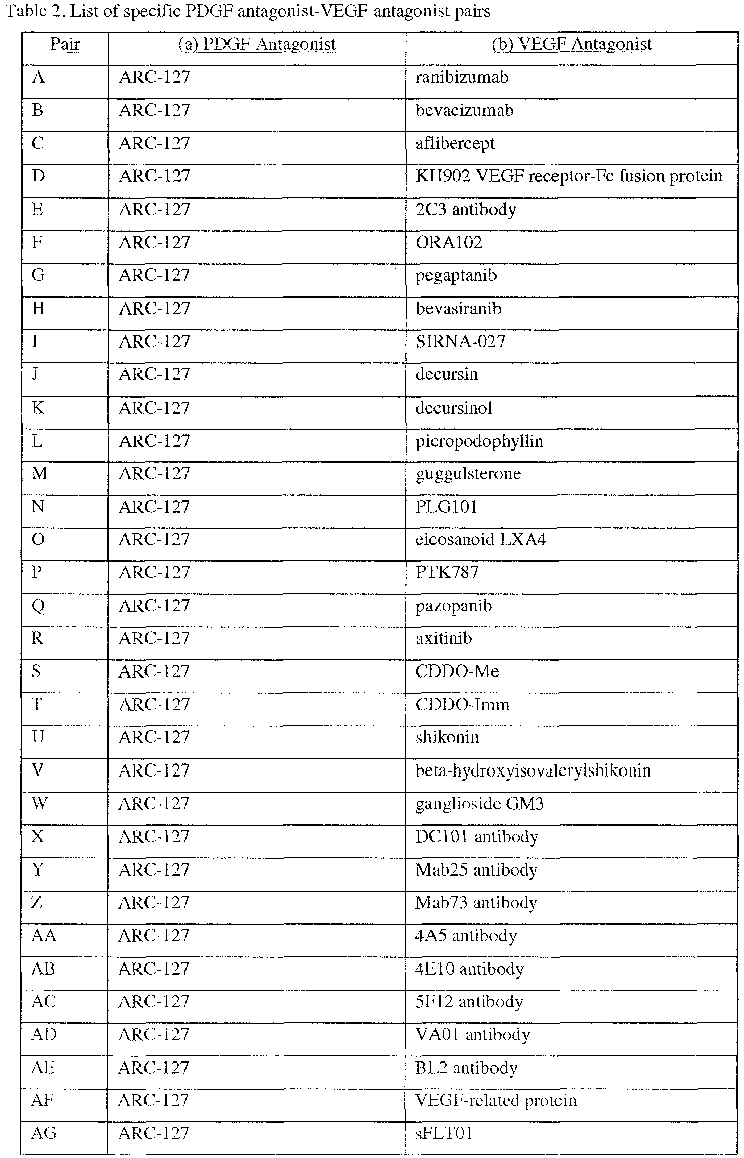

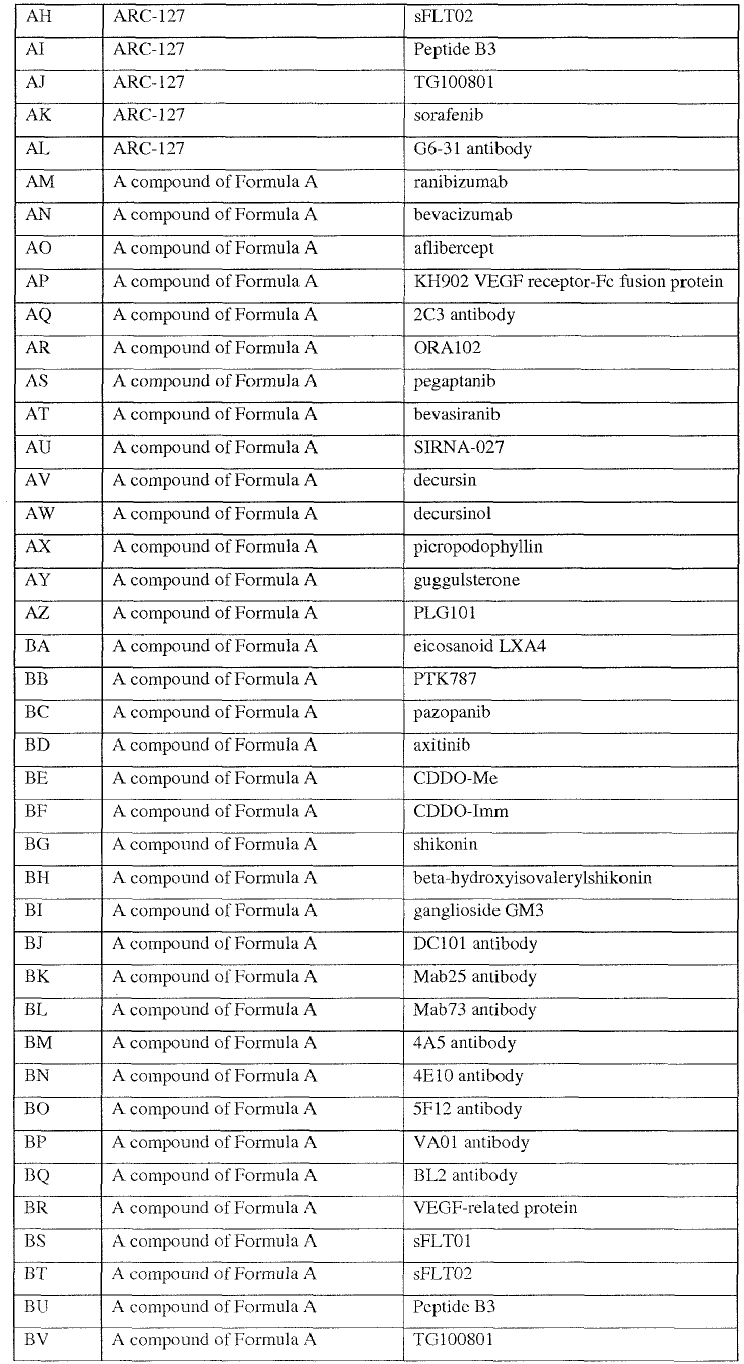

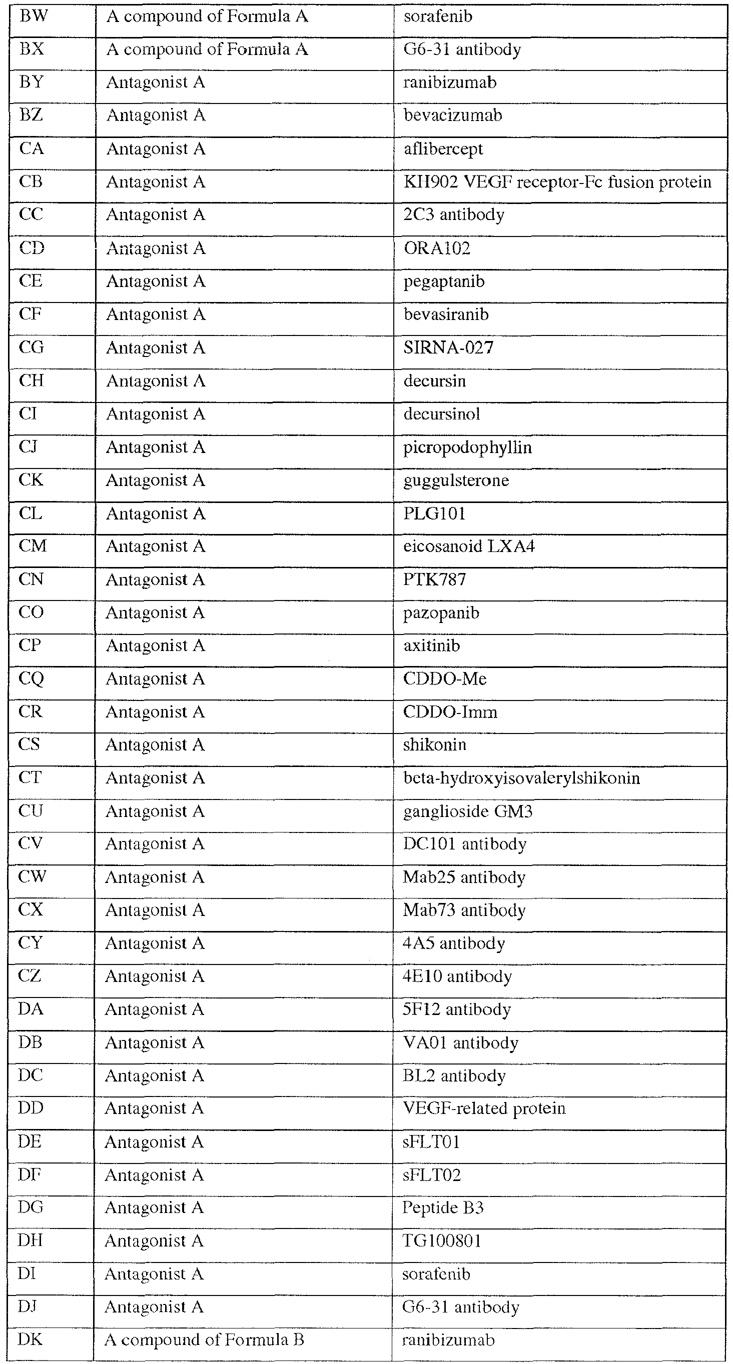

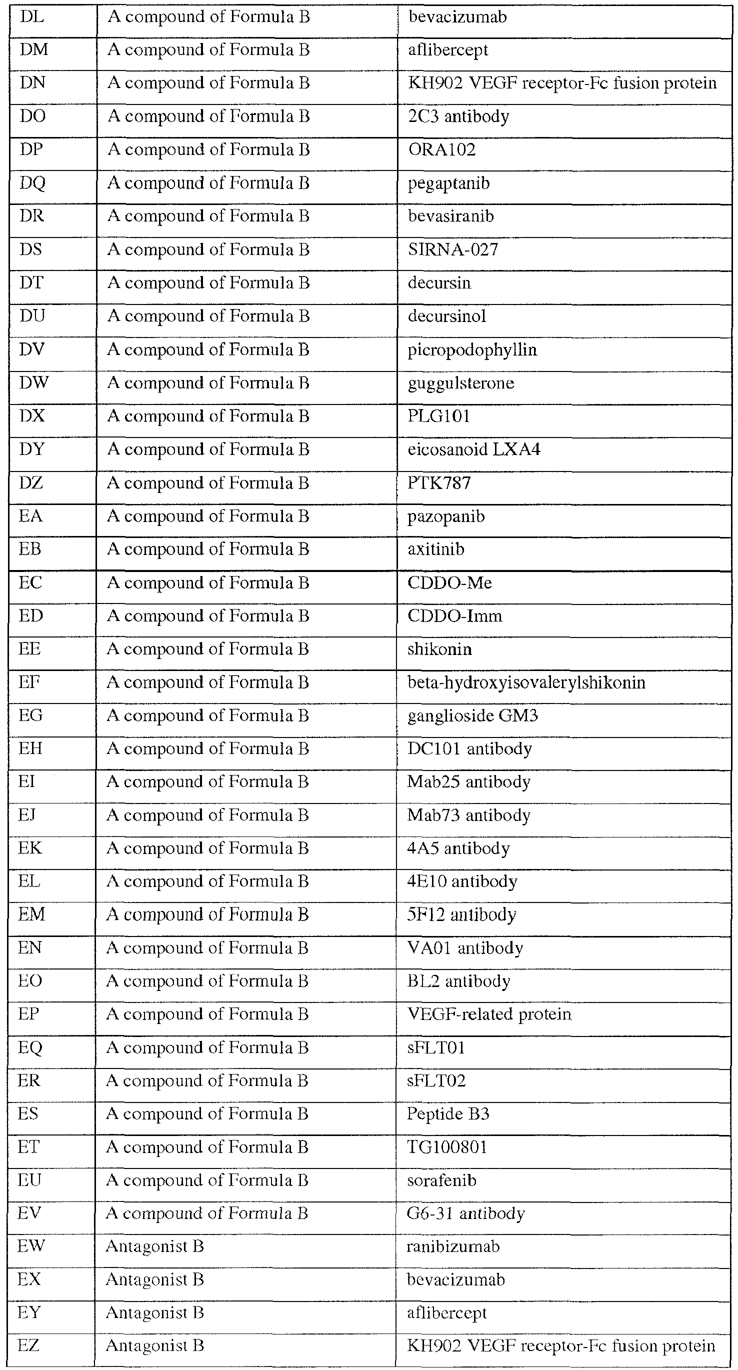

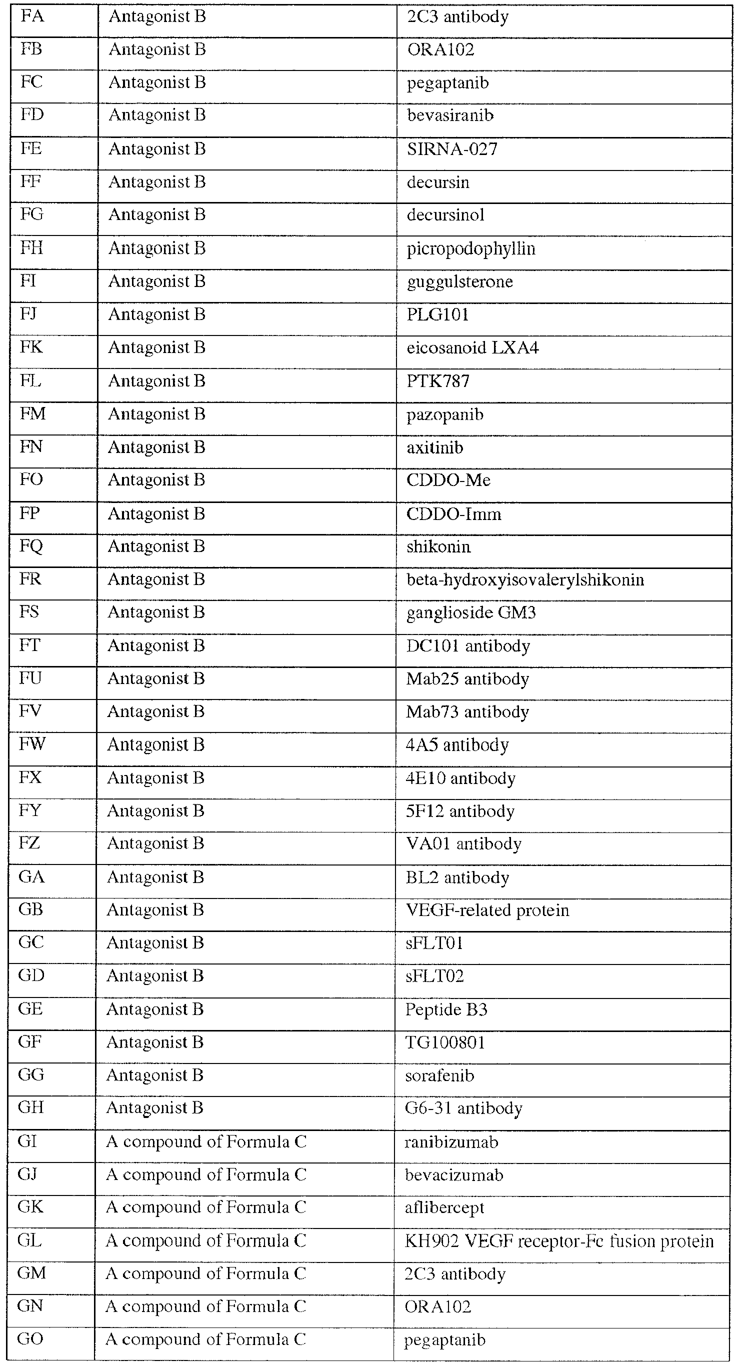

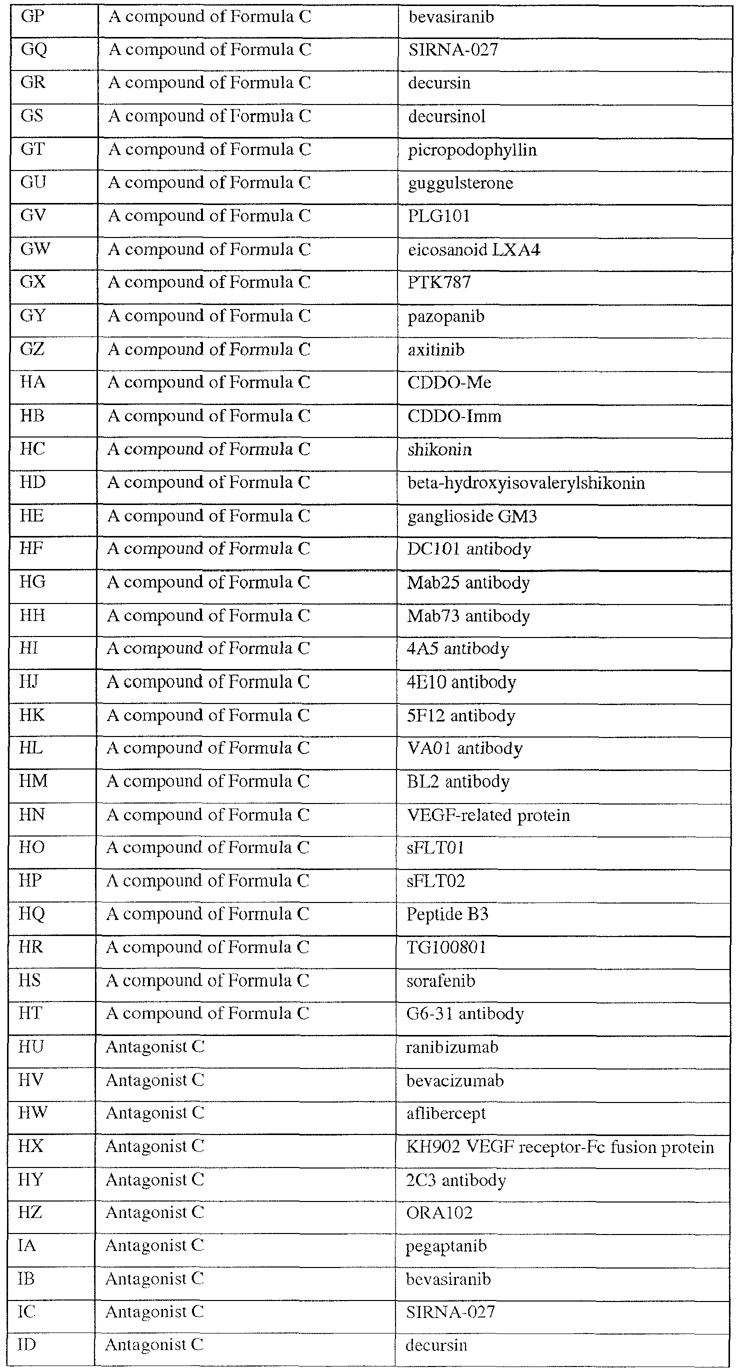

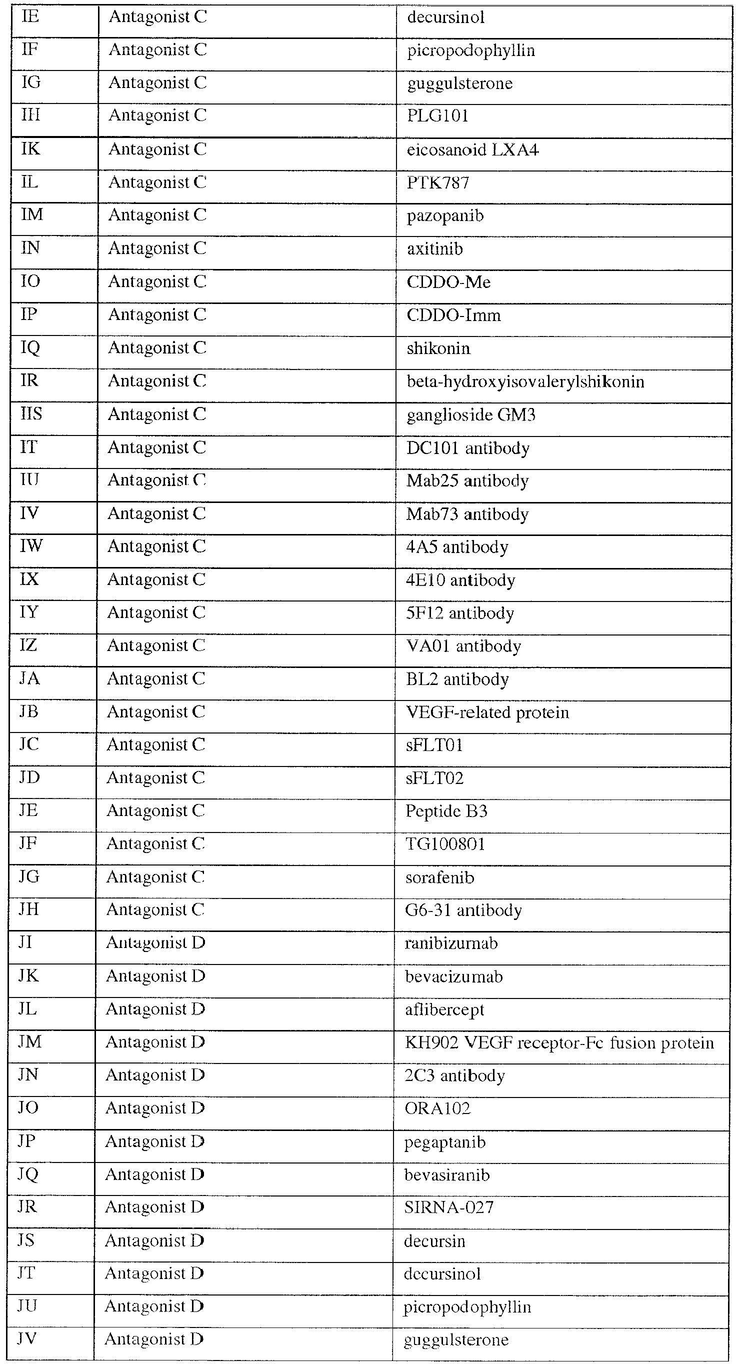

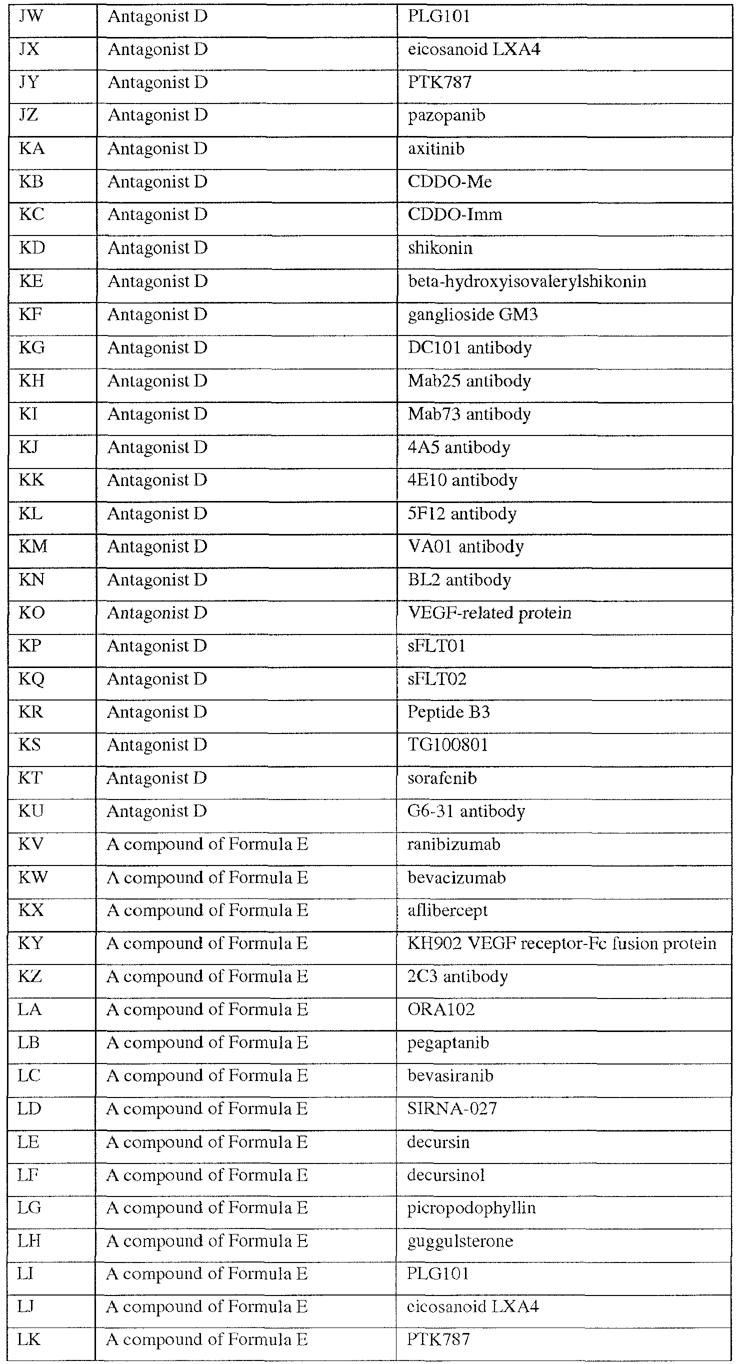

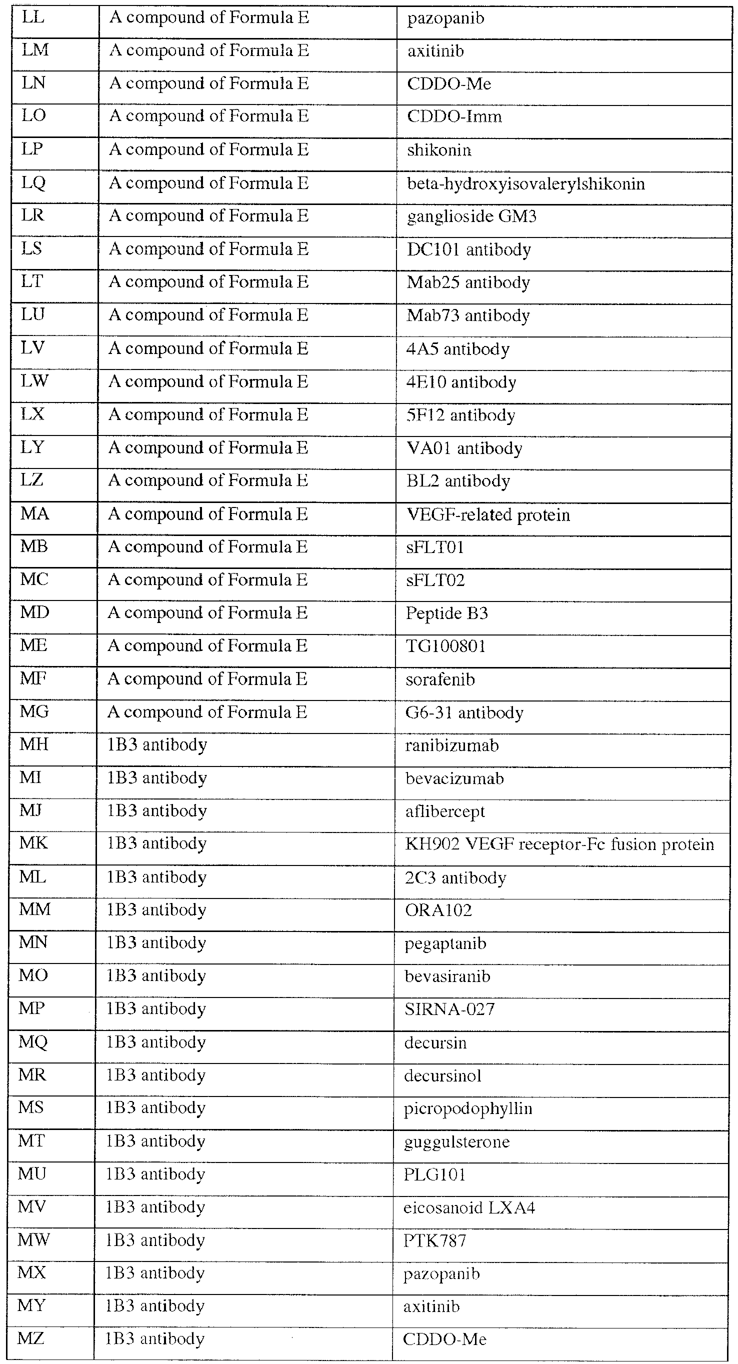

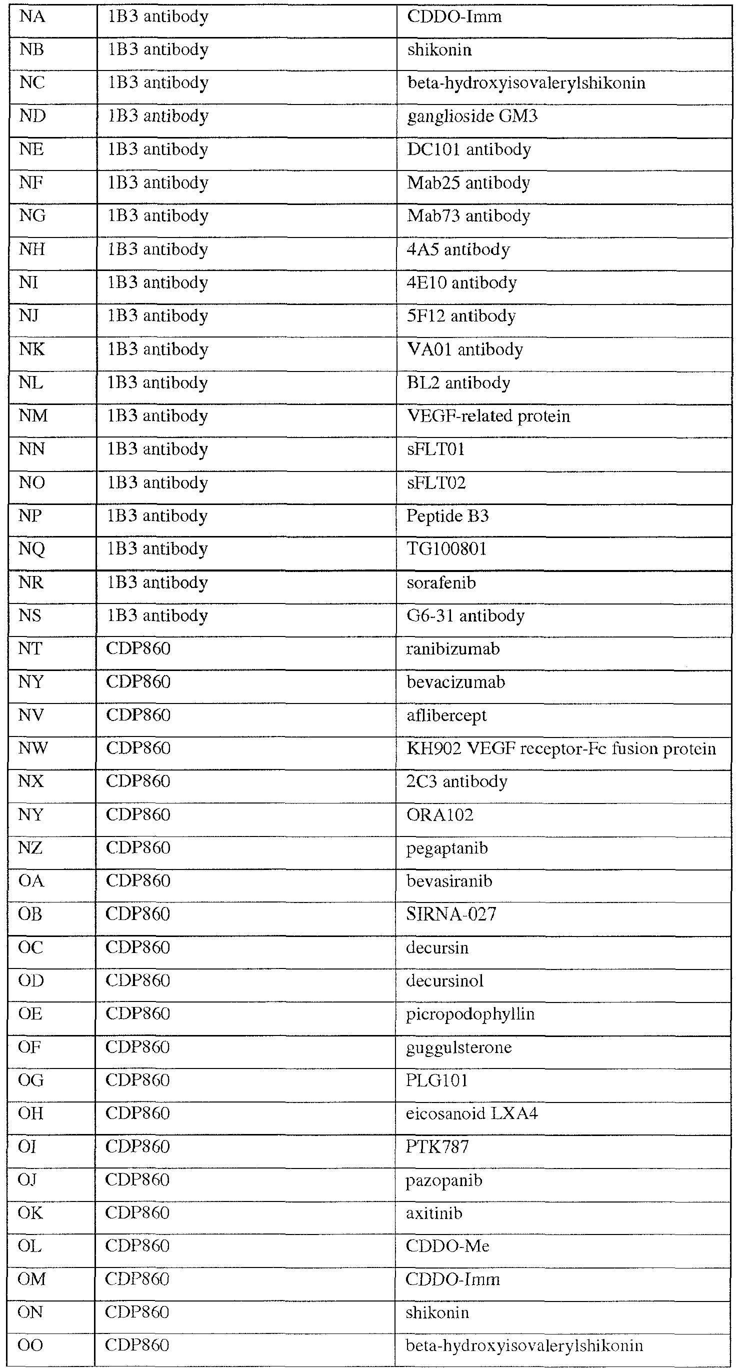

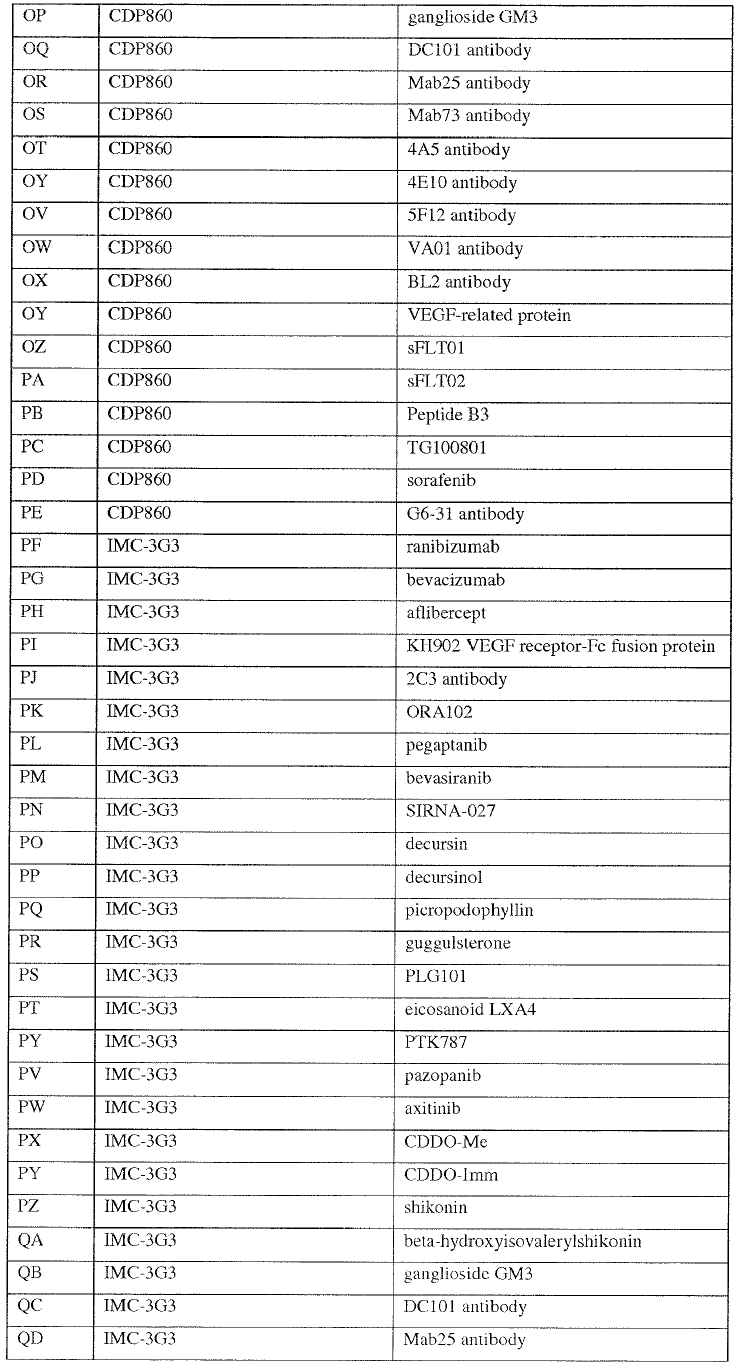

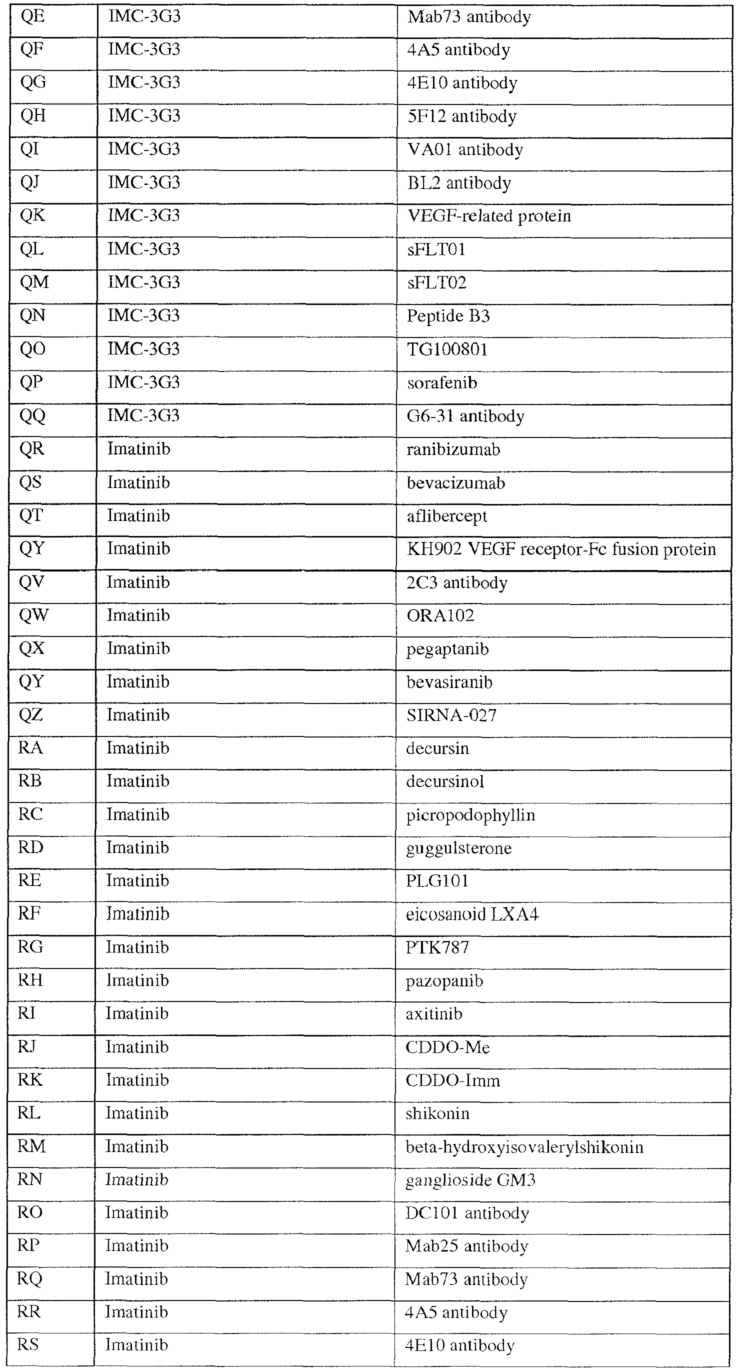

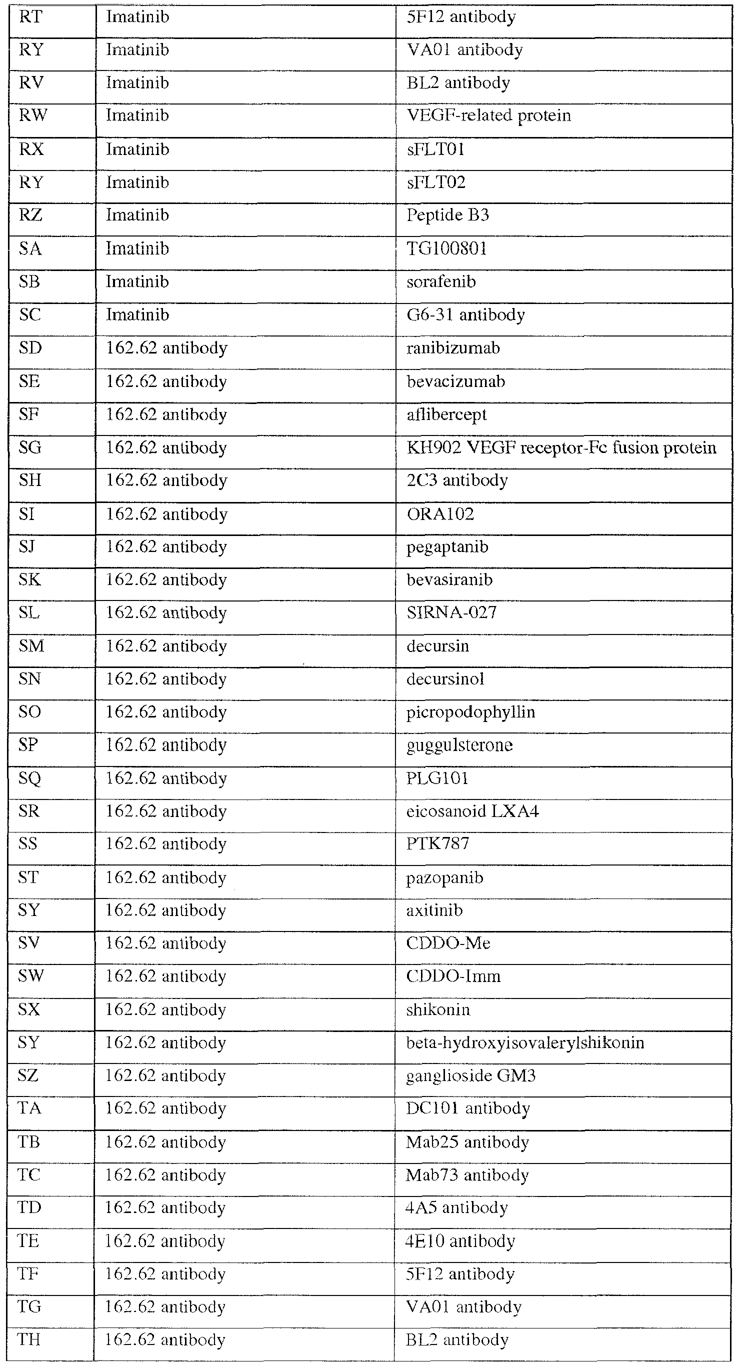

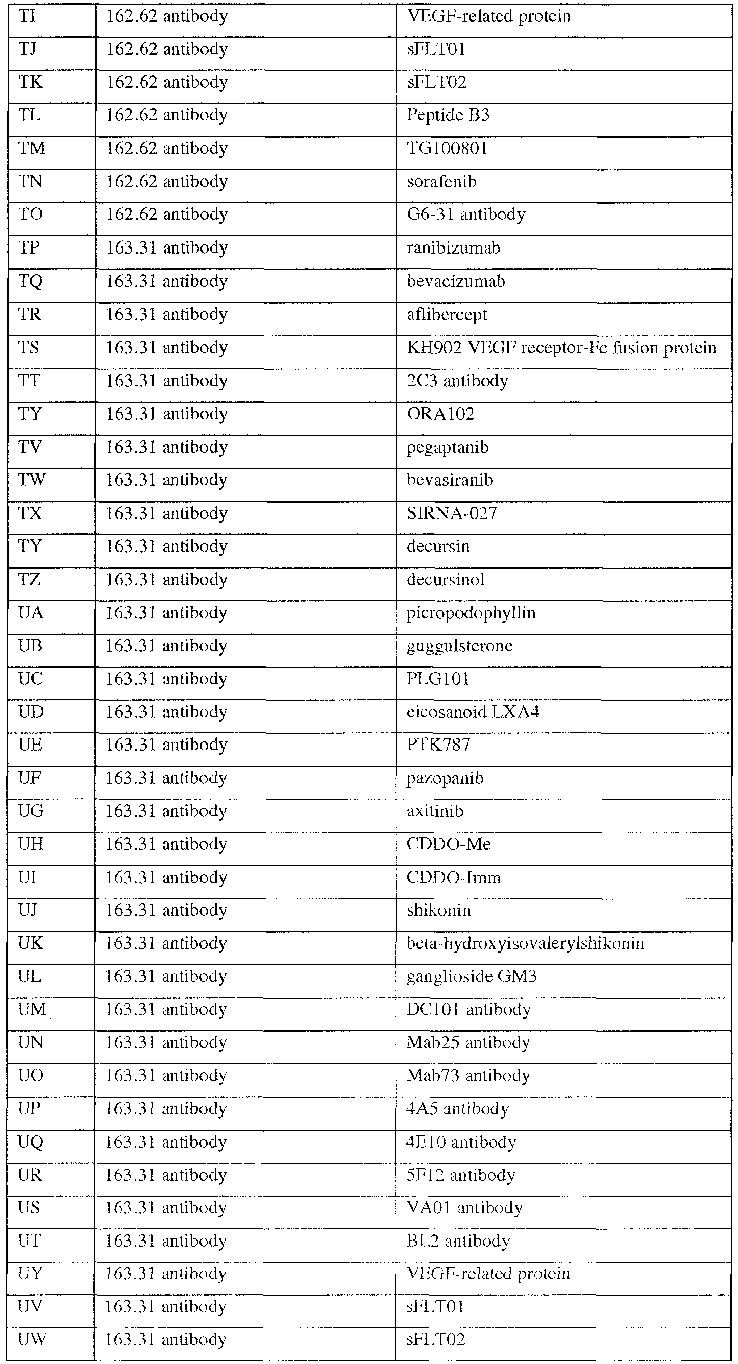

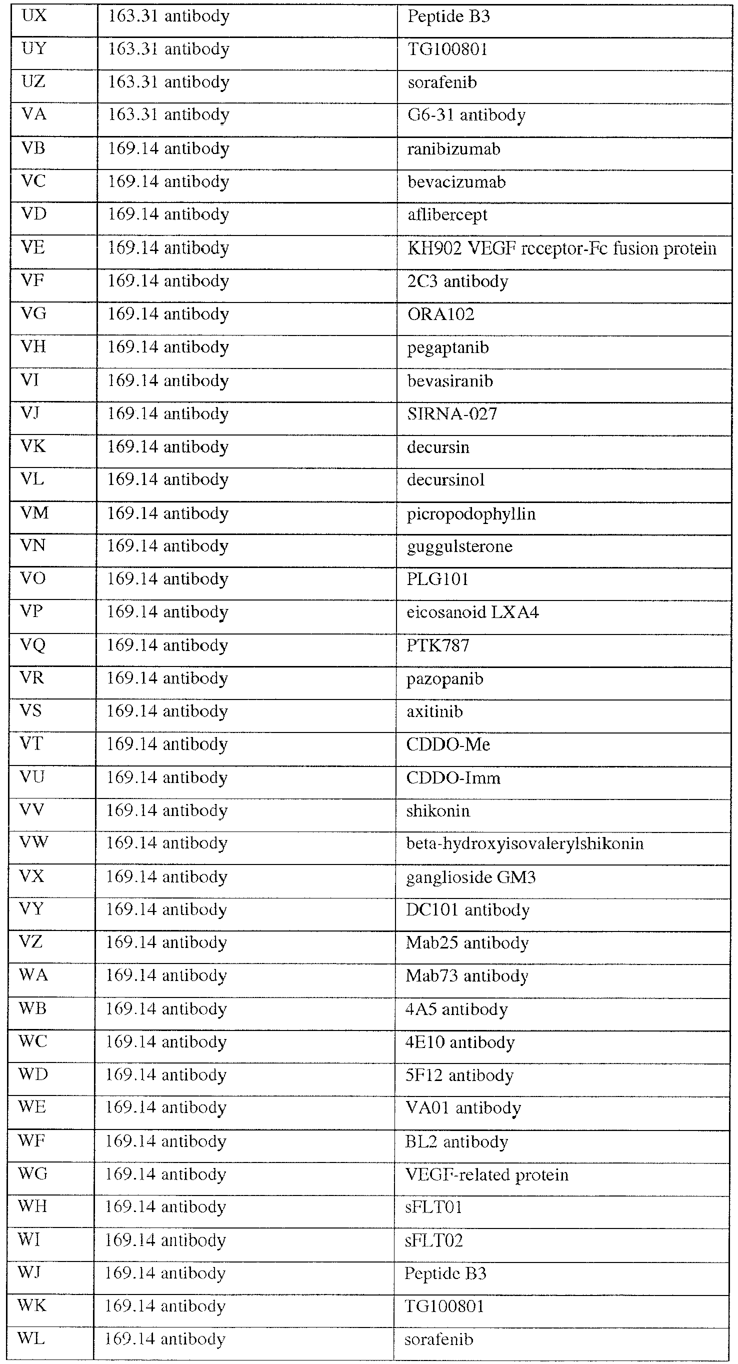

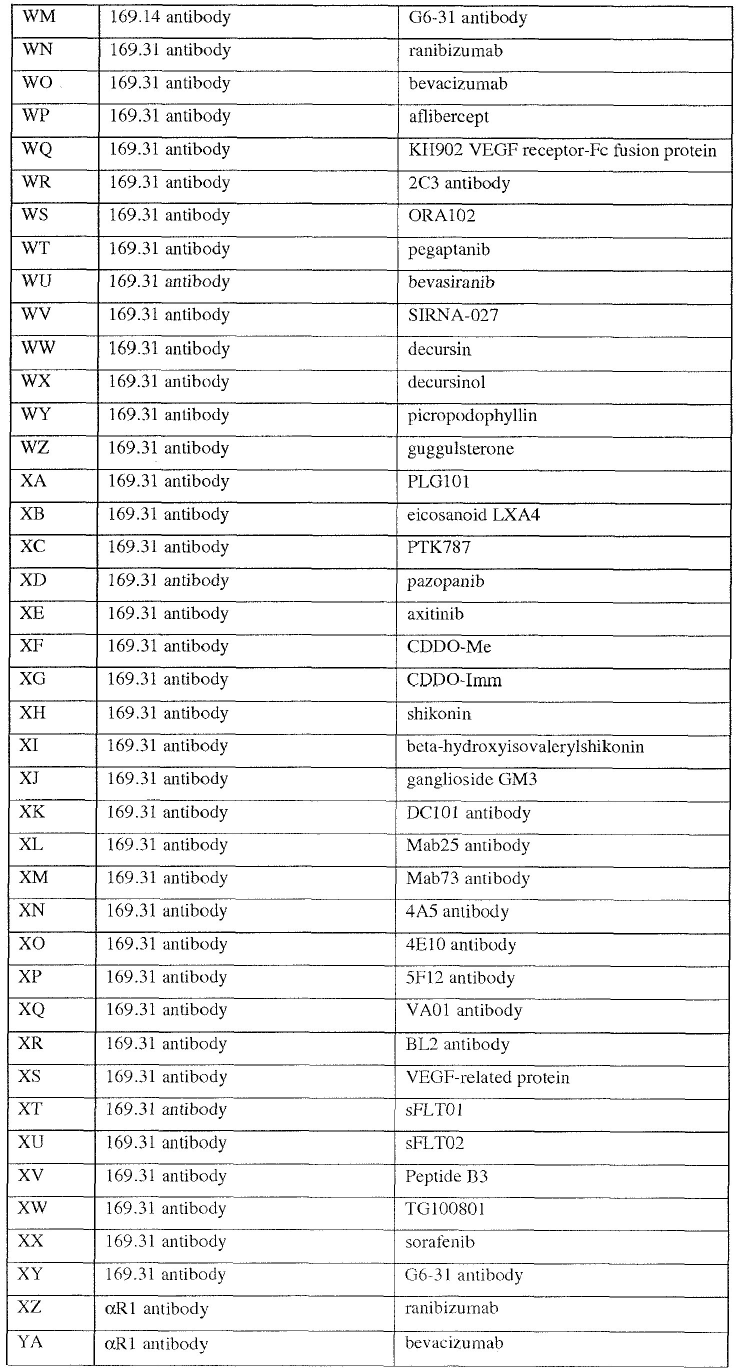

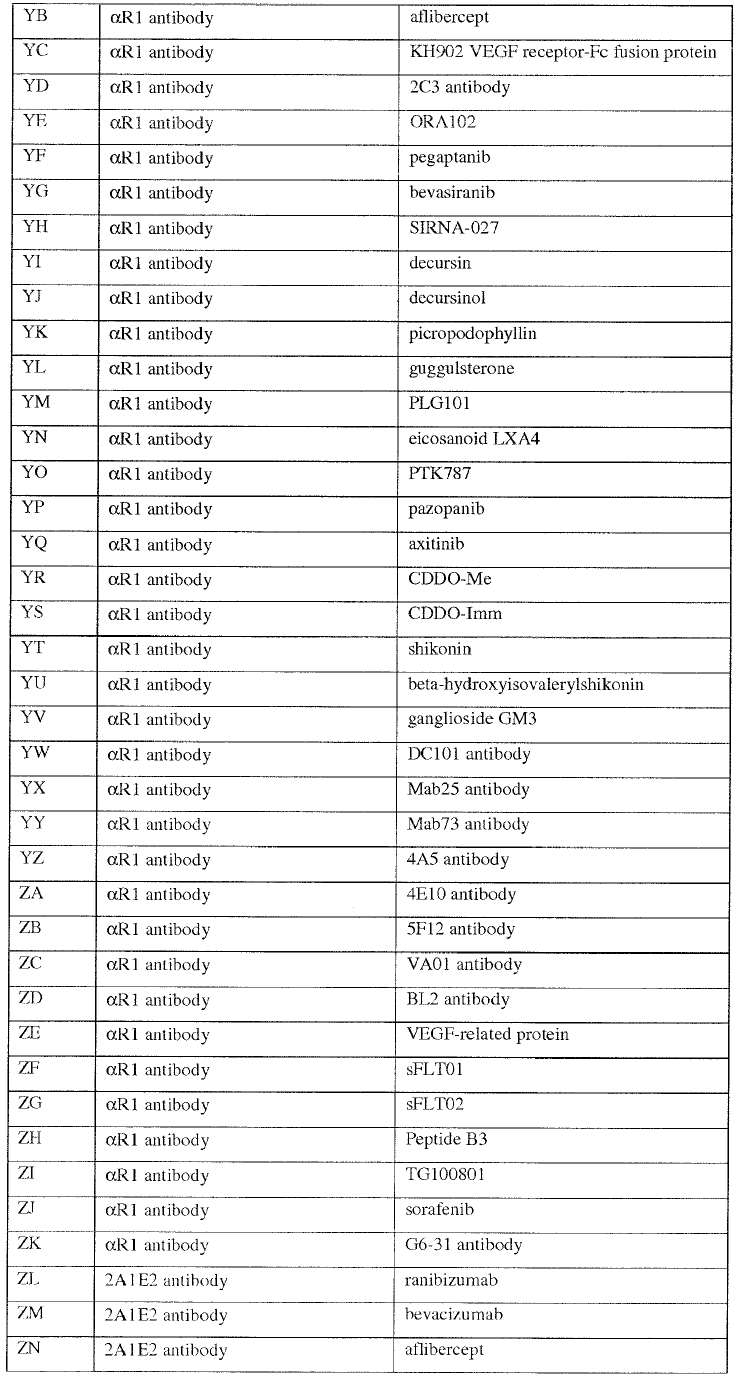

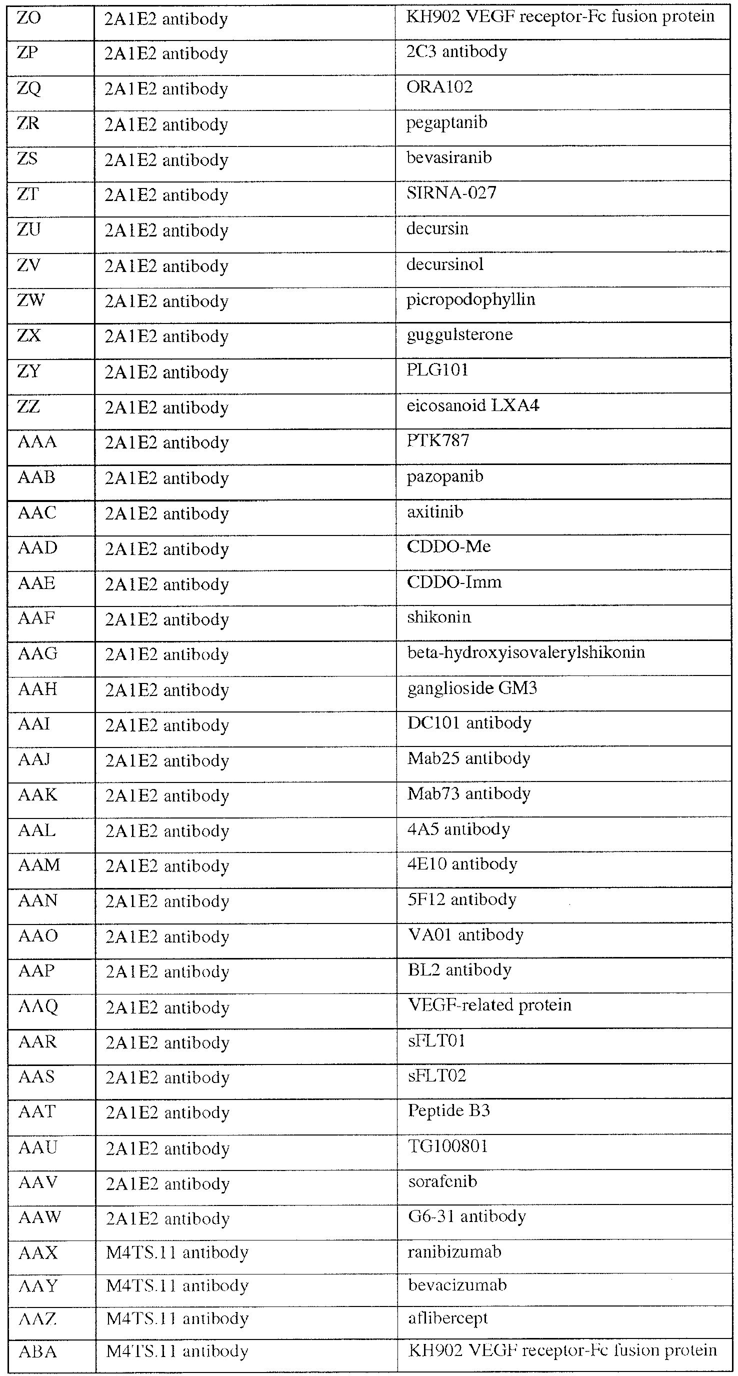

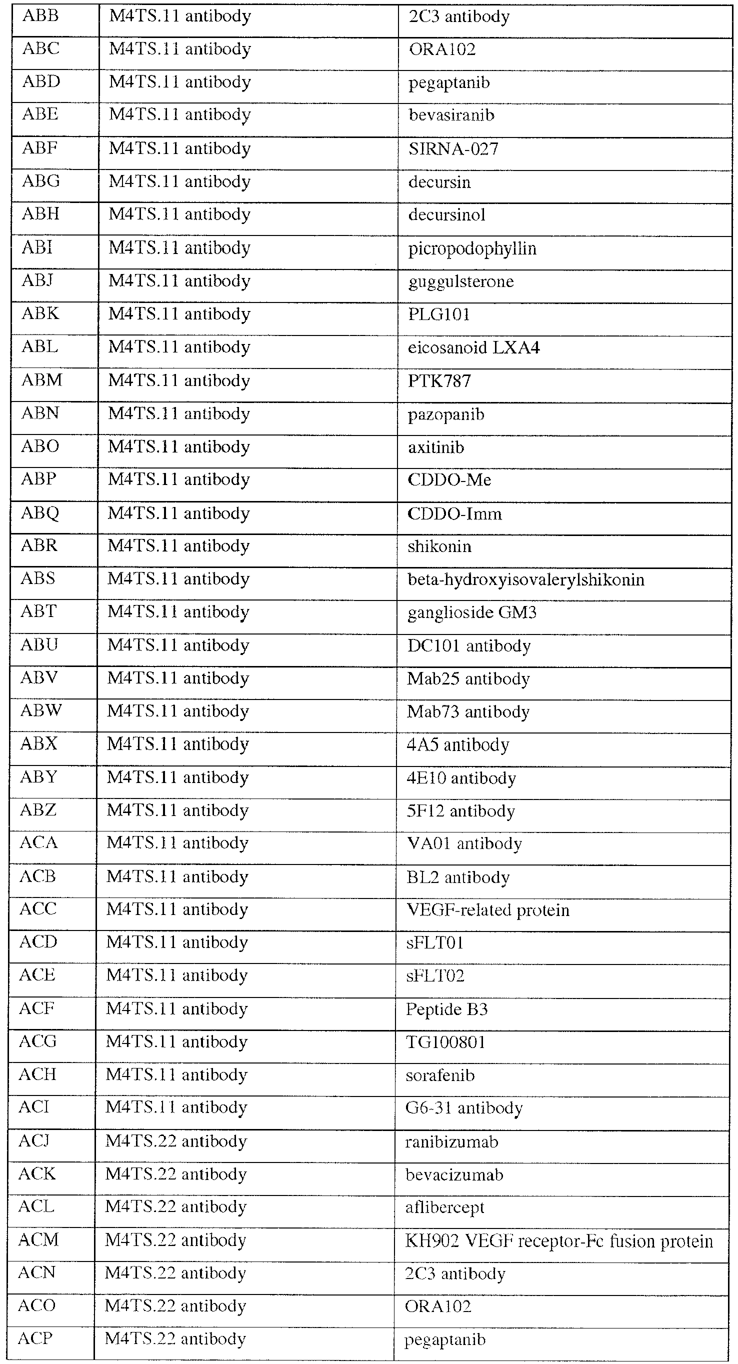

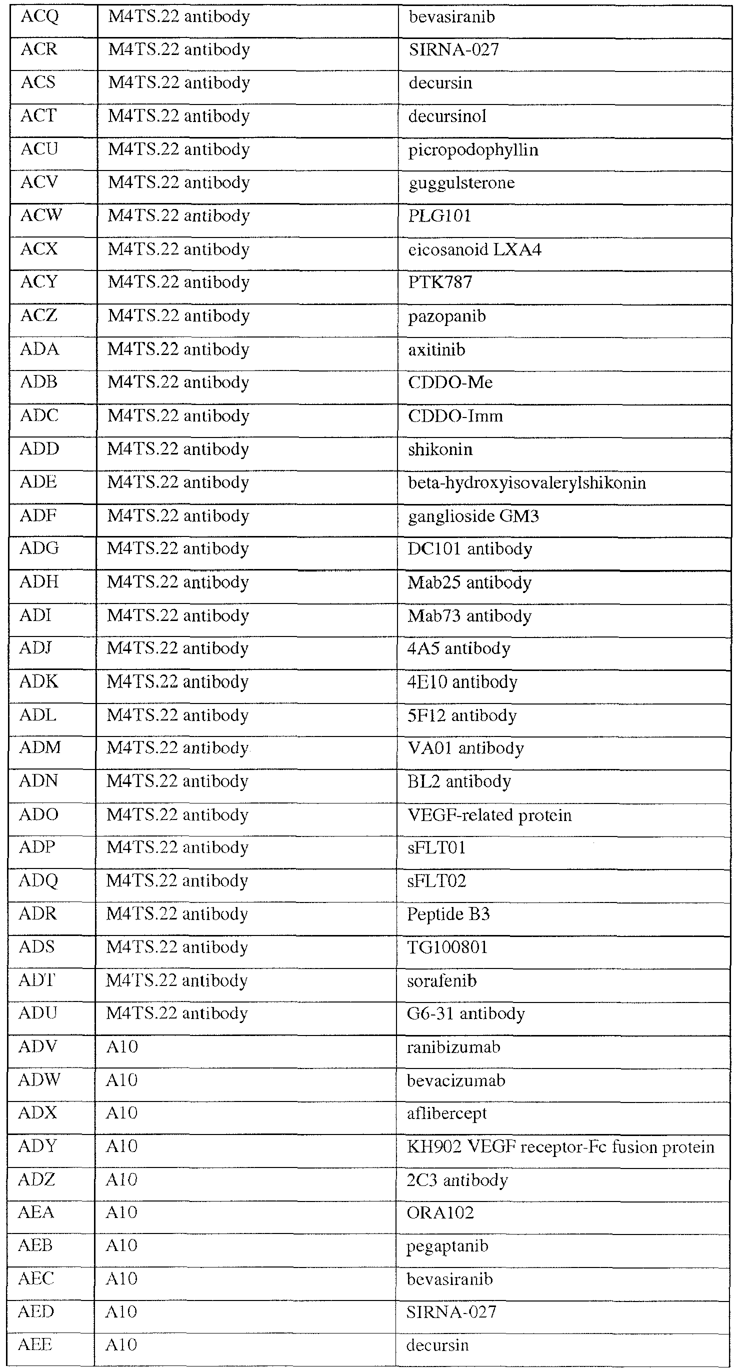

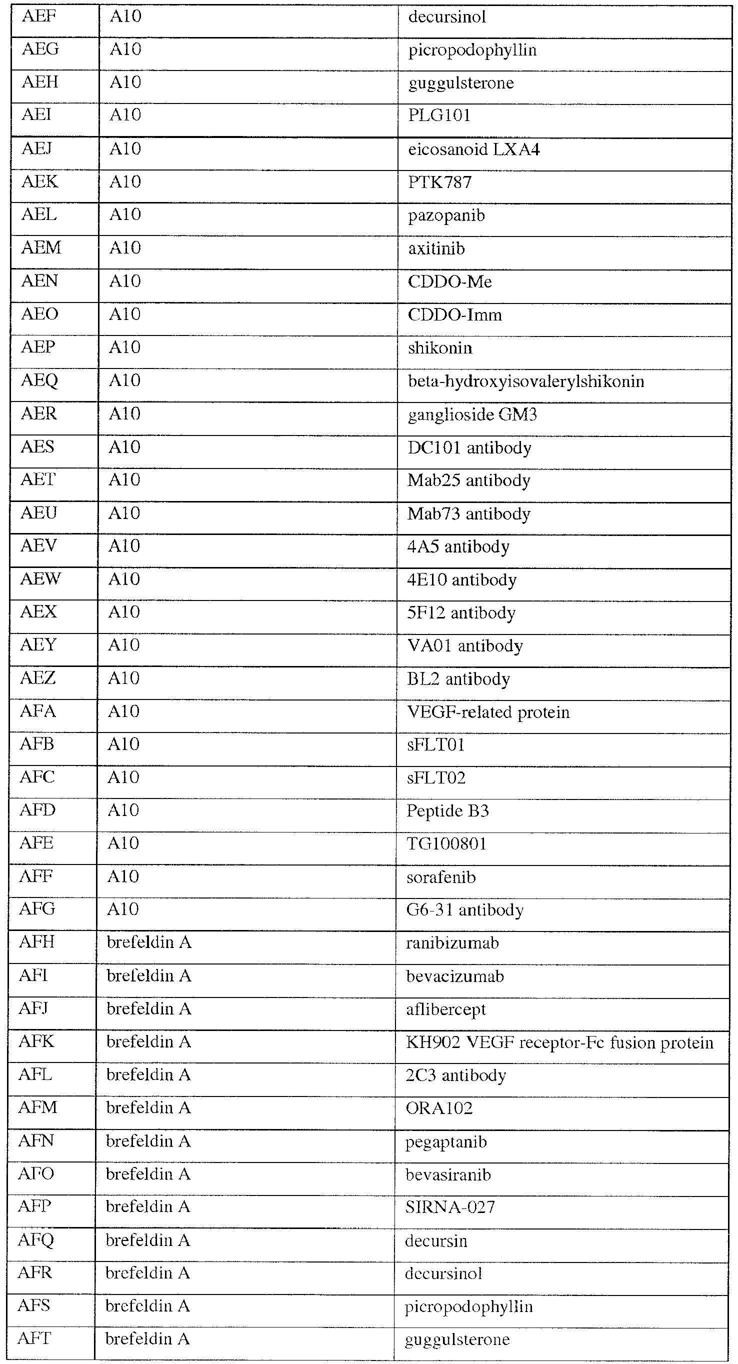

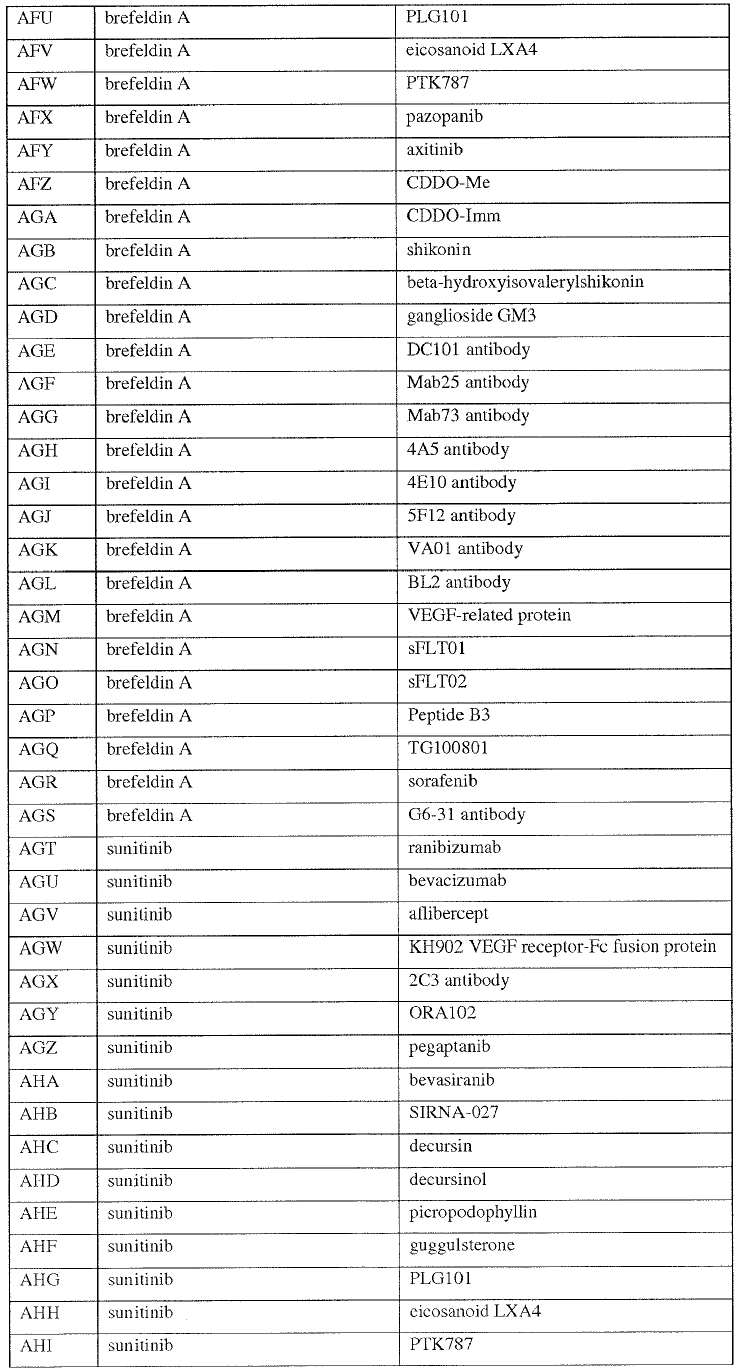

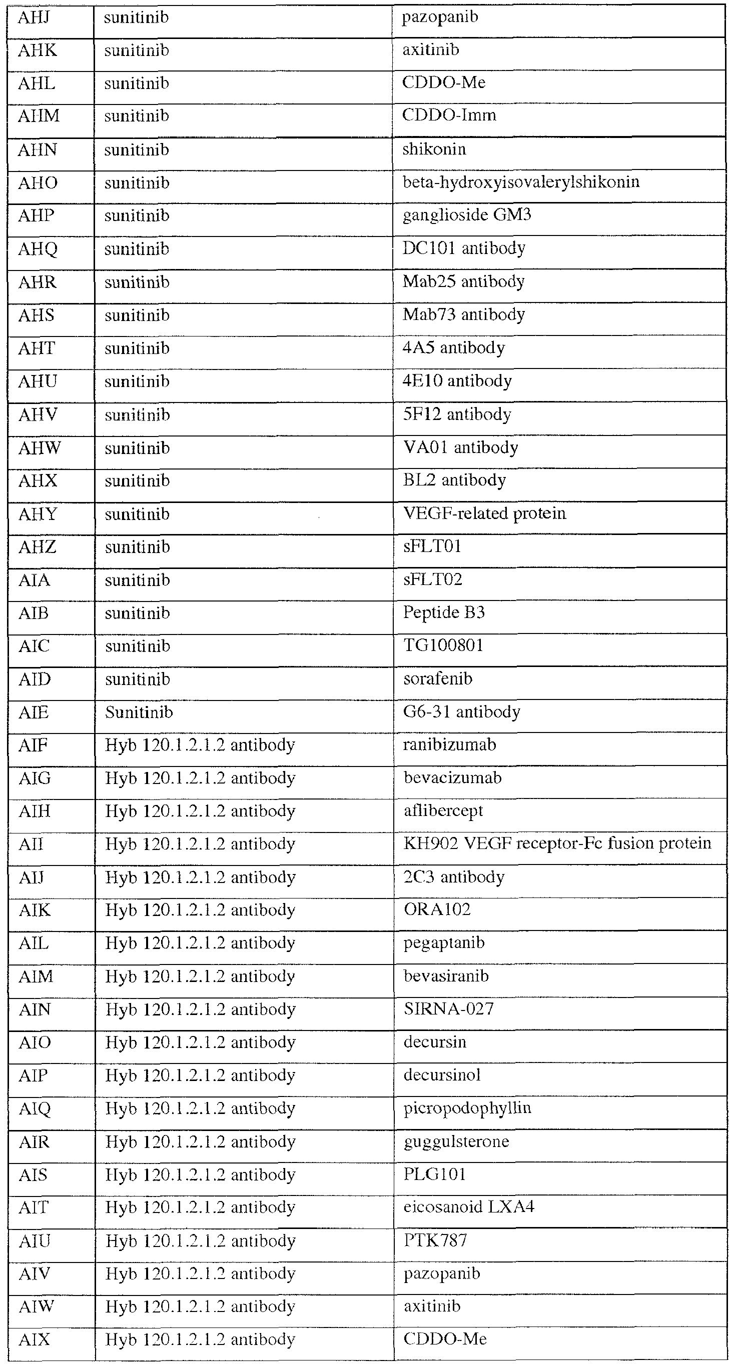

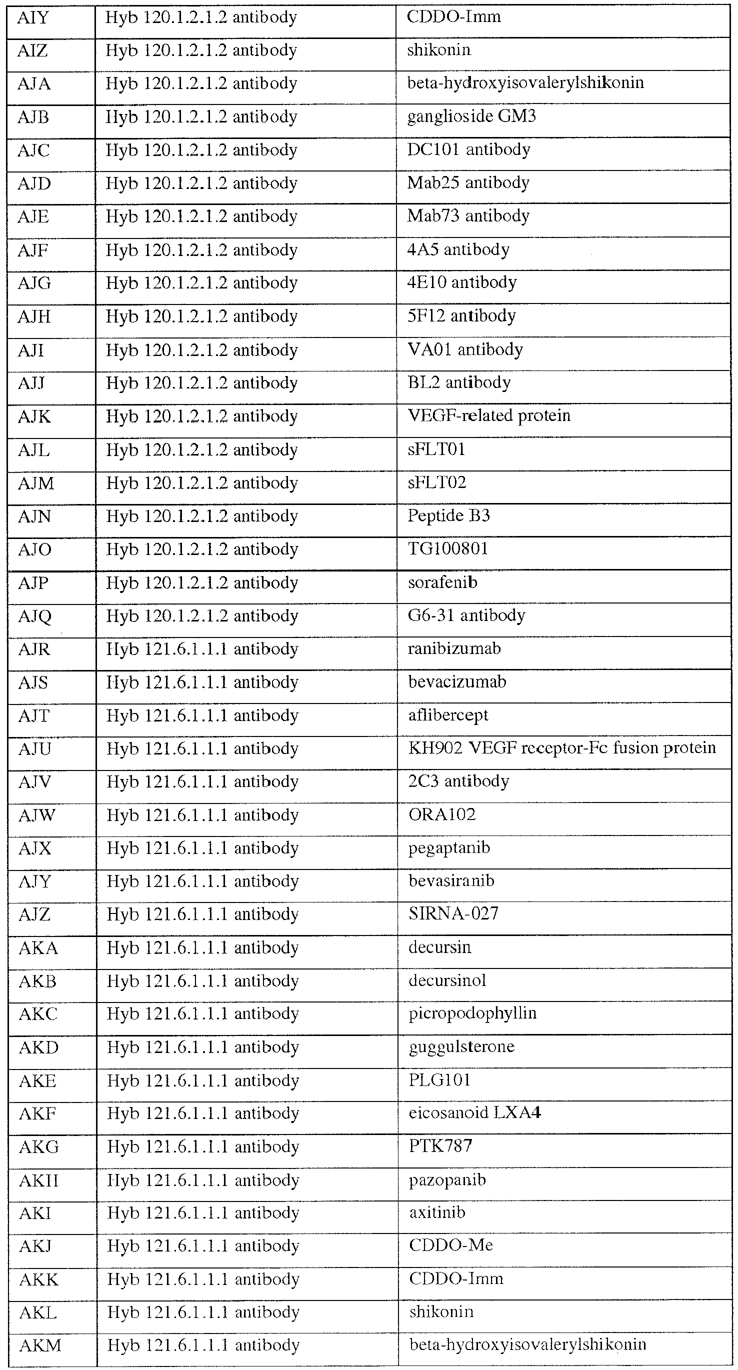

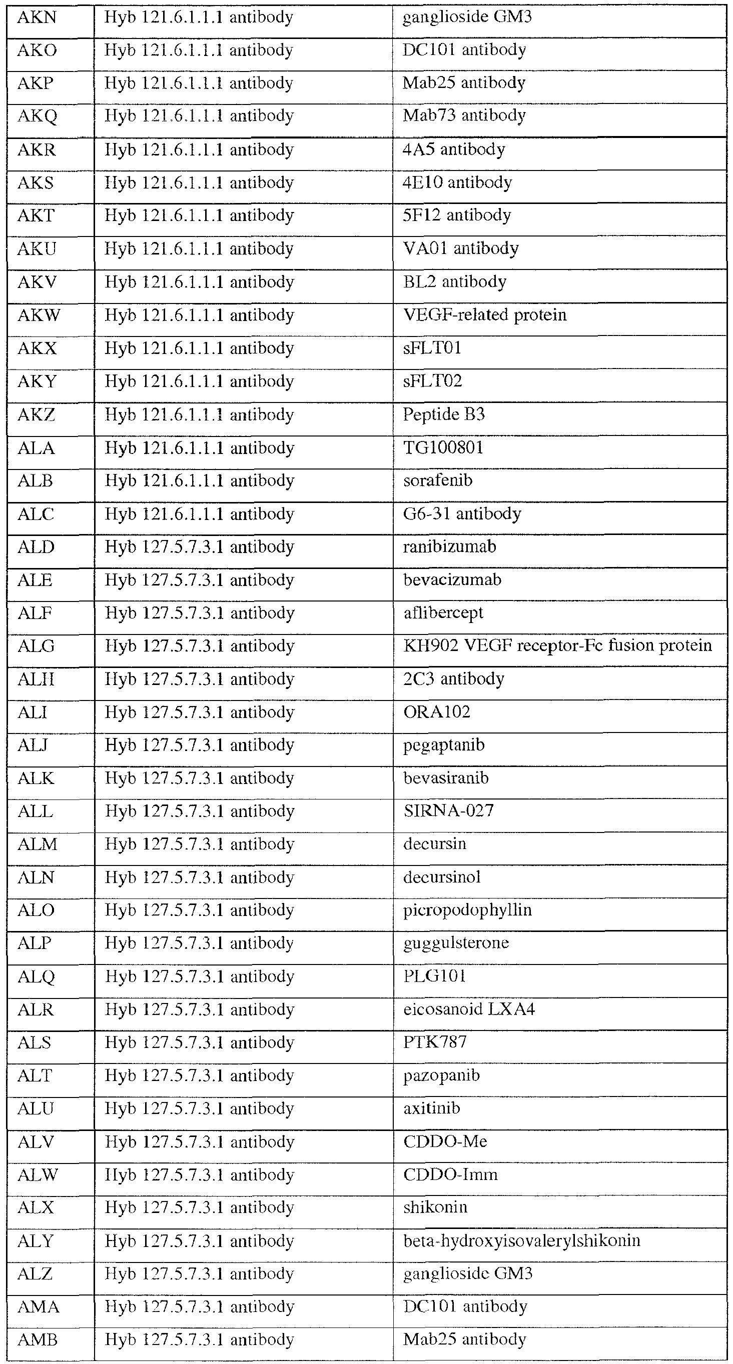

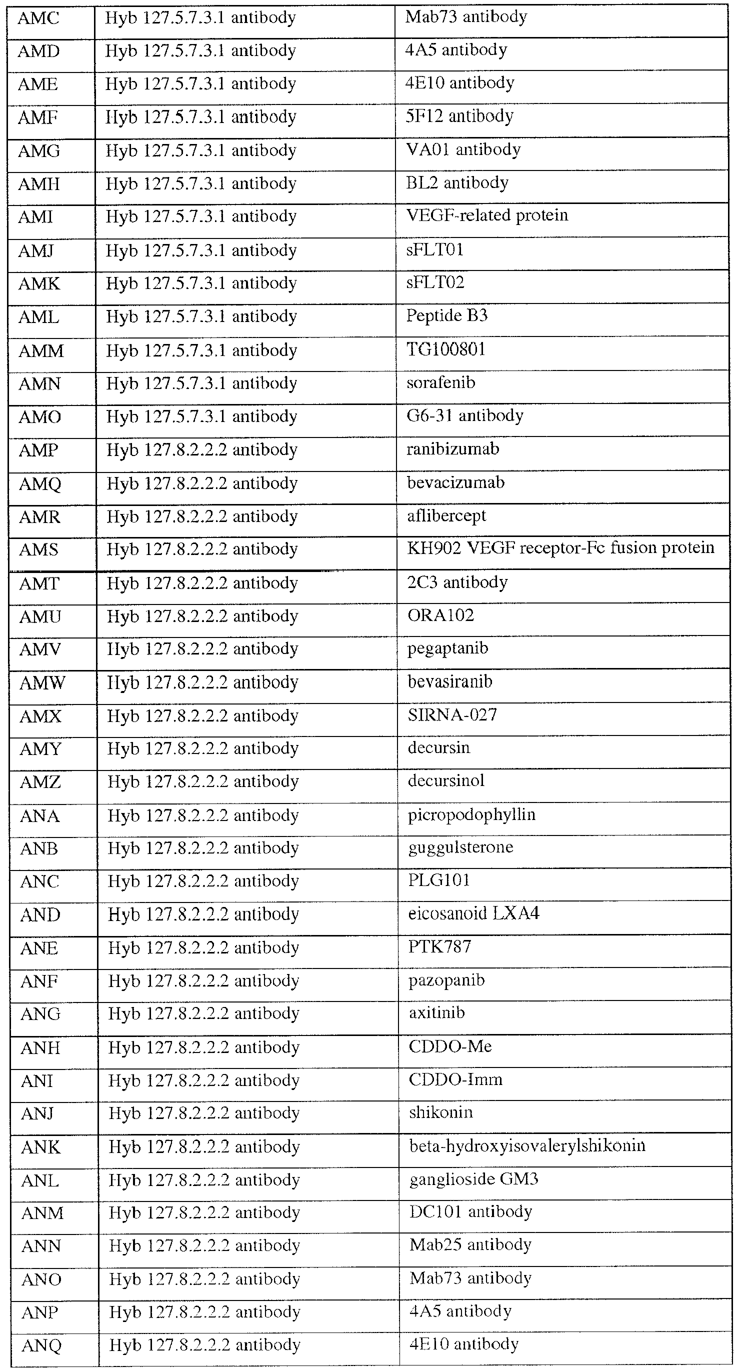

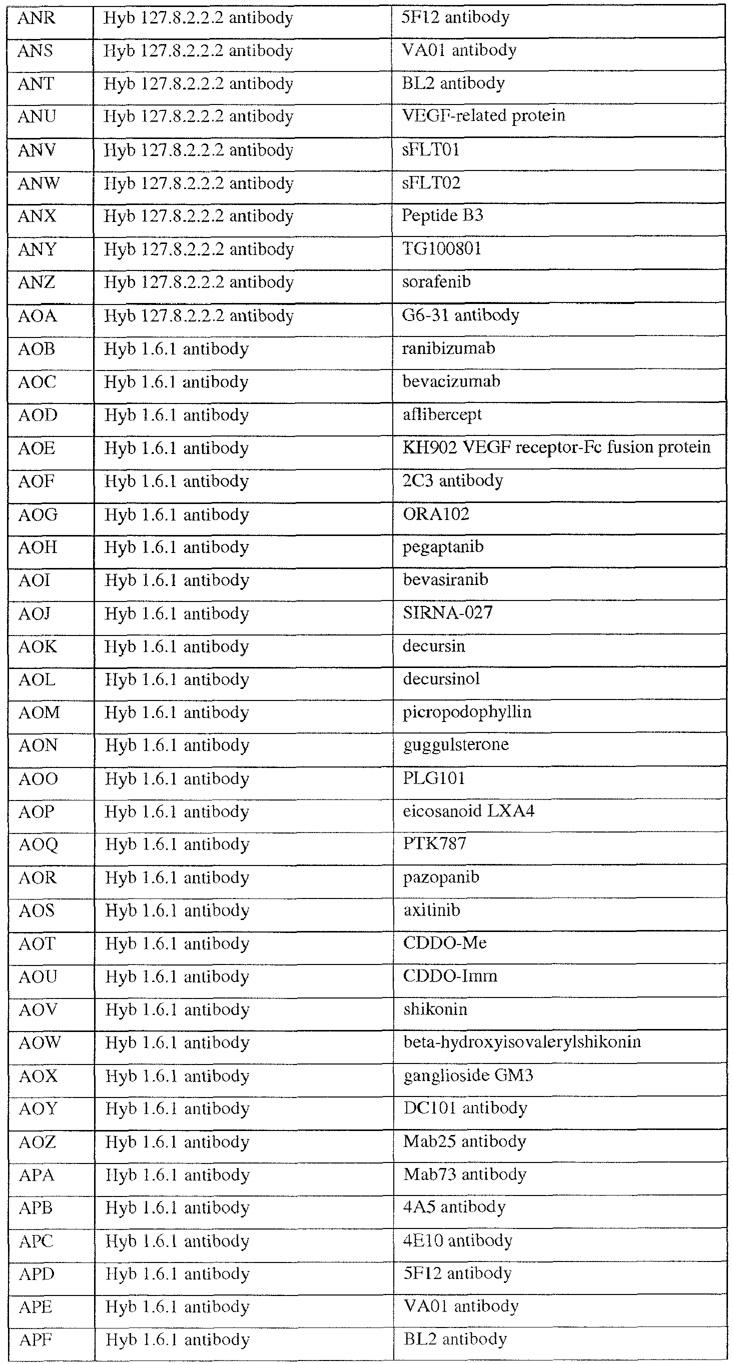

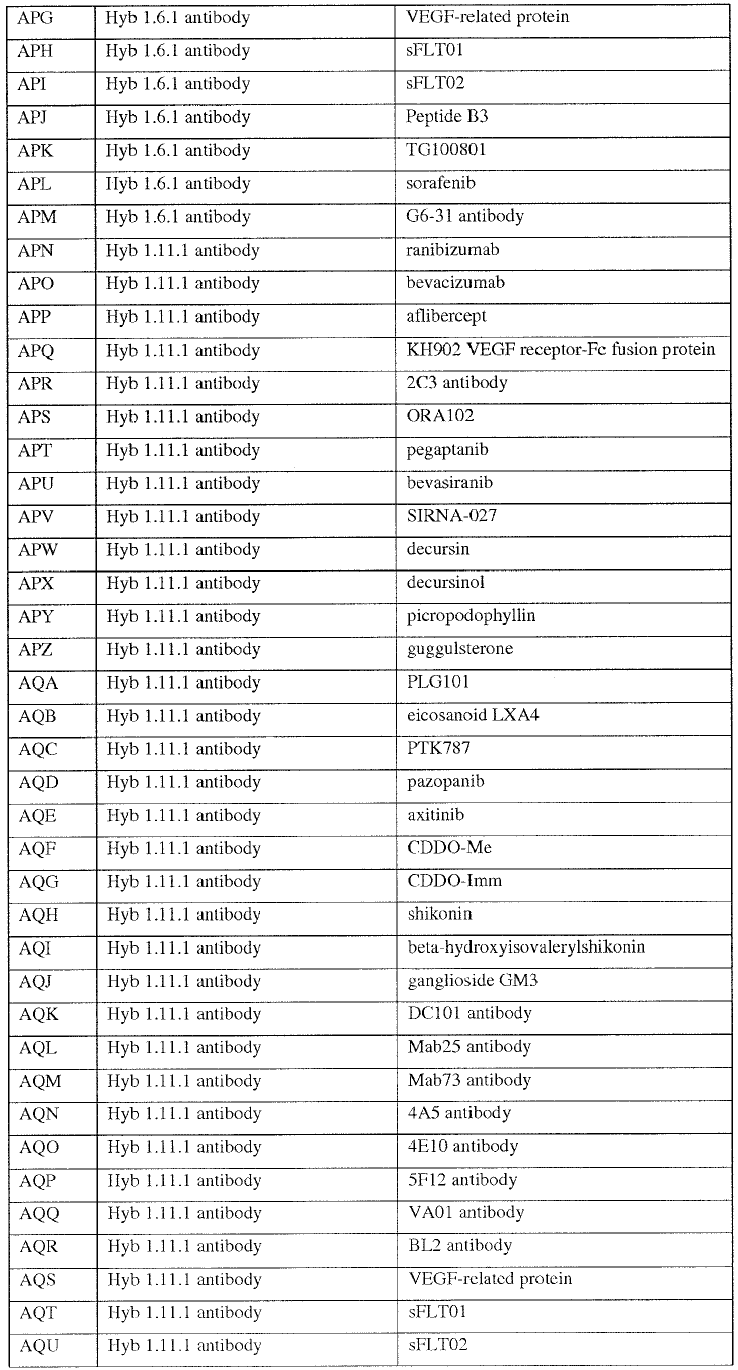

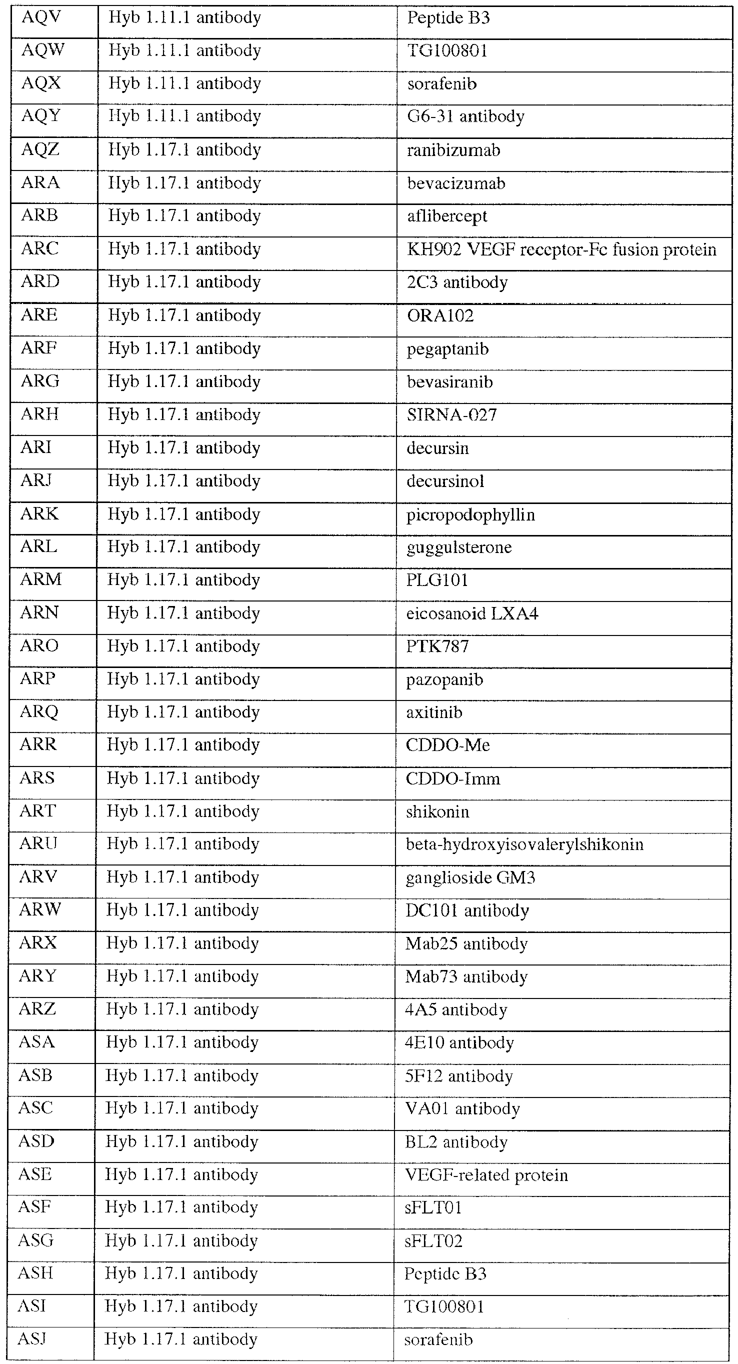

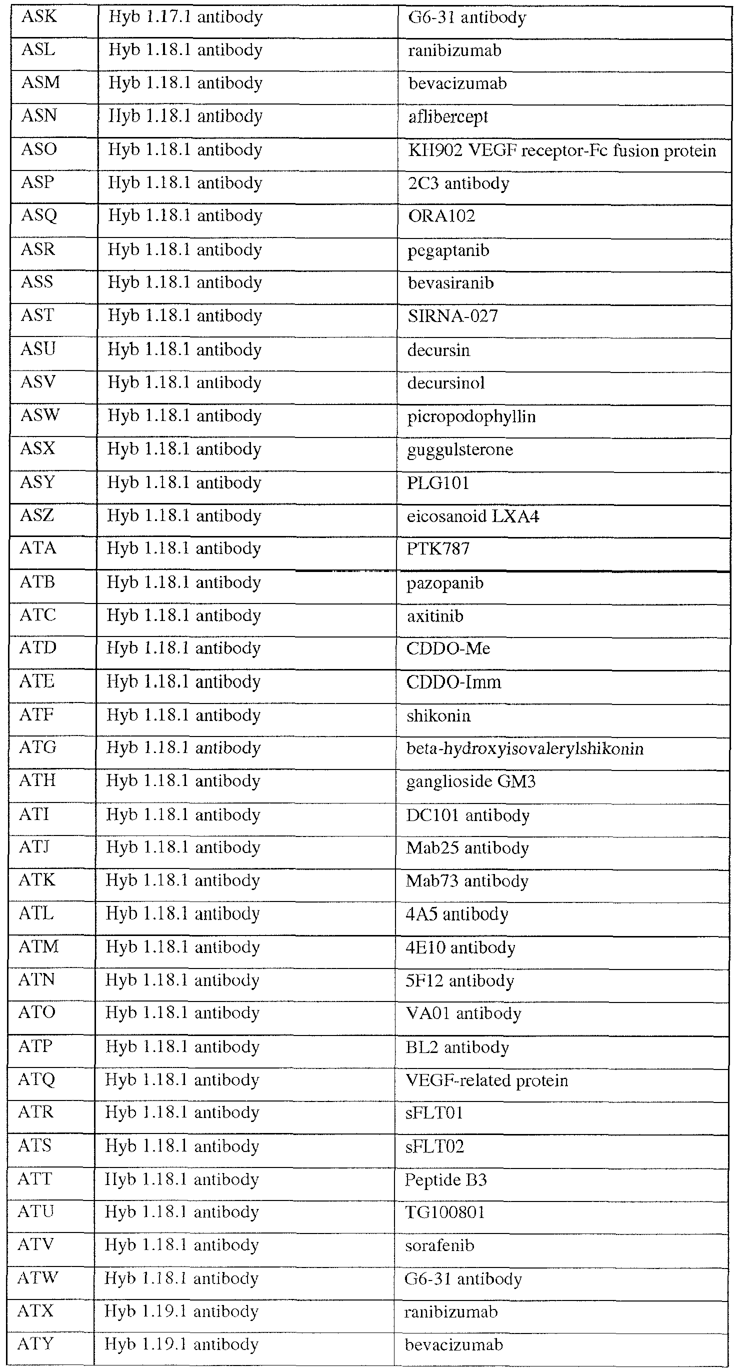

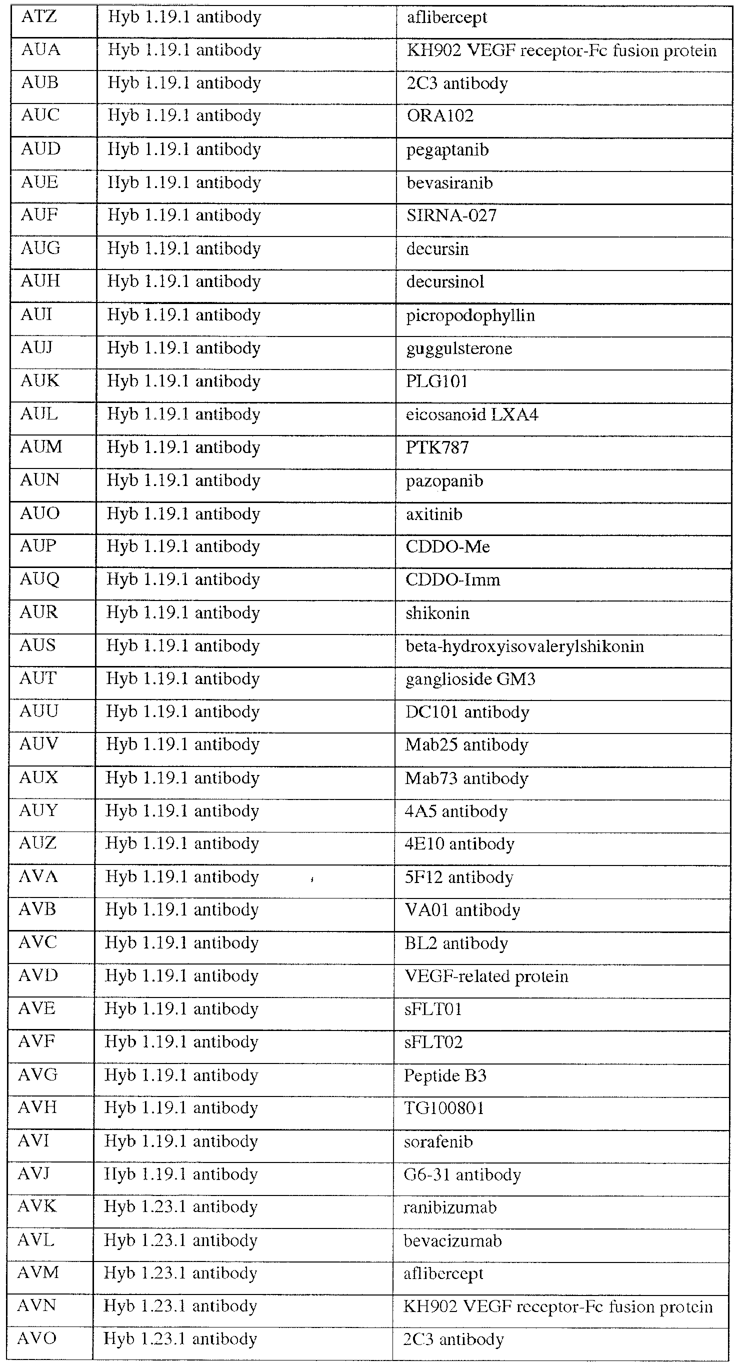

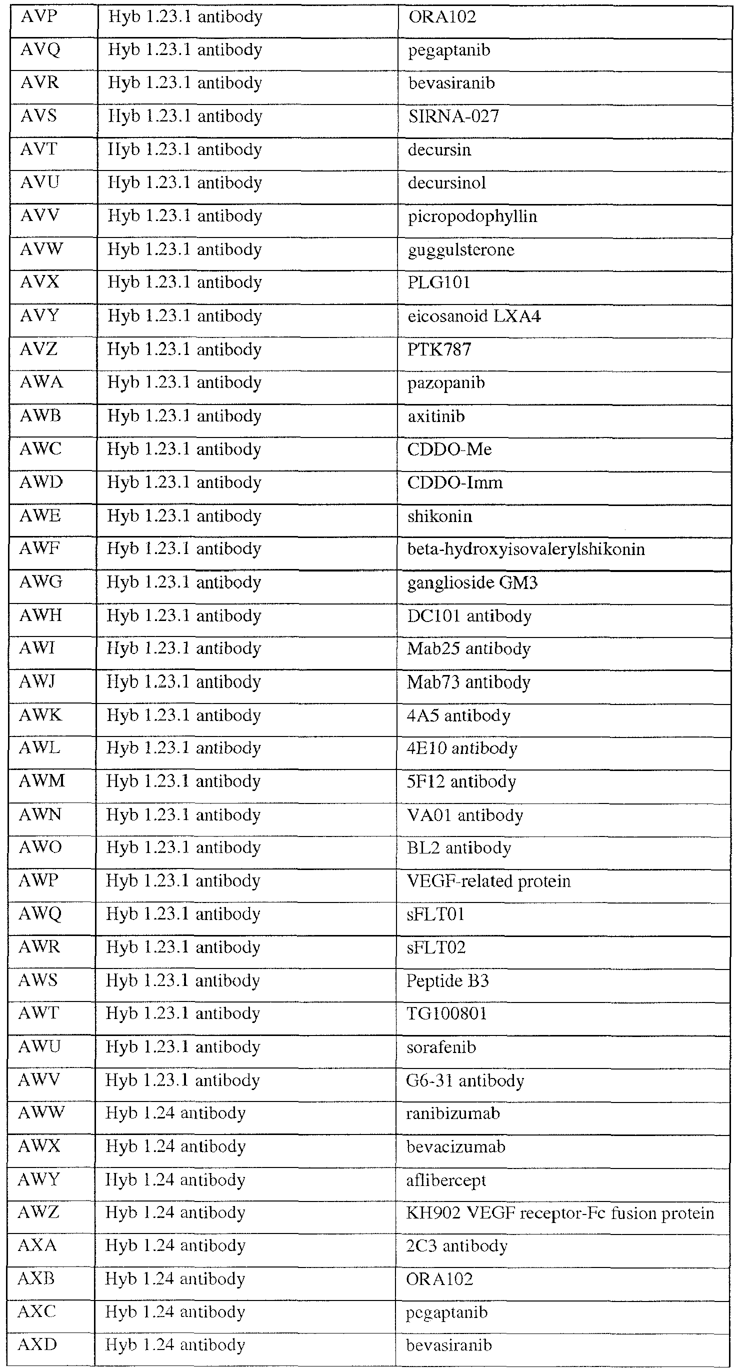

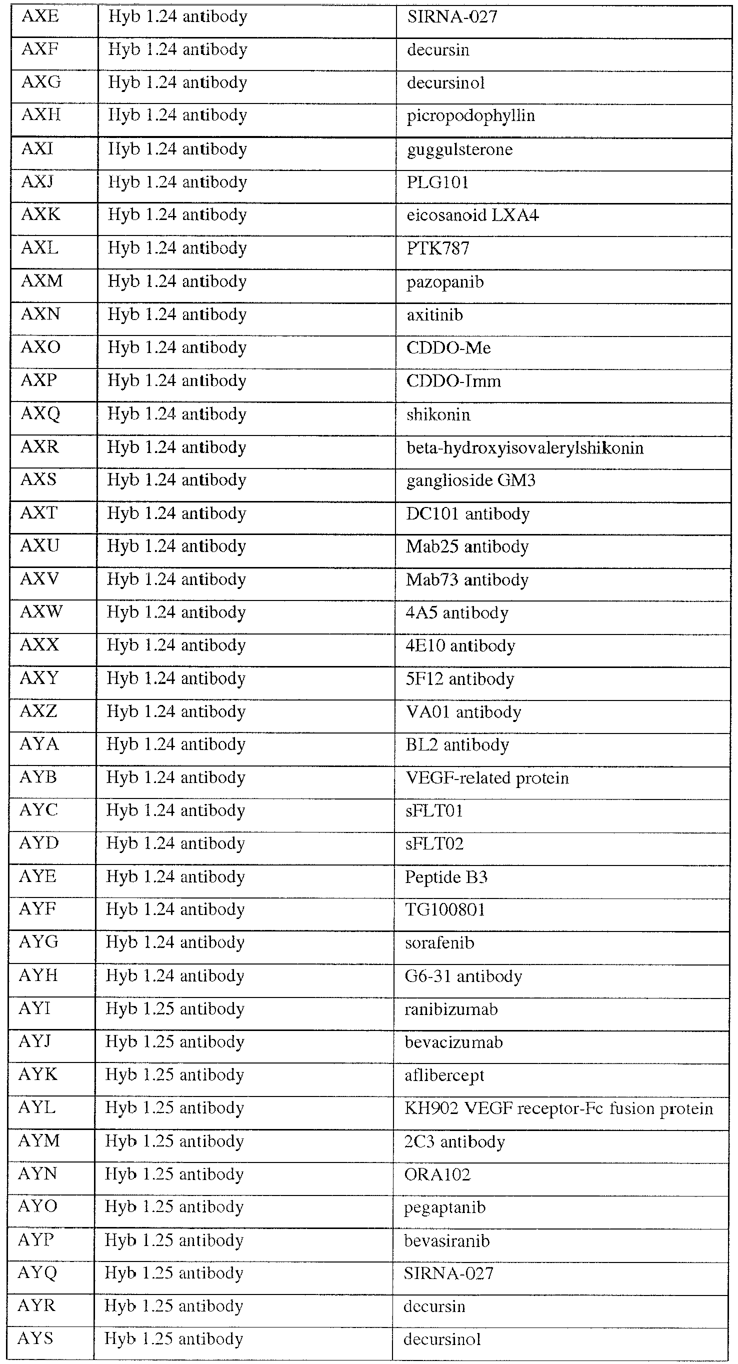

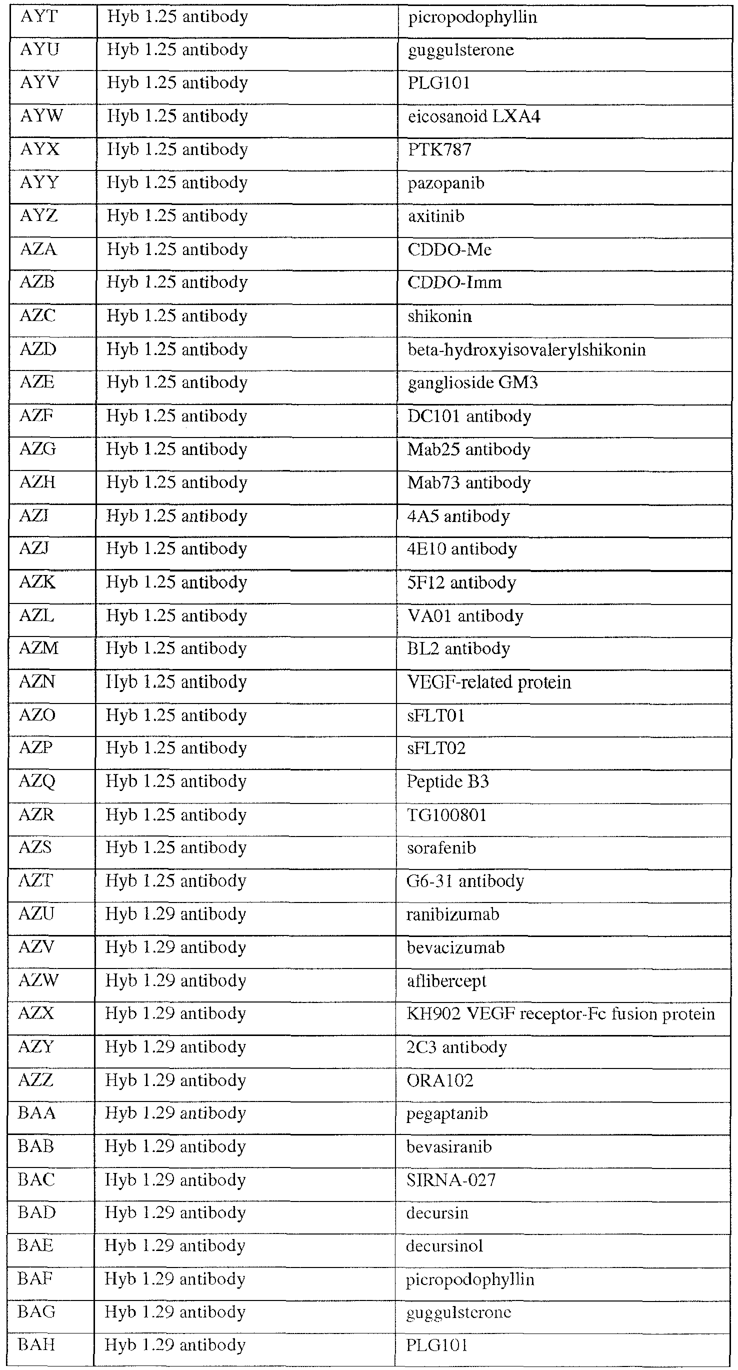

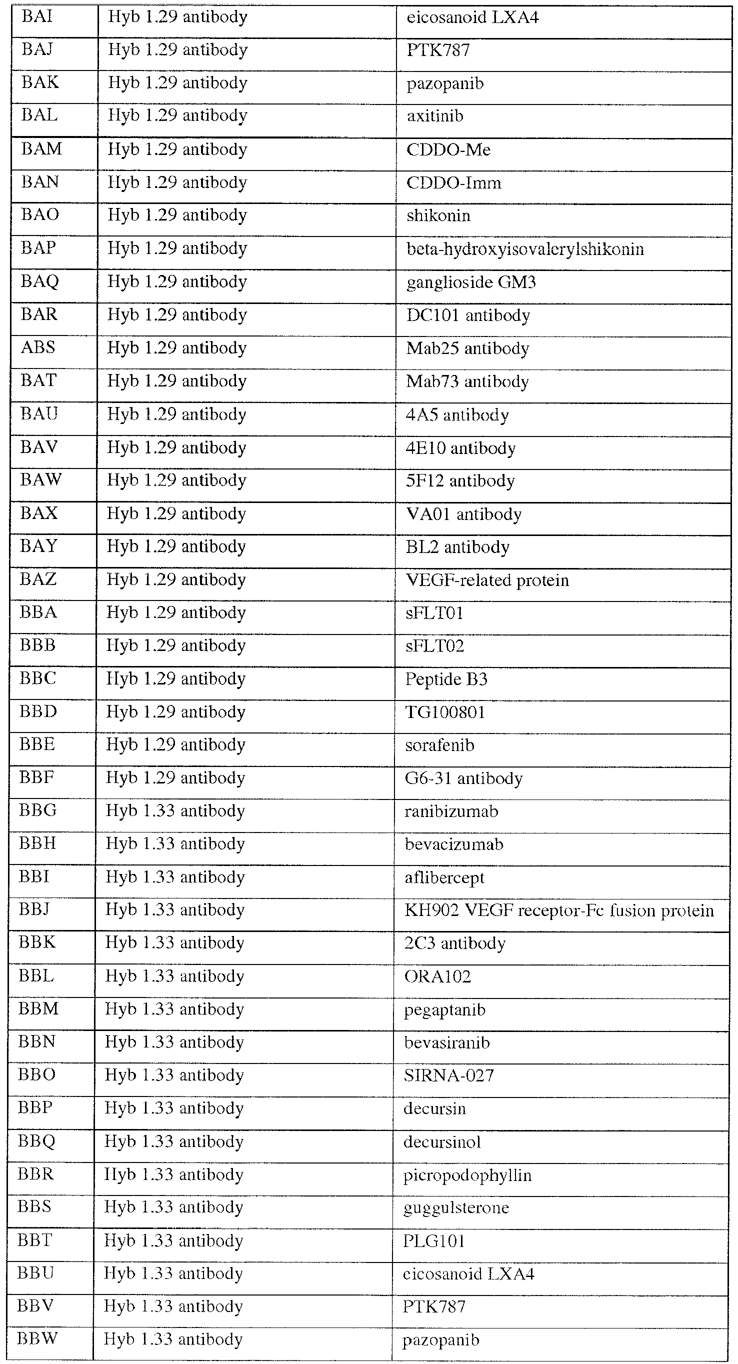

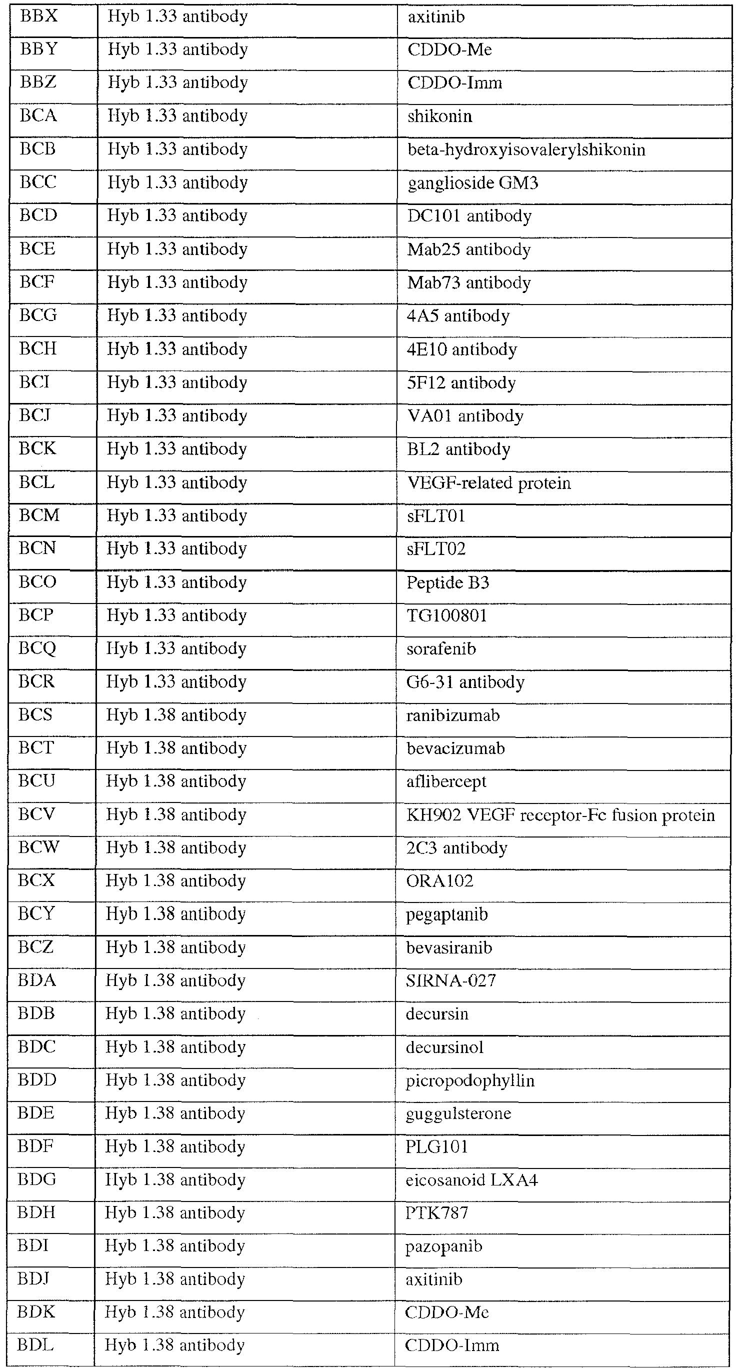

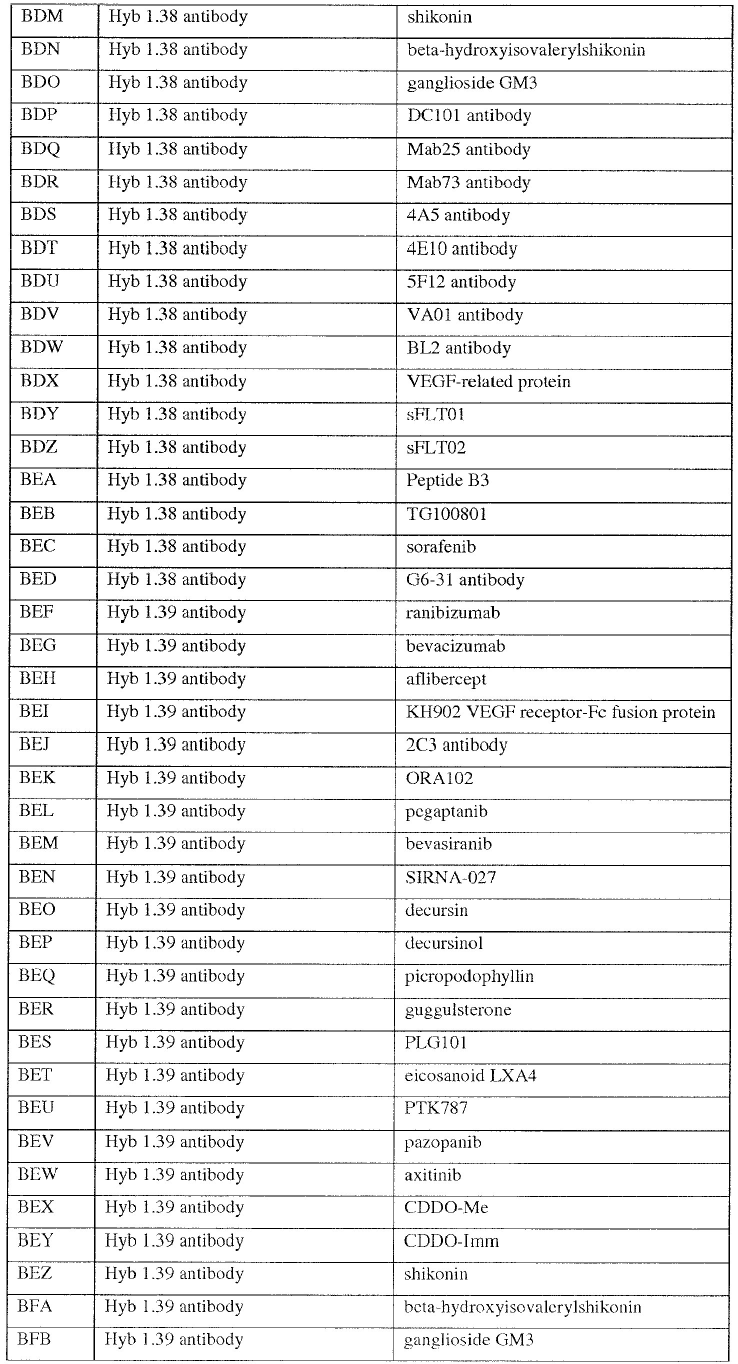

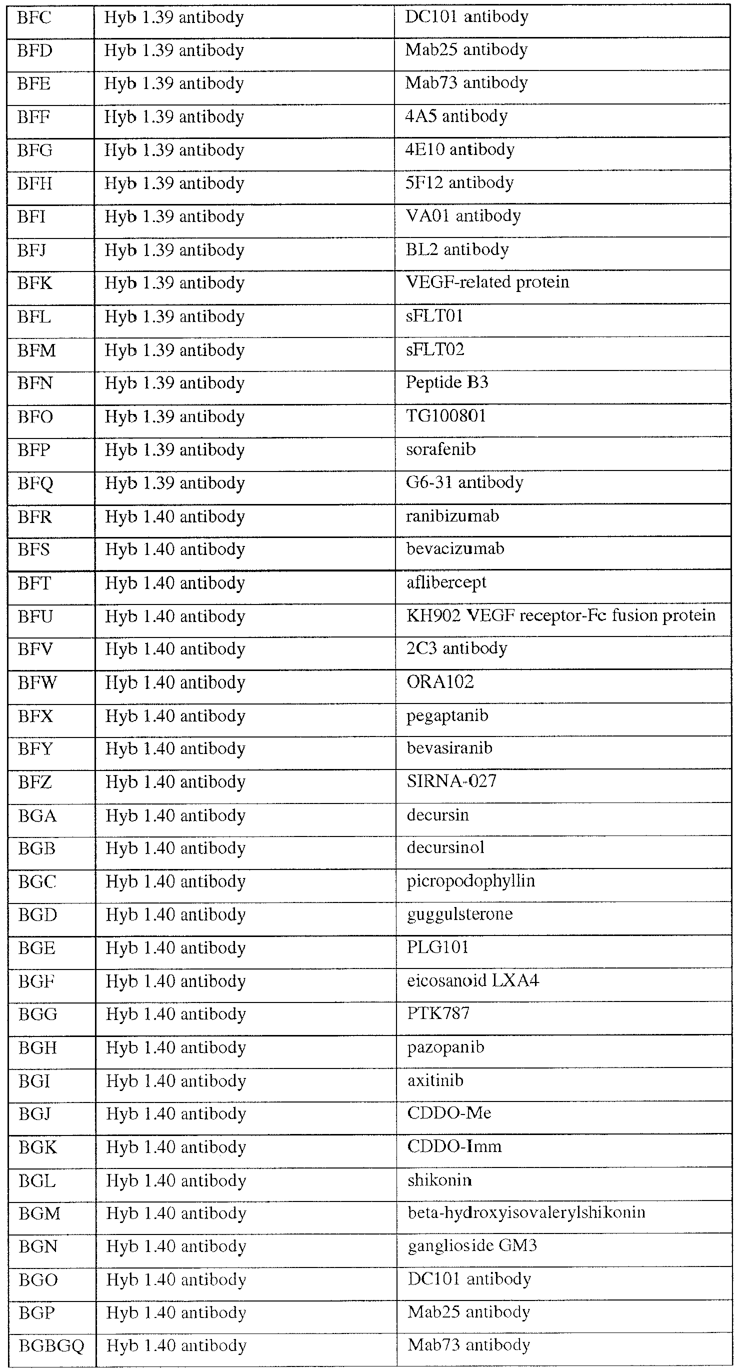

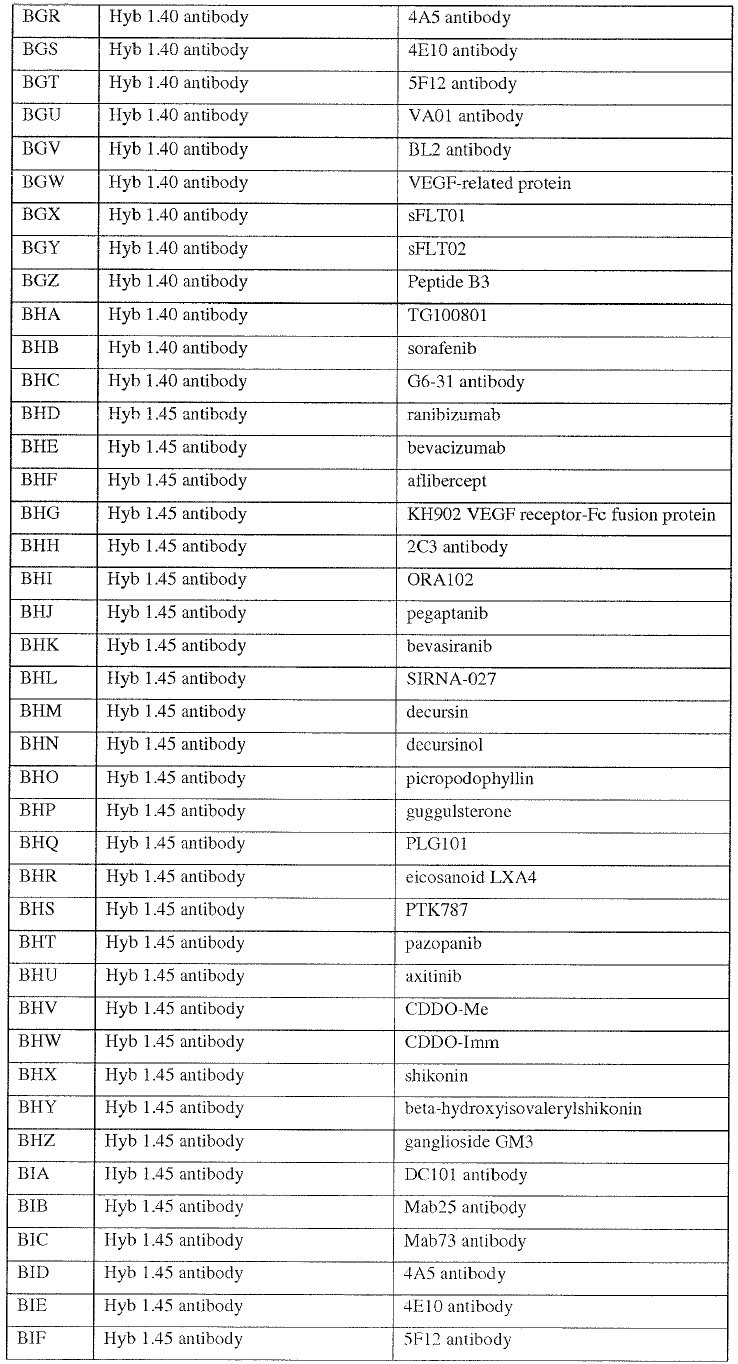

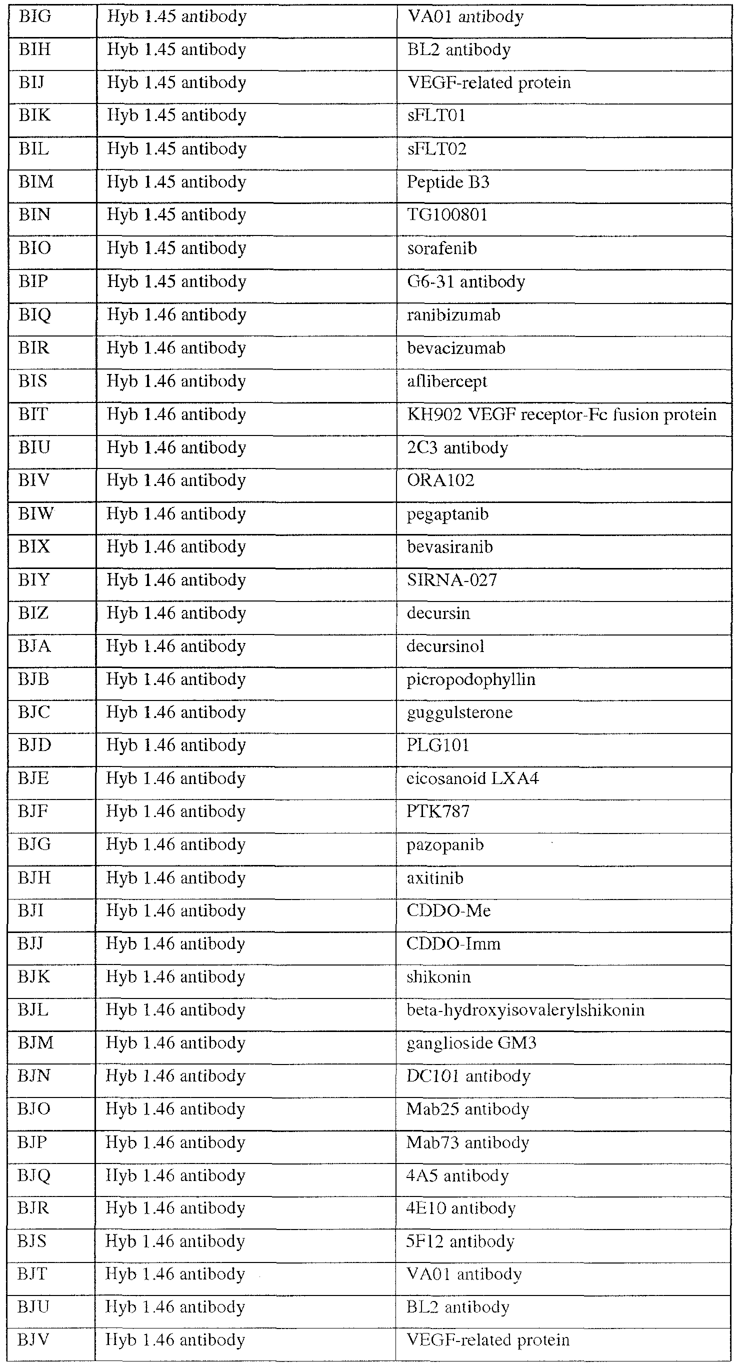

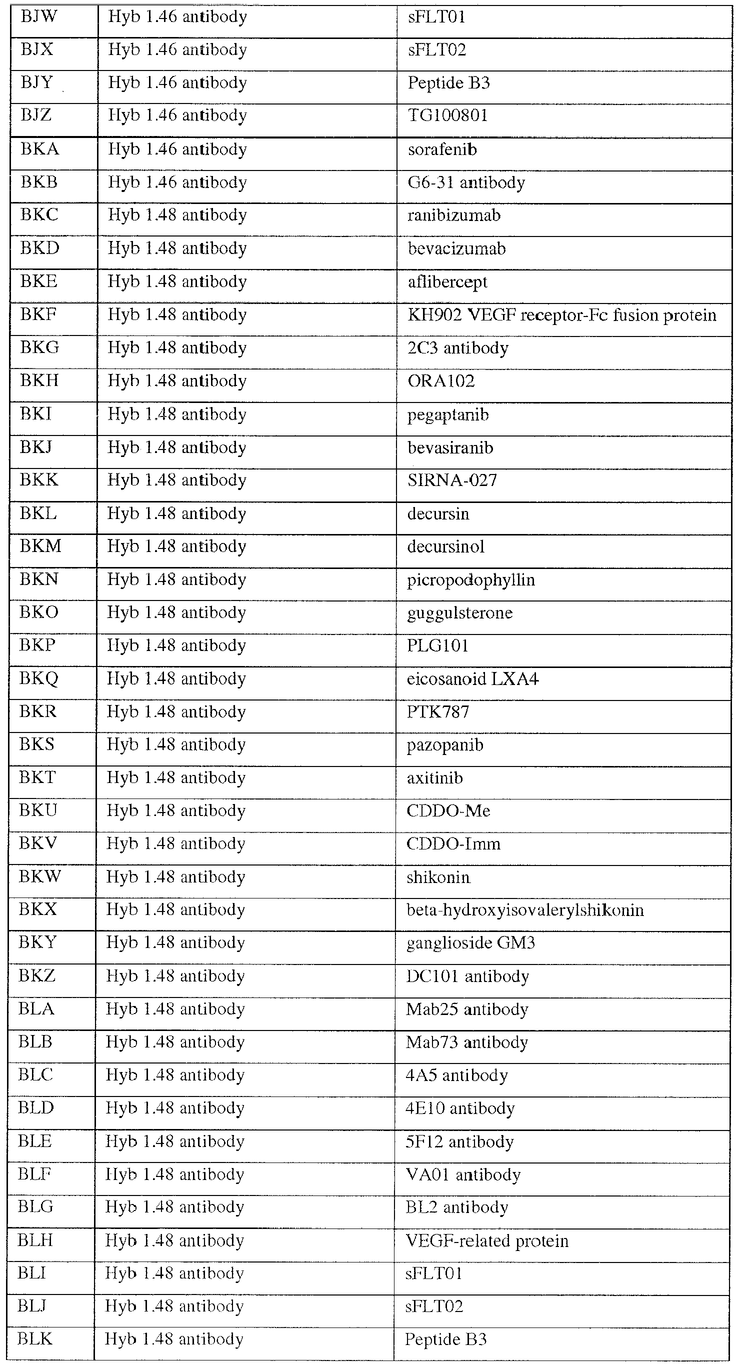

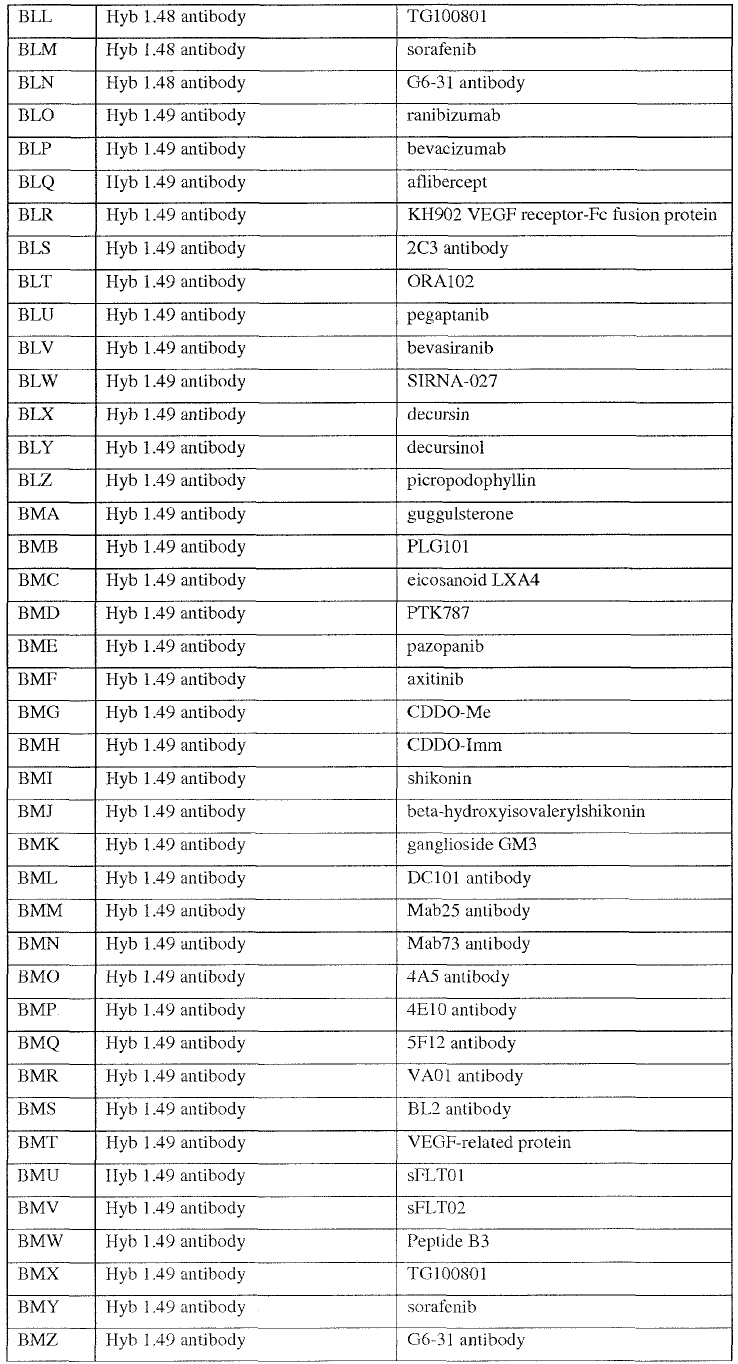

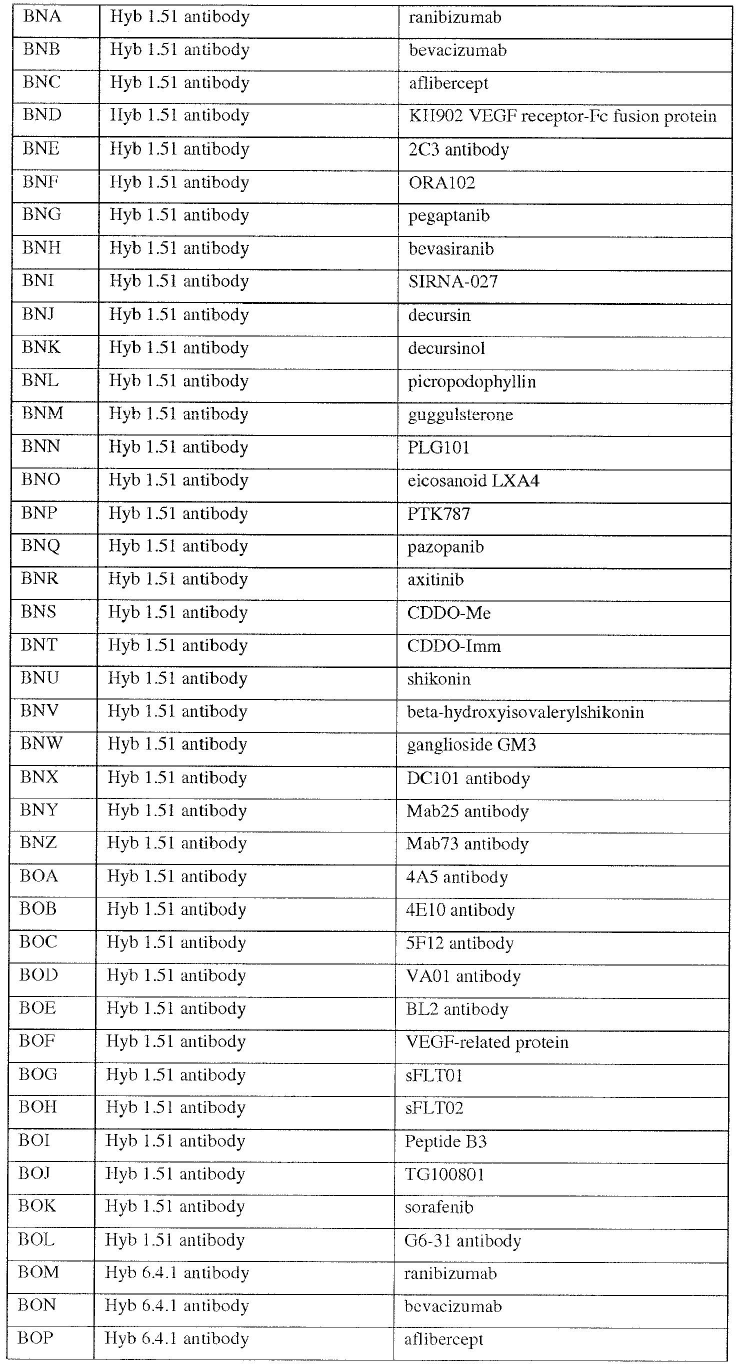

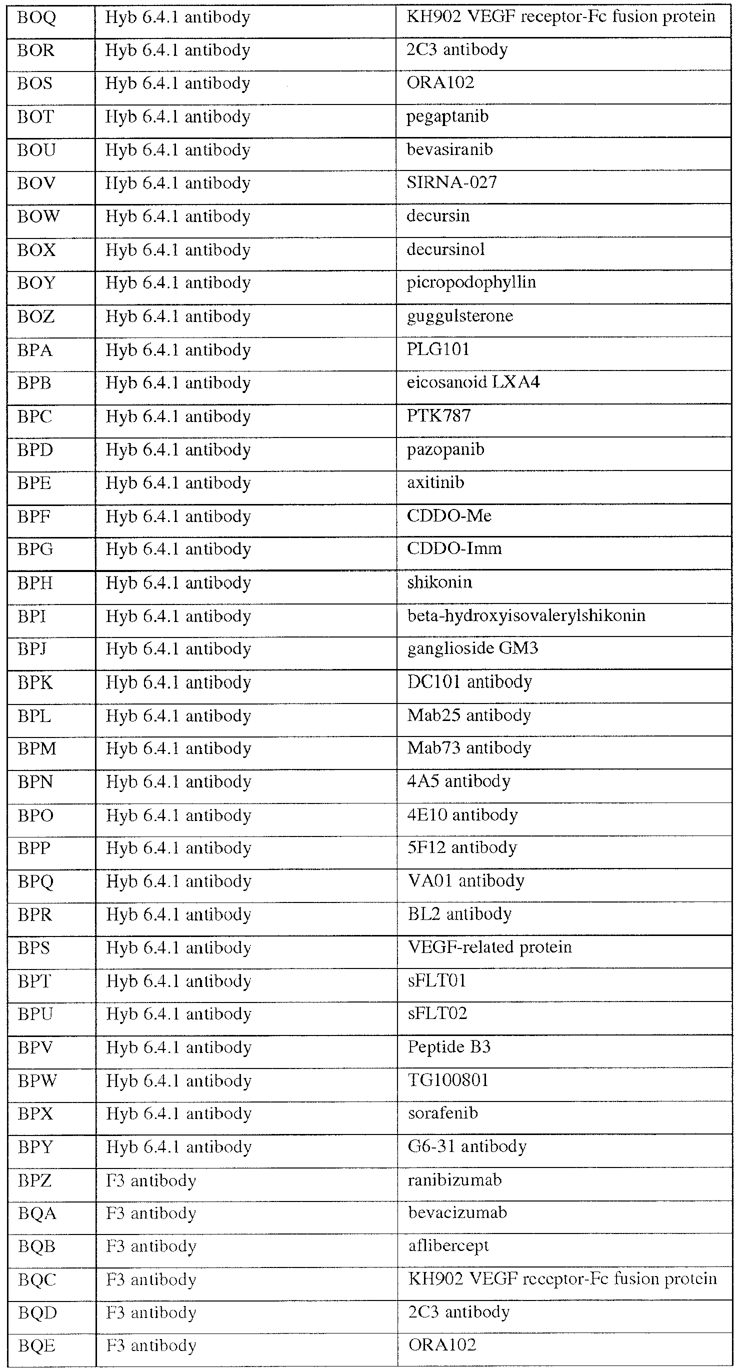

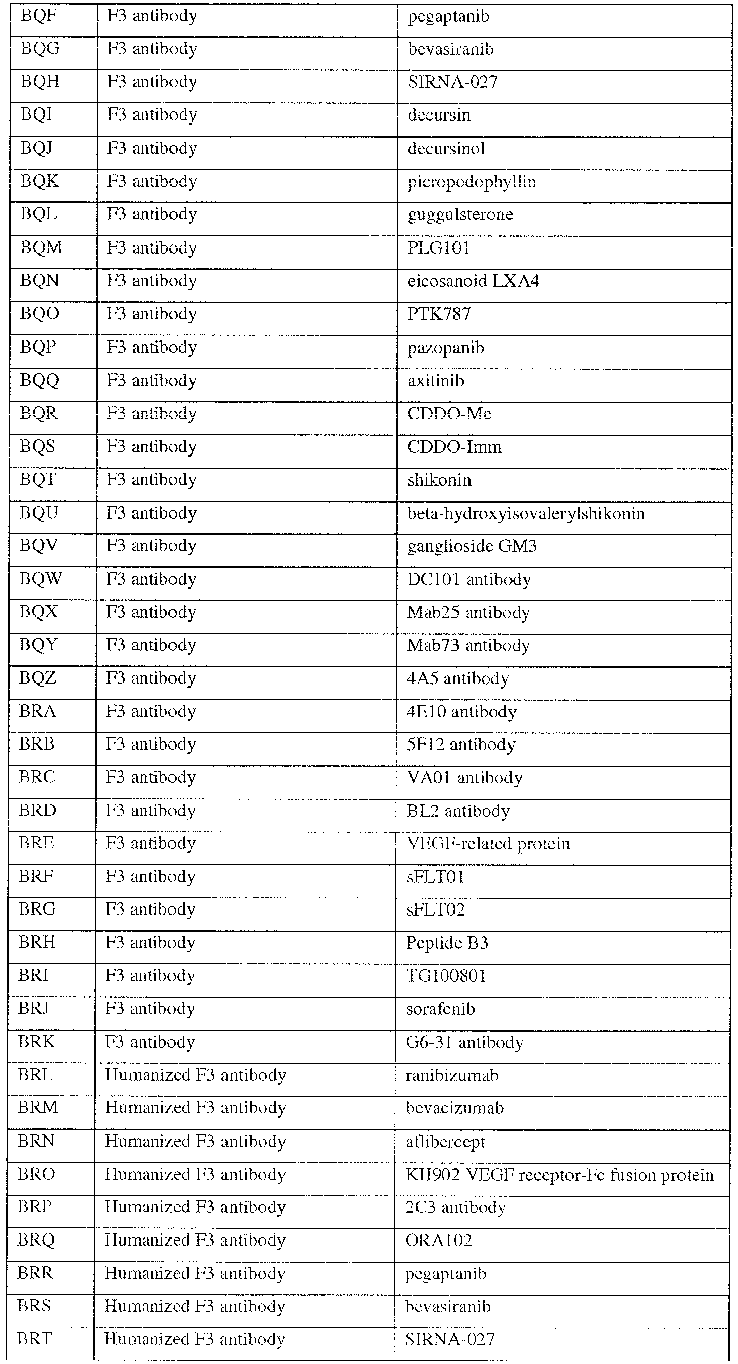

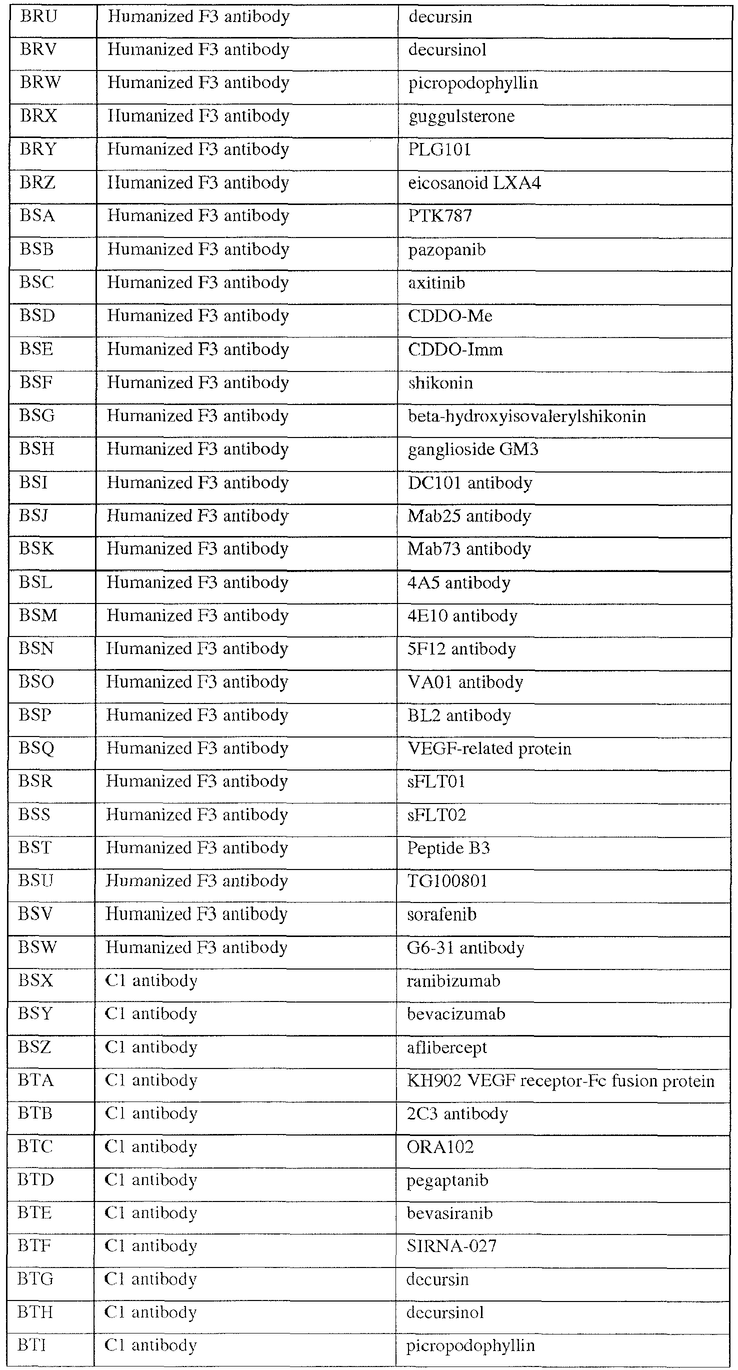

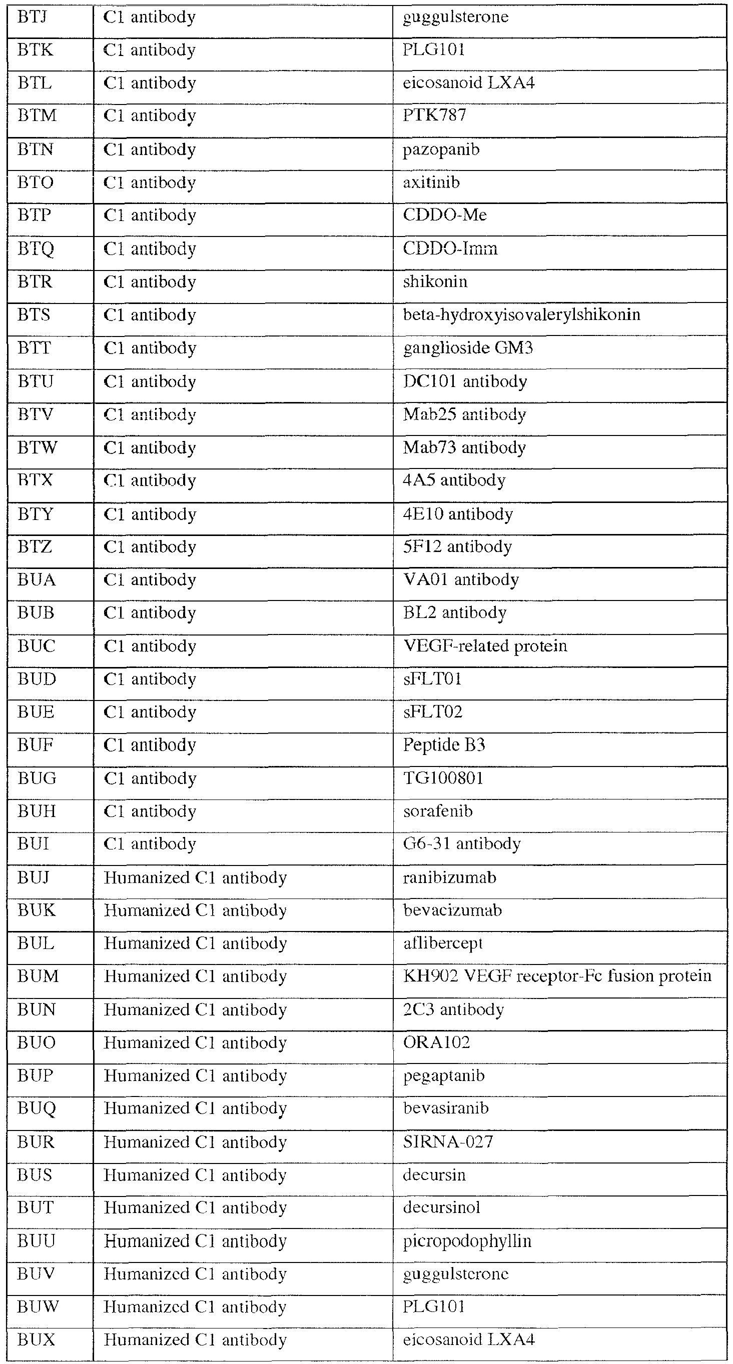

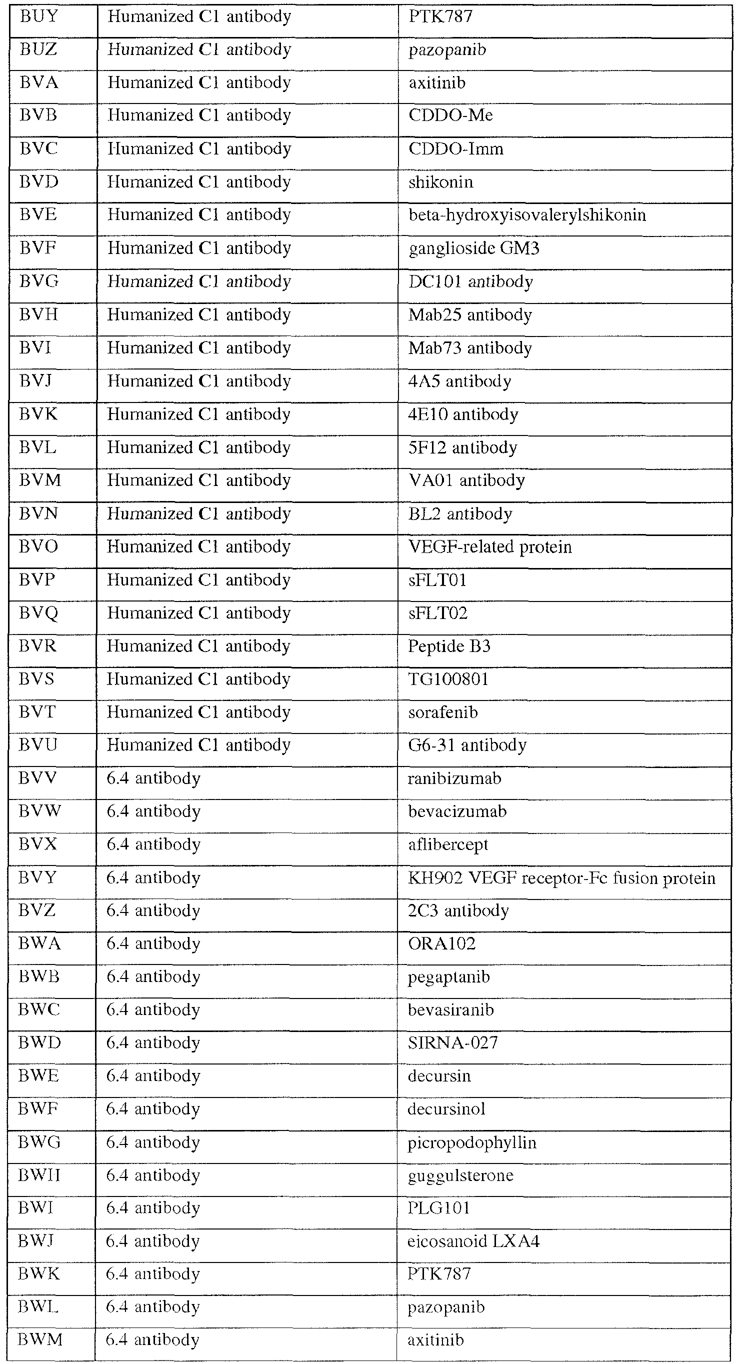

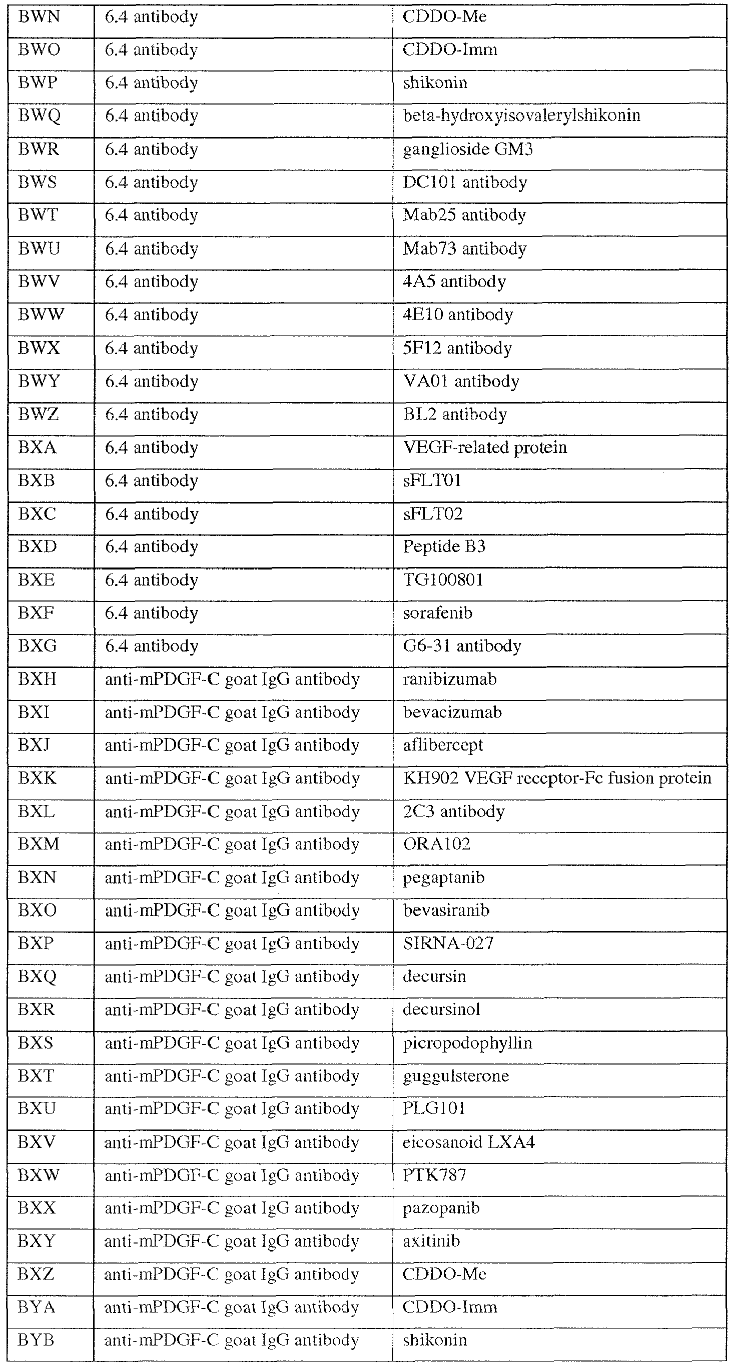

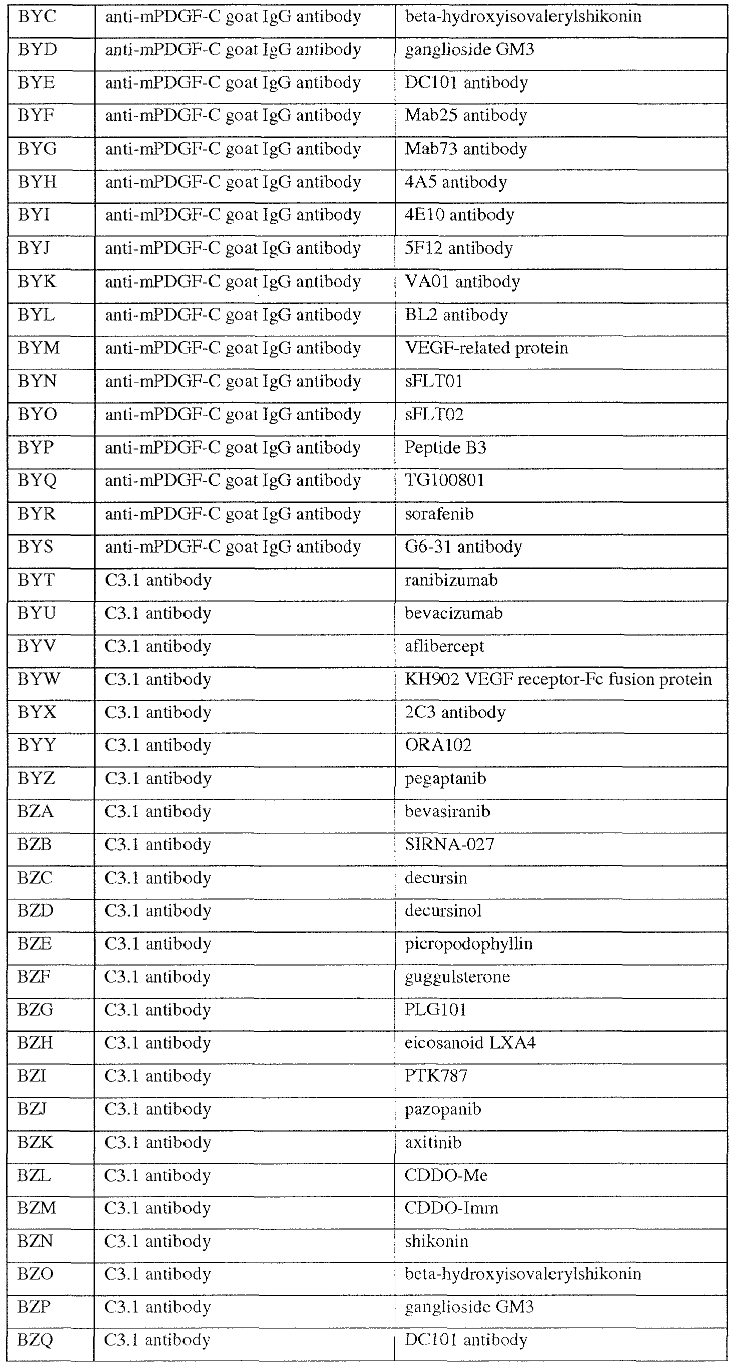

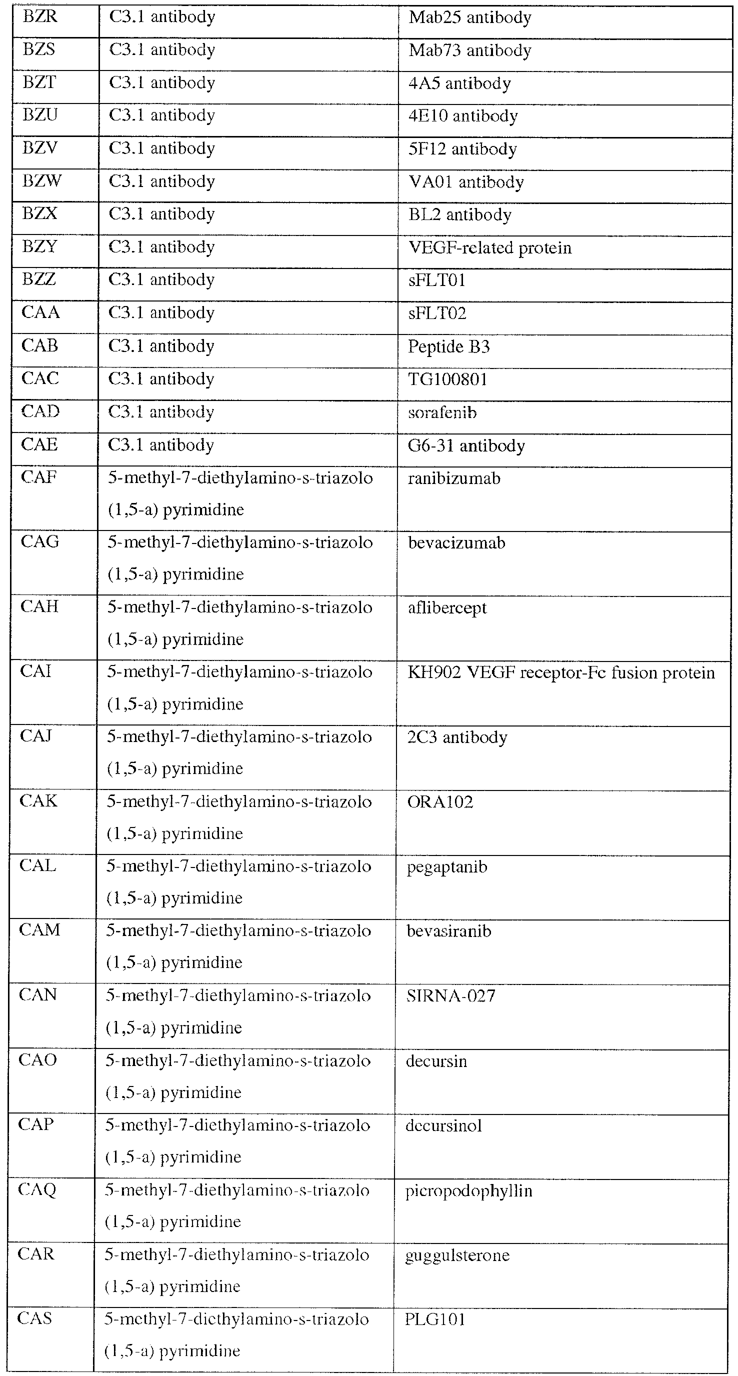

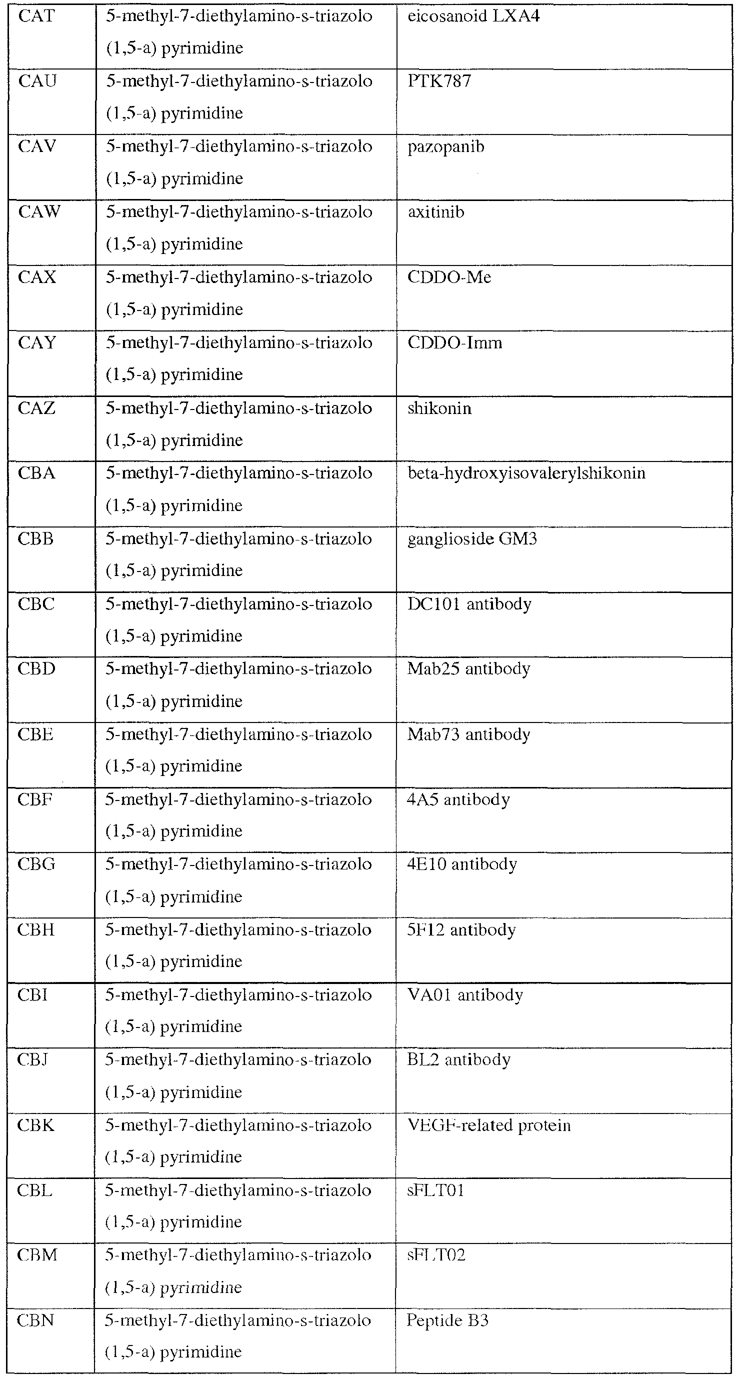

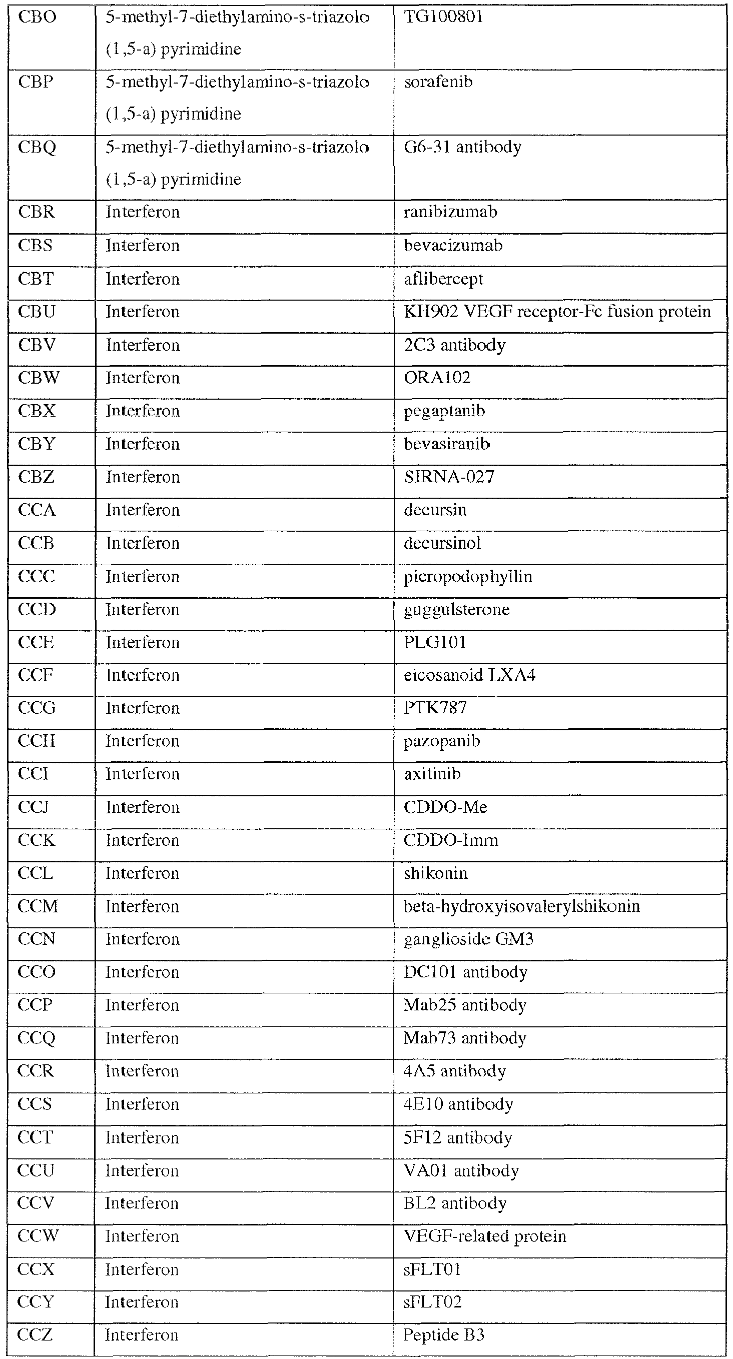

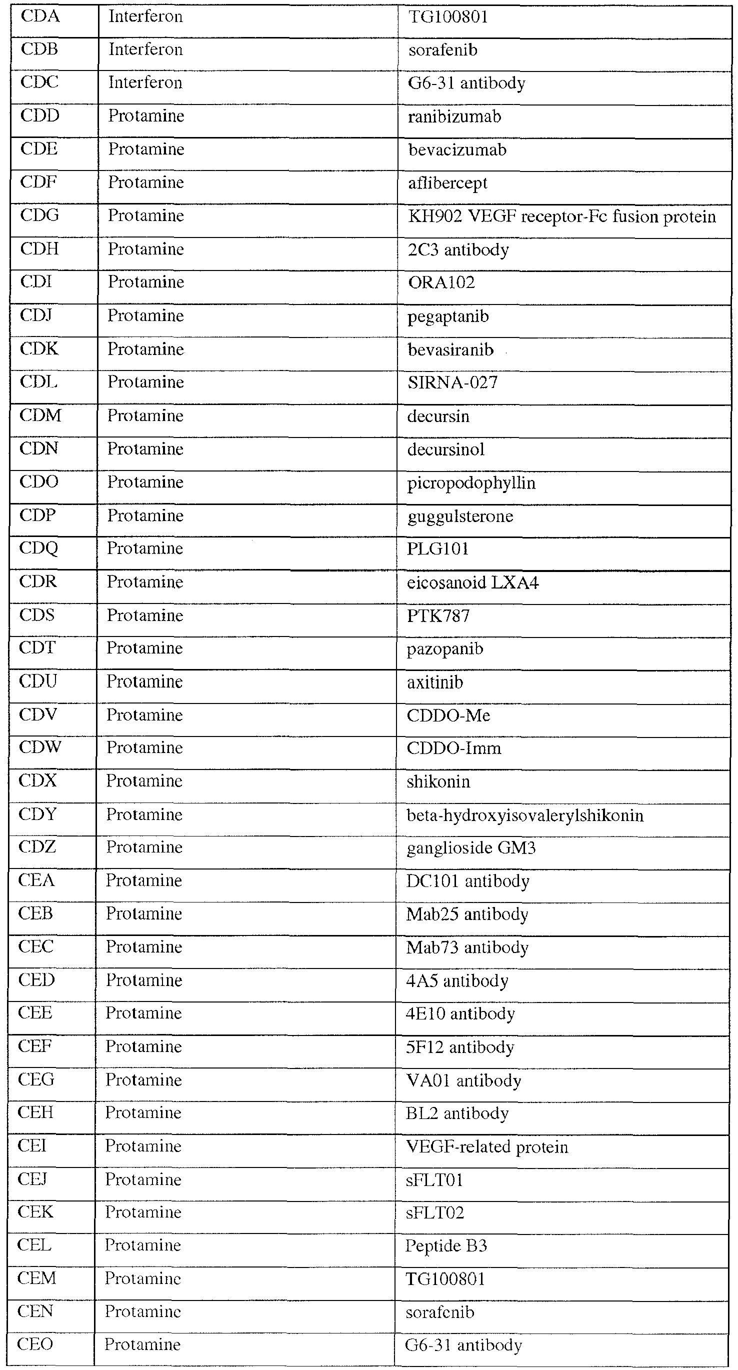

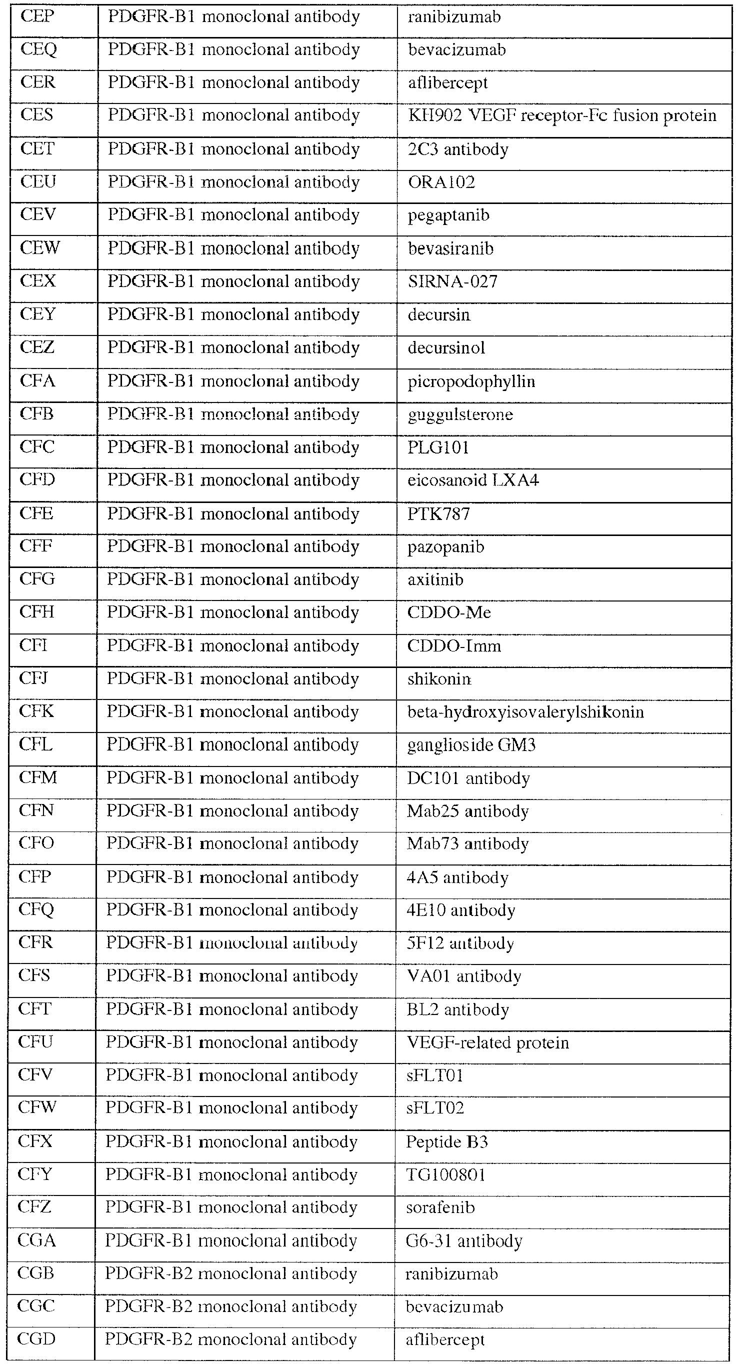

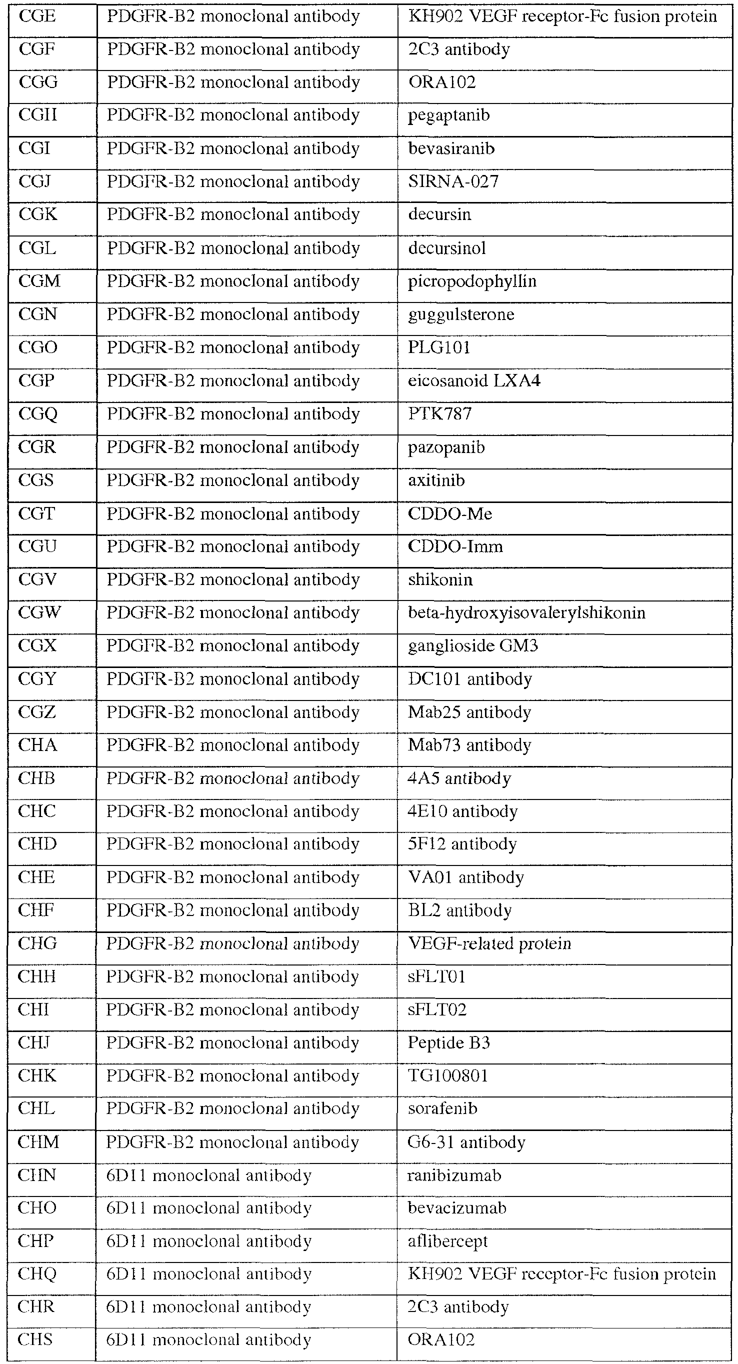

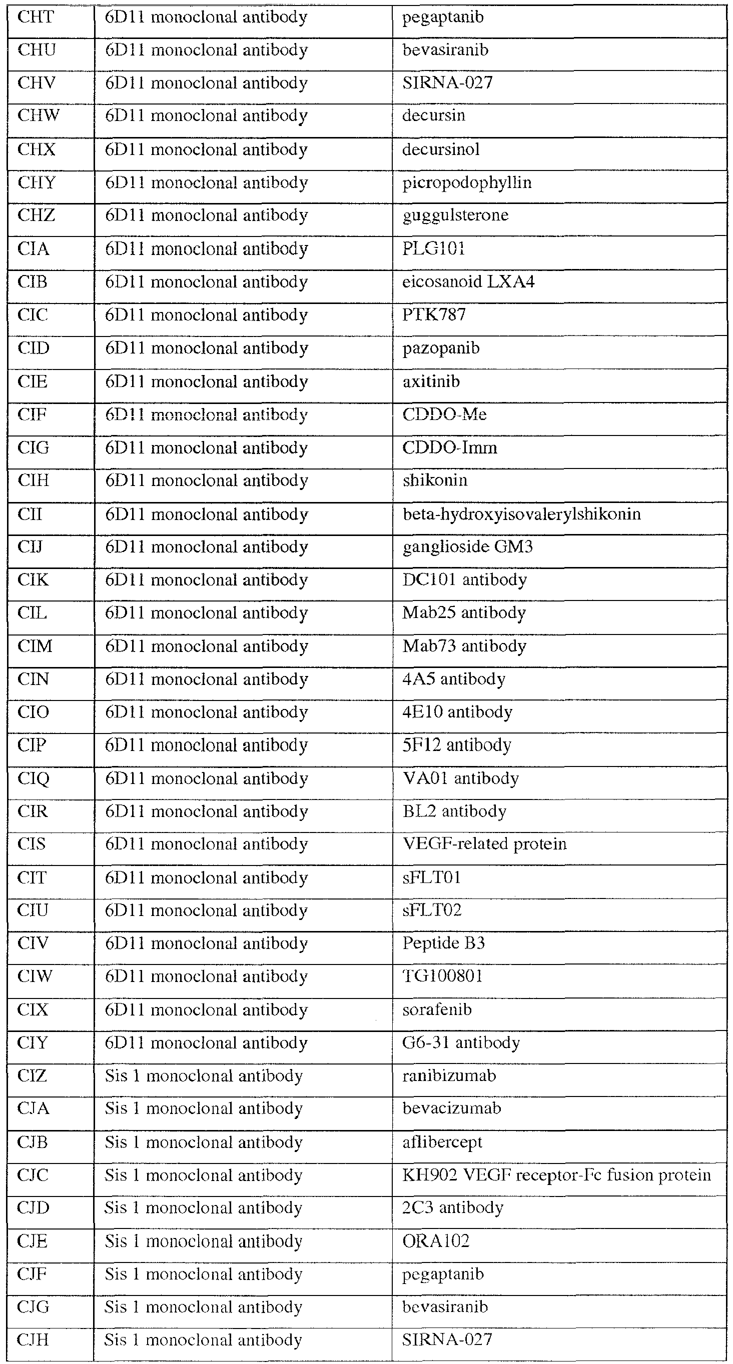

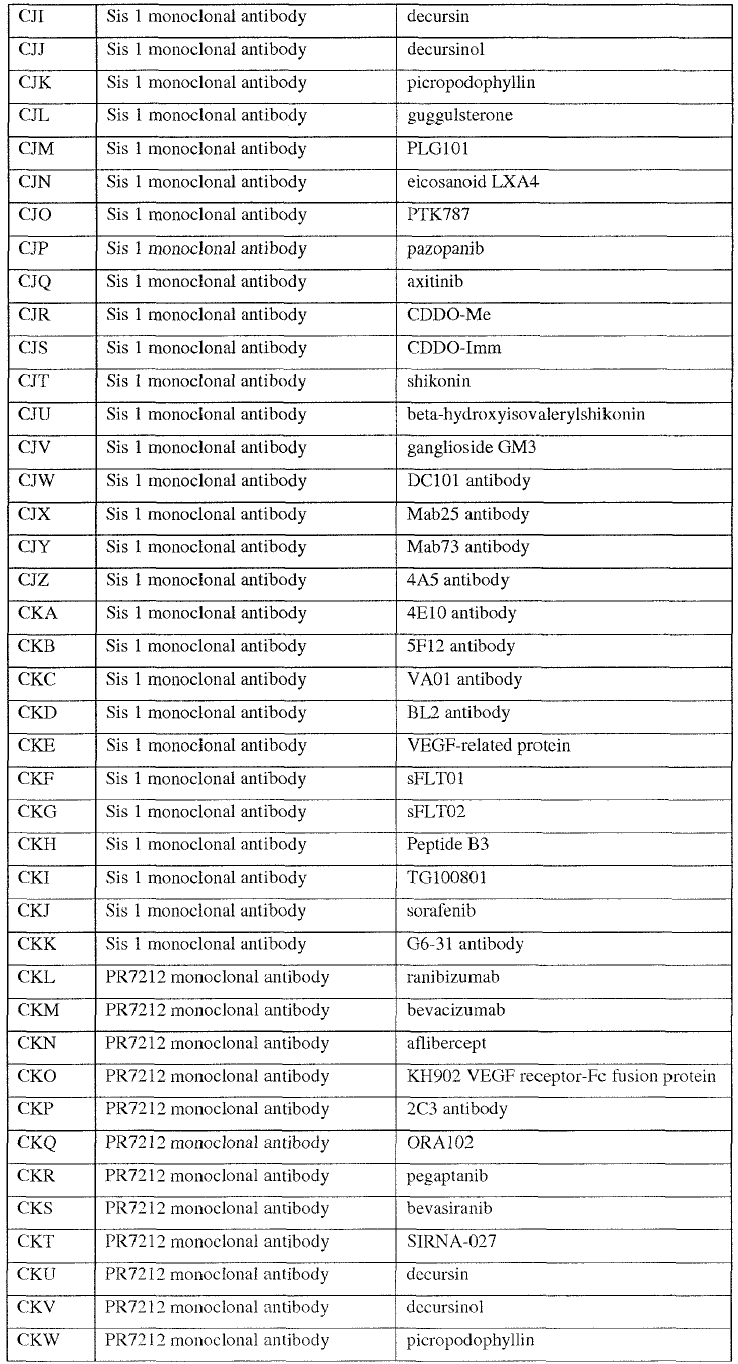

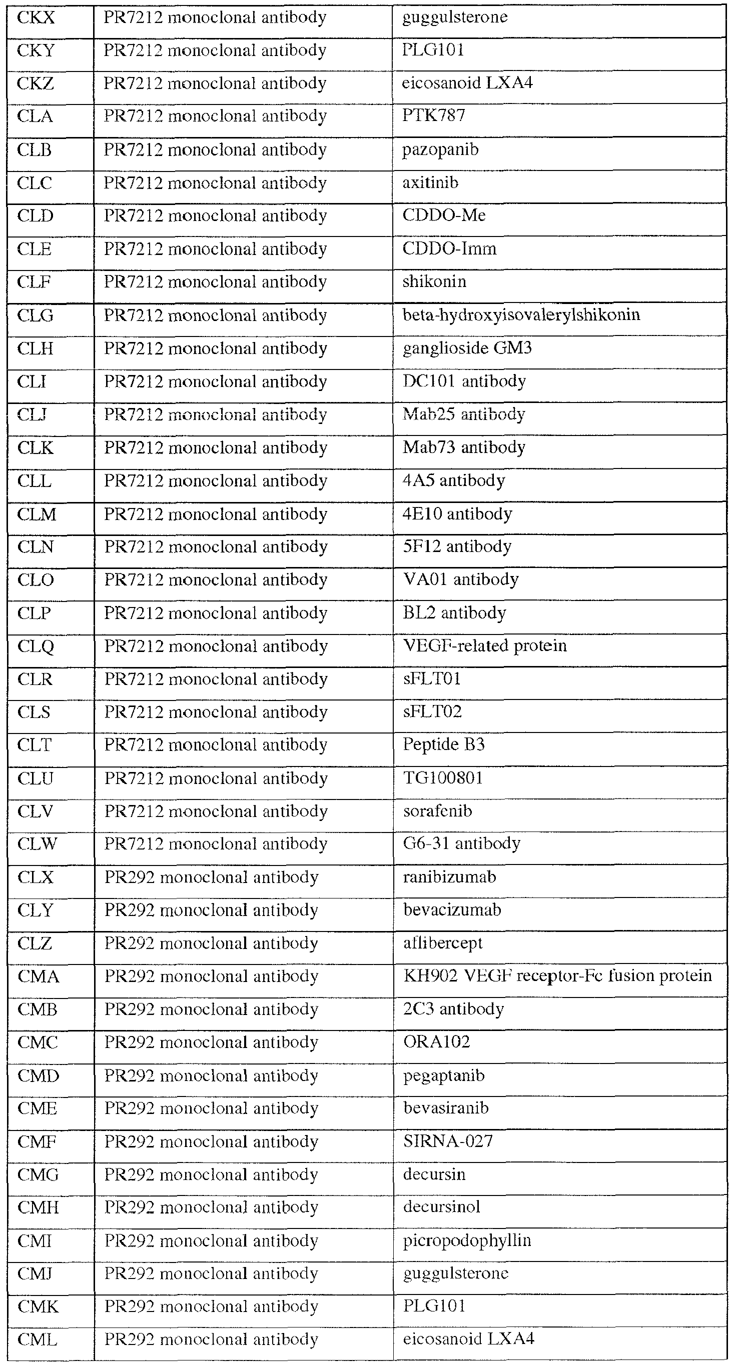

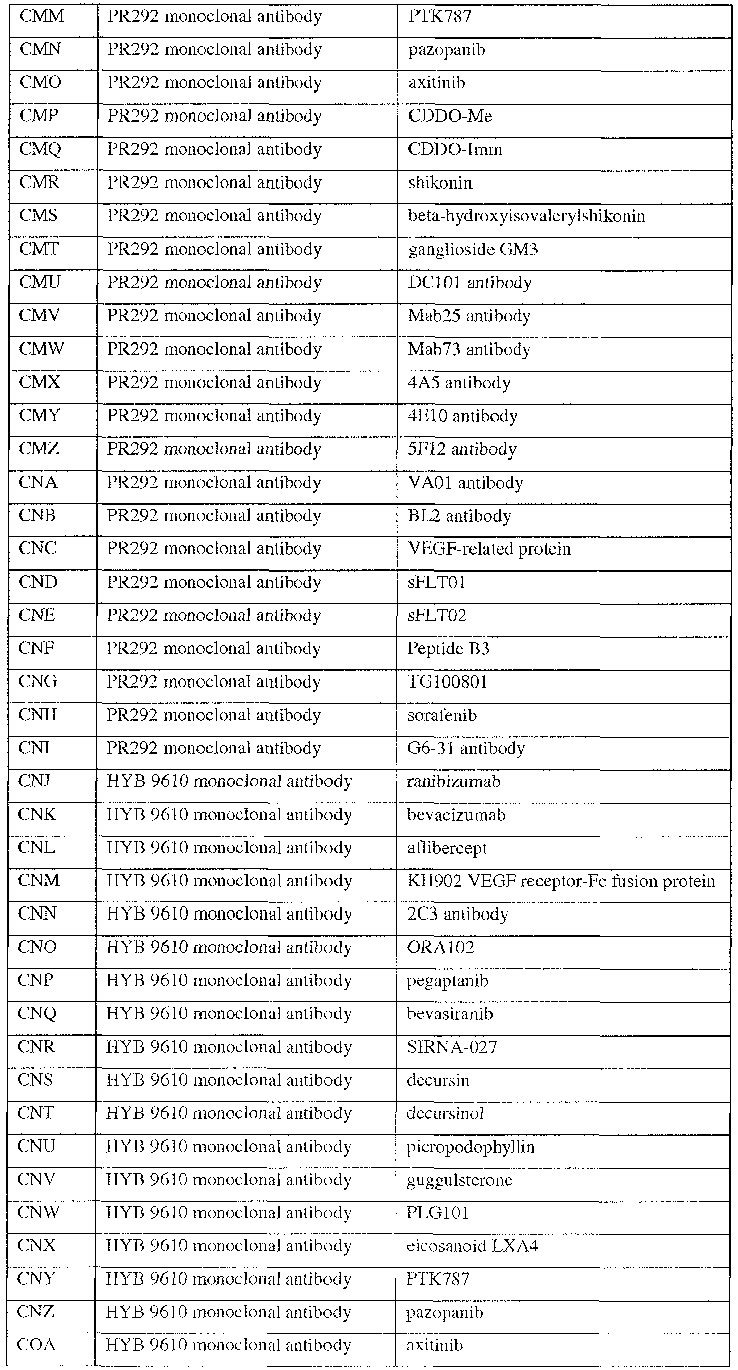

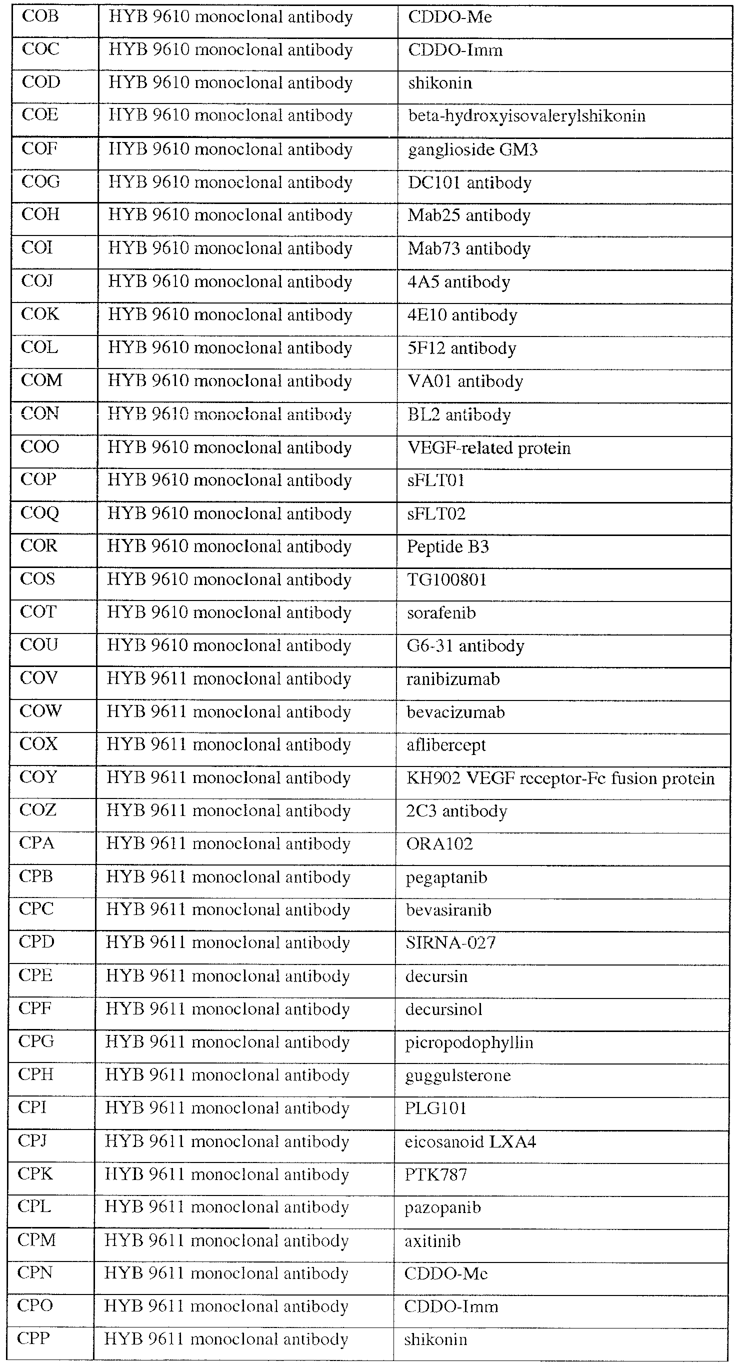

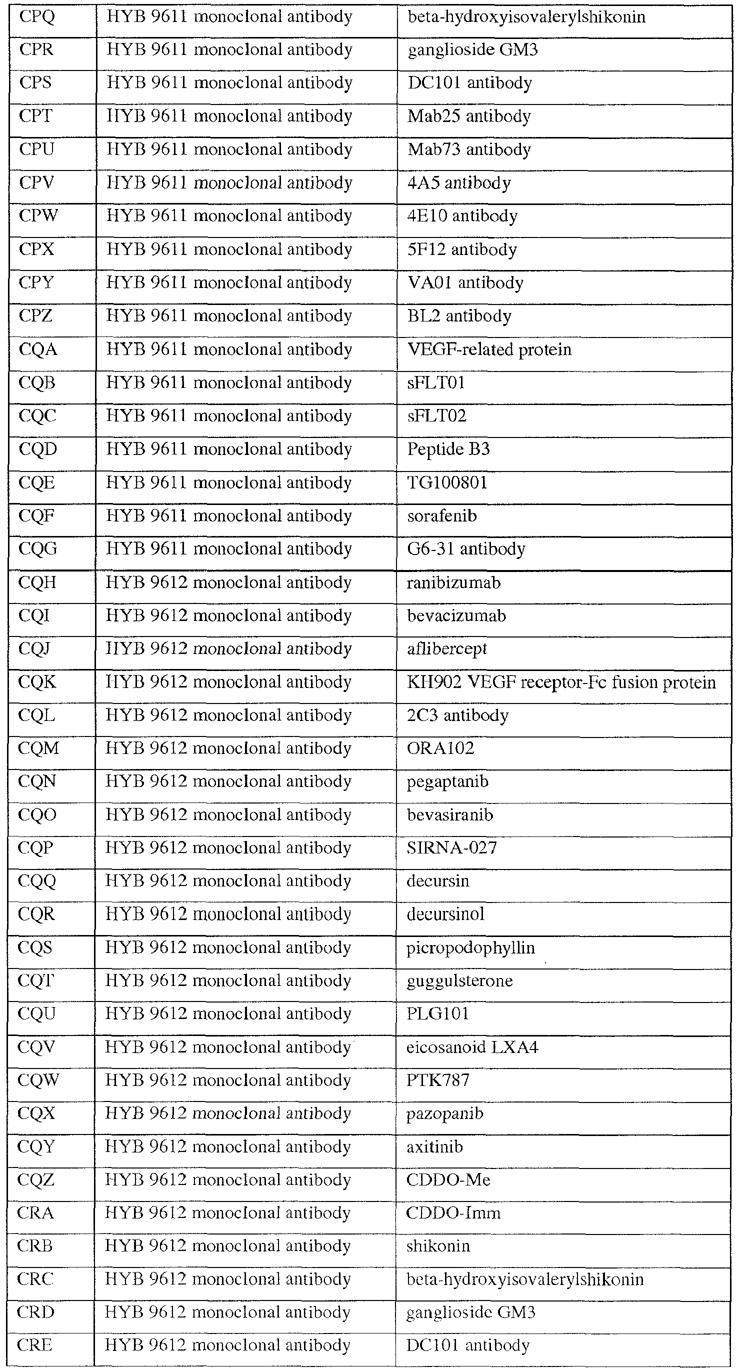

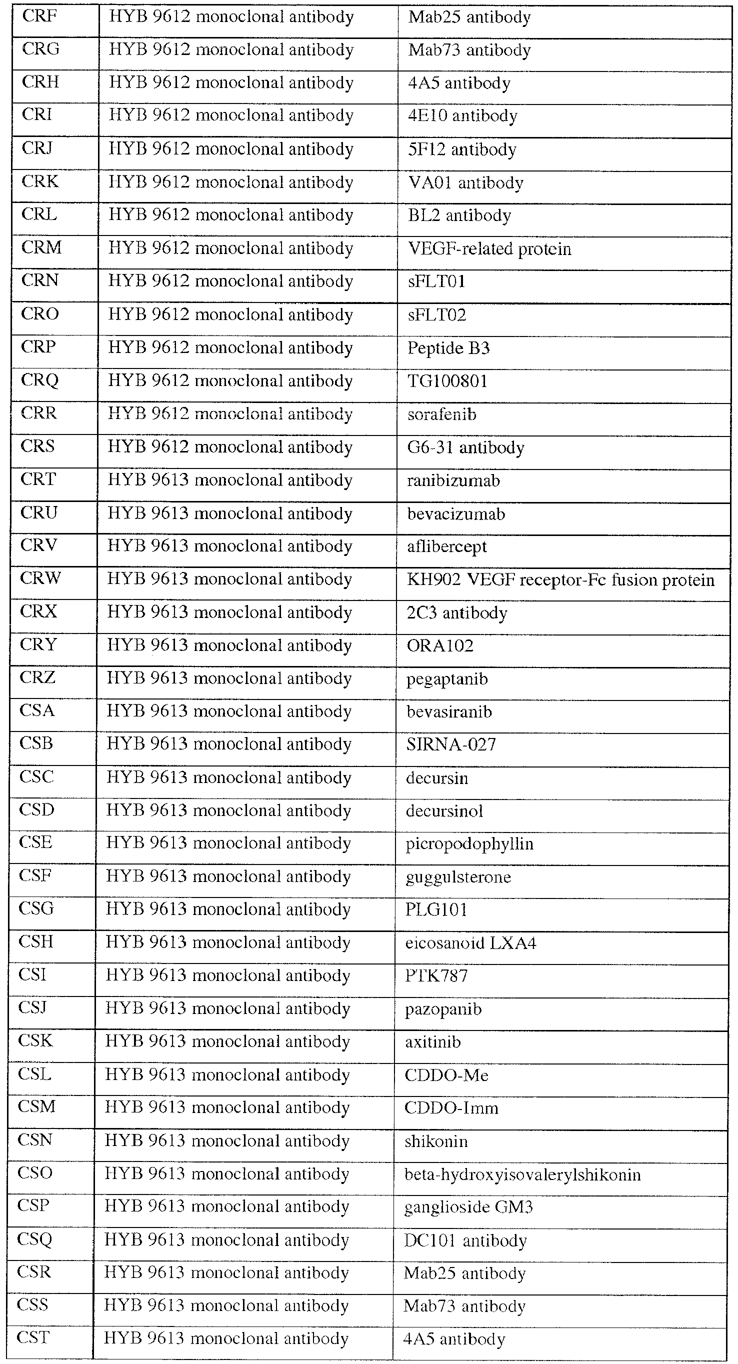

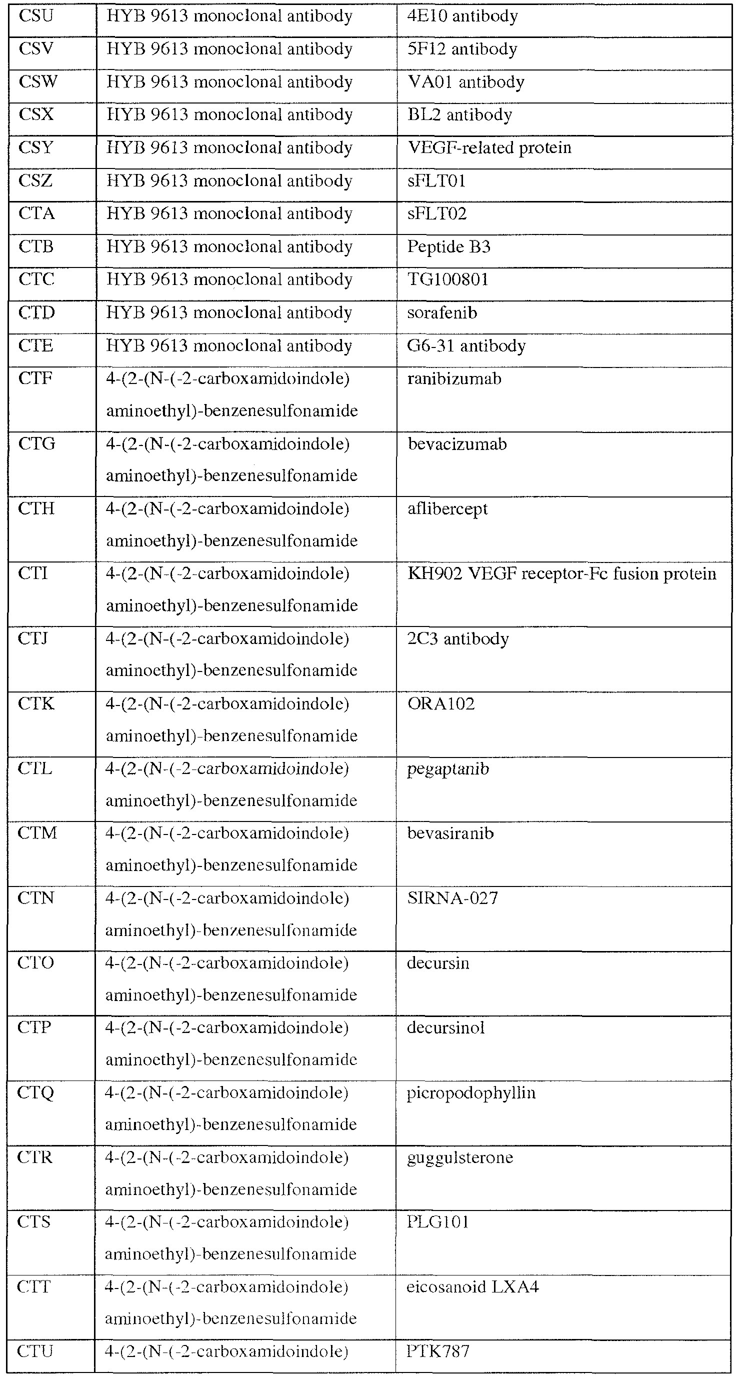

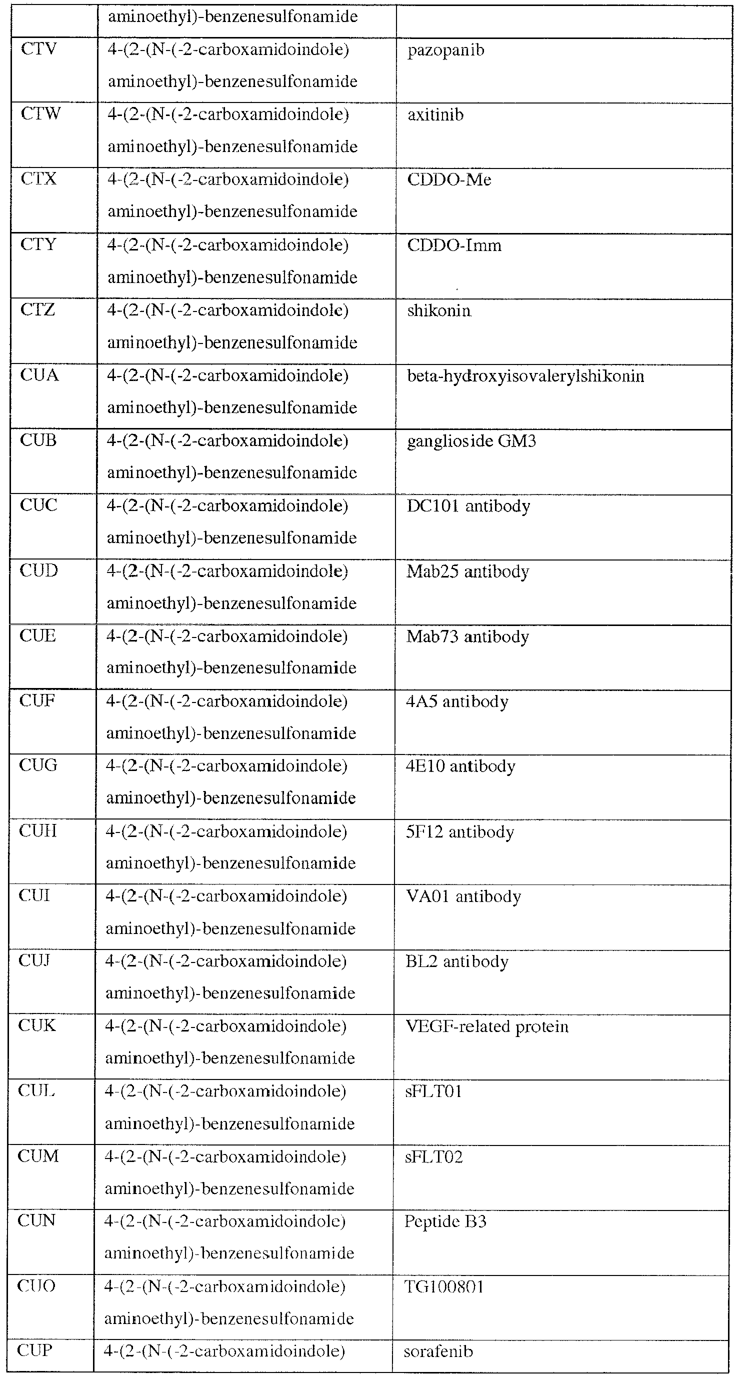

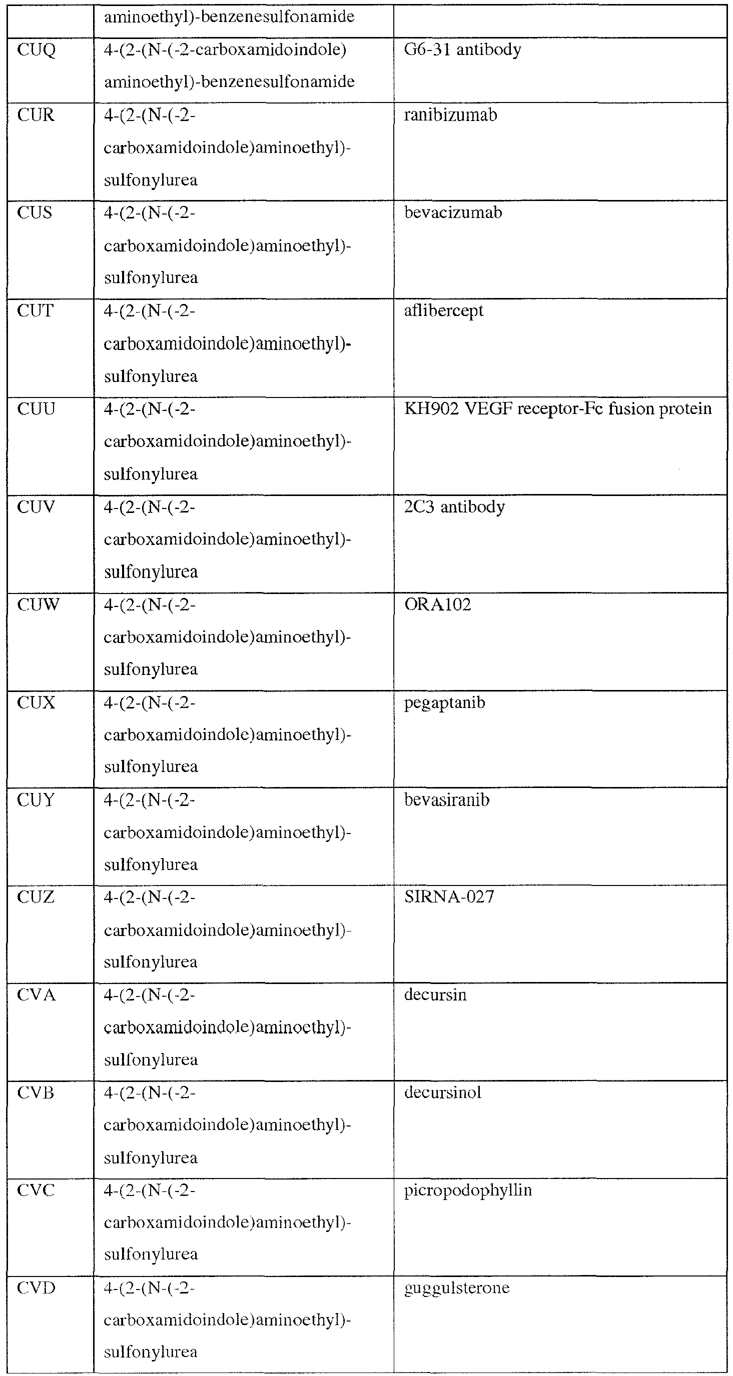

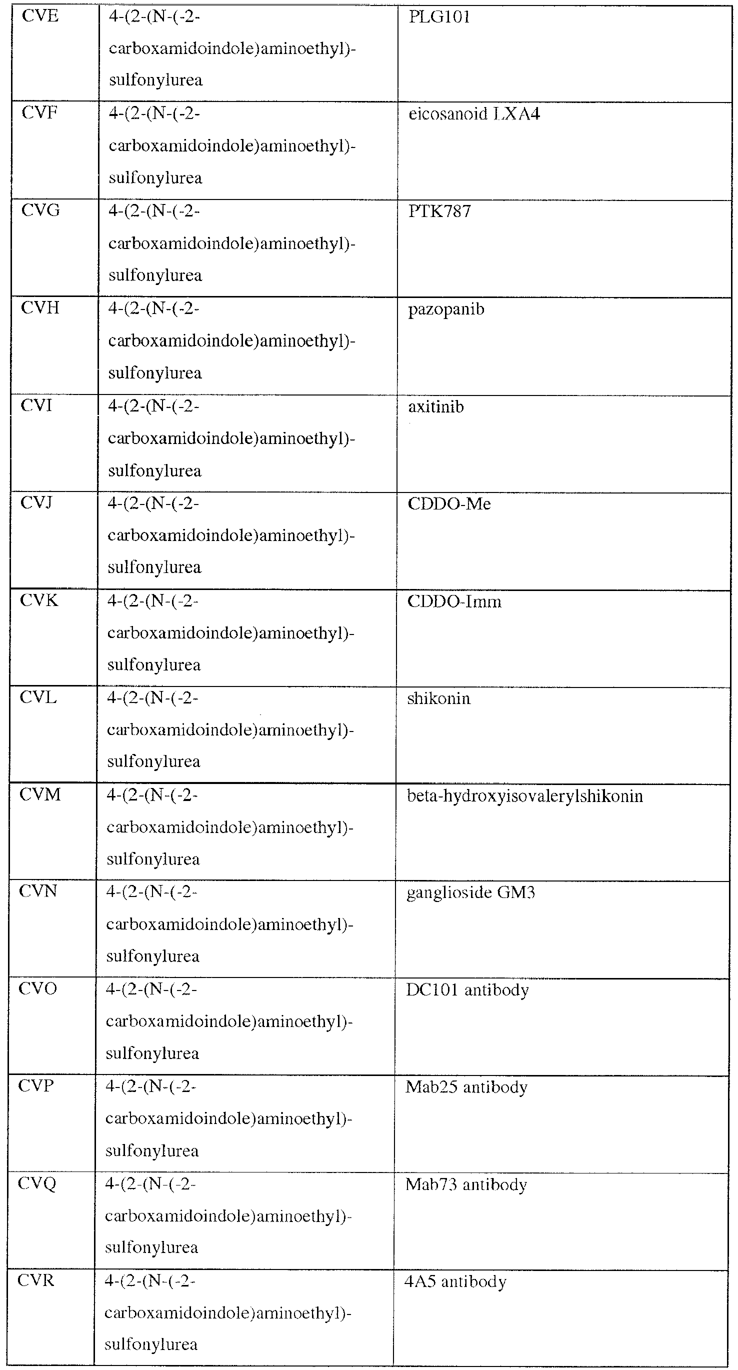

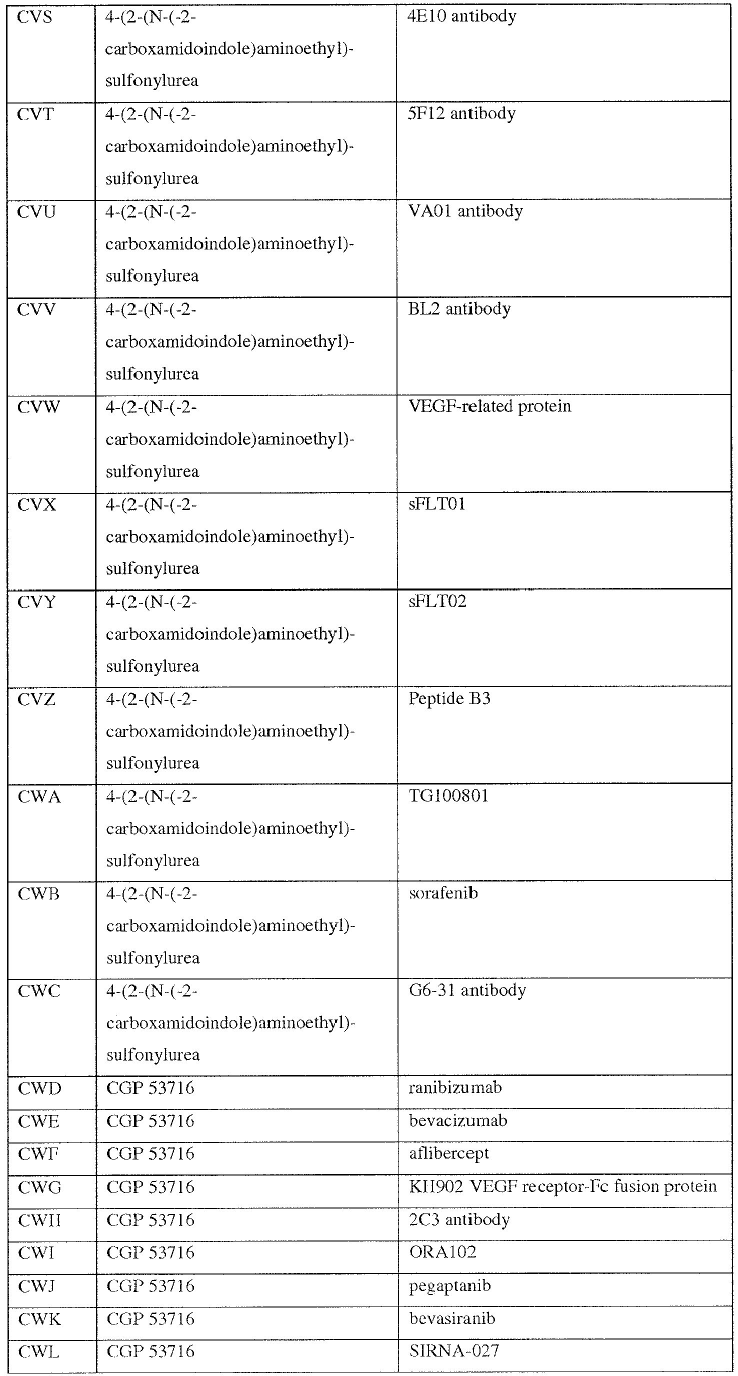

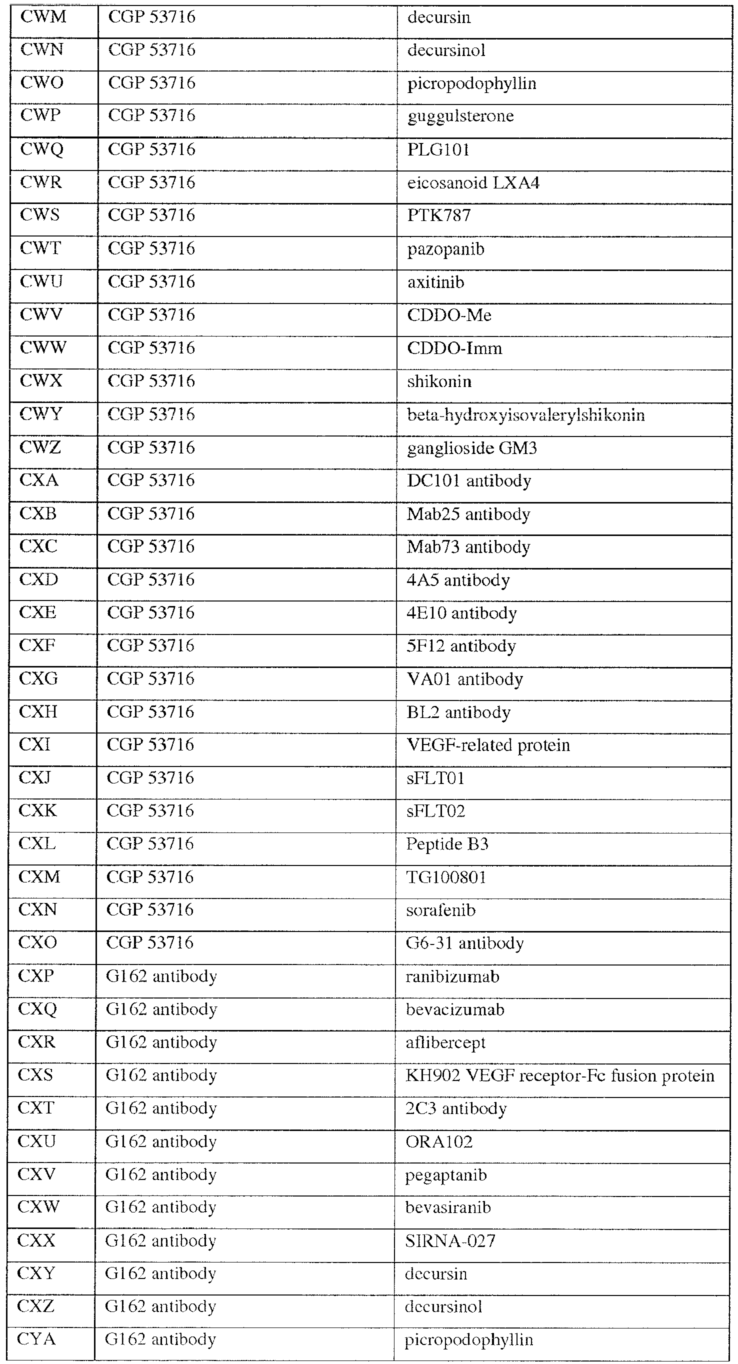

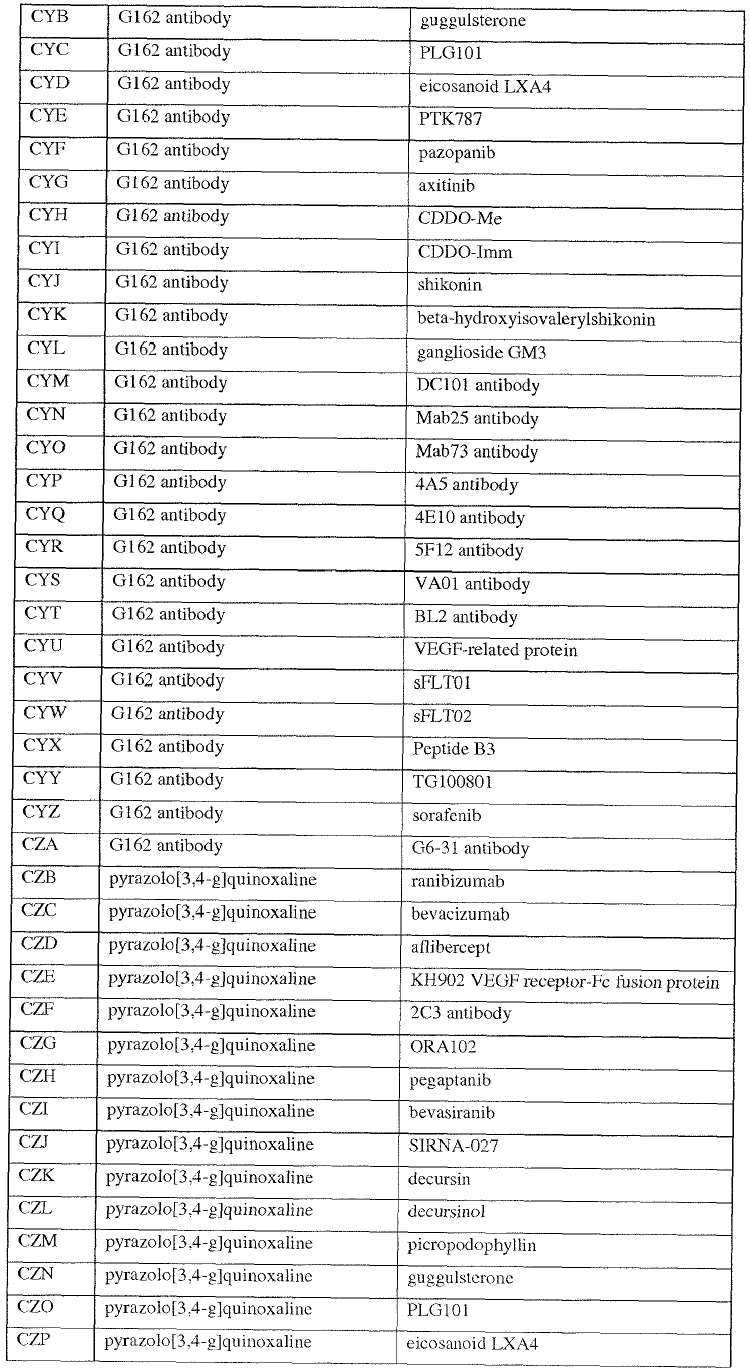

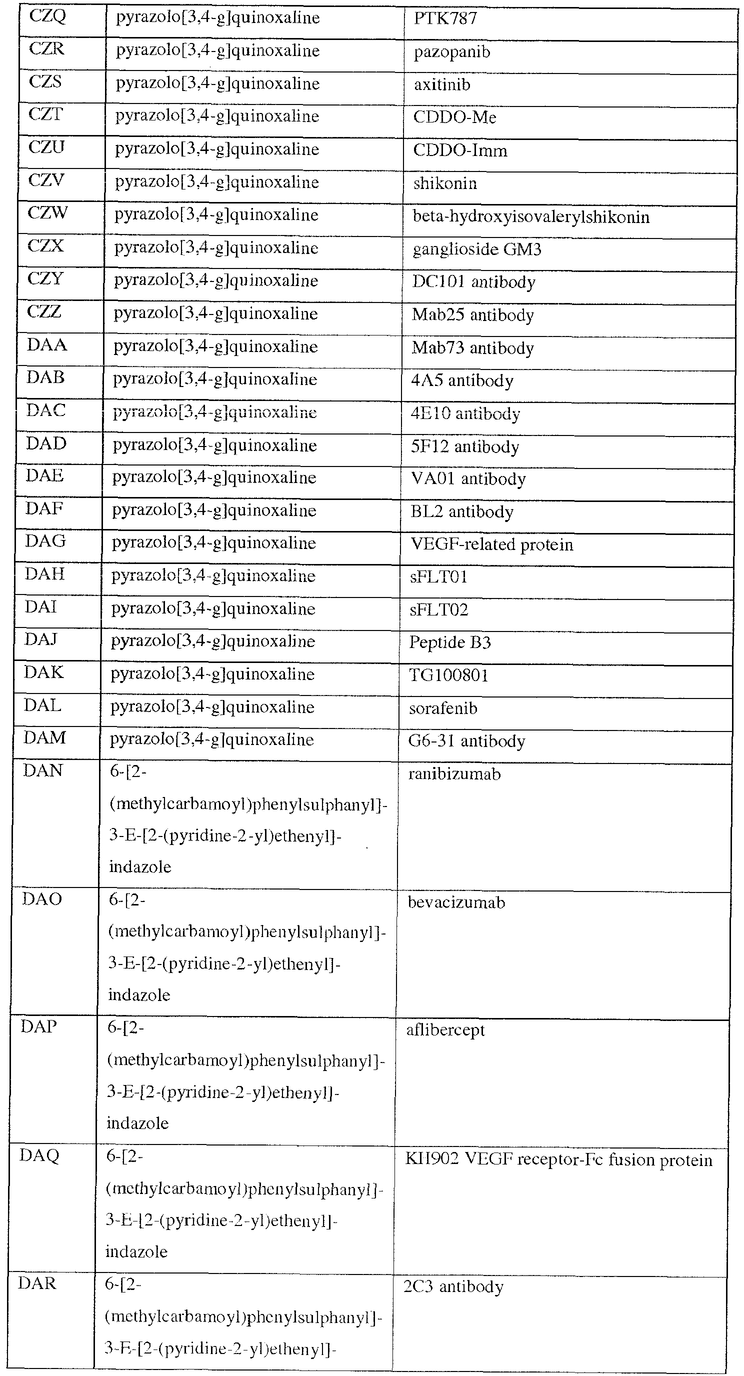

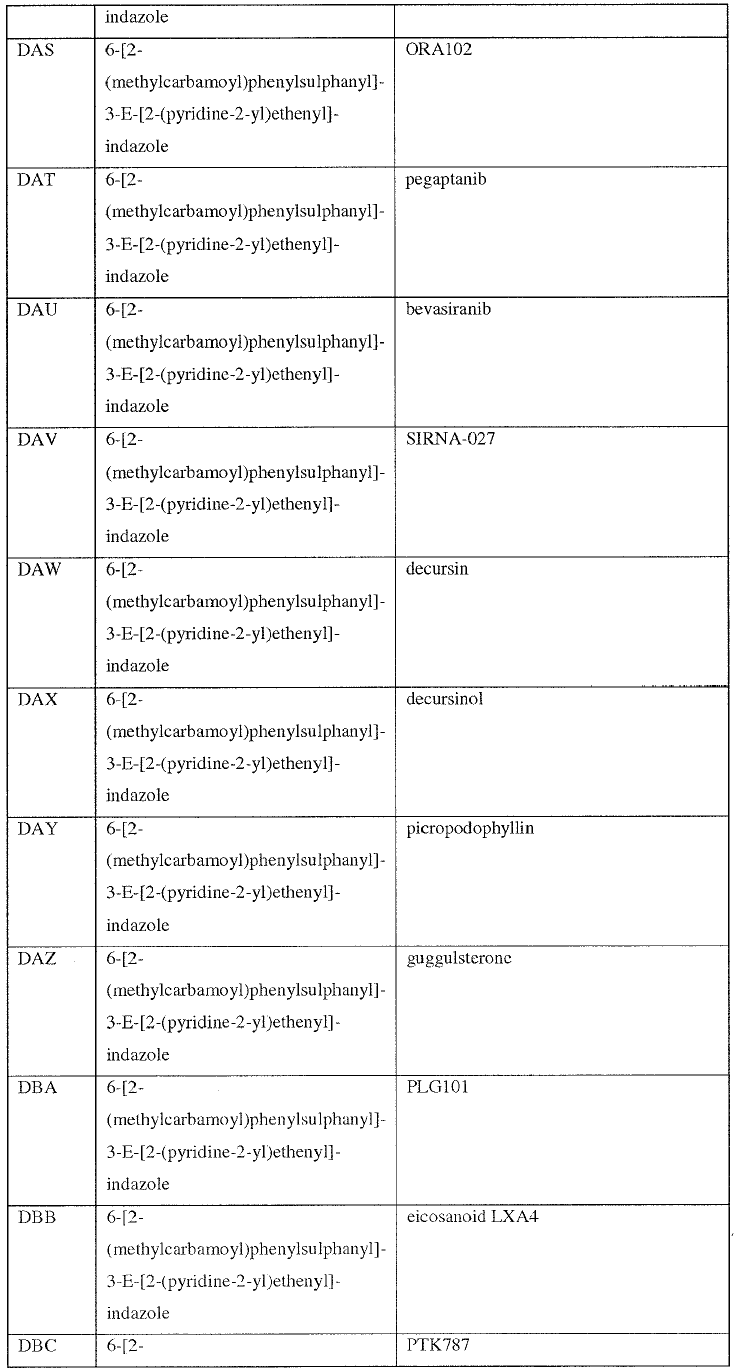

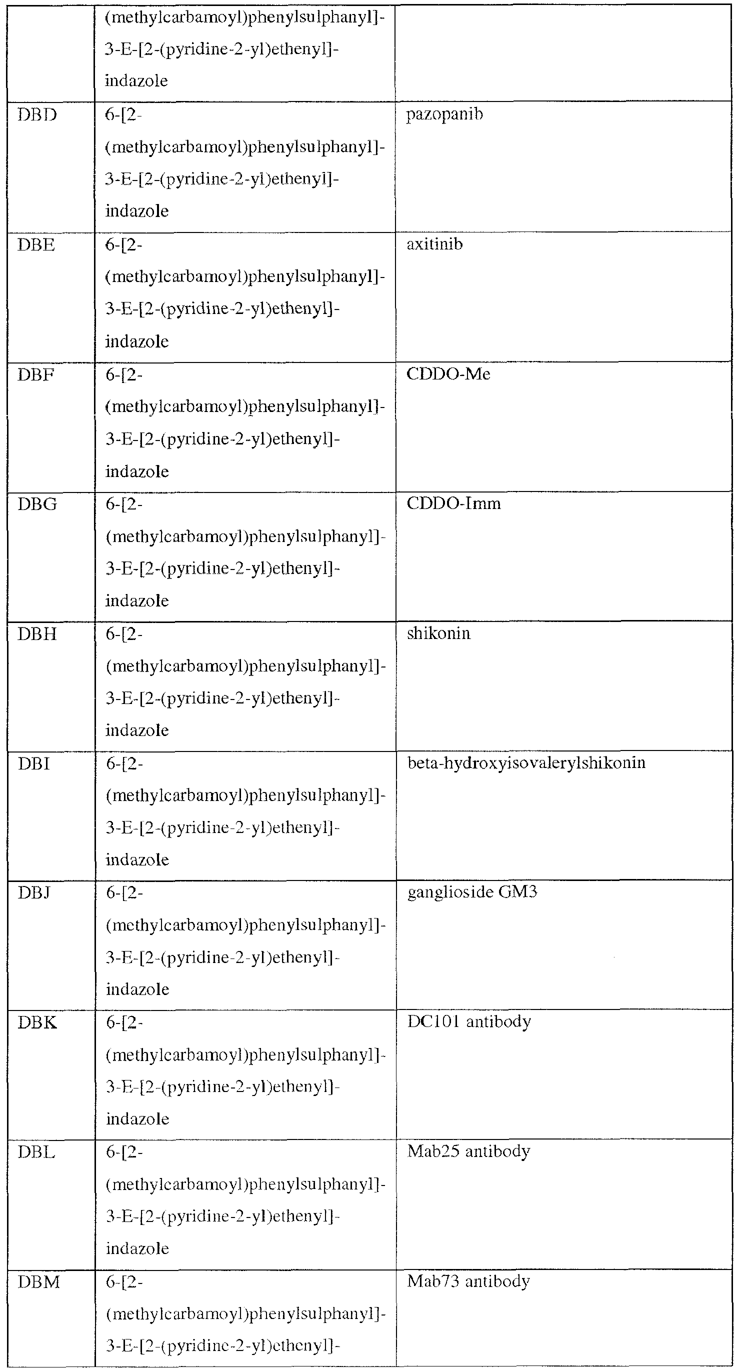

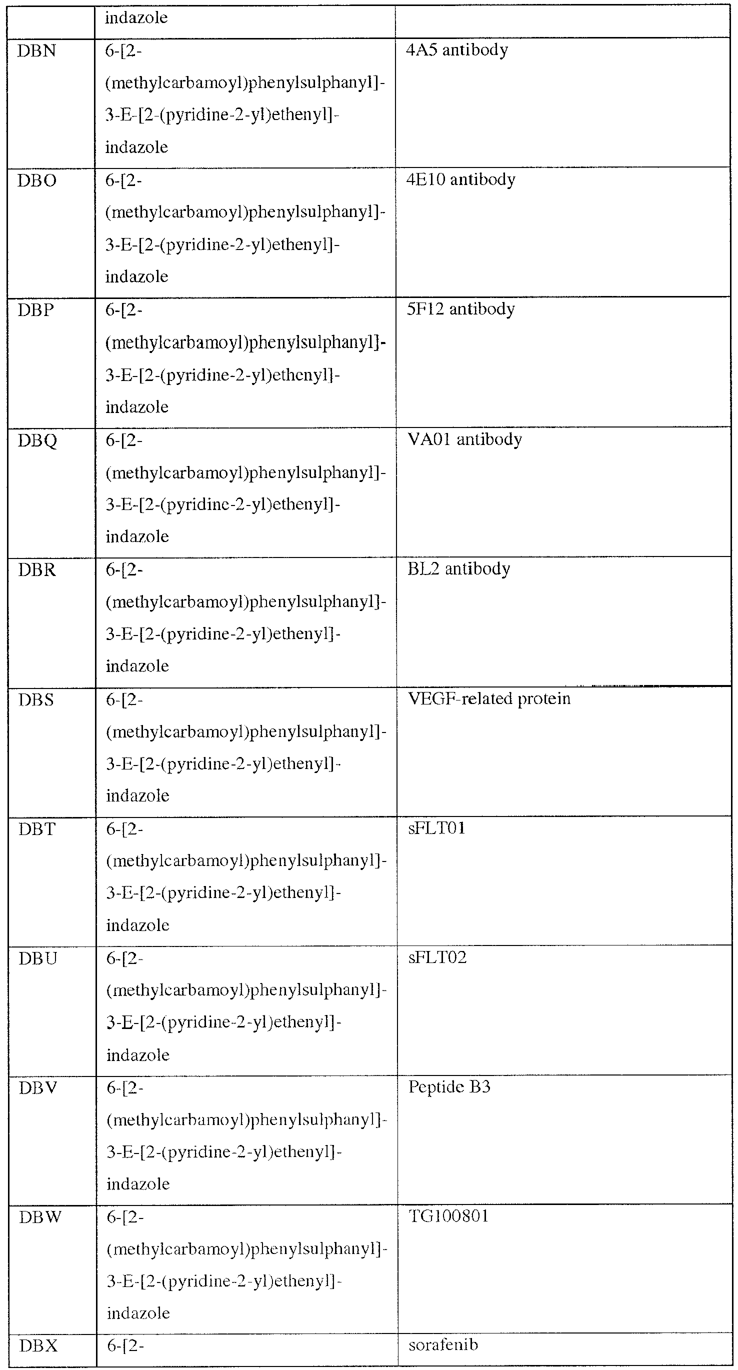

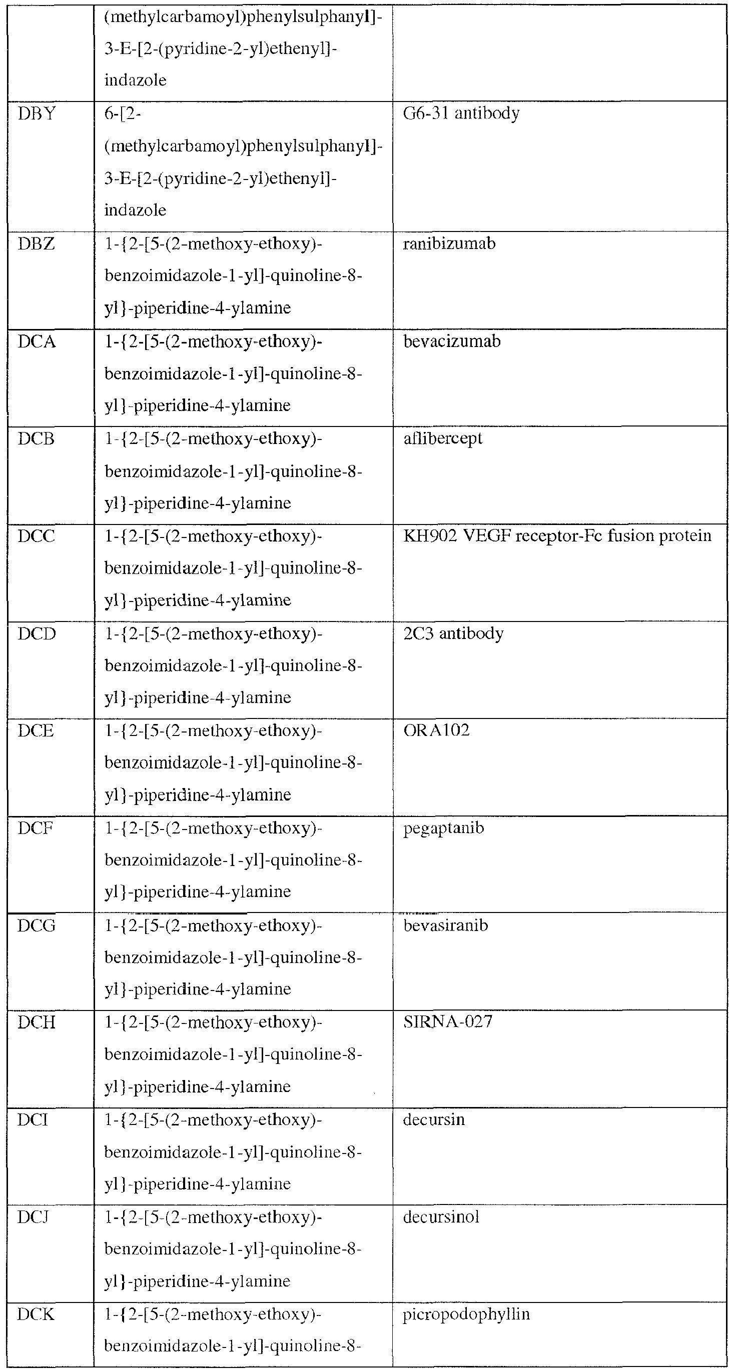

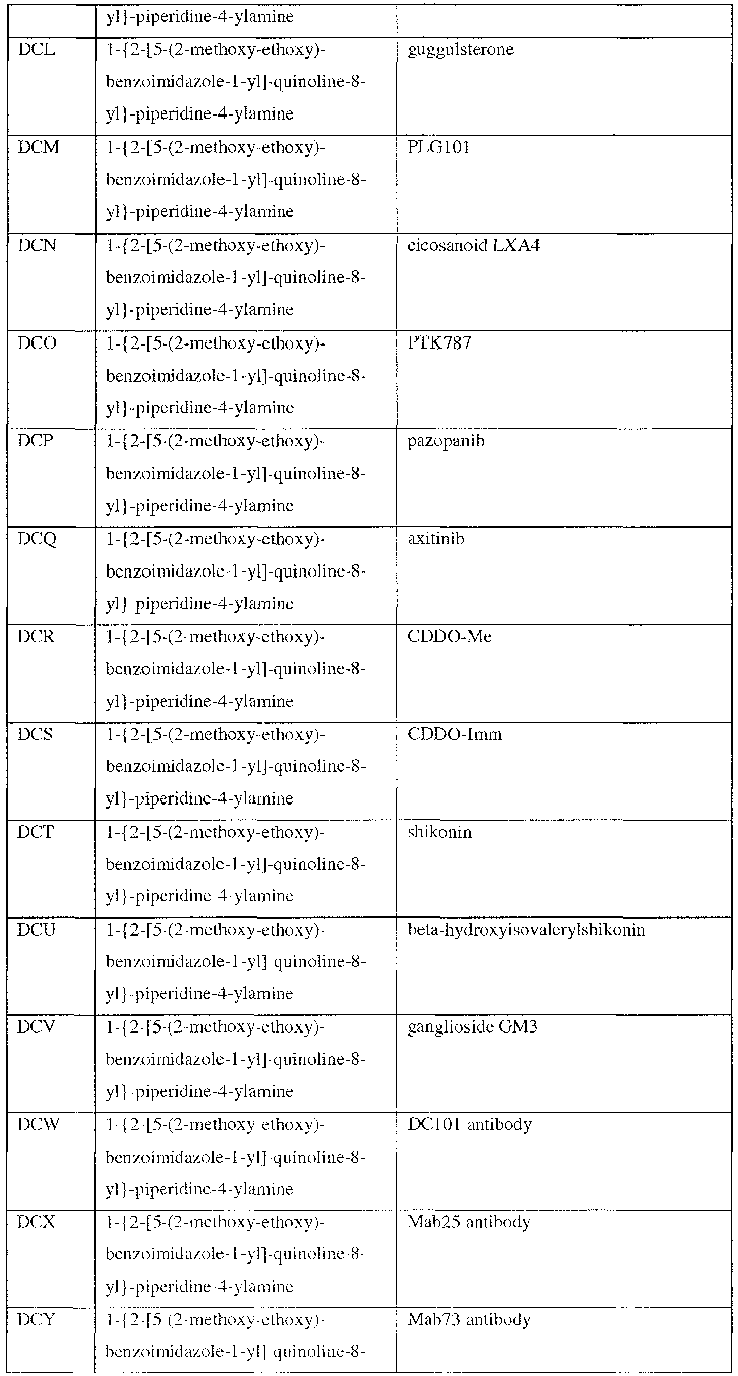

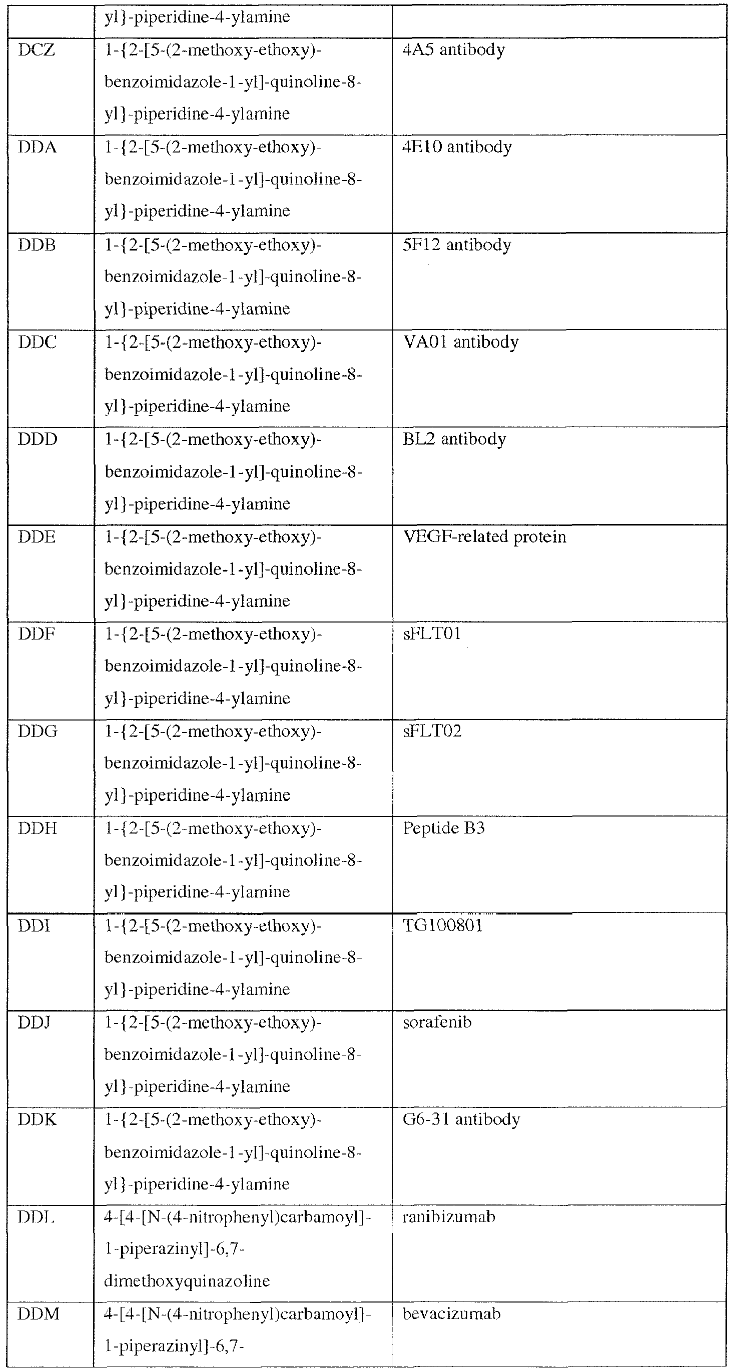

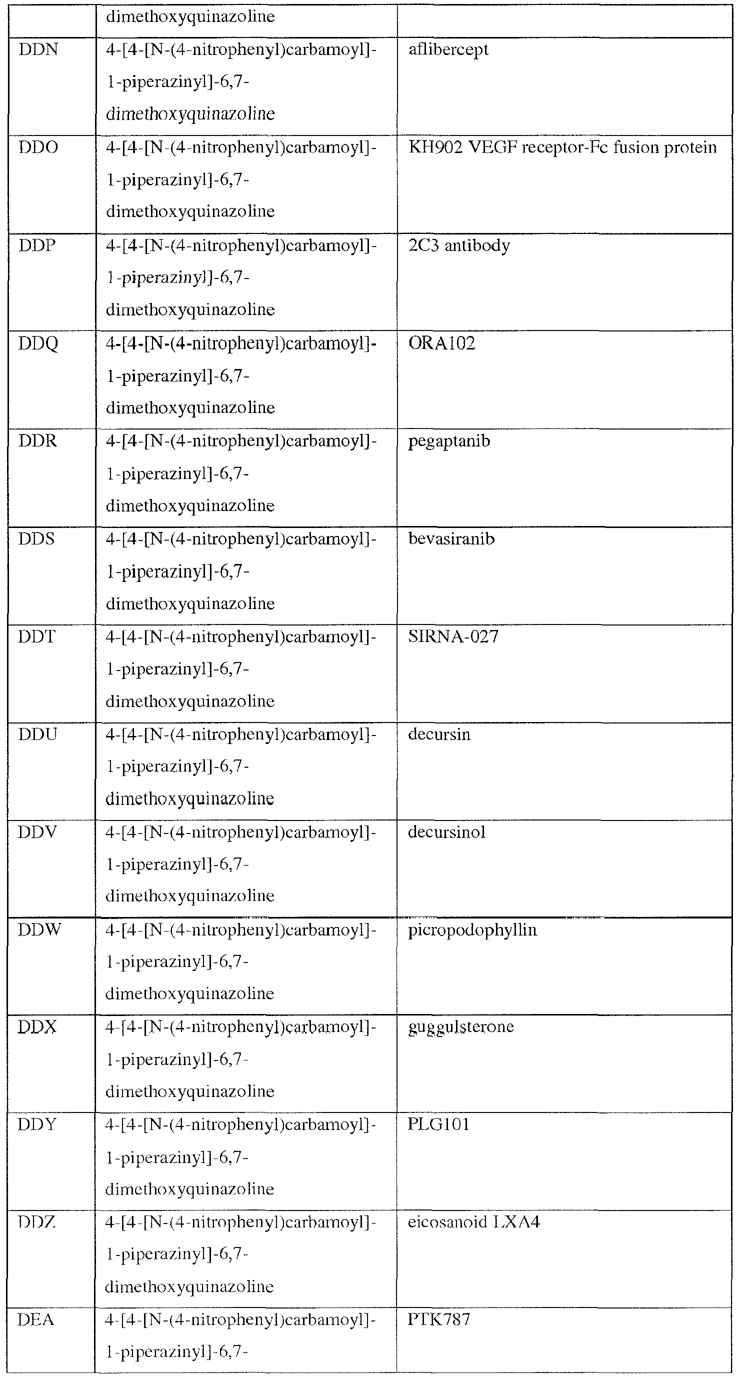

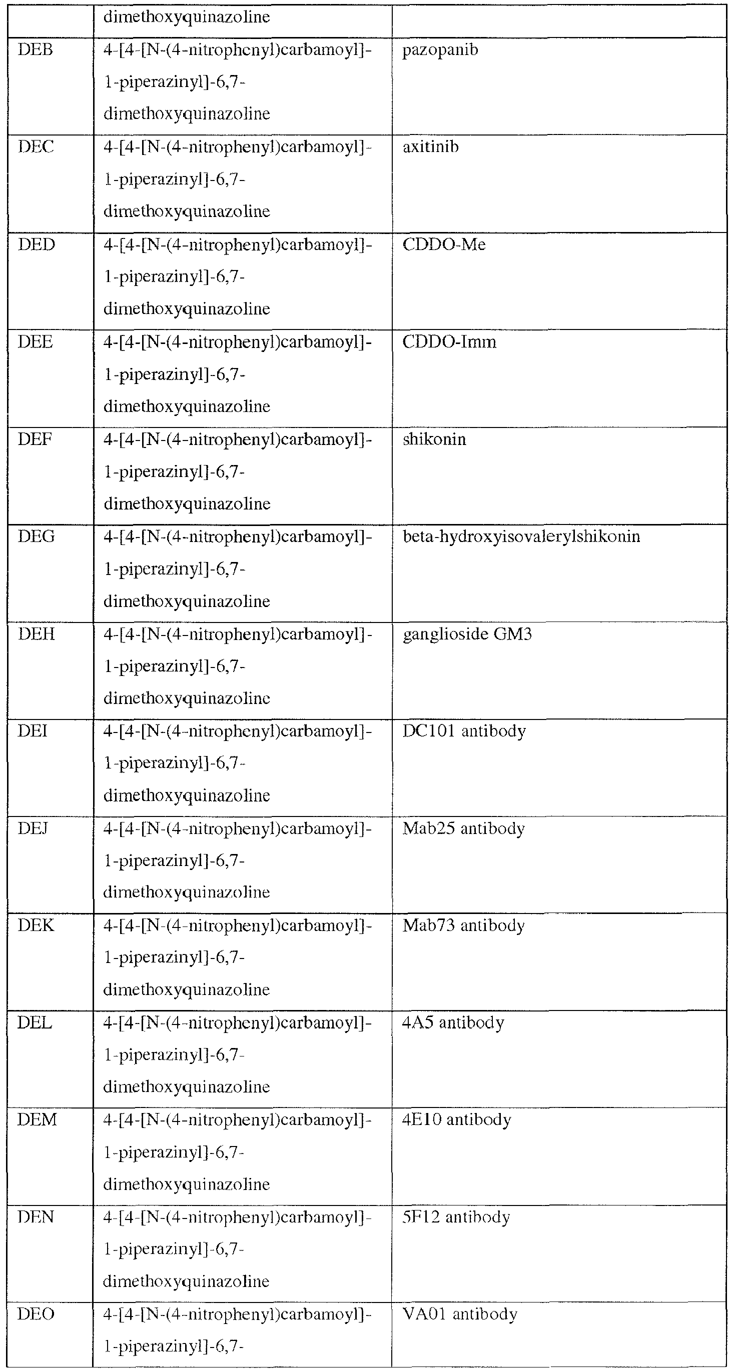

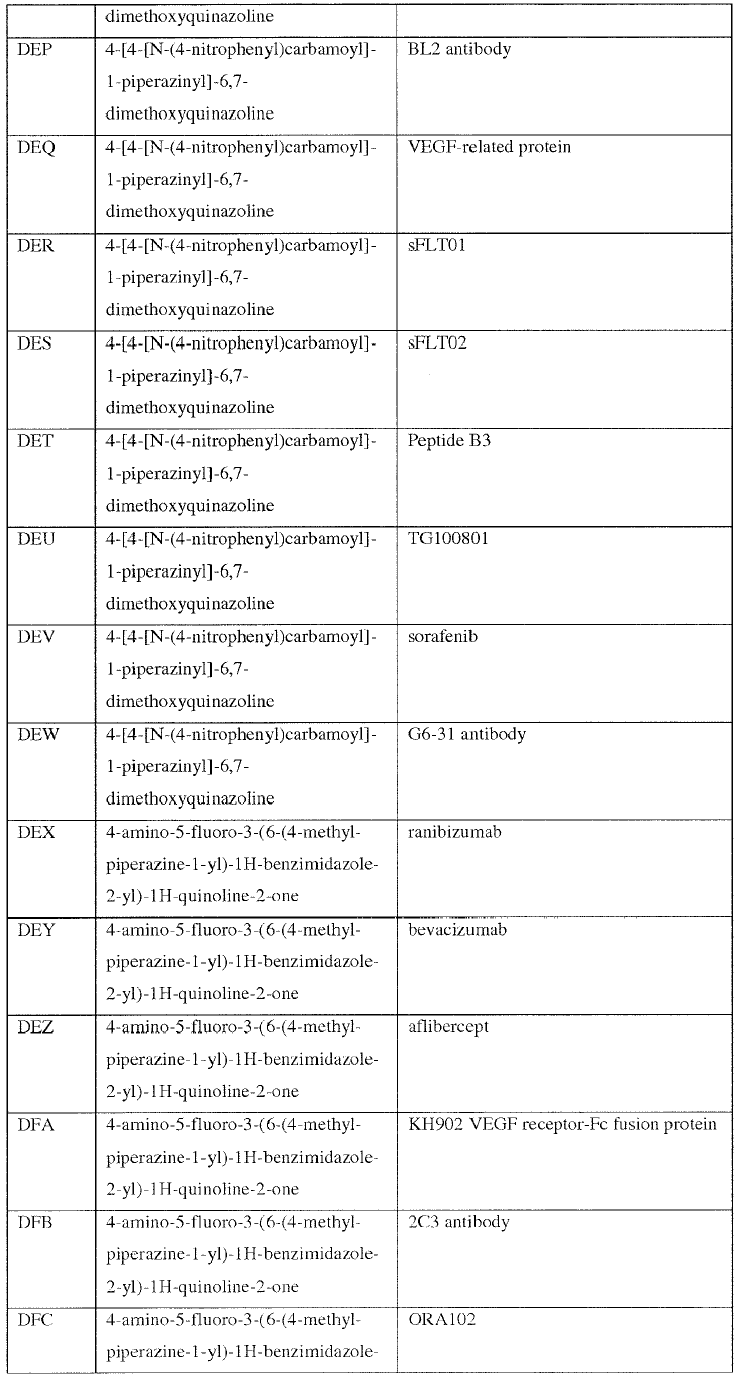

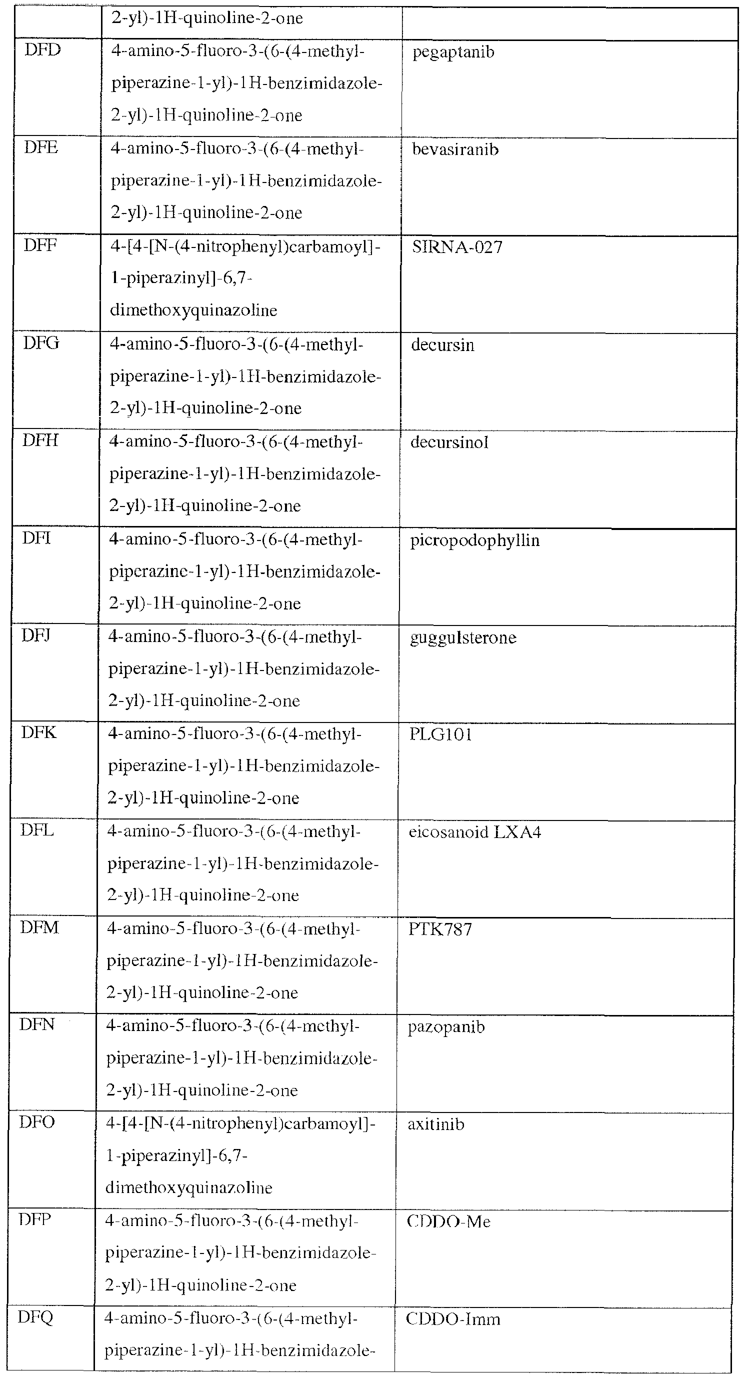

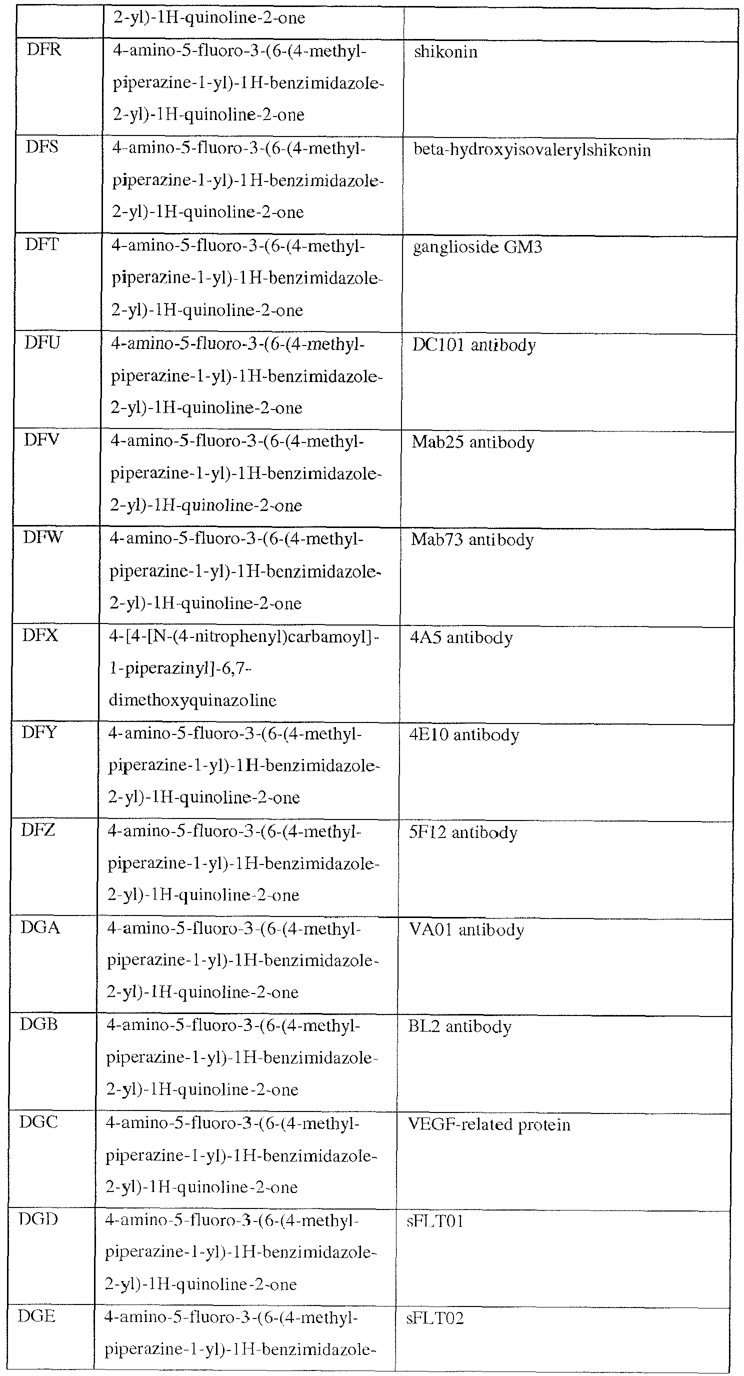

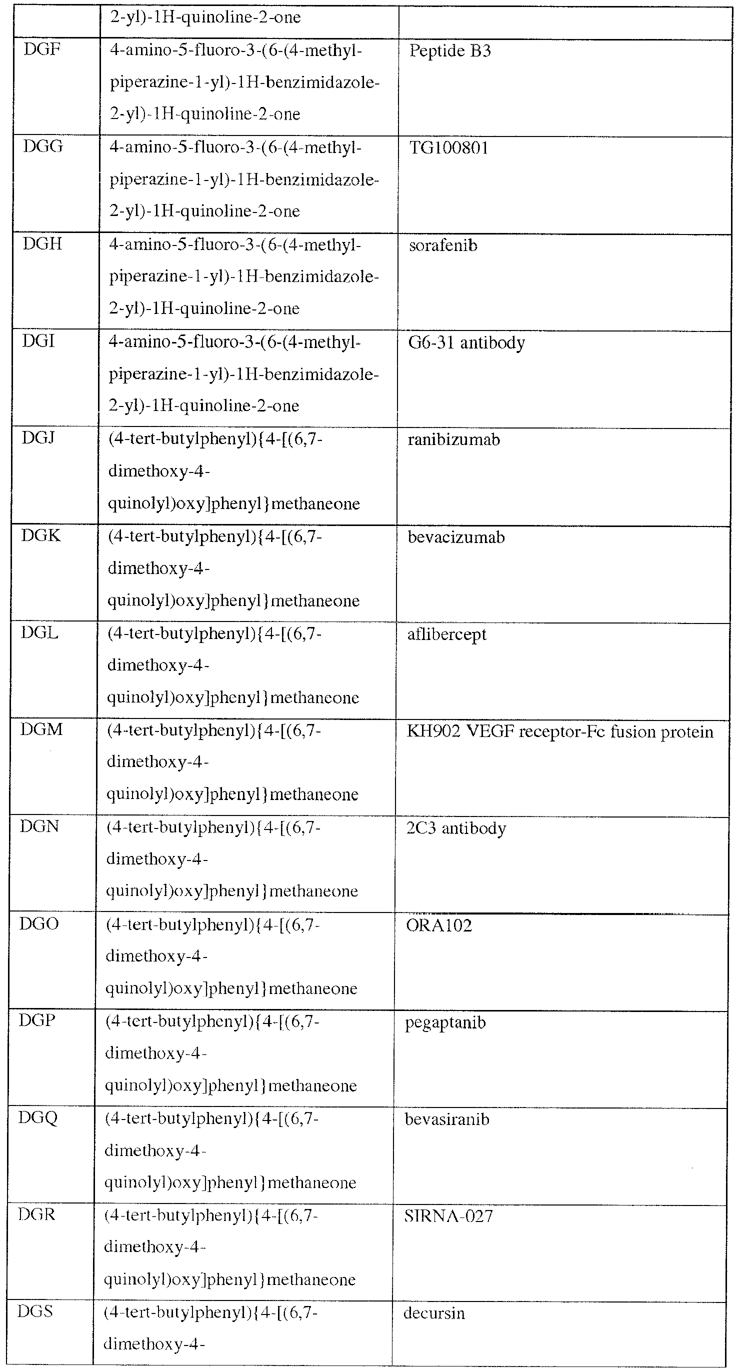

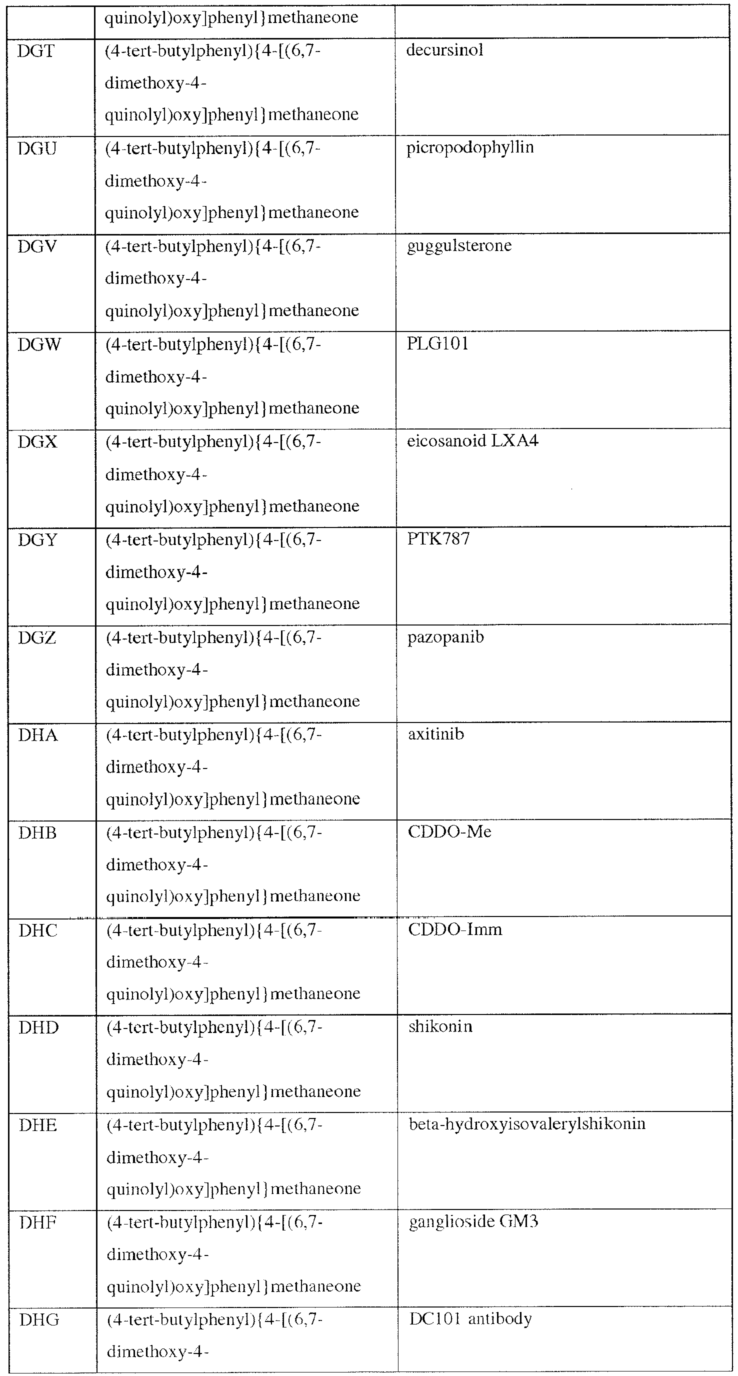

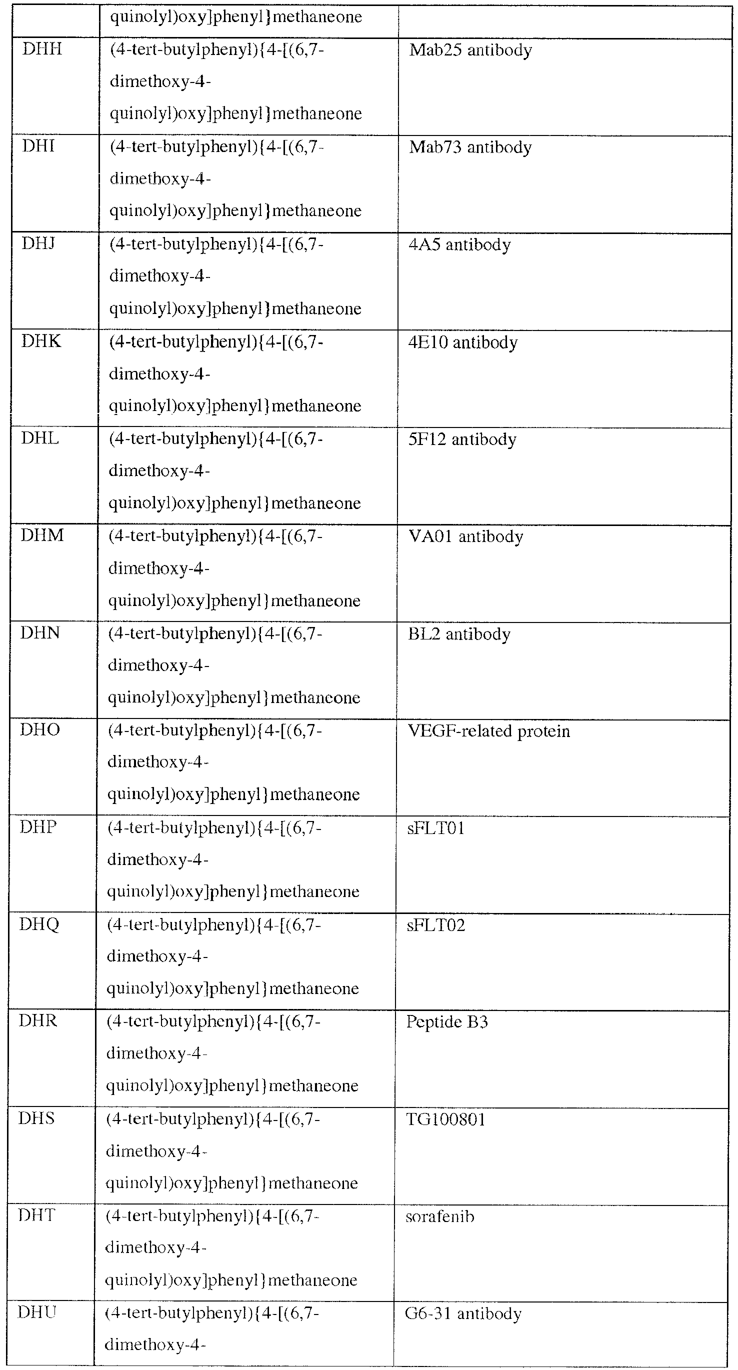

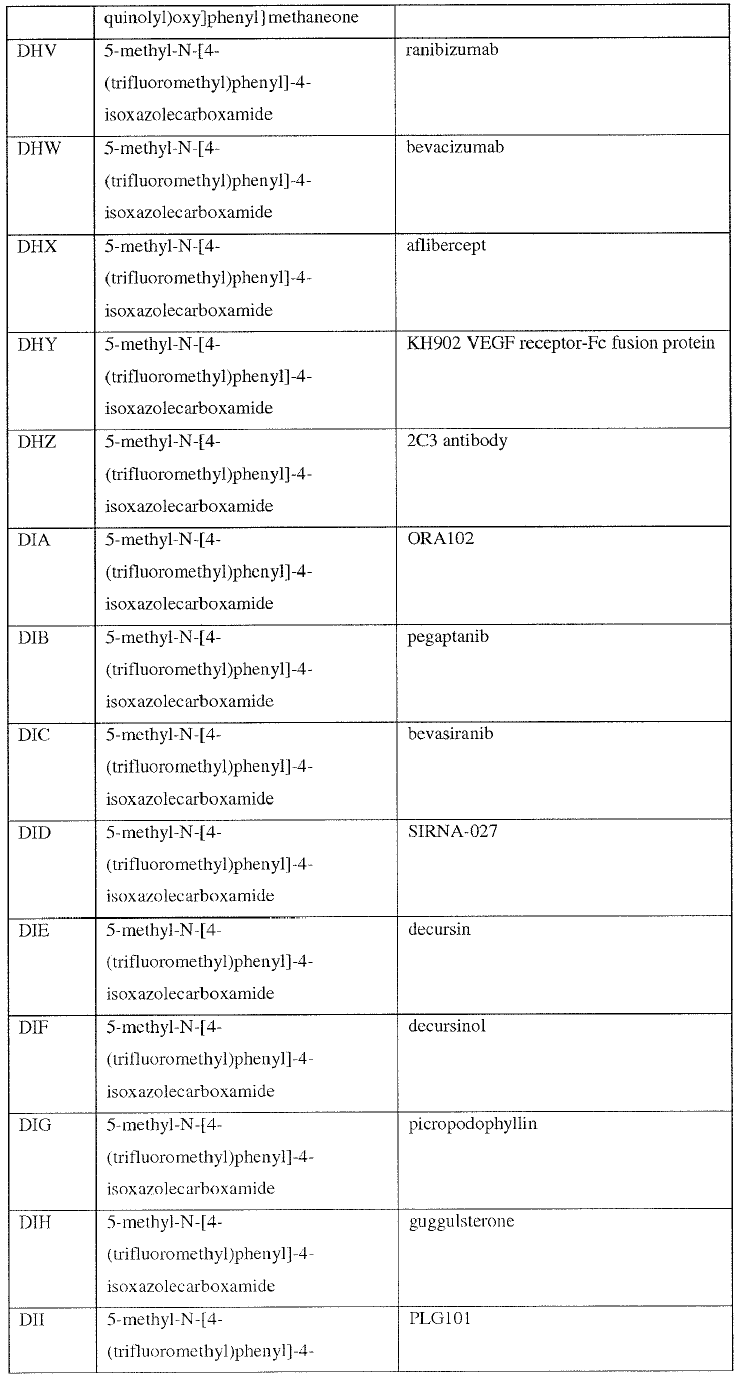

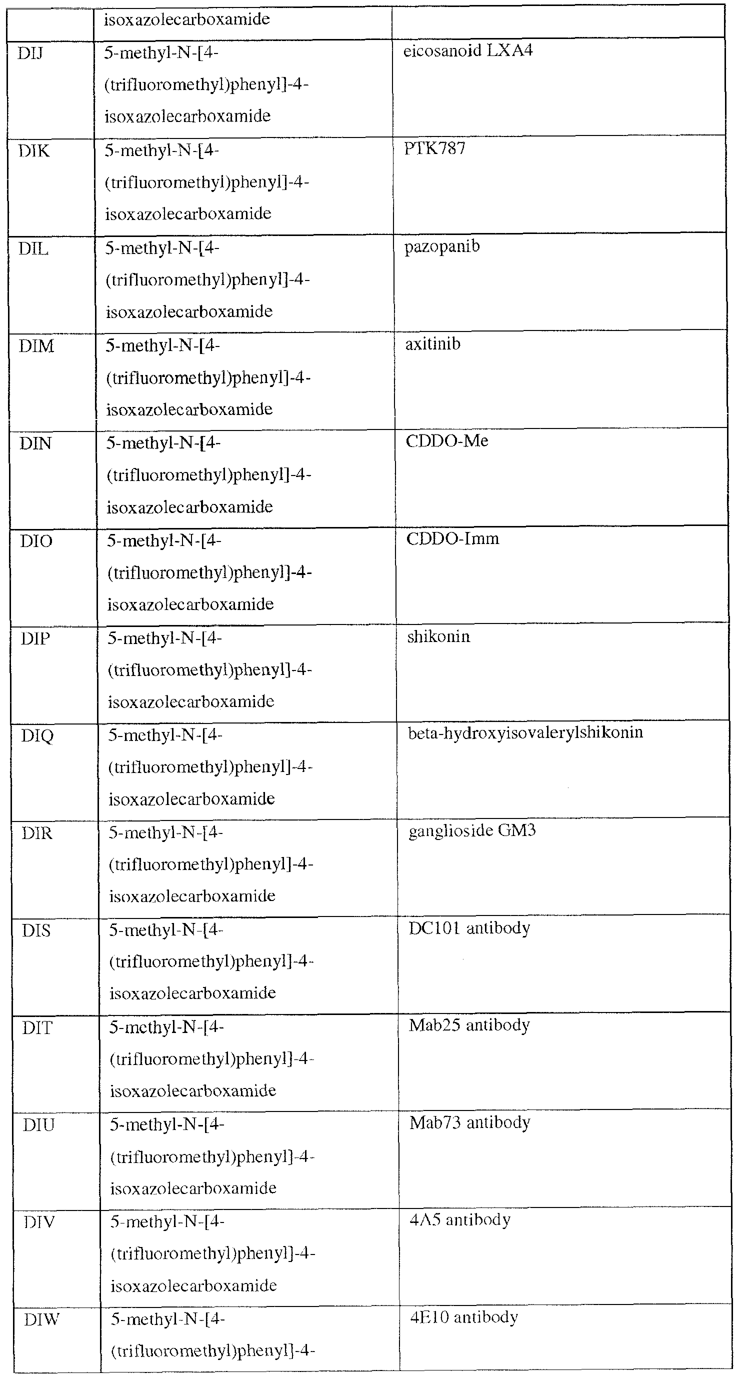

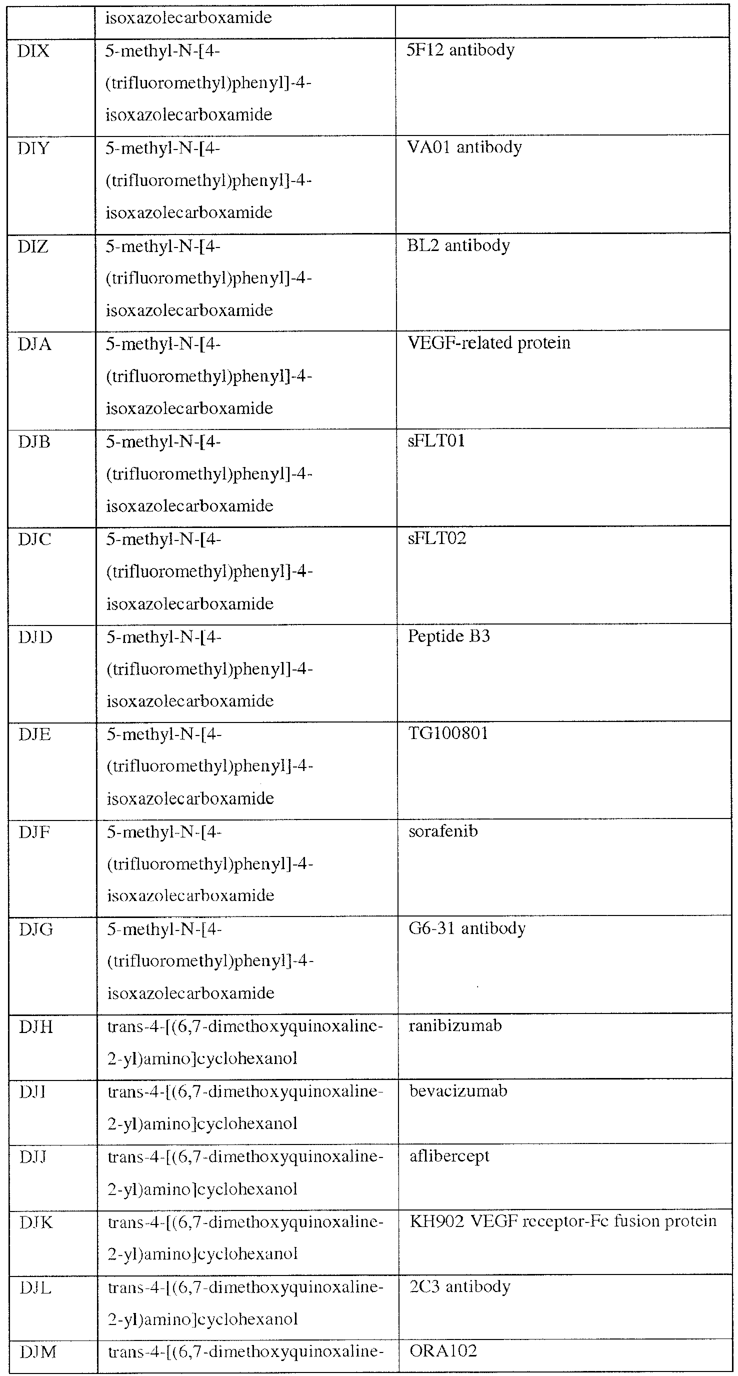

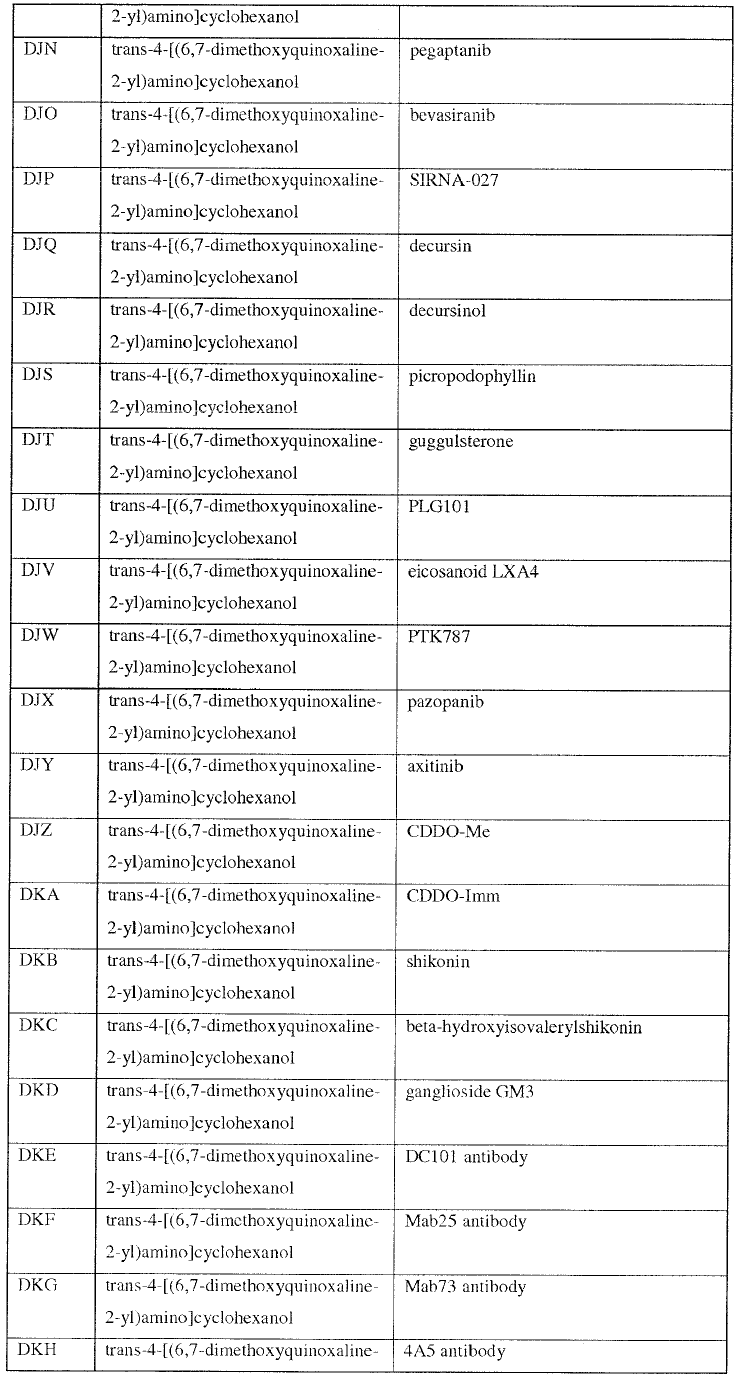

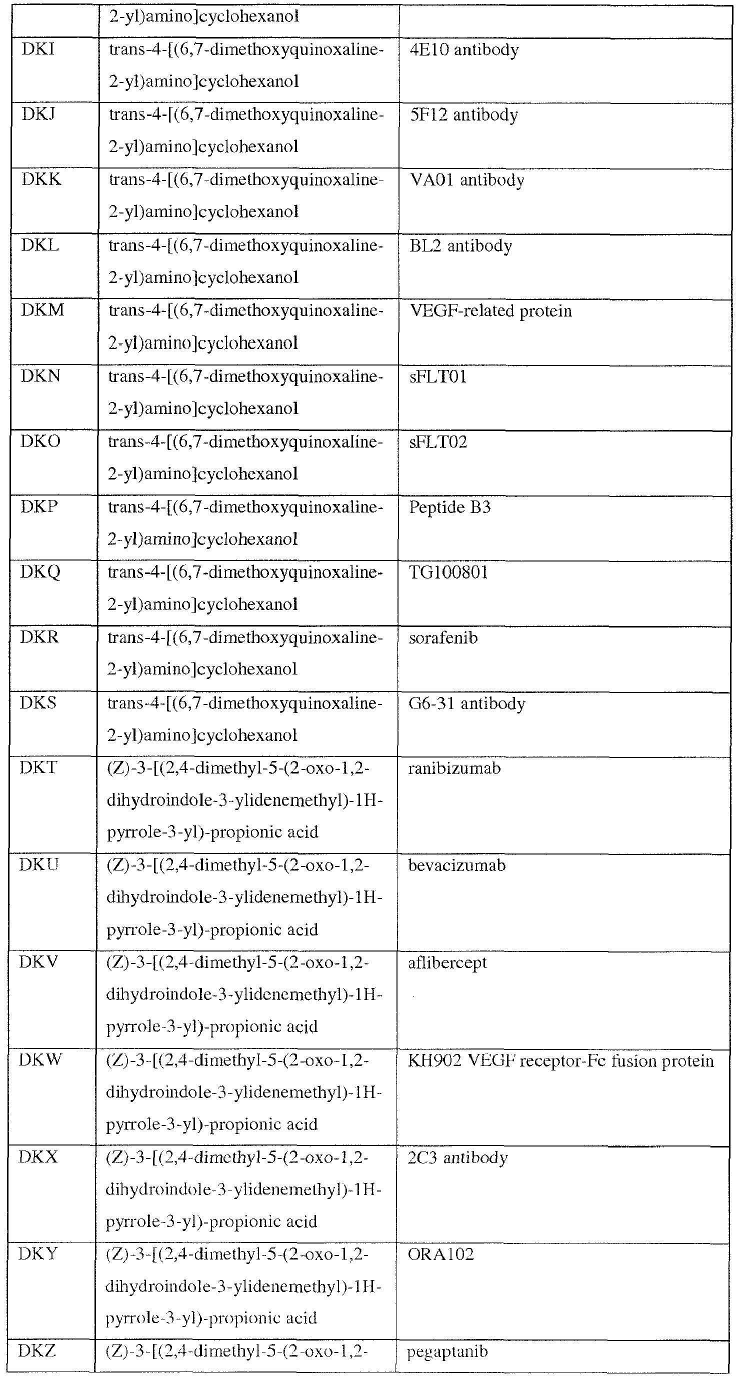

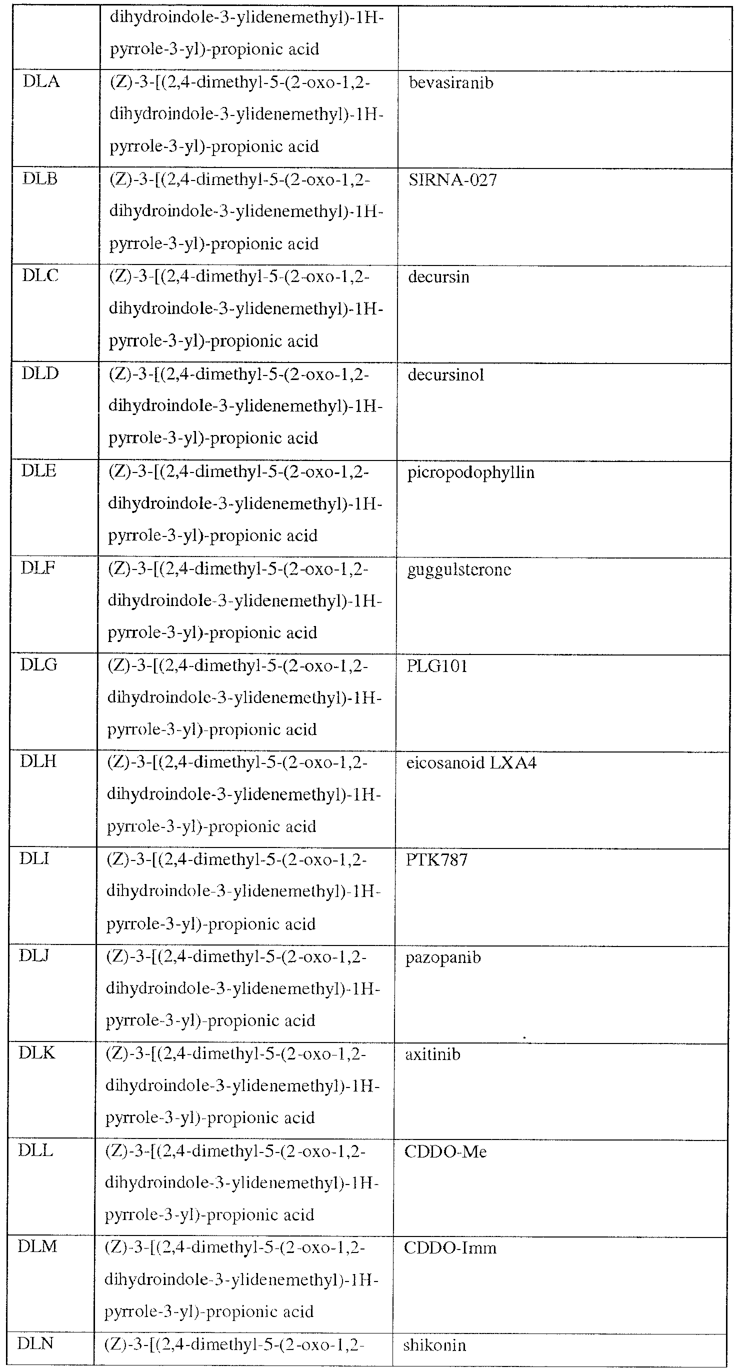

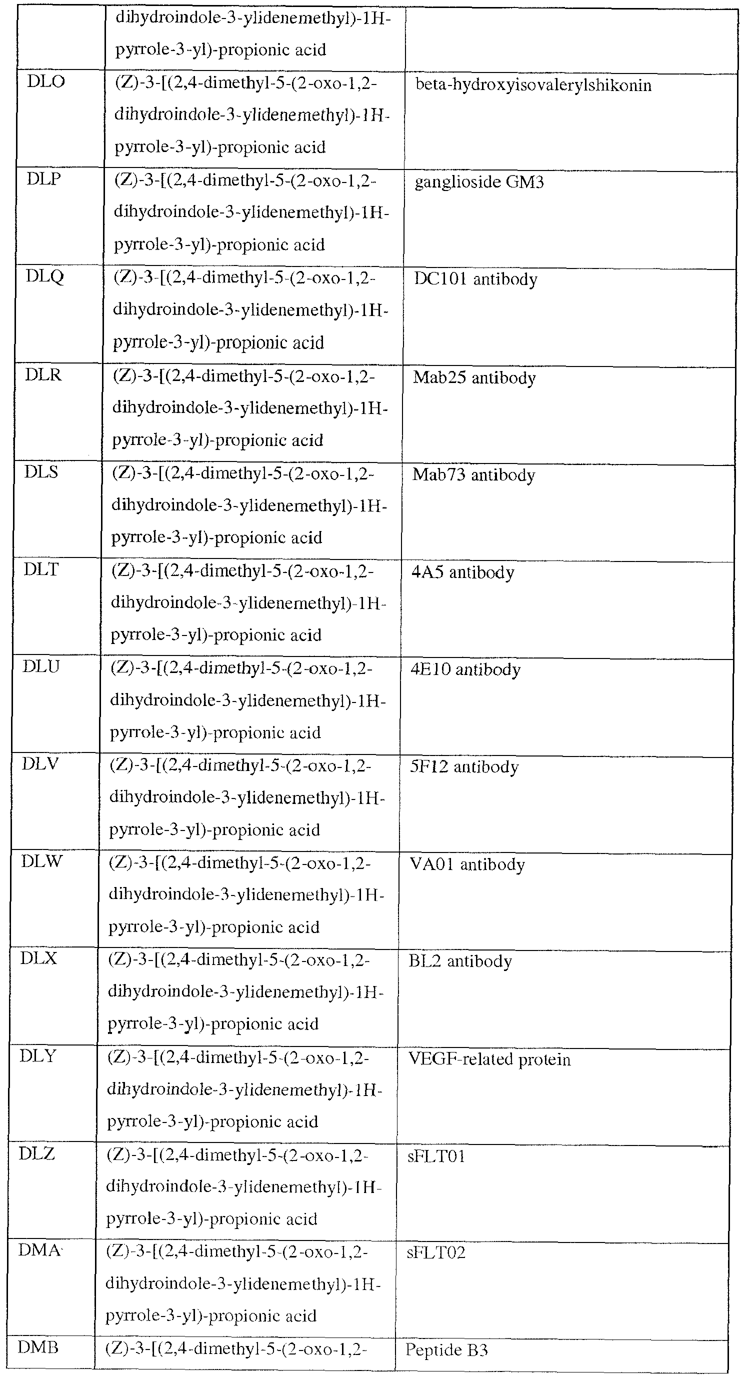

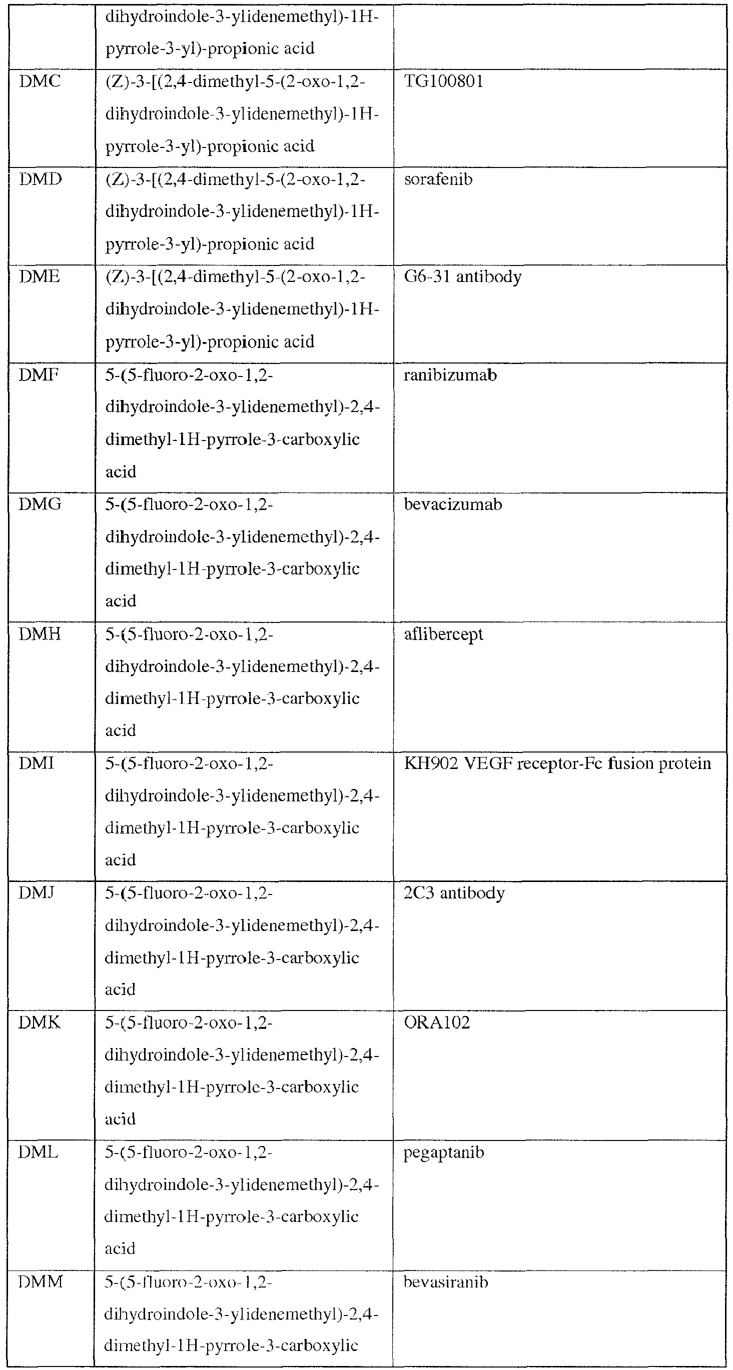

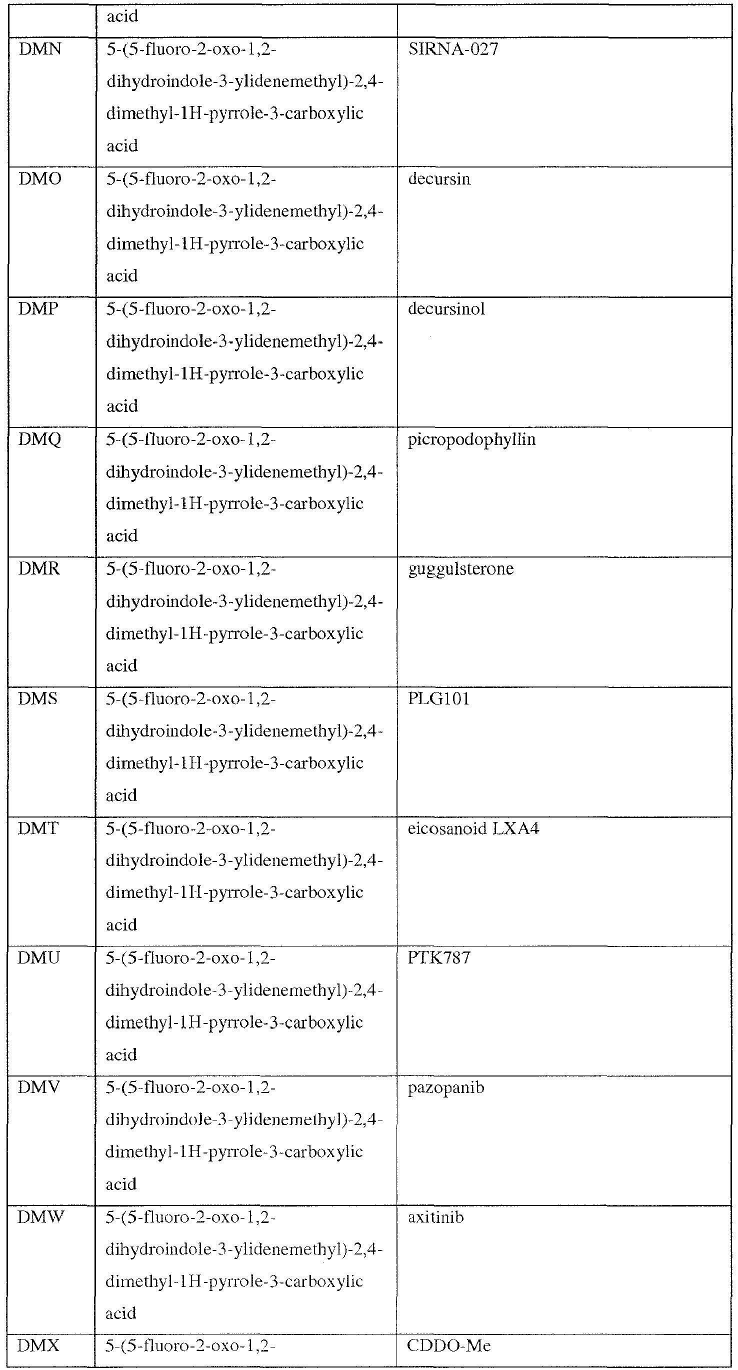

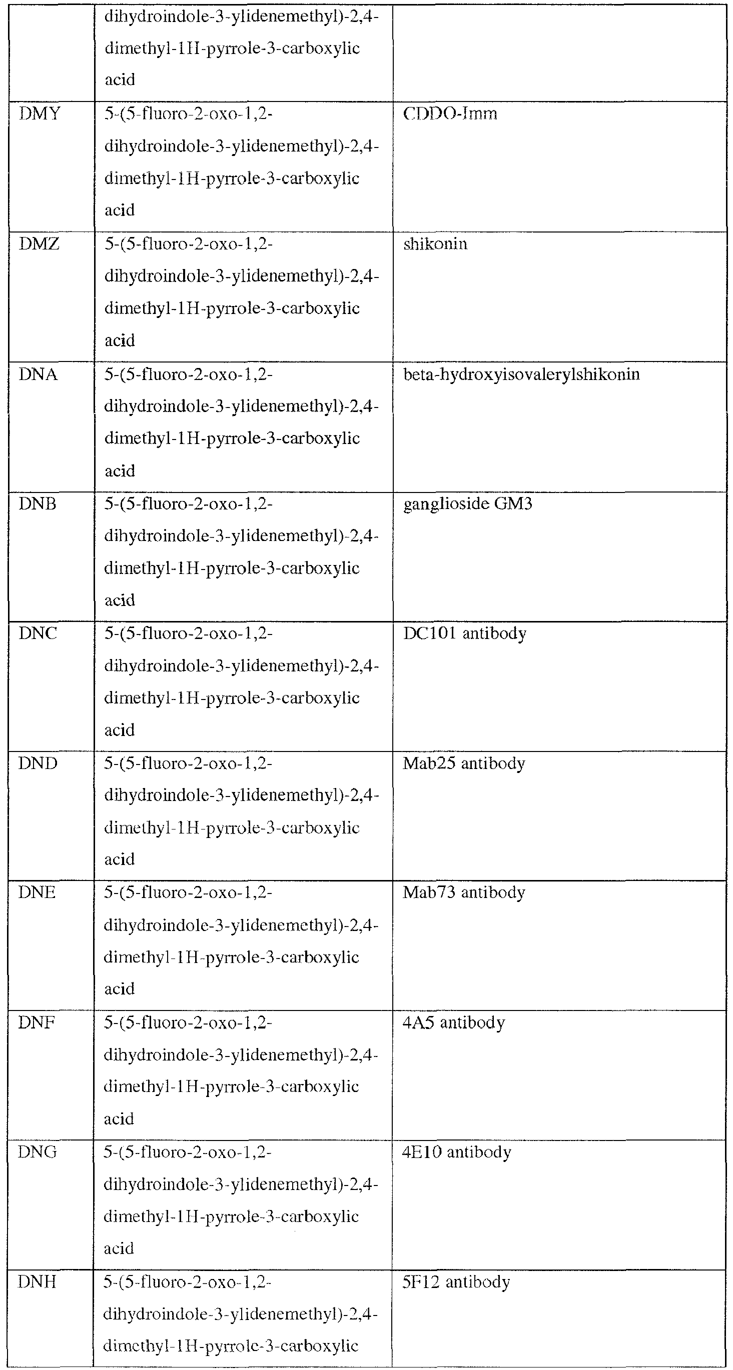

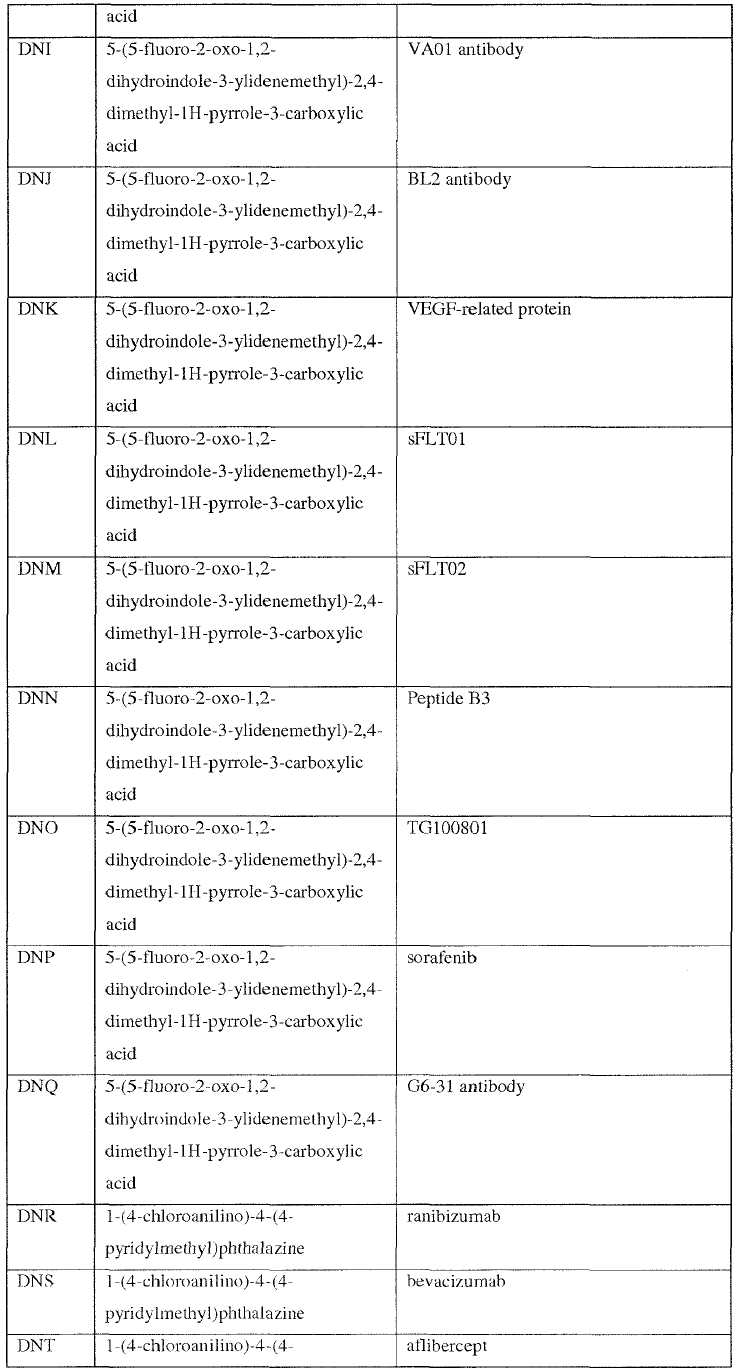

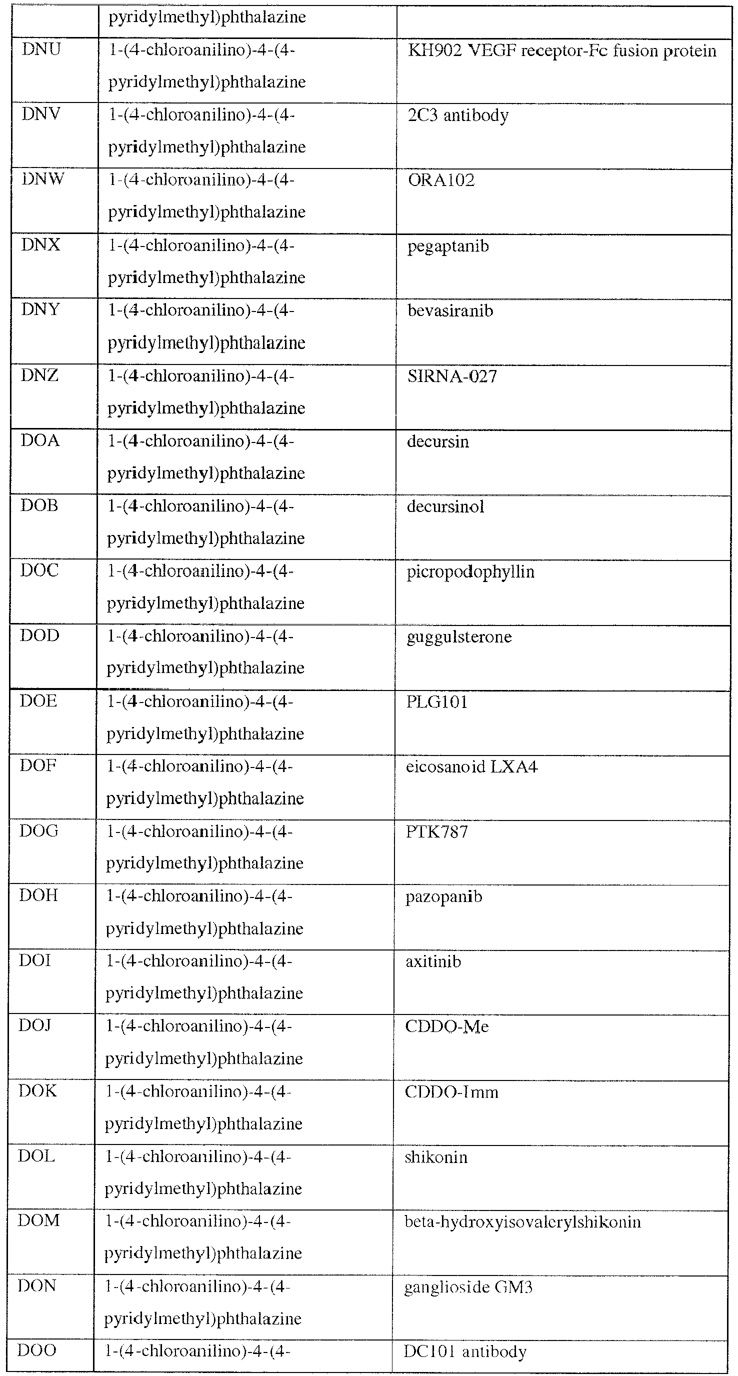

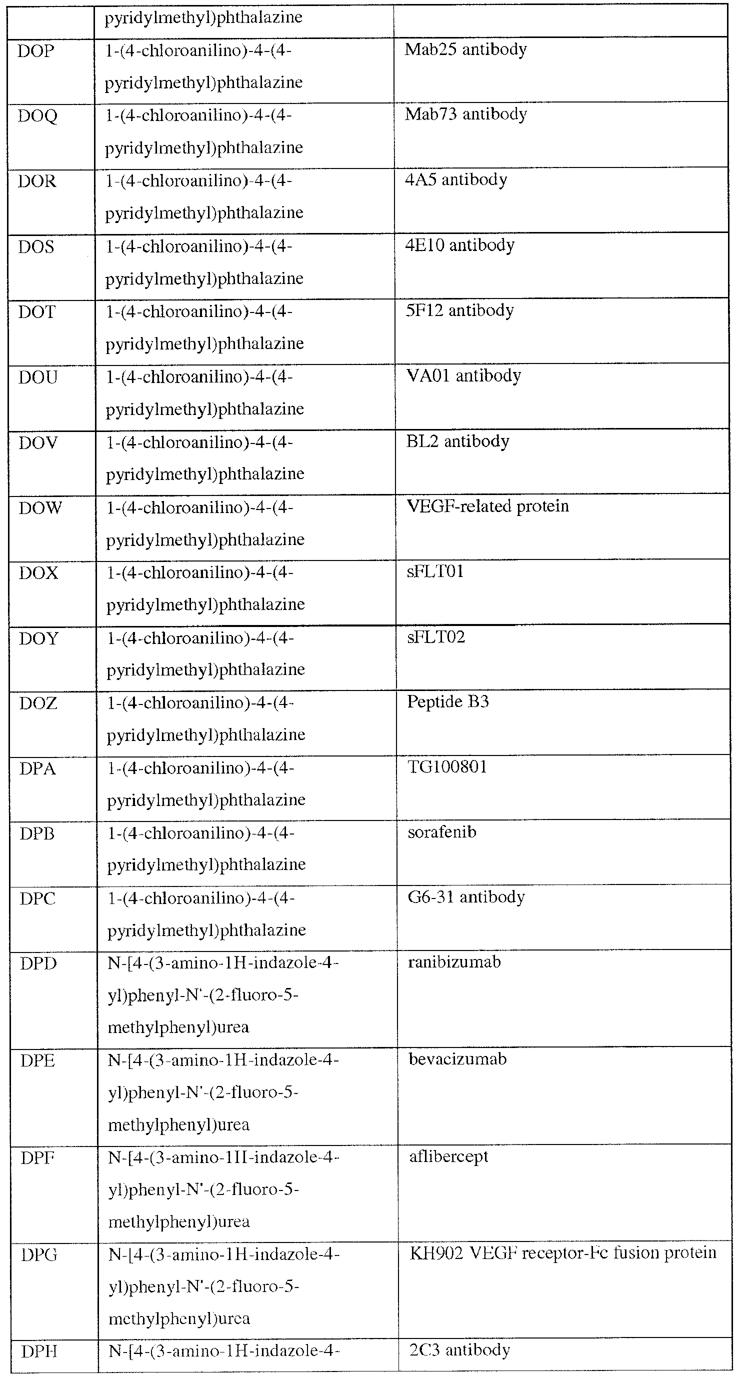

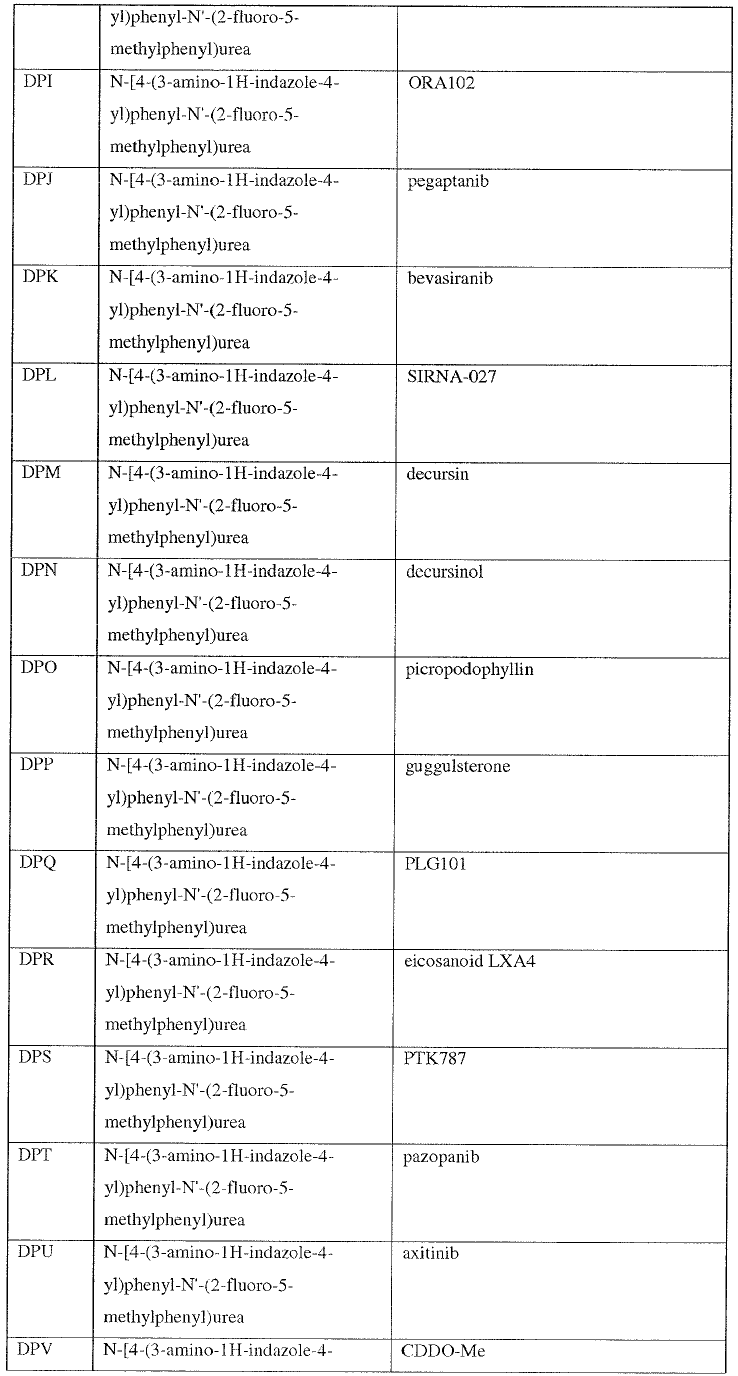

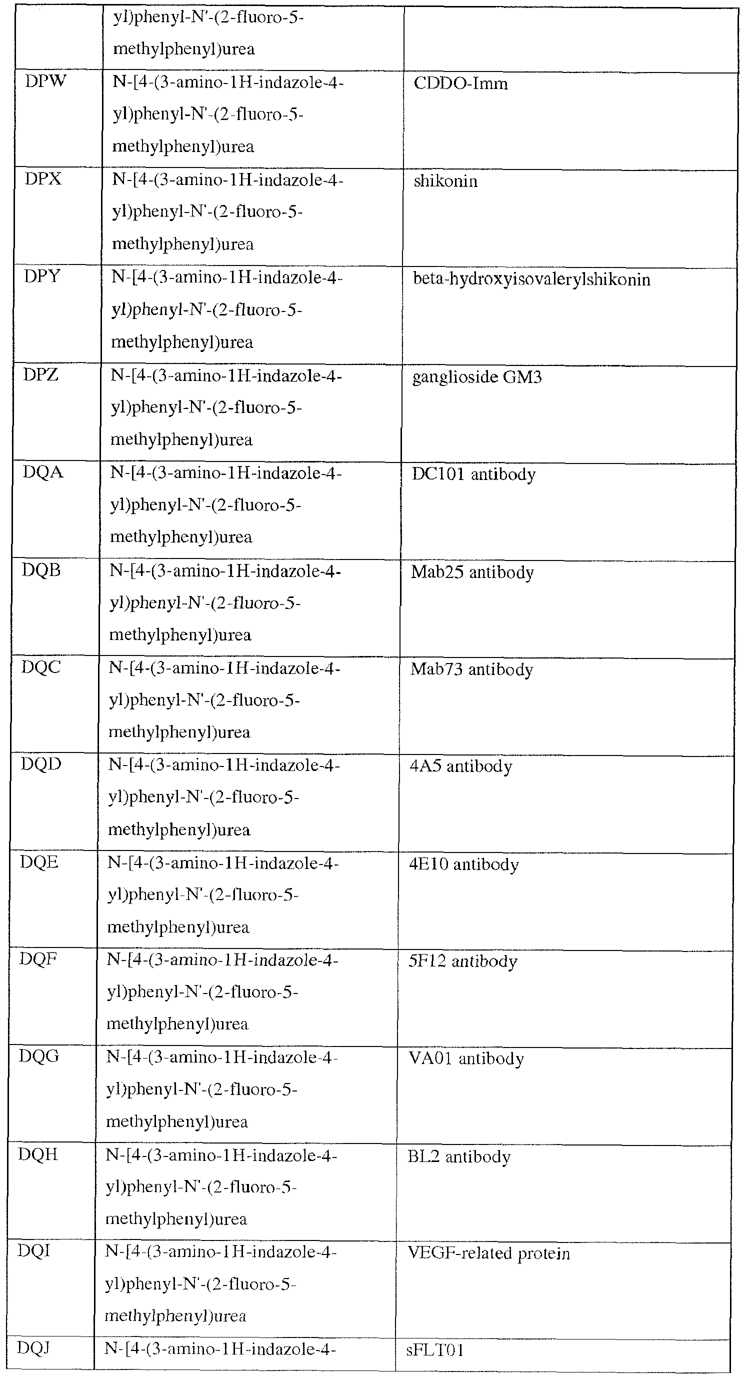

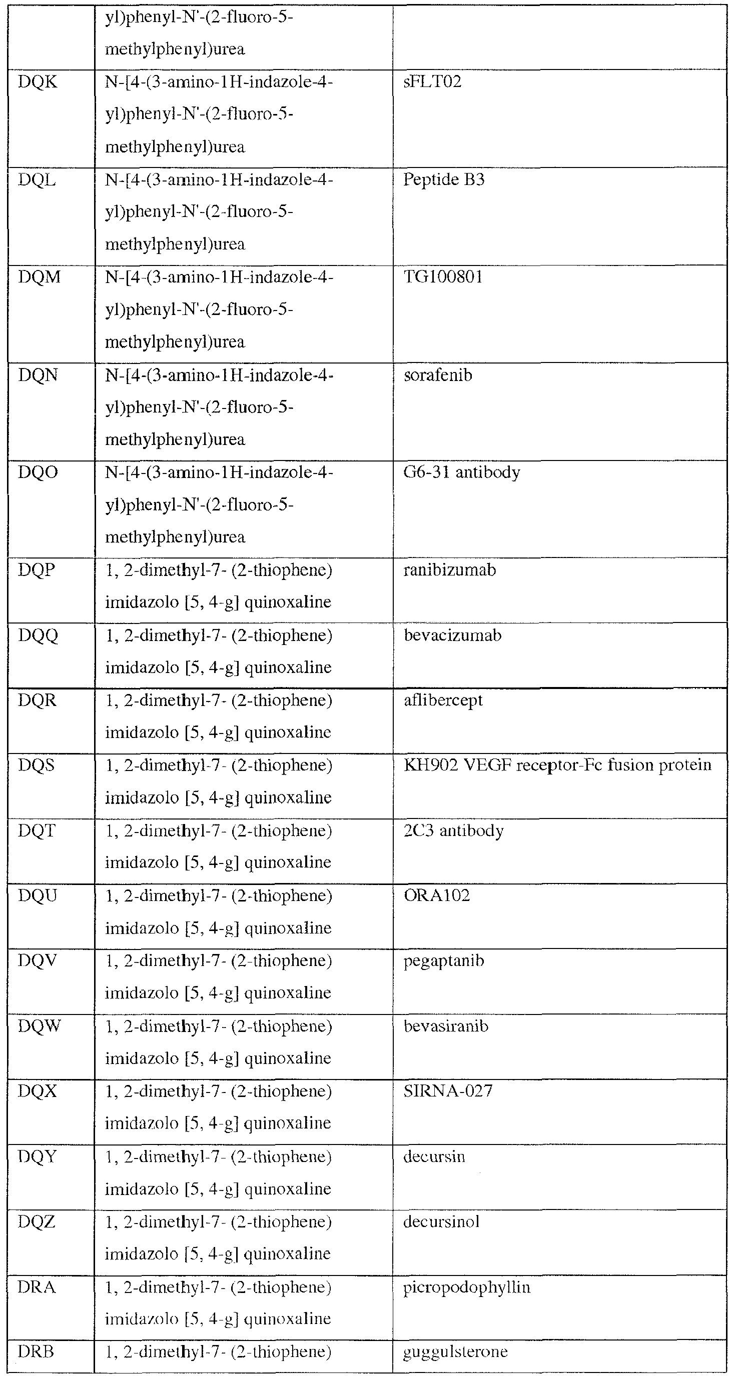

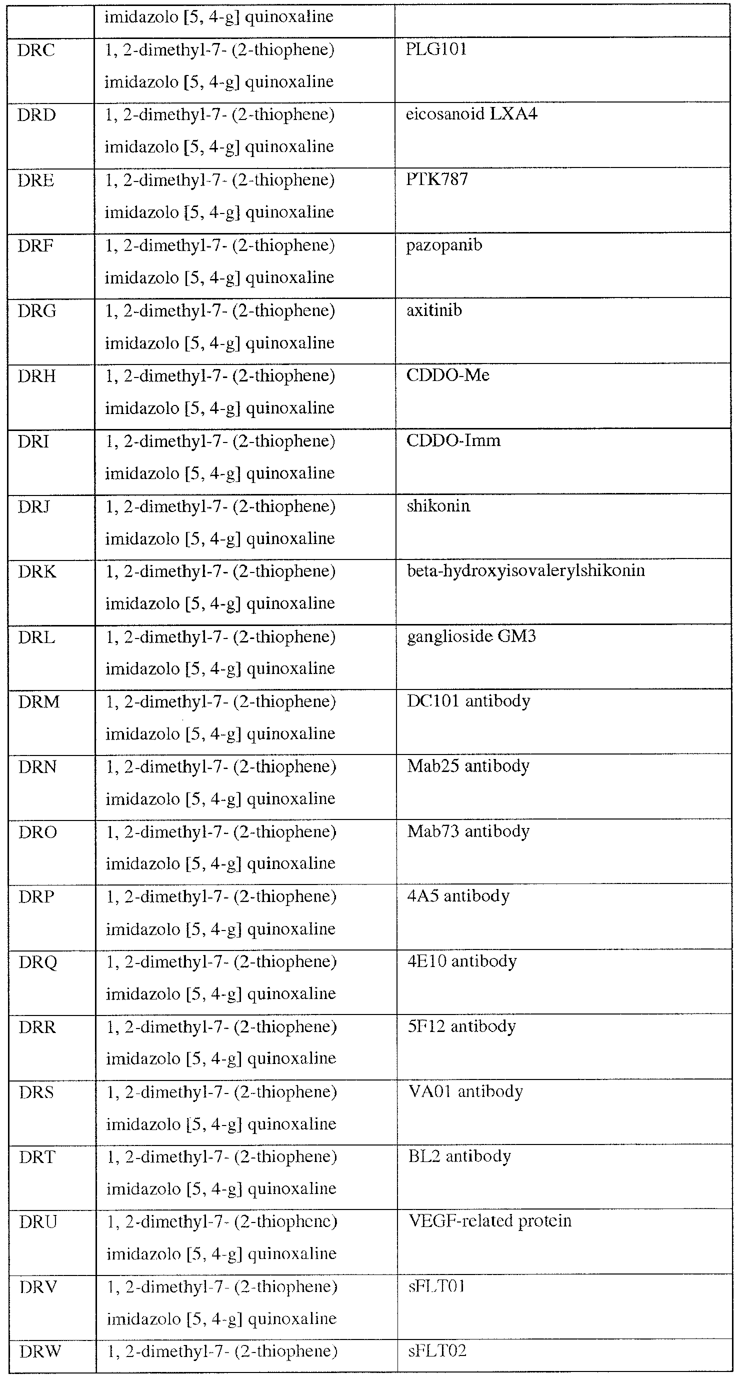

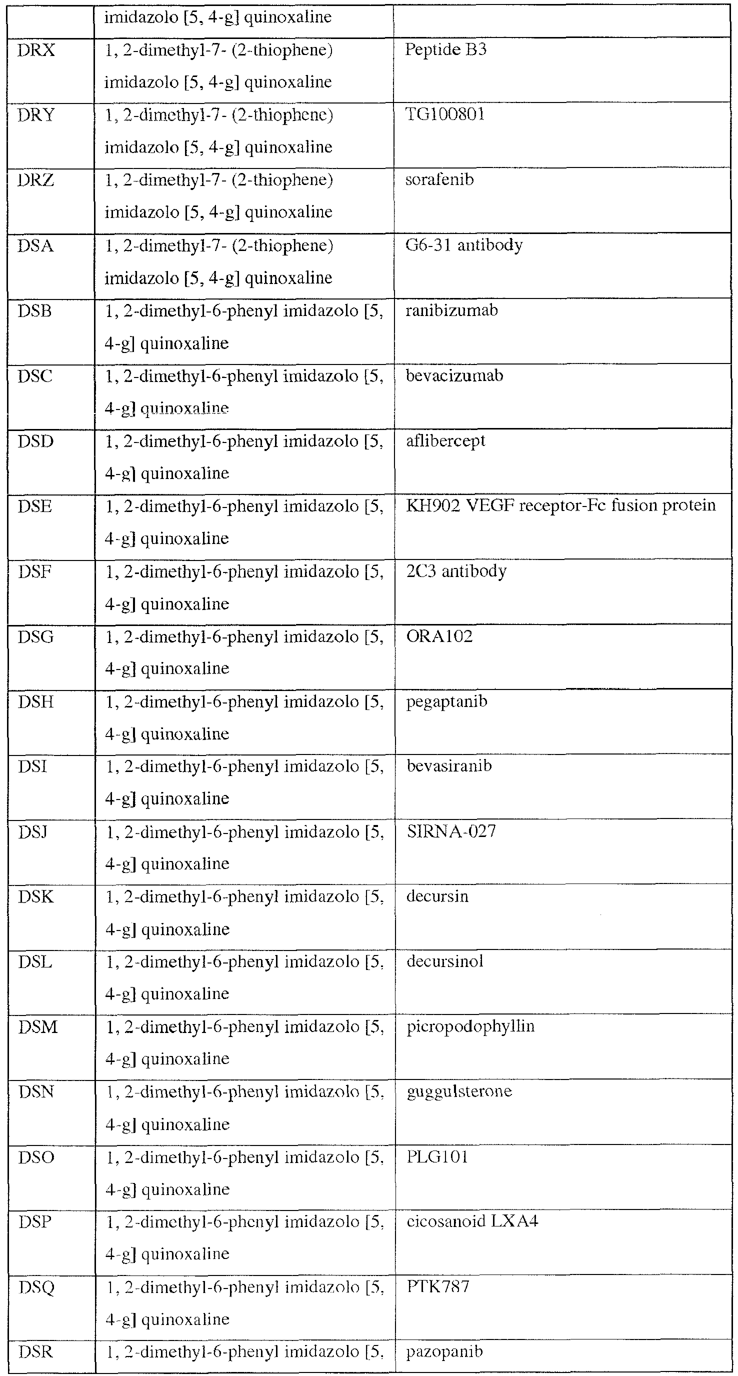

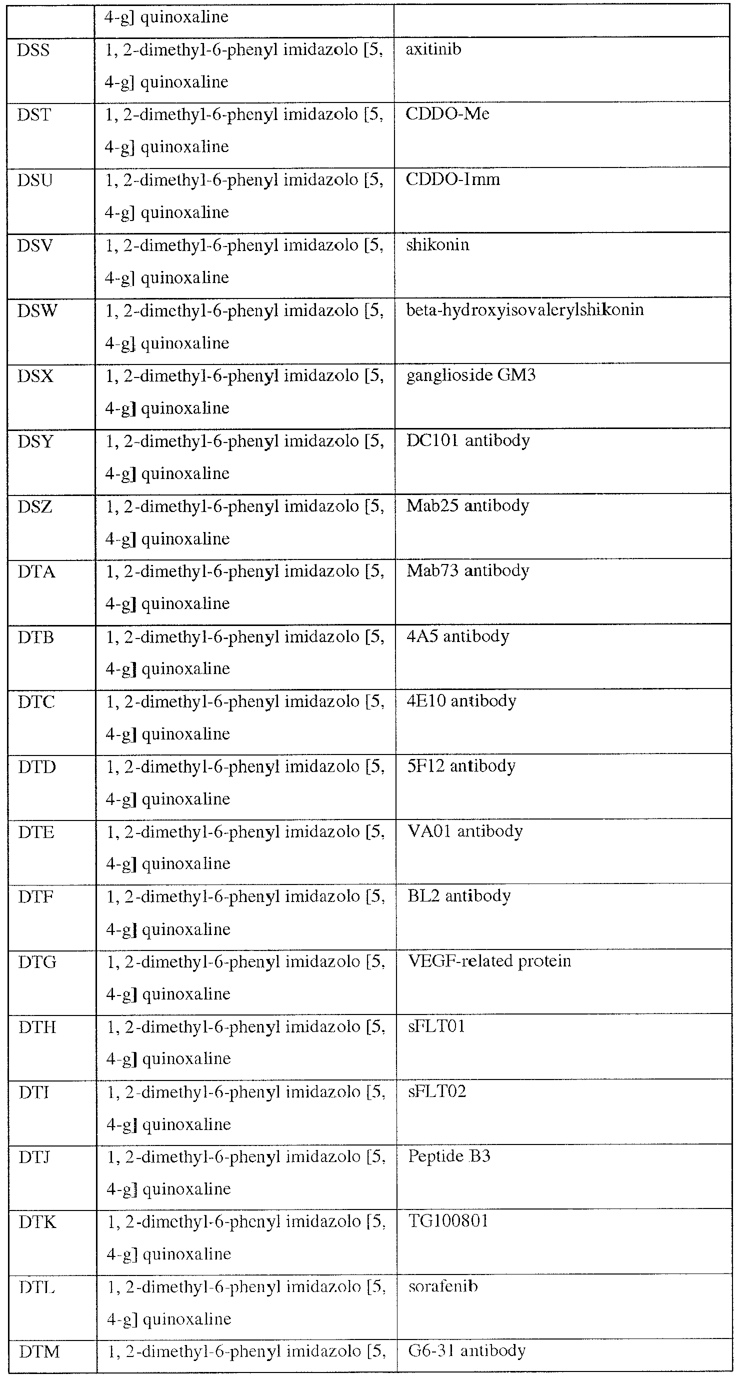

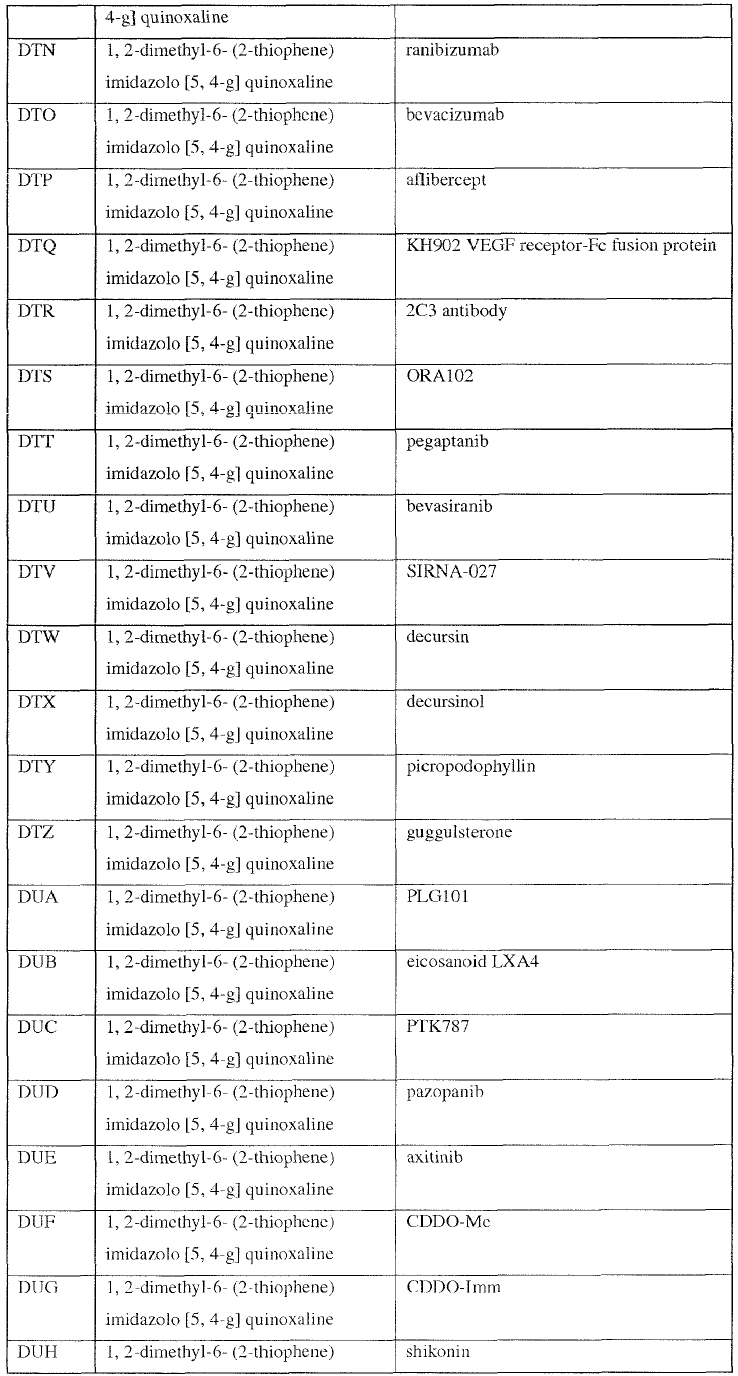

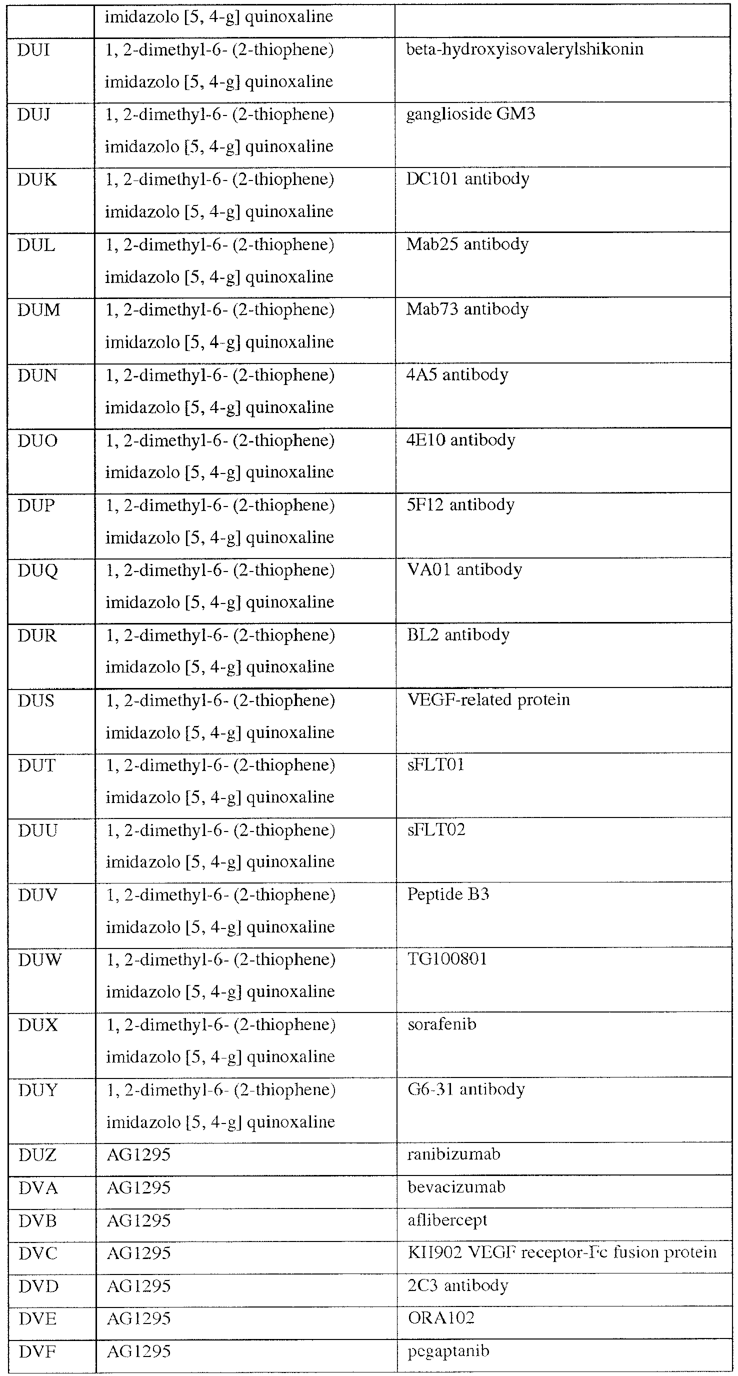

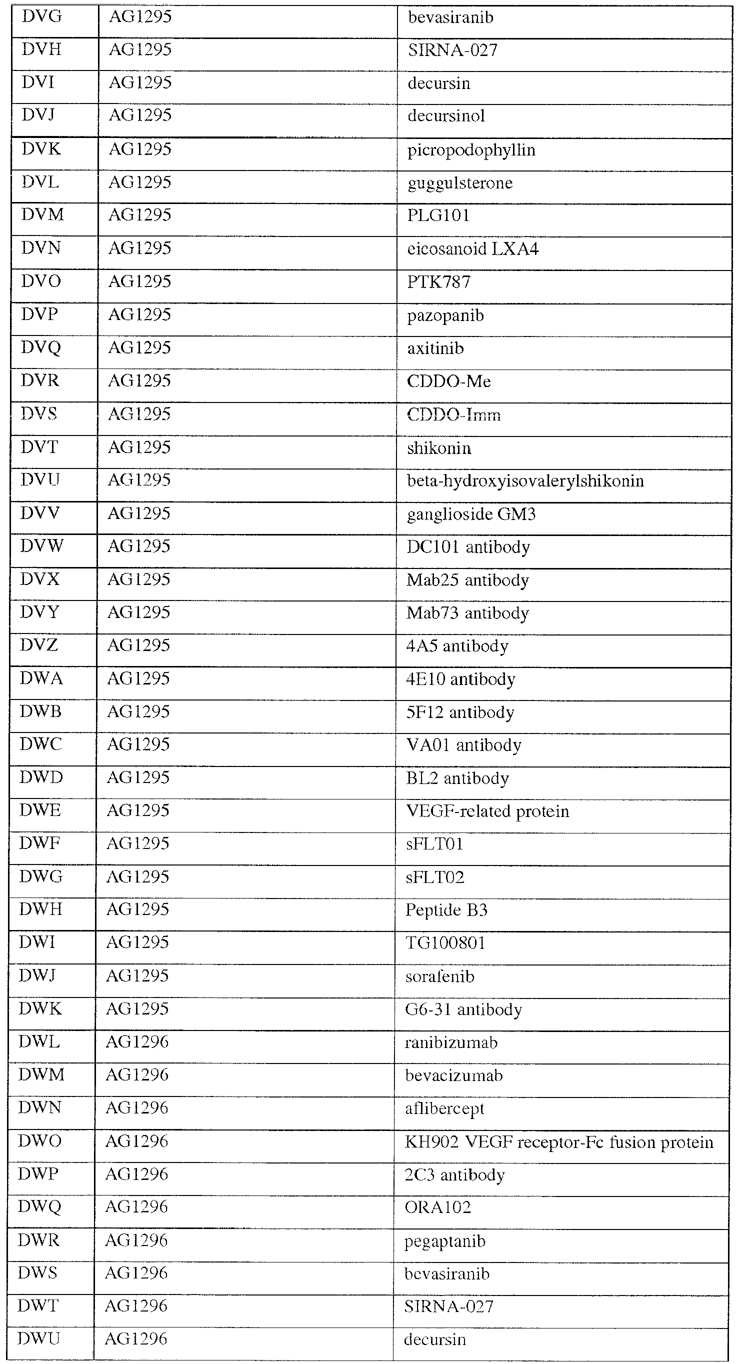

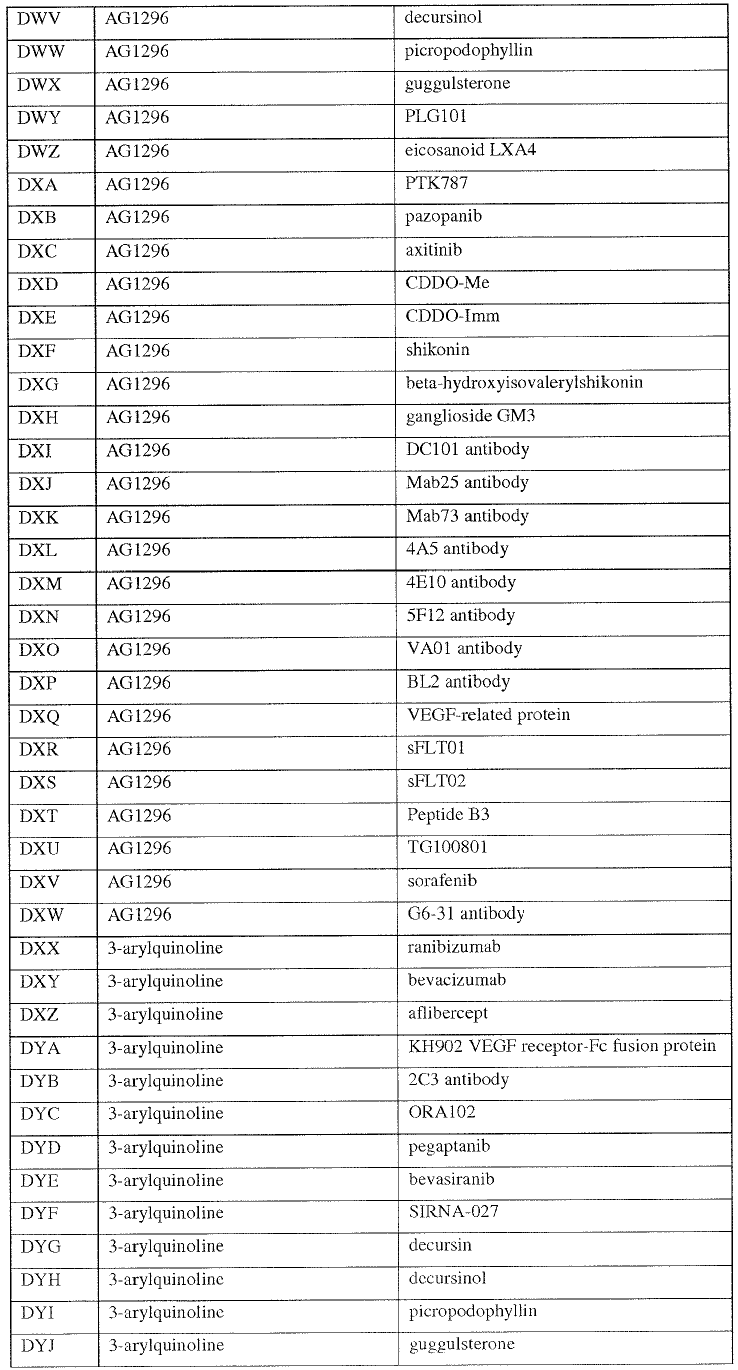

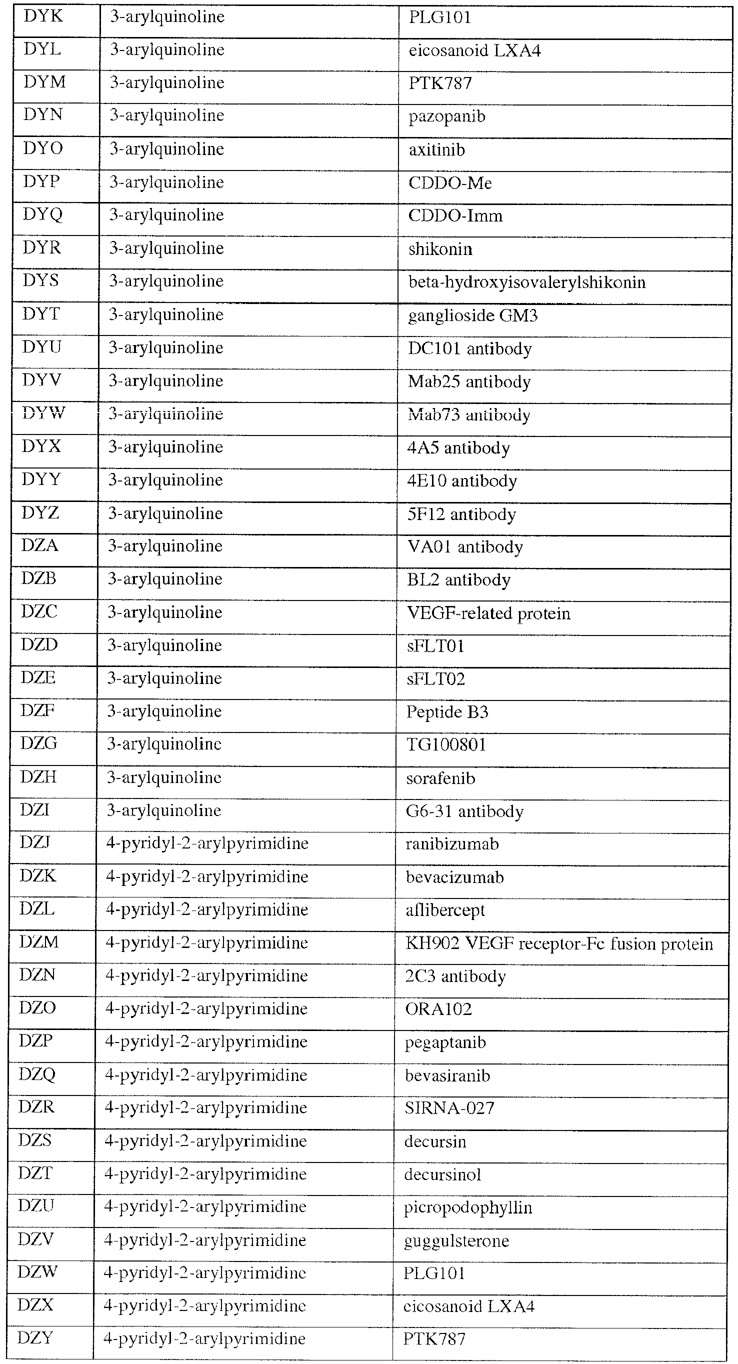

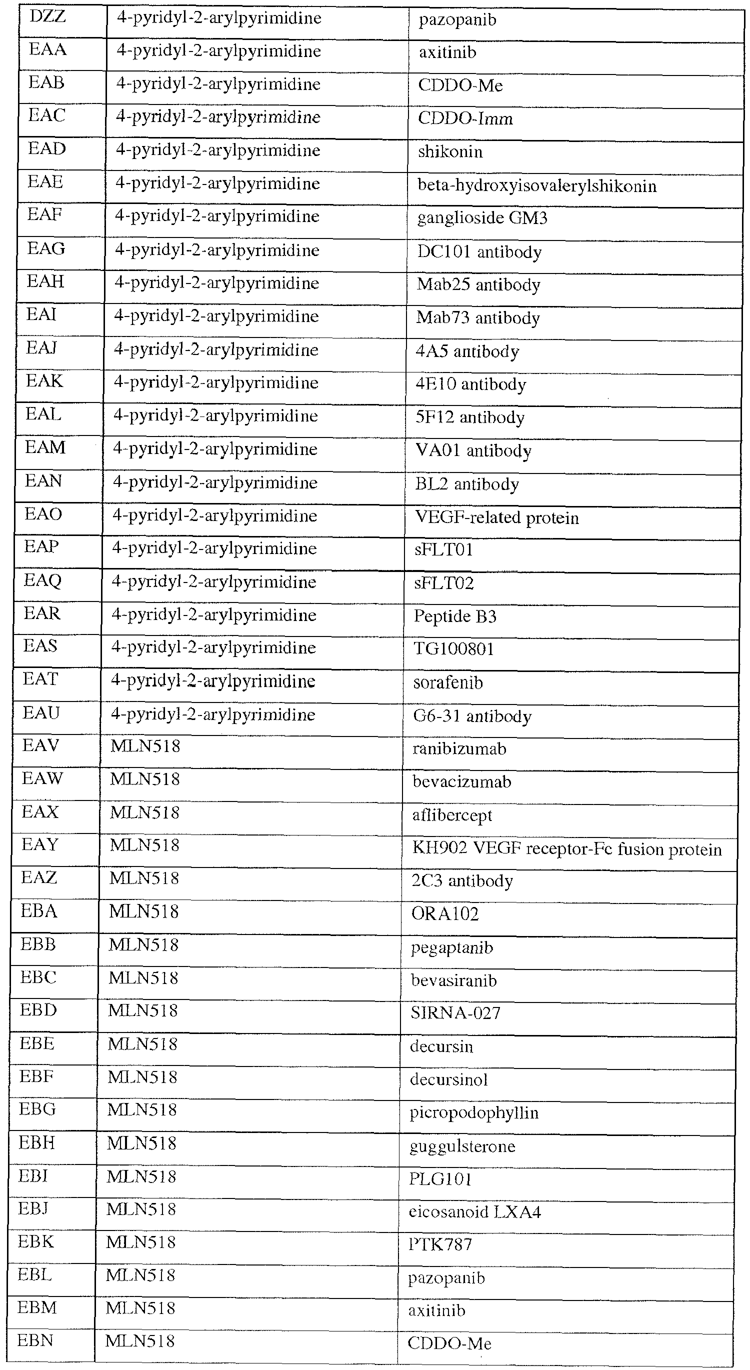

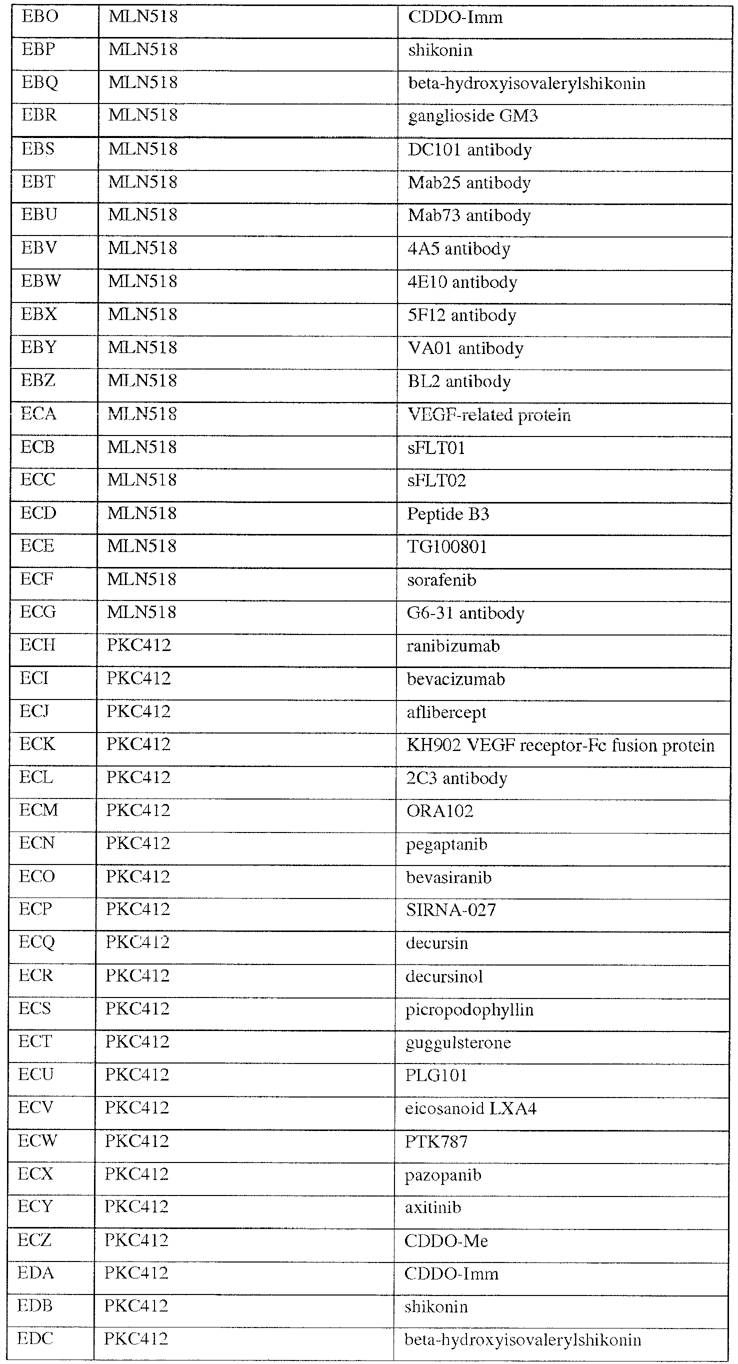

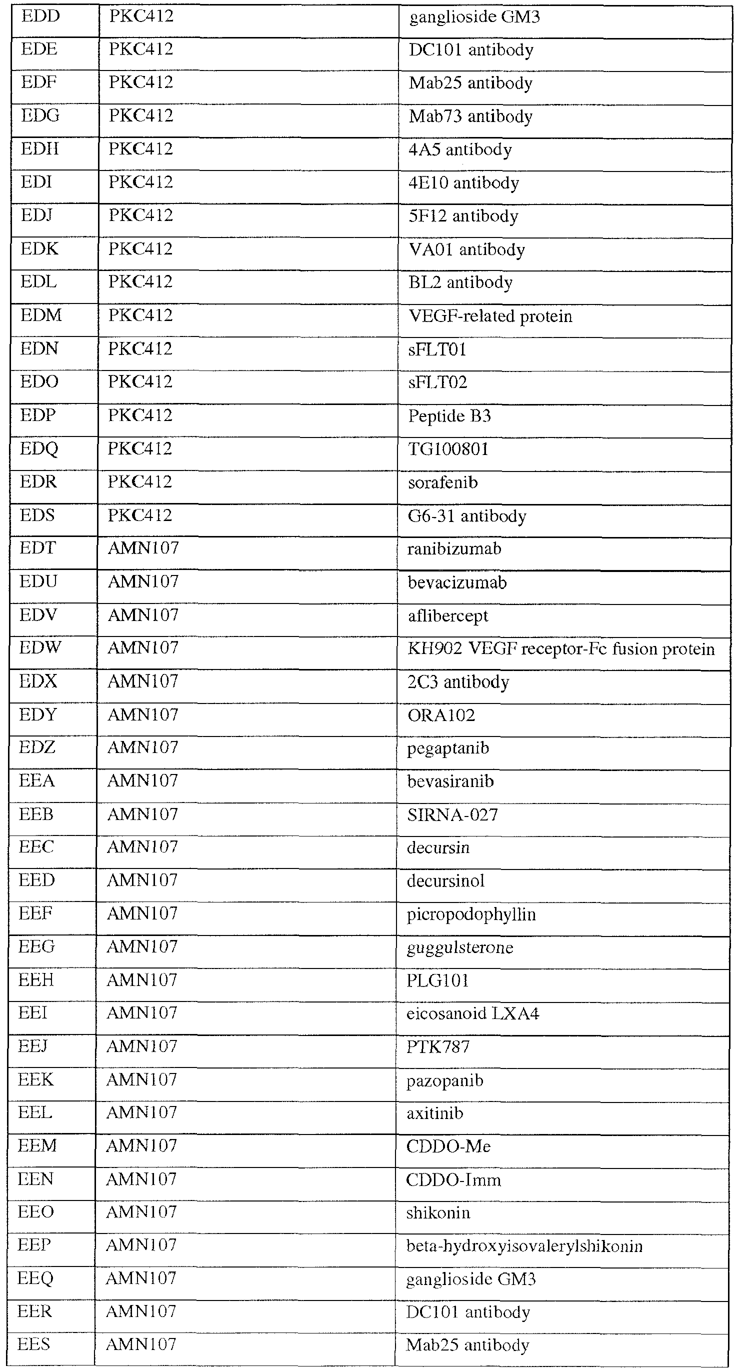

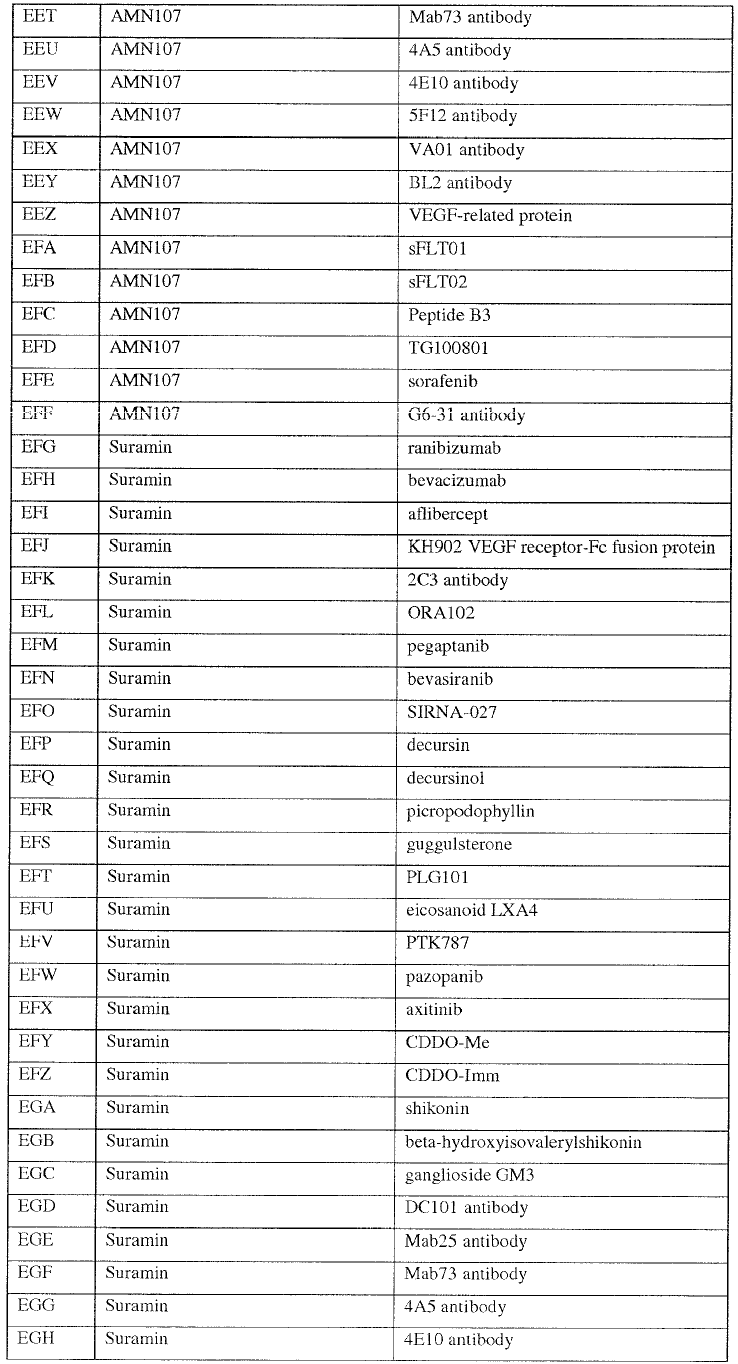

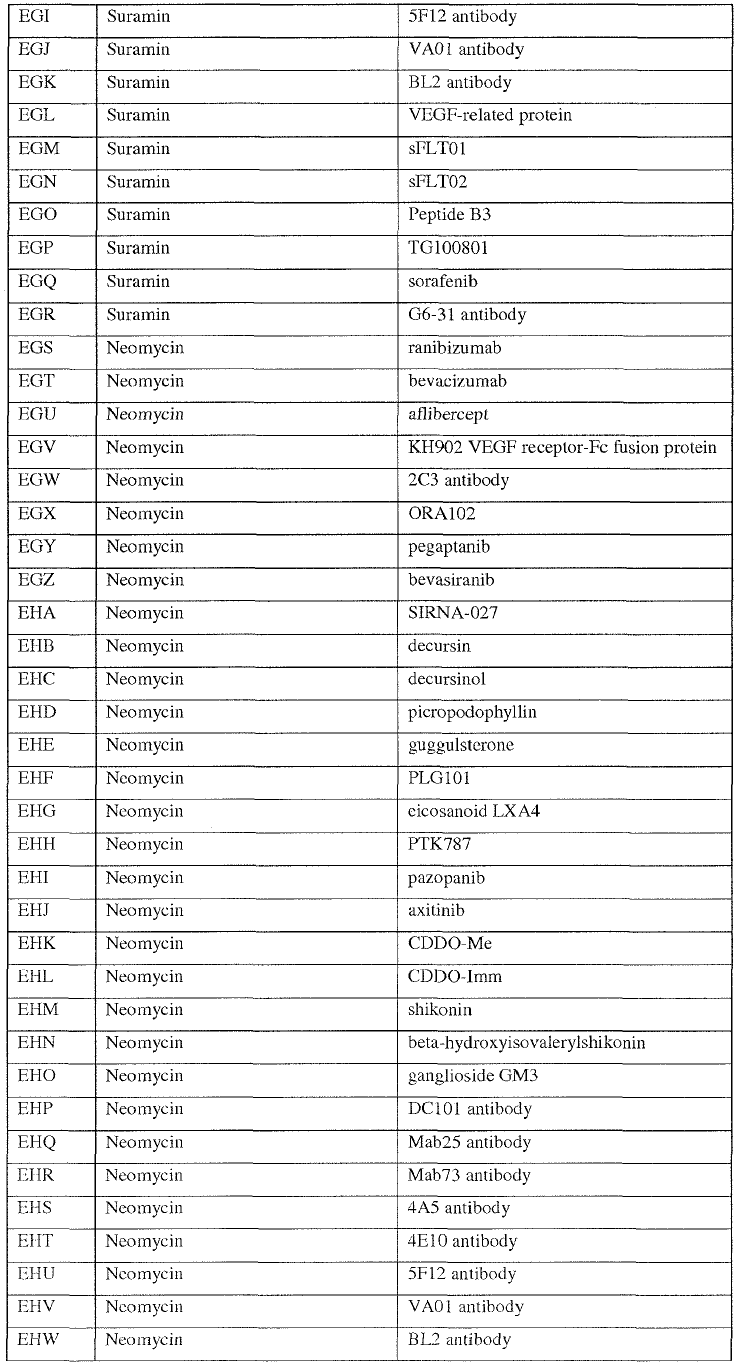

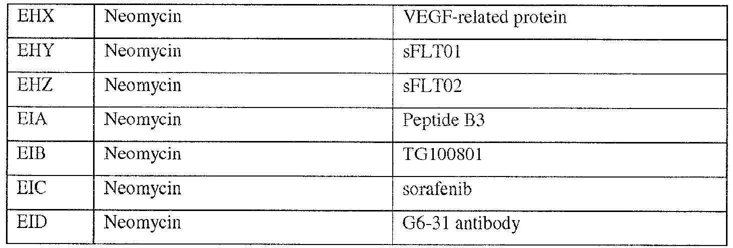

The invention further provides compositions comprising an effective amount of a PDGF antagonist and a VEGF antagonist of Table 1. The compositions are useful for treating or preventing an ophthalmological disease. In another embodiment, the PDGF antagonist and VEGF antagonist are those, respectively, of any of pairs A-EID set forth in Table 2. In a particular embodiment, the PDGF antagonist of the present compositions is Antagonist A or a pharmaceutically acceptable salt thereof. In a particular embodiment, the PDGF antagonist of the present compositions is Antagonist B or a pharmaceutically acceptable salt thereof. In a particular embodiment, the PDGF antagonist of the present compositions is Antagonist C or a pharmaceutically acceptable salt thereof. In a particular embodiment, the PDGF antagonist of the present compositions is Antagonist D or a pharmaceutically acceptable salt thereof. In another embodiment, the VEGF antagonist is ranibizumab, bevacizumab or aflibercept, or a pharmaceutically acceptable salt thereof.

The methods or compositions according to the invention can be administered alone or in conjunction with another therapy and can be provided at home, a doctor's office, a clinic, a hospital's outpatient department, or a hospital. Treatment can begin at a hospital so that the doctor can observe the therapy's effects closely and make any adjustments that are needed. The duration of the administration can depend on the type of ophthalmological disease being treated or prevented, the age and condition of the mammal, the stage and type of the mammal's disease, and how the mammal responds to the treatment. Additionally, a person having a greater risk of developing an ophthalmological disease (*e.g.,* a diabetic patient) can receive treatment to inhibit or delay the onset of symptoms. In one embodiment, the present methods or compositions allow for the administration of a relatively lower dose of each antagonist.

The dosage and frequency of administration of each antagonist can be controlled independently. For example, one antagonist can be administered three times per day, while the other antagonist can be administered once per day. Administration can be performed in on-and-off cycles that include rest periods so that the mammal's body has a chance to recover from a side effect, if any. The antagonists can also be present in the same composition.

### 5.3 Agents useful for treatmnet or prevention of an opthalmological disease

### 5.3.1 PDGF Antagonists

In one embodiment, the PDGF antagonist of Table 1 or 2 is ARC-127. ARC-127 is a 40kD PEGylated, anti-PDGF aptamer having the sequence CAGGCUACGN CGTAGAGCAU CANTGATCCU GT (see Examples 3 and 6 of US Patent Application No. 20050096257, incorporated herein by reference in its entirety) having 2'-fluoro-2'-deoxyuridine at positions 6, 20 and 30; 2'-fluoro-2'-deoxycytidine at positions 8, 21, 28, and 29; 2'-O-Methyl-2'-deoxyguanosine at positions 9, 15, 17, and 31; 2'-O-Methyl-2'-deoxyadenosine at position 22; "N" in positions 10 and 23 from a hexaethylene-glycol phosphoramidite; and an inverted orientation T (i.e., 3'-3'-linked) at position 32.

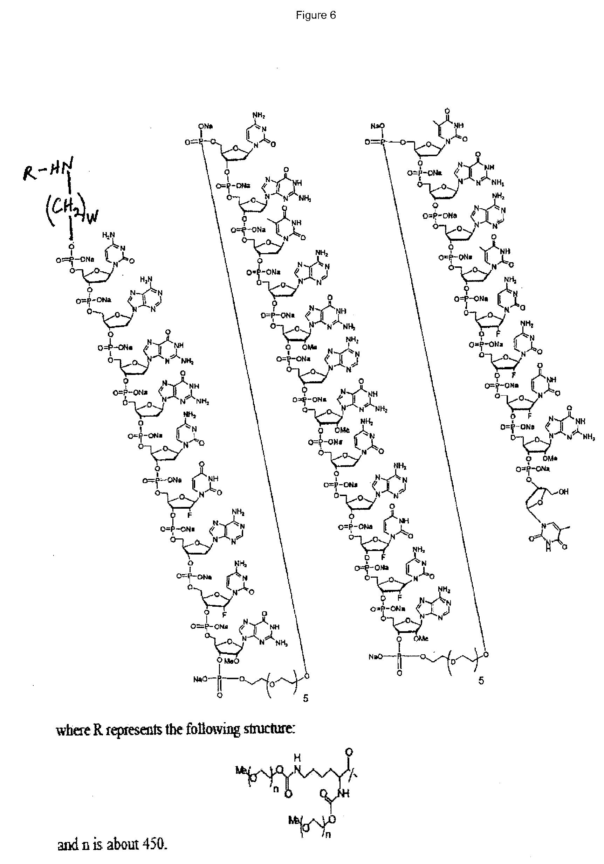

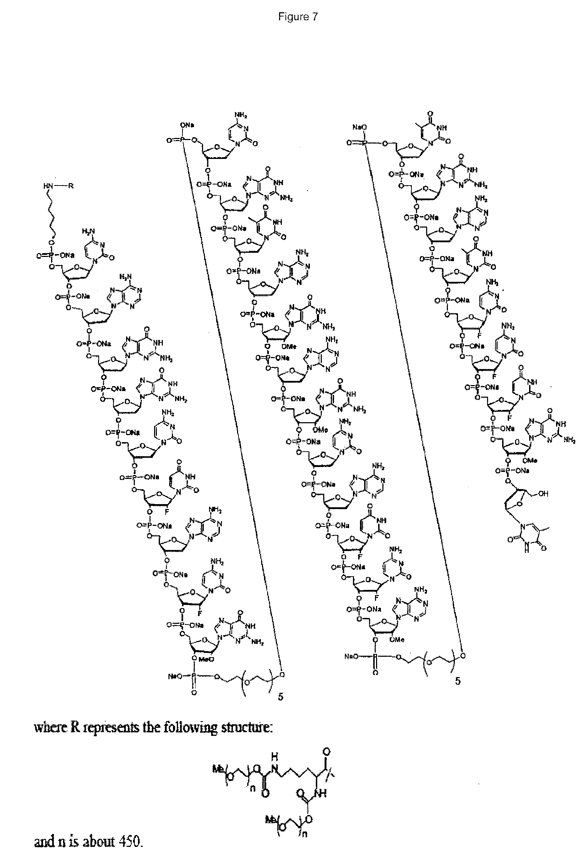

In another embodiment, the PDGF antagonist of Table 1 or 2 is a compound of Formula A (see Figure 6), wherein w is an integer from 2 to 12. In another embodiment, the PDGF antagonist is a compound of Formula A, wherein w is an integer from 4 to 10. In another embodiment, the PDGF antagonist is a compound of Formula A, wherein w is 5, 6, 7, or 8. In one embodiment, the PDGF antagonist is a compound of Formula A, wherein w is 5. In another embodiment, the PDGF antagonist is a compound of Formula A, wherein w is 6. In another embodiment, the PDGF antagonist is a compound of Formula A, wherein w is 7. In another embodiment, the PDGF antagonist is a compound of Formula A, wherein w is 8. In one embodiment, the PDGF antagonist has the structure of Figure 7.

In another embodiment, the PDGF antagonist of Table 1 or 2 is Antagonist A or a pharmaceutically acceptable salt thereof. The chemical name of Antagonist A is [(monomethoxy 20K polyethylene glycol carbamoyl-N2-) (monomethoxy 20K polyethylene glycol carbamoyl-N6-)]-lysine-amido-6-hexandilyl-(1-5')-2'-deoxycytidylyl-(3'-5')-2'-deoxyadenylyl-(3'-5')-2'-deoxyguanylyl-(3'-5')-2'-deoxyguanylyl-(3'-5')-2'-deoxycytidylyl-(3'-5')-2'-deoxy-2'-fluorouridylyl-(3'-5')-2'-deoxyadenylyl-(3'-5')-2'- deoxy-2'-fluorocytidylyl-(3'-5')-2'-deoxy-2'-methoxyguanylyl-(3'-1)-PO₃-hexa(ethyloxy)-(18-5')-2'-deoxycytidylyl-(3'-5')-2'-deoxyguanylyl-(3'-5')- thymidylyl-(3'-5')-2'-deoxyadenylyl-(3'-5')-2'- deoxy-2'-methoxyguanylyl-(3'-5')-2'-deoxyadenylyl-(3'-5')-2'- deoxy-2'-methoxyguanylyl-(3'-5')-2'-deoxycytidylyl-(3'-5')-2'-deoxyadenylyl-(3'-5')-2'- deoxy-2'-fluorouridylyl-(3'-5')-2'- deoxy-2'-fluorocytidylyl-(3'-5')-2'- deoxy-2'-methoxyadenylyl-(3'-1)- PO₃-hexa(ethyloxy)-(18-5')-thymidylyl-(3'-5')-2'-deoxyguanylyl-(3'-5')-2'-deoxyadenylyl-(3'-5')-thymidylyl-(3'-5')-2'- deoxy-2'-fluorocytidylyl-(3'-5')-2'-deoxy-2'-fluorocytidylyl-(3'-5')-2'-deoxy-2'-fluorouridylyl-(3'-5')-2'-methoxyguanylyl-(3'-3')-thymidine.

The structure of Antagonist A is shown in Figure 7.

The sequence of Antagonist A is: 
5'-[mPEG2 40kD]- [(HN-(CH2)6O] CAGGCUfACcfGm [(PO3(CH2CH2O)6] CGTAGmAGmCAUfCfAm
[O(CH2CH2O)6] TGATCfCfUfGm-iT-3'
 where:
[mPEG2 40 kD] represents two 20 kD polyethylene glycol (PEG) polymer chains, in one embodiment two about 20 kD PEG polymer chains, that are covalently attached to the two amino groups of a lysine residue via carbamate linkages. This moiety is in turn linked with the oligonucleotide via the amino linker described below.
[(HN-(CH₂)₆O] represents a bifunctional α-hydroxy-ω-amino linker that is covalently attached to the PEG polymer via an amide bond. The linker is attached to the oligonucleotide at the 5'-end of Antagonist A by a phosphodiester linkage.
[PO₃(CH₂CH₂O)₆] represents the hexaethylene glycol (HEX) moieties that join segments of the oligonucleotide via phosphodiester linkages. Antagonist A has two HEX linkages that join together the 9^{th} and 10^{th} nucleotides and 21^{st} and 22^{nd} nucleotides via phosphodiester linkages between the linker and the respective nucleotides.
C, A, G, and T represent the single letter code for the 2'-deoxy derivatives of cytosine, adenosine, guanosine, and thymidine nucleic acids, respectively. Antagonist A has four 2'-deoxyribocytosine, six 2'-deoxyriboadenosine, four 2'-deoxyriboguanosine, and four 2'-deoxyribothymidine.
Gₘ and Aₘ represent 2'-methoxy substituted forms of guanosine and adenosine, respectively. Antagonist A has four 2'-methoxyguanosines and one 2'-methoxyadenosine. C_{f} and U_{f} represent the 2'-fluoro substituted forms of cytosine and uridine, respectively. Antagonist A has four 2'-fluorocytosines and three 2'-fluorouridines.

The phosphodiester linkages in the oligonucleotide, with the exception of the 3'-terminus, connect the 5'-and 3'-oxygens of the ribose ring with standard nucleoside phosphodiester linkages. The phosphodiester linkage between the 3'-terminal thymidine and the penultimate Gₘ links their respective 3'-oxygens, which is referred to as the 3',3'-cap.

Antagonist A has a molecular weight from 40,000 to 60,000 Daltons, in one embodiment from about 40,000 to about 60,000 Daltons, and can be colorless to slightly yellow in solution. Antagonist A can be present in a solution of monobasic sodium phosphate monohydrate and dibasic sodium phosphate heptahydrate as buffering agents and sodium chloride as a tonicity adjuster. Antagonist A is a hydrophilic polymer. The Antagonist A sodium salt is soluble in water and in phosphate-buffered saline (PBS), as assessed by visual inspection, to at least 50 mg (based on oligonucleotide weight)/mL solution.

In one embodiment, Antagonist A is manufactured using an iterative chemical synthesis procedure to produce the oligonucleotide portion, which is then covalently bonded to a pegylation reagent, as further described in Example 4.

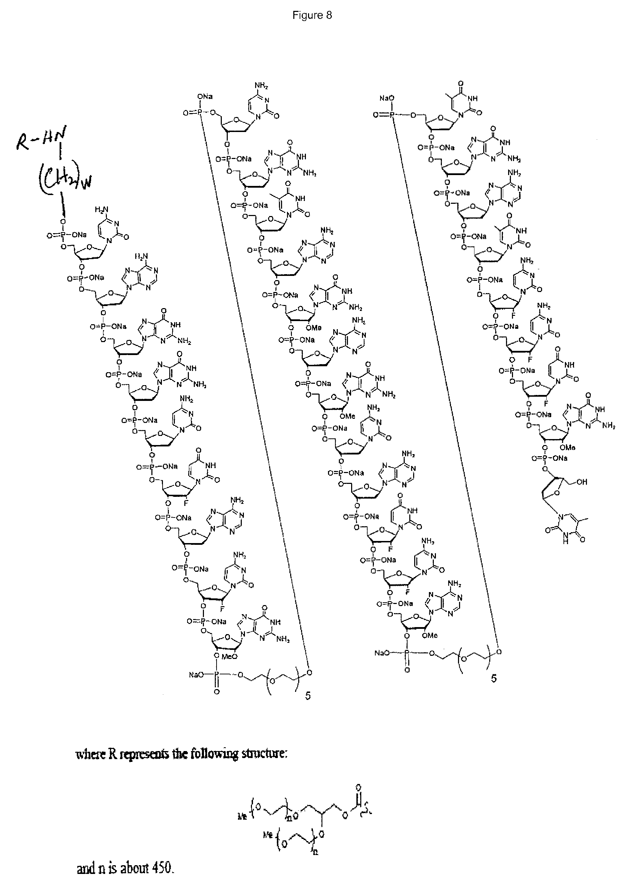

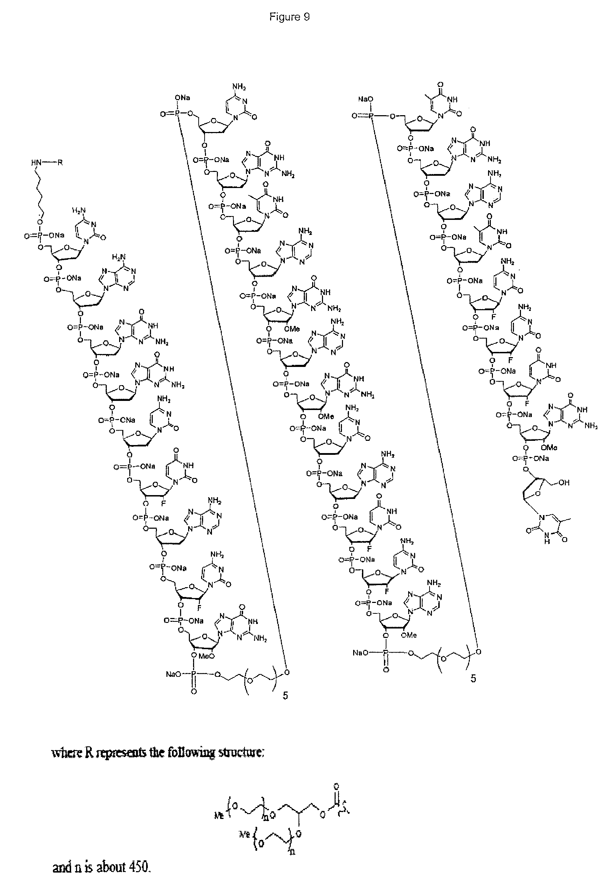

In another embodiment, the PDGF antagonist of Table 1 or 2 is a compound of Formula B (see Figure 8), wherein w is an integer from 2 to 12. In another embodiment, the PDGF antagonist is a compound of Formula B, wherein w is an integer from 4 to 10. In another embodiment, the PDGF antagonist is a compound of Formula B, wherein w is 5, 6, 7, or 8. In one embodiment, the PDGF antagonist is a compound of Formula B, wherein w is 5. In another embodiment, the PDGF antagonist is a compound of Formula B, wherein w is 6. In another embodiment, the PDGF antagonist is a compound of Formula B, wherein w is 7. In another embodiment, the PDGF antagonist is a compound of Formula B, wherein w is 8. In one embodiment, the PDGF antagonist is a compound of Formula B having two 20 kD polyethylene glycol (PEG) polymer chains. In one embodiment, the PDGF antagonist is a compound of Formula B having an α-hydroxy-ω-amino group. In one embodiment, the α-hydroxy-ω-amino group is attached to the oligonucleotide by a phosphodiester linkage. In one embodiment, the α-hydroxy-ω-amino group is attached at the 5'-end of the oligonucleotide. In one embodiment, the PDGF antagonist is a compound of Formula B having hexaethylene glycol (HEX) moieties that join segments of the oligonucleotide via phosphodiester linkages. In one embodiment, the PDGF antagonist hexaethylene glycol (HEX) moieties join together the 9th and 10th nucleotides and 21st and 22nd nucleotides of the oligonucleotide via phosphodiester linkages between the linker and the respective nucleotides. In one embodiment, the PDGF antagonist has the structure of Figure 9.

In another embodiment, the PDGF antagonist of Table 1 or 2 is Antagonist B or a pharmaceutically acceptable salt thereof.

The structure of Antagonist B is shown in Figure 9.

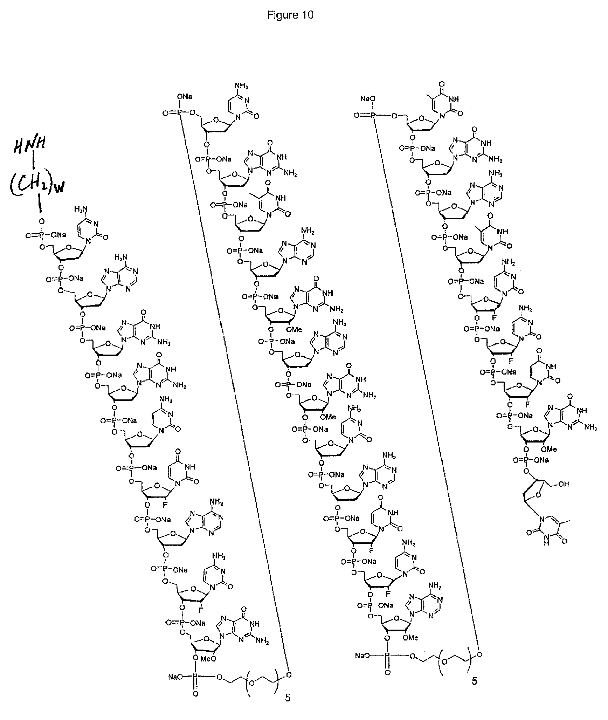

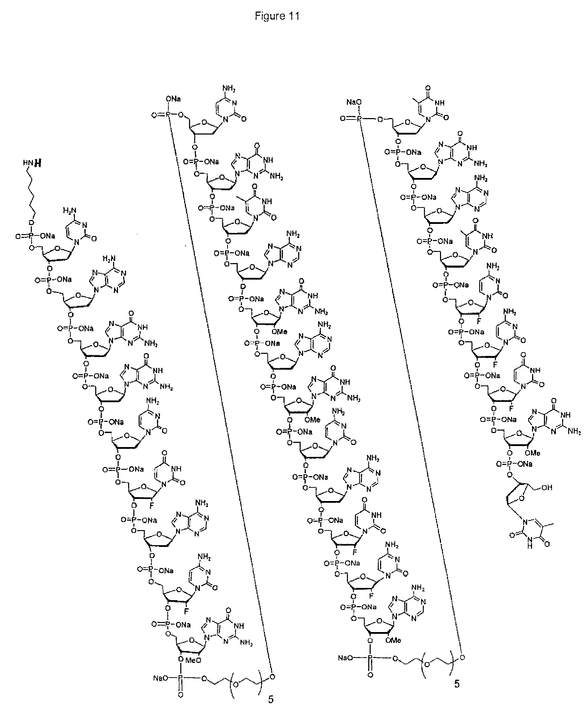

In another embodiment, the PDGF antagonist of Table 1 or 2 is a compound of Formula C (see Figure 10), wherein w is an integer from 2 to 12. In another embodiment, the PDGF antagonist is a compound of Formula C, wherein w is an integer from 4 to 10. In another embodiment, the PDGF antagonist is a compound of Formula C, wherein w is 5, 6, 7, or 8. In one embodiment, the PDGF antagonist is a compound of Formula C, wherein w is 5. In another embodiment, the PDGF antagonist is a compound of Formula C, wherein w is 6. In another embodiment, the PDGF antagonist is a compound of Formula C, wherein w is 7. In another embodiment, the PDGF antagonist is a compound of Formula C, wherein w is 8. In one embodiment, the PDGF antagonist is a compound of Formula C having an α-hydroxy-ω-amino group. In one embodiment, the α-hydroxy-ω-amino group is attached to the oligonucleotide by a phosphodiester linkage. In one embodiment, the α-hydroxy-ω-amino group is attached at the 5'-end of the oligonucleotide. In one embodiment, the PDGF antagonist is a compound of Formula C having hexaethylene glycol (HEX) moieties that join segments of the oligonucleotide via phosphodiester linkages. In one embodiment, the PDGF antagonist hexaethylene glycol (HEX) moieties join together the 9th and 10th nucleotides and 21st and 22nd nucleotides of the oligonucleotide via phosphodiester linkages between the linker and the respective nucleotides. In one embodiment, the PDGF antagonist has the structure of Figure 11.

In another embodiment, the PDGF antagonist of Table 1 or 2 is Antagonist C or a pharmaceutically acceptable salt thereof.

The structure of Antagonist C is shown in Figure 11.

The phosphodiester linkages in the oligonucleotide, with the exception of the 3'-terminus, connect the 5'-and 3'-oxygens of the ribose ring with standard nucleoside phosphodiester linkages. The phosphodiester linkage between the 3'-terminal thymidine and the penultimate Gₘ links their respective 3'-oxygens, which is referred to as the 3',3'-cap.

In another embodiment, the PDGF antagonist of Table 1 or 2 is Antagonist D or a pharmaceutically acceptable salt thereof.

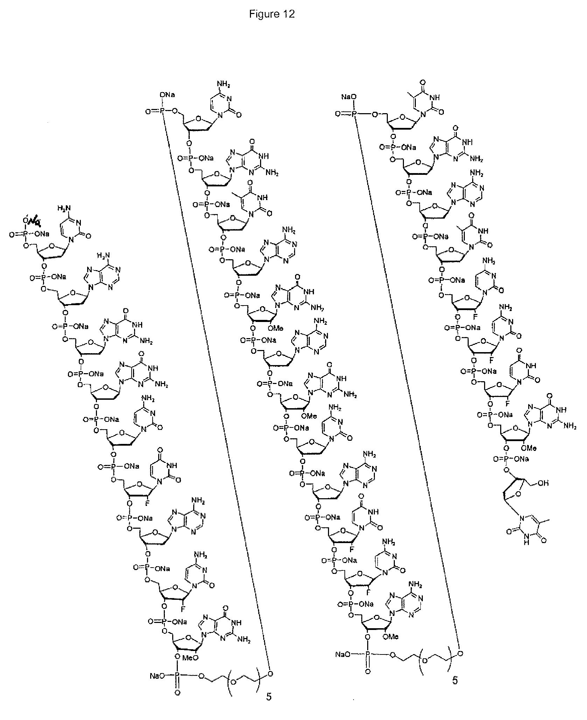

The structure of Antagonist D is shown in Figure 12.

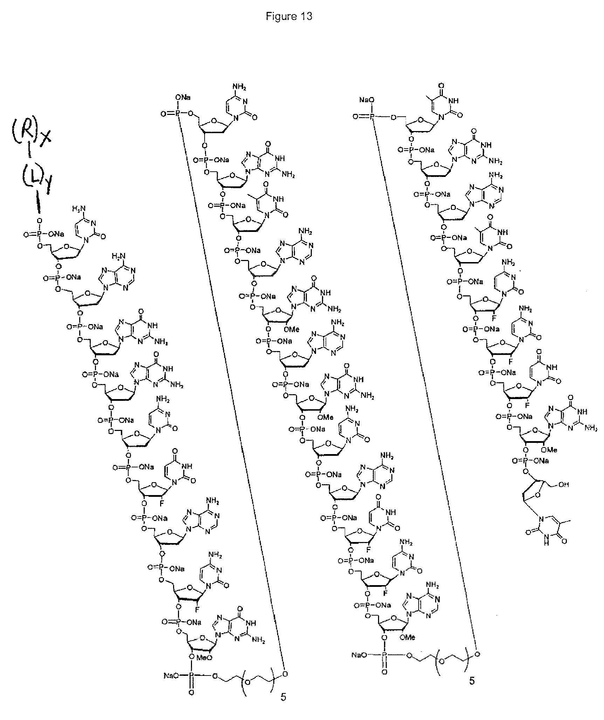

In another embodiment, the PDGF antagonist of Table 1 or 2 is a compound of Formula E (see Figure 13), wherein L is a linker, Y is 0 or 1, R is a nonphysiologically active group, lipophilic group or High Molecular Weight Compound, and X is an integer ranging from 1 to 4.

In another embodiment, the PDGF antagonist of Table 1 or 2 is the antibody 1B3 or a pharmaceutically acceptable salt thereof (US Patent Publication No. 20090053241 (paragraph 0073 and Table 1), which is hereby incorporated by reference in its entirety).

In another embodiment, the PDGF antagonist of Table 1 or 2 is the antibody CDP860 or a pharmaceutically acceptable salt thereof (Serruys et al. (2003) Int. J. Cardiovasc Intervent. 5:214-22, which is hereby incorporated by reference in its entirety).

In another embodiment, the PDGF antagonist of Table 1 or 2 is the antibody IMC-3G3 or a pharmaceutically acceptable salt thereof (Dolloff et al. (2007) Cancer Res. 67:555-62, which is hereby incorporated by reference in its entirety).

In one embodiment, the PDGF antagonist of Table 1 or 2 is imatinib or a pharmaceutically acceptable salt thereof. A composition comprising imatinib mesylate is commercially available under the trademark Gleevec.

In another embodiment, the PDGF antagonist of Table 1 or 2 is the antibody 162.62 or a pharmaceutically acceptable salt thereof (US Patent No. 5,976,534, which is hereby incorporated by reference in its entirety).

In another embodiment, the PDGF antagonist of Table 1 or 2 is the antibody 163.31 or a pharmaceutically acceptable salt thereof (US Patent No. 5,976,534, which is hereby incorporated by reference in its entirety).

In another embodiment, the PDGF antagonist of Table 1 or 2 is the antibody 169.14 or a pharmaceutically acceptable salt thereof (US Patent No. 5,976,534, which is hereby incorporated by reference in its entirety).

In another embodiment, the PDGF antagonist of Table 1 or 2 is the antibody 169.31 or a pharmaceutically acceptable salt thereof (US Patent No. 5,976,534, which is hereby incorporated by reference in its entirety).

In another embodiment, the PDGF antagonist of Table 1 or 2 is the antibody αR1 or a pharmaceutically acceptable salt thereof (US Patent No. 5,833,986 (Column 4, lines 46-51), which is hereby incorporated by reference in its entirety).

In another embodiment, the PDGF antagonist of Table 1 or 2 is the antibody 2A1E2 or a pharmaceutically acceptable salt thereof (US Patent No. 5,817,310 (Column 11, lines 52-59), which is hereby incorporated by reference in its entirety).

In another embodiment, the PDGF antagonist of Table 1 or 2 is the antibody M4TS.11 or a pharmaceutically acceptable salt thereof (US Patent No. 5,882,644 (Figure 7), which is hereby incorporated by reference in its entirety).

In another embodiment, the PDGF antagonist of Table 1 or 2 is the antibody M4TS.22 or a pharmaceutically acceptable salt thereof (US Patent No. 5,882,644 (Figure 1), which is hereby incorporated by reference in its entirety).

In another embodiment, the PDGF antagonist of Table 1 or 2 is A10 or a pharmaceutically acceptable salt thereof (US Patent No. 6,331,555 (Figure 1), which is hereby incorporated by reference in its entirety).

In another embodiment, the PDGF antagonist of Table 1 or 2 is brefeldin A or a pharmaceutically acceptable salt thereof (US Patent No. 5,618,837 (Column 2, lines 15-19), which is hereby incorporated by reference in its entirety).

In another embodiment, the PDGF antagonist of Table 1 or 2 is sunitinib or a pharmaceutically acceptable salt thereof. A composition comprising sunitinib malate is commercially available under the trademark Sutent.

In another embodiment, the PDGF antagonist of Table 1 or 2 is the antibody Hyb 120.1.2.1.2 or a pharmaceutically acceptable salt thereof (US Patent No. 5,094,941 (Example VI, col. 32, lines 1-15), which is hereby incorporated by reference in its entirety).

In another embodiment, the PDGF antagonist of Table 1 or 2 is the antibody Hyb 121.6.1.1.1 or a pharmaceutically acceptable salt thereof (US Patent No. 5,094,941 (Example VI, col. 32, lines 1-15), which is hereby incorporated by reference in its entirety).

In another embodiment, the PDGF antagonist of Table 1 or 2 is the antibody Hyb 127.5.7.3.1 or a pharmaceutically acceptable salt thereof (US Patent No. 5,094,941 (Example VII, col. 33, lines 1-15), which is hereby incorporated by reference in its entirety).

In another embodiment, the PDGF antagonist of Table 1 or 2 is the antibody Hyb 127.8.2.2.2 or a pharmaceutically acceptable salt thereof (US Patent No. 5,094,941 (Example VII, col. 33, lines 1-15), which is hereby incorporated by reference in its entirety).

In another embodiment, the PDGF antagonist of Table 1 or 2 is the antibody Hyb 1.6.1 or a pharmaceutically acceptable salt thereof (US Patent No. 7,135,174 (col. 32, lines 34-42), which is hereby incorporated by reference in its entirety).

In another embodiment, the PDGF antagonist of Table 1 or 2 is the antibody Hyb 1.11.1 or a pharmaceutically acceptable salt thereof (US Patent No. 7,135,174 (col. 32, lines 34-42), which is hereby incorporated by reference in its entirety).

In another embodiment, the PDGF antagonist of Table 1 or 2 is the antibody Hyb 1.17.1 or a pharmaceutically acceptable salt thereof (US Patent No. 7,135,174 (col. 32, lines 34-42), which is hereby incorporated by reference in its entirety).

In another embodiment, the PDGF antagonist of Table 1 or 2 is the antibody Hyb 1.18.1 or a pharmaceutically acceptable salt thereof (US Patent No. 7,135,174 (col. 32, lines 34-42), which is hereby incorporated by reference in its entirety).

In another embodiment, the PDGF antagonist of Table 1 or 2 is the antibody Hyb 1.19.1 or a pharmaceutically acceptable salt thereof (US Patent No. 7,135,174 (col. 32, lines 34-42), which is hereby incorporated by reference in its entirety).

In another embodiment, the PDGF antagonist of Table 1 or 2 is the antibody Hyb 1.23.1 or a pharmaceutically acceptable salt thereof (US Patent No. 7,135,174 (col. 32, lines 34-42), which is hereby incorporated by reference in its entirety).

In another embodiment, the PDGF antagonist of Table 1 or 2 is the antibody Hyb 1.24 or a pharmaceutically acceptable salt thereof (US Patent No. 7,135,174 (col. 32, lines 34-42), which is hereby incorporated by reference in its entirety).

In another embodiment, the PDGF antagonist of Table 1 or 2 is the antibody Hyb 1.25 or a pharmaceutically acceptable salt thereof (US Patent No. 7,135,174 (col. 32, lines 34-42), which is hereby incorporated by reference in its entirety).

In another embodiment, the PDGF antagonist of Table 1 or 2 is the antibody Hyb 1.29 or a pharmaceutically acceptable salt thereof (US Patent No. 7,135,174 (col. 32, lines 34-42), which is hereby incorporated by reference in its entirety).

In another embodiment, the PDGF antagonist of Table 1 or 2 is the antibody Hyb 1.33 or a pharmaceutically acceptable salt thereof (US Patent No. 7,135,174 (col. 32, lines 34-42), which is hereby incorporated by reference in its entirety).

In another embodiment, the PDGF antagonist of Table 1 or 2 is the antibody Hyb 1.38 or a pharmaceutically acceptable salt thereof (US Patent No. 7,135,174 (col. 32, lines 34-42), which is hereby incorporated by reference in its entirety).

In another embodiment, the PDGF antagonist of Table 1 or 2 is the antibody Hyb 1.39 or a pharmaceutically acceptable salt thereof (US Patent No. 7,135,174 (col. 32, lines 34-42), which is hereby incorporated by reference in its entirety).

In another embodiment, the PDGF antagonist of Table 1 or 2 is the antibody Hyb 1.40 or a pharmaceutically acceptable salt thereof (US Patent No. 7,135,174 (col. 32, lines 34-42), which is hereby incorporated by reference in its entirety).

In another embodiment, the PDGF antagonist of Table 1 or 2 is the antibody Hyb 1.45 or a pharmaceutically acceptable salt thereof (US Patent No. 7,135,174 (col. 32, lines 34-42), which is hereby incorporated by reference in its entirety).

In another embodiment, the PDGF antagonist of Table 1 or 2 is the antibody Hyb 1.46 or a pharmaceutically acceptable salt thereof (US Patent No. 7,135,174 (col. 32, lines 34-42), which is hereby incorporated by reference in its entirety).

In another embodiment, the PDGF antagonist of Table 1 or 2 is the antibody Hyb 1.48 or a pharmaceutically acceptable salt thereof (US Patent No. 7,135,174 (col. 32, lines 34-42), which is hereby incorporated by reference in its entirety).

In another embodiment, the PDGF antagonist of Table 1 or 2 is the antibody Hyb 1.49 or a pharmaceutically acceptable salt thereof (US Patent No. 7,135,174 (col. 32, lines 34-42), which is hereby incorporated by reference in its entirety).

In another embodiment, the PDGF antagonist of Table 1 or 2 is the antibody Hyb 1.51 or a pharmaceutically acceptable salt thereof (US Patent No. 7,135,174 (col. 32, lines 34-42), which is hereby incorporated by reference in its entirety).

In another embodiment, the PDGF antagonist of Table 1 or 2 is the antibody Hyb 6.4.1 or a pharmaceutically acceptable salt thereof (US Patent No. 7,135,174 (col. 32, lines 34-42), which is hereby incorporated by reference in its entirety).

In another embodiment, the PDGF antagonist of Table 1 or 2 is the antibody F3 or a pharmaceutically acceptable salt thereof (US Patent Publication No. 20030219839 (paragraph 144), which is hereby incorporated by reference in its entirety).

In another embodiment, the PDGF antagonist of Table 1 or 2 is the antibody Humanized F3 or a pharmaceutically acceptable salt thereof (US Patent Publication No. 20030219839 (paragraph 153-183), which is hereby incorporated by reference in its entirety).

In another embodiment, the PDGF antagonist of Table 1 or 2 is the antibody C1 or a pharmaceutically acceptable salt thereof (US Patent Publication No. 20030219839 (paragraph 192-196), which is hereby incorporated by reference in its entirety).

In another embodiment, the PDGF antagonist of Table 1 or 2 is the antibody Humanized C1 or a pharmaceutically acceptable salt thereof (US Patent Publication No. 20030219839 (paragraph 197-199), which is hereby incorporated by reference in its entirety).

In another embodiment, the PDGF antagonist of Table 1 or 2 is the antibody 6.4.1 or a pharmaceutically acceptable salt thereof (US Patent Publication No. 20040141969 (Example 4, paragraph 192-197), which is hereby incorporated by reference in its entirety).

In another embodiment, the PDGF antagonist of Table 1 or 2 is the anti-mPDGF-C goat IgG antibody or a pharmaceutically acceptable salt thereof (Crawford et al. (2009) Cancer Cell 15:21-34, which is hereby incorporated by reference in its entirety).

In another embodiment, the PDGF antagonist of Table 1 or 2 is the antibody C3.1 or a pharmaceutically acceptable salt thereof (Kawahara et al. (1987) Biochem. Biophys. Res. Commun. 147:839-845, which is hereby incorporated by reference in its entirety).

In another embodiment, the PDGF antagonist of Table 1 or 2 is 5-methyl-7-diethylamino-s-triazolo (1,5-a) pyrimidine or a pharmaceutically acceptable salt thereof (Ohnishi et al. (1983) Life Sci. 31:2595-2602, which is hereby incorporated by reference in its entirety).

In another embodiment, the PDGF antagonist of Table 1 or 2 is interferon or a pharmaceutically acceptable salt thereof (Zagari et al. (1988) Biochem. Biophys 150:1207-12, which is hereby incorporated by reference in its entirety).

In another embodiment, the PDGF antagonist of Table 1 or 2 is protamine or a pharmaceutically acceptable salt thereof (Huang (1984) J. Cell. Biol. 26:205-220, which is hereby incorporated by reference in its entirety).

In another embodiment, the PDGF antagonist of Table 1 or 2 is the monoclonal antibody PDGFR-B1 or a pharmaceutically acceptable salt thereof (Ronnestrand, L. and Terracio, L. (1988) J. Biol. Chem. 263: 10429-10435, which is hereby incorporated by reference in its entirety).

In another embodiment, the PDGF antagonist of Table 1 or 2 is the monoclonal antibody PDGFR-B2 or a pharmaceutically acceptable salt thereof (Ronnestrand, L. and Terracio, L. (1988) J. Biol. Chem. 263: 10429-10435, which is hereby incorporated by reference in its entirety).

In another embodiment, the PDGF antagonist of Table 1 or 2 is the monoclonal antibody 6D11 or a pharmaceutically acceptable salt thereof (Vassbotn et al. (1990) Biochim. Biophy. Acta, 1054: 246-249, which is hereby incorporated by reference in its entirety).

In another embodiment, the PDGF antagonist of Table 1 or 2 is the monoclonal antibody Sis 1 or a pharmaceutically acceptable salt thereof (La Rochelle et al. (1989) Mol. Cell. Bio., 9: 3538-3542, which is hereby incorporated by reference in its entirety).

In another embodiment, the PDGF antagonist of Table 1 or 2 is the monoclonal antibody PR7212 or a pharmaceutically acceptable salt thereof (Seifert et al. (1989) J. Biol. Chem. 264: 8771-8778, which is hereby incorporated by reference in its entirety).

In another embodiment, the PDGF antagonist of Table 1 or 2 is the monoclonal antibody PR292 or a pharmaceutically acceptable salt thereof (La Rochelle et al. (1993) Cell Growth Differ. 4:547-53, which is hereby incorporated by reference in its entirety).

In another embodiment, the PDGF antagonist of Table 1 or 2 is the monoclonal antibody HYB 9610 or a pharmaceutically acceptable salt thereof (EP0798002 (see para (0023)), which is hereby incorporated by reference in its entirety).

In another embodiment, the PDGF antagonist of Table 1 or 2 is the monoclonal antibody HYB 9611 or a pharmaceutically acceptable salt thereof (EP0798002 (see para (0023)), which is hereby incorporated by reference in its entirety).

In another embodiment, the PDGF antagonist of Table 1 or 2 is the monoclonal antibody HYB 9612 or a pharmaceutically acceptable salt thereof (EP0798002 (see para (0023)), which is hereby incorporated by reference in its entirety).

In another embodiment, the PDGF antagonist of Table 1 or 2 is the monoclonal antibody HYB 9613 or a pharmaceutically acceptable salt thereof (EP0798002 (see para (0023)), which is hereby incorporated by reference in its entirety).

In another embodiment, the PDGF antagonist of Table 1 or 2 is 4-(2-(N-(-2-carboxamidoindole) aminoethyl)-benzenesulfonamide or a pharmaceutically acceptable salt thereof (EP0835115, which is hereby incorporated by reference in its entirety).

In another embodiment, the PDGF antagonist of Table 1 or 2 is 4-(2-(N-(-2-carboxamidoindole)aminoethyl)-sulfonylurea or a pharmaceutically acceptable salt thereof (EP0835115, which is hereby incorporated by reference in its entirety).

In another embodiment, the PDGF antagonist of Table 1 or 2 is CGP 53716 or a pharmaceutically acceptable salt thereof (Buchdunger, et al. (1995) Proc. Natl. Acad. Sci.;92:2558-562, which is hereby incorporated by reference in its entirety).

In another embodiment, the PDGF antagonist of Table 1 or 2 is the antibody g162 or a pharmaceutically acceptable salt thereof (WO1998025971 (see Example 7), which is hereby incorporated by reference in its entirety).

In another embodiment, the PDGF antagonist of Table 1 or 2 is pyrazolo[3,4-g]quinoxaline or a pharmaceutically acceptable salt thereof (US Pat No. 5476851, which is hereby incorporated by reference in its entirety).

In another embodiment, the PDGF antagonist of Table 1 or 2 is 6-[2-(methylcarbamoyl)phenylsulphanyl]-3-E-[2-(pyridine-2-yl)ethenyl]-indazole or a pharmaceutically acceptable salt thereof (EP1925941 (see para (0121)), which is hereby incorporated by reference in its entirety).

In another embodiment, the PDGF antagonist of Table 1 or 2 is 1-{2-[5-(2-methoxy-ethoxy)-benzoimidazole-1-yl]-quinoline-8-yl}-piperidine-4-ylamine or a pharmaceutically acceptable salt thereof (EP 1925941 (see para (0121)), which is hereby incorporated by reference in its entirety).

In another embodiment, the PDGF antagonist of Table 1 or 2 is 4-[4-[N-(4-nitrophenyl)carbamoyl]-1-piperazinyl]-6,7-dimethoxyquinazoline or a pharmaceutically acceptable salt thereof (EP1925941 (see para (0121)), which is hereby incorporated by reference in its entirety).

In another embodiment, the PDGF antagonist of Table 1 or 2 is 4-amino-5-fluoro-3-(6-(4-methyl-piperazine-1-yl)-1H-benzimidazole-2-yl)-1H-quinoline-2-one or a pharmaceutically acceptable salt thereof (EP1925941 (see para (0121)), which is hereby incorporated by reference in its entirety).

In another embodiment, the PDGF antagonist of Table 1 or 2 is (4-tert-butylphenyl){4-[(6,7-dimethoxy-4-quinolyl)oxy]phenyl}methaneone or a pharmaceutically acceptable salt thereof (EP1925941 (see para (0121)), which is hereby incorporated by reference in its entirety).

In another embodiment, the PDGF antagonist of Table 1 or 2 is 5-methyl-N-[4-(trifluoromethyl)phenyl]-4-isoxazolecarboxamide or a pharmaceutically acceptable salt thereof (EP1925941 (see para (0121)), which is hereby incorporated by reference in its entirety).

In another embodiment, the PDGF antagonist of Table 1 or 2 is trans-4-[(6,7-dimethoxyquinoxaline-2-yl)amino]cyclohexanol or a pharmaceutically acceptable salt thereof (EP1925941 (see para (0121)), which is hereby incorporated by reference in its entirety).

In another embodiment, the PDGF antagonist of Table 1 or 2 is (Z)-3-[(2,4-dimethyl-5-(2-oxo-1,2-dihydroindole-3-ylidenemethyl)-1H-pyrrole-3-yl)-propionic acid or a pharmaceutically acceptable salt thereof (EP1925941 (see para (0121)), which is hereby incorporated by reference in its entirety).

In another embodiment, the PDGF antagonist of Table 1 or 2 is 5-(5-fluoro-2-oxo-1,2-dihydroindole-3-ylidenemethyl)-2,4-dimethyl-1H-pyrrole-3-carboxylic acid or a pharmaceutically acceptable salt thereof (EP1925941 (see para (0121)), which is hereby incorporated by reference in its entirety).

In another embodiment, the PDGF antagonist of Table 1 or 2 is 1-(4-chloroanilino)-4-(4-pyridylmethyl)phthalazine or a pharmaceutically acceptable salt thereof (EP1925941 (see para (0121)), which is hereby incorporated by reference in its entirety).

In another embodiment, the PDGF antagonist of Table 1 or 2 is N-[4-(3-amino-1H-indazole-4-yl)phenyl-N'-(2-fluoro-5-methylphenyl)urea or a pharmaceutically acceptable salt thereof (EP1925941 (see para (0121)), which is hereby incorporated by reference in its entirety).

In another embodiment, the PDGF antagonist of Table 1 or 2 is 1, 2-dimethyl-7- (2-thiophene) imidazolo [5, 4-g] quinoxaline or a pharmaceutically acceptable salt thereof (US Patent No. 6,358,954 (see Figure 4), which is hereby incorporated by reference in its entirety).

In another embodiment, the PDGF antagonist of Table 1 or 2 is 1, 2-dimethyl-6-phenyl imidazolo [5, 4-g] quinoxaline or a pharmaceutically acceptable salt thereof (US Patent No. 6,358,954 (see Figure 6), which is hereby incorporated by reference in its entirety).

In another embodiment, the PDGF antagonist of Table 1 or 2 is 1, 2-dimethyl-6- (2-thiophene) imidazolo [5, 4-g] quinoxaline or a pharmaceutically acceptable salt thereof (US Patent No. 6,358,954 (see Figure 2), which is hereby incorporated by reference in its entirety).

In another embodiment, the PDGF antagonist of Table 1 or 2 is AG1295 or a pharmaceutically acceptable salt thereof (Kovalenko et al. (1994) Cancer Research 54: 6106-6114, which is hereby incorporated by reference in its entirety).

In another embodiment, the PDGF antagonist of Table 1 or 2 is AG1296 or a pharmaceutically acceptable salt thereof (Kovalenko et al. (1994) Cancer Research 54: 6106-6114, which is hereby incorporated by reference in its entirety).

In another embodiment, the PDGF antagonist of Table 1 or 2 is 3-arylquinoline or a pharmaceutically acceptable salt thereof (Dolle et al. (1994) J. Med. Chem. 37, 2627-2629, which is hereby incorporated by reference in its entirety).

In another embodiment, the PDGF antagonist of Table 1 or 2 is 4-pyridyl-2-arylpyrimidine or a pharmaceutically acceptable salt thereof (Buchdunger et al. (1995) Proc. Natl. Acad. Sci. USA. 92: 2558-62, which is hereby incorporated by reference in its entirety).

In another embodiment, the PDGF antagonist of Table 1 or 2 is sorafenib or a pharmaceutically acceptable salt thereof (US2009081709 (see para (0007)), which is hereby incorporated by reference in its entirety).

In another embodiment, the PDGF antagonist of Table 1 or 2 is MLN518 or a pharmaceutically acceptable salt thereof (US2009081709 (see para (0007)), which is hereby incorporated by reference in its entirety).

In another embodiment, the PDGF antagonist of Table 1 or 2 is PKC412 or a pharmaceutically acceptable salt thereof (US2009081709 (see para (0007)), which is hereby incorporated by reference in its entirety).

In another embodiment, the PDGF antagonist of Table 1 or 2 is AMN107 or a pharmaceutically acceptable salt thereof (US2009081709 (see para (0007)), which is hereby incorporated by reference in its entirety).

In another embodiment, the PDGF antagonist of Table 1 or 2 is suramin or a pharmaceutically acceptable salt thereof (Williams et al. (1984) J. Biol. Chem. 259:287-5294, which is hereby incorporated by reference in its entirety).

In another embodiment, the PDGF antagonist of Table 1 or 2 is neomycin or a pharmaceutically acceptable salt thereof (Vassbotn et al. (1992) J. Biol. Chem. 267:15635-15641, which is hereby incorporated by reference in its entirety).

In another embodiment, the PDGF antagonist of Table 1 or 2 is an antibody or an antibody fragment which binds to an epitope PDGF-C (SEQ ID NO:11), PDGF-C (SEQ ID NO:12), PDGF-D (SEQ ID NO:13) or PDGF-D (SEQ ID NO:14), or any portion of the epitopes.

PDGF-C epitope: Arg Lys Ser Arg Val Val Asp Leu Asn Leu Leu Thr Glu Glu Val Arg Leu Tyr Ser Cys Thr Pro Arg Asn Phe Ser Val Ser Ile Arg Glu Glu Leu Lys Arg Thr Asp Thr Ile Phe Trp Pro Gly Cys (SEQ ID NO:11)

PDGF-C epitope: Arg Lys Ser Arg Val Val Asp Leu Asn Leu Leu Thr Glu Glu Val Arg Leu Tyr Ser Cys (SEQ ID NO: 12)

PDGF-D epitope: Arg Lys Ser Lys Val Asp Leu Asp Arg Leu Asn Asp Asp Ala Lys Arg Tyr Ser Cys Thr Pro Arg Asn Tyr Ser Val Asn Ile Arg Glu Glu Leu Lys Leu Ala Asn Val Val Phe Phe Pro Arg Cys (SEQ ID NO:13)

PDGF-D epitope: Cys Lys Ser Lys Val Asp Leu Asp Arg Leu Asn Asp Asp Ala Lys Arg Tyr Ser Cys (SEQ ID NO: 14)

In another embodiment, the PDGF antagonist of Table 1 or 2 is an antibody or an antibody fragment which binds to an epitope of PDGF, such as an epitope of PDGF-A, PDGF-B, PDGF-C, or PDGF-D. In some embodiments, the PDGF antagonist binds to an epitope of PDGF such that binding of PDGF and PDGFR are inhibited. In one embodiment, the epitope encompasses a component of the three dimensional structure of PDGF that is displayed, such that the epitope is exposed on the surface of the folded PDGF molecule. In one embodiment, the epitope is a linear amino acid sequence from PDGF.

### 5.3.2 VEGF Antagonists

In one embodiment, the VEGF antagonist of Table 1 or 2 is the antibody ranibizumab or a pharmaceutically acceptable salt thereof (see US Patent No. 7,060,269 (Figure 1) for the heavy chain and light chain variable region sequences, which is hereby incorporated by reference in its entirety). Ranibizumab is commercially available under the trademark Lucentis.

In another embodiment, the VEGF antagonist of Table 1 or 2 is the antibody bevacizumab or a pharmaceutically acceptable salt thereof (see US Patent No. 6,054,297 (Figure 1) for the heavy chain and light chain variable region sequences, which is hereby incorporated by reference in its entirety). Bevacizumab is commercially available under the trademark Avastin.

In another embodiment, the VEGF antagonist of Table 1 or 2 is aflibercept or a pharmaceutically acceptable salt thereof (Do et al. (2009) Br J Ophthalmol. 93:144-9, which is hereby incorporated by reference in its entirety).

In another embodiment, the VEGF antagonist of Table 1 or 2 is KH902 or a pharmaceutically acceptable salt thereof (Zhang et al. (2008) Mol Vis. 14:37-49, which is hereby incorporated by reference in its entirety).

In another embodiment, the VEGF antagonist of Table 1 or 2 is the antibody 2C3 or a pharmaceutically acceptable salt thereof (US Patent No. 6,342,221 (Column 8, lines 48-67, Column 9, lines 1-21), which is hereby incorporated by reference in its entirety).

In another embodiment, the VEGF antagonist is ORA102 or a pharmaceutically acceptable salt thereof (Ora Bio, Ltd).

In one embodiment, the VEGF antagonist of Table 1 or 2 is pegaptanib or a pharmaceutically acceptable salt thereof (US Patent No. 6,051,698 (Figure 1), which is hereby incorporated by reference in its entirety). A composition comprising pegaptanib is commercially available under the trademark Macugen.

In another embodiment, the VEGF antagonist of Table 1 or 2 is bevasiranib or a pharmaceutically acceptable salt thereof (Dejneka et al. (2008) Mol Vis. 14:997-1005, which is hereby incorporated by reference in its entirety).

In another embodiment, the VEGF antagonist of Table 1 or 2 is Sirna-027 or a pharmaceutically acceptable salt thereof (Shen et al. (2006) Gene Ther. 13:225-34, which is hereby incorporated by reference in its entirety).

In another embodiment, the VEGF antagonist of Table 1 or 2 is decursin or a pharmaceutically acceptable salt thereof (US Patent No. 6,525,089 (Column 3, lines 5-16), which is hereby incorporated by reference in its entirety).

In another embodiment, the VEGF antagonist of Table 1 or 2 is decursinol or a pharmaceutically acceptable salt thereof (Ahn et al. (1997) Planta Med. 63:360-1, which is hereby incorporated by reference in its entirety).

In another embodiment, the VEGF antagonist of Table 1 or 2 is picropodophyllin or a pharmaceutically acceptable salt thereof (Economou (2008) Investigative Ophthalmology & Visual Science. 49:2620-6, which is hereby incorporated by reference in its entirety).

In another embodiment, the VEGF antagonist of Table 1 or 2 is guggulsterone or a pharmaceutically acceptable salt thereof (Kim et al. (2008) Oncol. Rep. 20:1321-7, which is hereby incorporated by reference in its entirety).

In another embodiment, the VEGF antagonist of Table 1 or 2 is PLG101 or a pharmaceutically acceptable salt thereof (Ahmadi and Lim (2008) Expert Opin Pharmacother. 9:3045-52, which is hereby incorporated by reference in its entirety).

In another embodiment, the VEGF antagonist of Table 1 or 2 is PLG201 or a pharmaceutically acceptable salt thereof (Ahmadi and Lim (2008) Expert Opin Pharmacother. 9:3045-52, which is hereby incorporated by reference in its entirety).

In another embodiment, the VEGF antagonist of Table 1 or 2 is eicosanoid LXA4 or a pharmaceutically acceptable salt thereof (Baker et al (2009) J Immun. 182:3819-26, which is hereby incorporated by reference in its entirety).

In another embodiment, the VEGF antagonist of Table 1 or 2 is PTK787 or a pharmaceutically acceptable salt thereof (Barakat and Kaiser (2009) Expert Opin Investig Drugs 18:637-46, which is hereby incorporated by reference in its entirety). A composition comprising PTK787 is commercially available under the trademark Vitalanib.

In another embodiment, the VEGF antagonist of Table 1 or 2 is pazopanib or a pharmaceutically acceptable salt thereof (Takahashi et al. (2009) Arch Ophthalmol. 127:494-9, which is hereby incorporated by reference in its entirety).

In another embodiment, the VEGF antagonist of Table 1 or 2 is axitinib or a pharmaceutically acceptable salt thereof (Hu-Lowe et al. (2008) Clin Cancer Res. 14:7272-83, which is hereby incorporated by reference in its entirety).

In another embodiment, the VEGF antagonist of Table 1 or 2 is CDDO-Me or a pharmaceutically acceptable salt thereof (Sogno et al. (2009) Recent Results Cancer Res. 181:209-12, which is hereby incorporated by reference in its entirety).

In another embodiment, the VEGF antagonist of Table 1 or 2 is CDDO-Imm or a pharmaceutically acceptable salt thereof (Sogno ct al. (2009) Recent Results Cancer Res. 181:209-12, which is hereby incorporated by reference in its entirety).

In another embodiment, the VEGF antagonist of Table 1 or 2 is shikonin or a pharmaceutically acceptable salt thereof (Hisa et al. (1998) Anticancer Res. 18:783-90, which is hereby incorporated by reference in its entirety).

In another embodiment, the VEGF antagonist of Table 1 or 2 is beta-hydroxyisovalerylshikonin or a pharmaceutically acceptable salt thereof (Hisa et al. (1998) Anticancer Res. 18:783-90, which is hereby incorporated by reference in its entirety).

In another embodiment, the VEGF antagonist of Table 1 or 2 is ganglioside GM3 or a pharmaceutically acceptable salt thereof (Chung et al. (2009) Glycobio. 19:229-39, which is hereby incorporated by reference in its entirety).

In another embodiment, the VEGF antagonist of Table 1 or 2 is the antibody DC101 or a pharmaceutically acceptable salt thereof (US Patent No. 6,448,077 (Column 2, lines 61-65), which is hereby incorporated by reference in its entirety).

In another embodiment, the VEGF antagonist of Table 1 or 2 is the antibody Mab25 or a pharmaceutically acceptable salt thereof (US Patent No. 6,448,077 (Column 2, lines 61-65), which is hereby incorporated by reference in its entirety).

In another embodiment, the VEGF antagonist of Table 1 or 2 is the antibody Mab73 or a pharmaceutically acceptable salt thereof (US Patent No. 6,448,077 (Column 2, lines 61-65), which is hereby incorporated by reference in its entirety).

In another embodiment, the VEGF antagonist of Table 1 or 2 is the antibody 4A5 or a pharmaceutically acceptable salt thereof (US Patent No. 6,383,484 (Column 12, lines 50-54), which is hereby incorporated by reference in its entirety).

In another embodiment, the VEGF antagonist of Table 1 or 2 is the antibody 4E10 or a pharmaceutically acceptable salt thereof (US Patent No. 6,383,484 (Column 10, 1 ines 66-67, Column 11, lines 1-2), which is hereby incorporated by reference in its entirety).

In another embodiment, the VEGF antagonist of Table 1 or 2 is the antibody 5F12 or a pharmaceutically acceptable salt thereof (US Patent No. 6,383,484 (Column 10, lines 62-65), which is hereby incorporated by reference in its entirety).

In another embodiment, the VEGF antagonist of Table 1 or 2 is the antibody VA01 or a pharmaceutically acceptable salt thereof (US Patent No. 5,730,977 (Column 6, lines 26-30), which is hereby incorporated by reference in its entirety).

In another embodiment, the VEGF antagonist of Table 1 or 2 is the antibody BL2 or a pharmaceutically acceptable salt thereof (US Patent No. 5,730,977 (Column 6, lines 30-32), which is hereby incorporated by reference in its entirety).

In one embodiment, the VEGF antagonist of Table 1 or 2 is VEGF-related protein or a pharmaceutically acceptable salt thereof (US Patent No. 6,451,764 (Figure 1), which is hereby incorporated by reference in its entirety).

In another embodiment, the VEGF antagonist of Table 1 or 2 is sFLT01 or a pharmaceutically acceptable salt thereof (Pechan et al. (2009) Gene Ther. 16:10-6, which is hereby incorporated by reference in its entirety).

In another embodiment, the VEGF antagonist of Table 1 or 2 is sFLT02 or a pharmaceutically acceptable salt thereof (Pechan et al. (2009) Gene Ther. 16:10-6, which is hereby incorporated by reference in its entirety).

In another embodiment, the VEGF antagonist of Table 1 or 2 is Peptide B3 or a pharmaceutically acceptable salt thereof (Lacal et al. (2008) Eur J Cancer 44:1914-21, which is hereby incorporated by reference in its entirety).

In another embodiment, the VEGF antagonist of Table 1 or 2 is TG100801 or a pharmaceutically acceptable salt thereof (Palanki et al. (2008) J Med Chem. 51:1546-59, which is hereby incorporated by reference in its entirety).

In another embodiment, the VEGF antagonist of Table 1 or 2 is sorafenib or a pharmaceutically acceptable salt thereof (Kernt et al. (2008) Acta Ophthalmol. 86:456-8, which is hereby incorporated by reference in its entirety). A composition comprising sorafenib is commercially available under the trademark Nexavar.

In another embodiment, the VEGF antagonist of Table 1 or 2 is G6-31 antibody or a pharmaceutically acceptable salt thereof (Crawford et al. (2009) Cancer Cell 15:21-34, which is hereby incorporated by reference in its entirety).

In another embodiment, the VEGF antagonist of Table I or 2 is an antibody or an antibody fragment which binds to an epitope VEGF-A (SEQ ID NO:15) or VEGF-B (SEQ ID NO:16), or any portion of the epitopes.

VEGP-A epitope: Cys Asn Asp Glu Gly Leu Glu Cys Val Pro Thr Glu Glu Ser Asn Ile (SEQ ID NO:15)

VEGF-B epitope: Cys Pro Asp Asp Gly Lue Glu Cys Val Pro Thr Gly Gin His Gln Val (SEQ ID NO:16)

In one embodiment, the PDGF or VEGF antagonist of Table 1 or 2 is an antibody or antibody fragment that binds to one or more of an epitope of PDGF (e.g. SEQ ID NO:11-14) and one or more of an epitope of VEGF (e.g., SEQ ID NO:15-16)

In another embodiment, the VEGF antagonist of Table 1 or 2 is an antibody or an antibody fragment which binds to an epitope of VEGF, such as an epitope of VEGF-A, VEGF-B, VEGF-C, VEGF-D, or VEGF-E. In some embodiments, the VEGF antagonist binds to an epitope of VEGF such that binding of VEGF and VEGFR are inhibited. In one embodiment, the epitope encompasses a component of the three dimensional structure of VEGF that is displayed, such that the epitope is exposed on the surface of the folded VEGF molecule. In one embodiment, the epitope is a linear amino acid sequence from VEGF.

### 5.3.3 Other agents for treatment or prevention of an ophthalmological disease

In another embodiment, another agent useful for treating or preventing an ophthalmological disease is volociximab or a pharmaceutically acceptable salt thereof (Ramakrishnan et al. (2008) J Exp Ther Oncol. 5:273-86, which is hereby incorporated by reference in its entirety).

### 5.4 Modification of antagonist agents

### Aptamer Antagonists

Where an antagonist of the present invention is an aptamer, the invention emcompasses modified versions thereof, as set forth below. In some embodiments, an aptamer can have chemically modified nucleotides, including 5-X and/or 2'-Y substitutions in pyrimidine bases and 8-X and/or 2'-Y substitutions in purine bases. 2'-Modifications, such as 2'-fluoro and 2'-O-Me, can be utilized for stabilization against nucleases without compromising the aptamer binding interaction with the target. See, e.g., Lin et al., Nucleic Acids Res., 22, 5229-5234 (1994); Jellinek et al., Biochemistry, 34, 11363-1137 (1995); Lin et al., Nucleic Acids Res., 22, 5229-5234 (1994); Kubik et al., J. Immunol., 159(1), 259-267 (1997); Pagratis et al., Nat. Biotechnol., 1, 68-73 (1997); and Wilson et al., Curr Opin Chem Biol, 10(6), 607-614 (2006). In some embodiments, the chemical substitution can be a chemical substitution at a sugar position; a chemical substitution at a base position or a chemical substitution at a phosphate position.

Modifications of aptamers of this invention include, but are not limited to, those which provide other chemical groups that incorporate additional charge, polarizability, hydrophobicity, hydrogen bonding, electrostatic interaction, or fluxionality to the aptamer bases or to the aptamer as a whole. Such modifications include, but are not limited to, 2'-position sugar modifications, 5-position pyrimidine modifications, 8-position purine modifications, modifications at exocyclic amines, substitution of 4-thiouridine, substitution of 5-bromo or 5-iodo-uracil; backbone modifications, phosphorothioate or alkyl phosphate modifications, methylations, unusual base-pairing combinations such as the isobases isocytidine and isoguanidine and the like. Modifications can also include 3' and 5' modifications such as capping or modification with sugar moieties. In some embodiments of the instant invention, the aptamers are RNA molecules that are 2'-fluoro (2'-F) modified on the sugar moiety of pyrimidine residues.

The stability of the aptamer can be increased by the introduction of such modifications and as well as by modifications and substitutions along the phosphate backbone of the RNA. In addition, a variety of modifications can be made on the nucleobases themselves which both inhibit degradation and which can increase desired nucleotide interactions or decrease undesired nucleotide interactions. Accordingly, once the sequence of an aptamer is known, modifications or substitutions can be made by the synthetic procedures described below or by procedures known to those of skill in the art.

Other modifications include the incorporation of modified bases (or modified nucleoside or modified nucleotides) that are variations of standard bases, sugars and/or phosphate backbone chemical structures occurring in ribonucleic (i.e., A, C, G and U) and deoxyribonucleic (*i.e*., A, C, G and T) acids. Included within this scope are, for example: Gm (2'-methoxyguanylic acid), Am (2'-methoxyadenylic acid), Cf (2'-fluorocytidylic acid), Uf (2'-fluorouridylic acid), Ar (riboadenylic acid). The aptamers can also include cytosine or any cytosine-related base including 5-methylcytosine, 4-acetylcytosine, 3-methylcytosine, 5-hydroxymethyl cytosine, 2-thiocytosine, 5-halocytosine (e.g., 5-fluorocytosine, 5-bromocytosine, 5-chlorocytosine, and 5-iodocytosine), 5-propynyl cytosine, 6-azocytosine, 5-trifluoromethylcytosine, N4, N4-ethanocytosine, phenoxazine cytidine, phenothiazine cytidine, carbazole cytidine or pyridoindole cytidine. The aptamer can further include guanine or any guanine-related base including 6-methylguanine, 1-methylguanine, 2,2-dimethylguanine, 2-methylguanine, 7-methylguanine, 2-propylguanine, 6-propylguanine, 8-haloguanine (*e.g*., 8-fluoroguanine, 8-bromoguanine, 8-chloroguanine, and 8-iodoguanine), 8-aminoguanine, 8-sulfhydrylguanine, 8-thioalkylguanine, 8-hydroxylguanine, 7-methylguanine, 8-azaguaninc, 7-deazaguaninc or 3-deazaguanine. The aptamer may still further include adenine or any adenine-related base including 6-methyladenine, N6-isopentenyladenine, N6-methyladenine, 1-methyladenine, 2-methyladenine, 2-methylthio-N6-isopentenyladenine, 8-haloadenine (e*.g.,* 8-fluoroadenine, 8-bromoadenine, 8-chloroadenine, and 8-iodoadenine), 8-aminoadenine, 8-sulfhydryladenine, 8-thioalkyladenine, 8-hydroxyladenine, 7-methyladenine, 2-haloadenine (e.g., 2-fluoroadenine, 2-bromoadenine, 2-chloroadenine, and 2-iodoadenine), 2-aminoadenine, 8-azaadenine, 7-deazaadenine or 3-deazaadenine. Also included are uracil or any uracil-related base including 5-halouracil (*e.g.,* 5-fluorouracil, 5-bromouracil, 5-chlorouracil, 5-iodouracil), 5-(carboxyhydroxylmethyl)uracil, 5-carboxymethylaminomethyl-2-thiouracil, 5-carboxymethylaminomethyluracil, dihydrouracil, 1-methylpseudouracil, 5-methoxyaminomethyl-2-thiouracil, 5'-methoxycarbonylmethyluracil, 5-methoxyuracil, 5-methyl-2-thiouracil, 2-thiouracil, 4-thiouracil, 5-methyluracil, uracil-5-oxyacetic acid methylester, uracil-5-oxyacetic acid, pseudouracil, 5-methyl-2-thiouracil, 2-thiouracil, 3-(3-amino-3-N-2-carboxypropyl)uracil, 5-methylaminomethyluracil, 5-propynyl uracil, 6-azouracil, or 4-thiouracil.

Examples of other modified base variants known in the art include, without limitation, 4-acetylcytidine, 5-(carboxyhydroxylmethyl) uridine, 2'-methoxycytidine, 5-carboxymethylaminomethyl-2-thioridine, 5-carboxymethylaminomethyluridine, dihydrouridine, 2'-O-methylpseudouridine, b-D-galactosylqueosine, inosine, N6-isopentenyladenosine, 1-methyladenosine, 1-methylpseudouridine, 1-methylguanosine, 1-methylinosine, 2,2-dimethylguanosine, 2-methyladenosine, 2-methylguanosine, 3-methylcytidine, 5-methylcytidine, N6-methyladenosine, 7-methylguanosine, 5-methylaminomethyluridine, 5-methoxyaminomethyl-2-thiouridine, b-D-mannosylqueosine, 5-methoxycarbonylmethyluridine, 5-methoxyuridine, 2-methylthio-N6-isopentenyladenosine, N-((9-b-D-ribofuranosyl-2-methylthiopurinc-6-yl)carbamoyl)threonine, N-((9-b-D-ribofuranosylpurine-6-yl)N-methylcarbamoyl)threonine, urdine-5-oxyacetic acid methylester, uridine-5-oxyacetic acid, wybutoxosine, pseudouridine, queosine, 2-thiocytidine, 5-methyl-2-thiouridine, 2-thiouridine, 4-thiouridine, 5-methyluridine, N-((9-b-D-ribofuranosylpurine-6-yl)carbamoyl)threonine, 2'-O-methyl-5-methyluridine, 2'-O-methyluridine, wybutosine, 3-(3-amino-3-carboxypropyl)uridine.

Examples of modified nucleoside and nucleotide sugar backbone variants known in the art include, without limitation, those having, *e.g*., 2' ribosyl substituents such as F, SH, SCH3, OCN, Cl, Br, CN, CF3, OCF3, SOCH3, SO2, CH3, ONO2, NO2 N3, NH2, OCH2CH3OCH3, O(CH2)2ON(CH3)2, OCH2OCH2N(CH3)2, O(C1-10 alkyl), O(C2-10 alkenyl), O(C2-10 alkynyl), S(C1-10 alkyl), S(C2-10 alkenyl), S(C2-10 alkynyl), NH(C1-10 alkyl), NH(C2-10 alkenyl), NH(C2-10 alkynyl), and O-alkyl-O-alkyl. Desirable 2'ribosyl substituents include 2'-methoxy (2'-OCH3), 2'-aminopropoxy (2' OCH2CH2CH2NH2), 2'-allyl (2'-CH2-CH=CH2), 2'-O-allyl (2'-O-CH2-CH=CH2), 2'-amino (2'-NH2), and 2'-fluoro (2'-F). The 2'-substituent may be in the arabino (up) position or ribo (down) position.

Examples of modifications include: a purine substitution for a pyrimidine; a 2'-deoxy dihydrouridine substitution for a uridine; a 3'-deoxy-5-methyl cytidine for a cytidine; a 2-amino purine substitution for a purine; a phosphorothioate substituted for a phosphodiester; a phosphorodithioate substituted for a phosphodiester; a deoxynucleotide substituted for a 2'-OH nucleotide; a 2'-OMe nucleotide, a 2'-fluoro nucleotide or a 2'-O-methoxyethyl nucleotide substituted for a 2'-OH or deoxynucleotide; the addition of a PEG or PAG polymer; the addition of a large steric molecule; the addition of a 3' cap; or any other modification known to block nuclease degradation. See, for example, U.S. Patent Publication No. 20090075342, which is incorporated by reference in its entirety.

The aptamers of the invention may be made up of nucleotides and/or nucleotide analogs such as described above, or a combination of both, or are oligonucleotide analogs. The aptamers of the invention may contain nucleotide analogs at positions which do not affect the function of the oligomer, for example, to bind PDGF or VEGF (or their cognate receptors).

The aptamers described herein can be linked with one or more non-physiologically active groups, such as a lipophilic compound (e.g., cholesterol); non-immunogenic high molecular weight compounds; or attached to or encapsulated in a complex comprising a lipophilic component (eg., a liposome). In one embodiment, the linked aptamers enhance the cellular uptake of the aptamers by a cell for delivery of the aptamers to an intracellular target. U.S. Patent No. 6,011,020 describes a method for preparing a therapeutic or diagnostic compounds of an aptamer linked with lipophilic compound or a non-immunogenic, high molecular weight compound.

The invention further encompasses linking selected aptamers with one or more non-physiologically active group, such as lipophilic or Non-Immunogenic, High Molecular Weight compounds, in a diagnostic or therapeutic complex as described in U.S. Patent No. 6,011,020. Aptamers that are linked with a Lipophilic Compound, such as diacyl glycerol or dialkyl glycerol, in a diagnostic or therapeutic complex are described in U.S. Patent No. 5,859,228. Aptamers that are linked with a Lipophilic Compound, such as a glycerol lipid, or a Non-Immunogenic, High Molecular Weight Compound, such as polyalkylene glycol, are further described in U.S. Patent No. 6,051,698. Aptamers that are linked with a Non-Immunogenic, High Molecular Weight compound or a lipophilic compound are also further described in PCT/US97/18944, filed Oct. 17, 1997, entitled "Vascular Endothelial Growth Factor (VEGF) Nucleic Acid Ligand Complexes." Each of the above described patents and patent applications are specifically incorporated by reference herein in its entirety.

Certain embodiments of the present invention provide compounds comprising one or more aptamers covalently linked with a Non-Immunogenic, High Molecular Weight compound or lipophilic compound. A Non-Immunogenic, High Molecular Weight compound can be a compound that has a molecular weight of about 100 Da to 1,000,000 Da, about 1000 Da to 500,000 Da, or about 1000 Da to 200,000 Da, that typically does not generate an immunogenic response. For the purposes of this invention, an immunogenic response is one that causes the organism to make antibody proteins directed to the non-physiologically active group. In one embodiment, the Non-Immunogenic, High Molecular Weight compound can be a polyalkylene glycol. In another embodiment, the polyalkylene glycol can be polyethylene glycol (PEG). In some embodiments, the PEG has a molecular weight of about 10-80K or a molecular weight of about 20-45K. In some embodiments, the Non-Immunogenic, High Molecular Weight compound can be an aptamer.

Another embodiment of the invention is directed to compounds comprising an aptamer linked with lipophilic compound. Lipophilic compounds are compounds that have the propensity to associate with or partition into lipid and/or other materials or phases having a low dielectric constant, including compounds based mostly on lipophilic components. Lipophilic compounds include lipids as well as non-lipid containing compounds that have the propensity to associate with lipids (and/or other materials or phases with low dielectric constants). Cholesterol, phospholipid, and glycerol lipids, such as dialkyl glycerol, diacyl glycerol, and glycerol amide lipids are further examples of lipophilic compounds. In one embodiment, the lipophilic compound is a glycerol lipid.

The Non-Immunogenic, High Molecular Weight compound or lipophilic compound can be covalently bound to a variety of positions on the aptamer, such as to an exocyclic amino group on a nucleotide's base, the 5-position of a pyrimidine nucleotide, the 8-position of a purine nucleotide, the hydroxyl group of a nucleotide's phosphate, or a hydroxyl group or other group at the 5' or 3' terminus of the aptamer. In some embodiments where the lipophilic compound is a glycerol lipid, or the Non-Immunogenic, High Molecular Weight compound is polyalkylene glycol or polyethylene glycol, the Non-Immunogenic, High Molecular Weight compound can be bonded to the 5' or 3' hydroxyl of the phosphate group thereof. In one embodiment, the lipophilic compound or Non-Immunogenic, High Molecular Weight compound is bonded to the 5' phosphate group of the aptamer. Attachment of the Non-Immunogenic, High Molecular Weight compound or lipophilic compound to the aptamer can be done directly or with the utilization of one or more linkers that interpose between the aptamer and lipophilic compound or Non-Immunogenic, High Molecular Weight compound. When attachment is done directly, on the other hand, no linker is present.

A linker is a molecular entity that connects two or more molecular entities through covalent bonds or noncovalent interactions, and can allow spatial separation of the molecular entities in a manner that preserves the functional properties of one or more of the molecular entities.

In one embodiment of the invention, the Non-Immunogenic, High Molecular Weight Compound covalently linked with the aptamer is a polyalkylene glycol and has the structure R(O(CH₂)ₓ)ₙ O--, where R is independently selected from the group consisting of H and CH₃, x=2-5, and n ≈ MW of the Polyalkylene Glycol/(16+14x). In one embodiment of the present invention, the molecular weight of the polyalkylene glycol is about between 10-80 kDa. In another embodiment, the molecular weight of the polyalkylene glycol is about between 20-45 kDa. In yet another embodiment, x=2 and n=9X10². There can be one or more Polyalkylene Glycols attached to the same aptamer.

In one embodiment, a Complex of the present invention is a PDGF aptamer covalently linked with a Non-Immunogenic, High Molecular Weight Compound such as Polyalkylene Glycol or PEG. In this embodiment, the pharmacokinetic properties of the Complex are improved relative to the PDGF aptamer alone. The Polyalkylene Glycol or PEG can be covalently bound to a variety of positions on the PDGF aptamer. In embodiments where Polyalkylene Glycol or PEG are used, the PDGF aptamer can be bonded through the 5' hydroxyl group via a phosphodiester linkage.

In some embodiments, a plurality of aptamers can be associated with a single Non-Immunogenic, High Molecular Weight Compound, such as Polyalkylene Glycol or PEG, or a Lipophilic Compound, such as a glycerolipid. The aptamers can all be to one target or to different targets. In embodiments where a compound comprises more than one PDGF aptamer, there can be an increase in avidity due to multiple binding interactions with a target, such as PDGF or VEGF. In yet further embodiments, a plurality of Polyalkylene Glycol, PEG, glycerol lipid molecules can be attached to each other. In these embodiments, one or more aptamers can be associated with each Polyalkylene Glycol, PEG, or glycerol lipid. This can result in an increase in avidity of each aptamer to its target. In addition, in embodiments where there are aptamers to PDGF- or aptamers to PDGF and different Targets associated with Polyalkylene Glycol, PEG, or glycerol lipid, a drug can also be associated with, e.g., covalently bonded to, Polyalkylene Glycol, PEG, or glycerol lipid. Thus the compound would provide targeted delivery of the drug, with Polyalkylene Glycol, PEG, or glycerol lipid serving as a Linker, optionally, with one or more additional linkers.

Aptamers can be 5'-capped and/or 3'-capped with a 5'-5' inverted nucleoside cap structure at the 5' end and/or a 3'-3' inverted nucleoside cap structure at the 3' end. In several embodiments, Antagonist A, Antagonist B, Antagonist C, Antagonist D, pegaptanib, bevasiranib and Sirna-027 are 5' or 3' end-capped.

### Antibody Antagonists

Where the PDGF antagonist or VEGF antagonist of Table 1 or 2 is an antibody, such as for example 1B3, CDP860, 162.62, 163.31, 169.14, 169.31, αR1, 2A1E2, M4TS.11, M4TS.22, Hyb 120.1.2.1.2 antibody, Hyb 121.6.1.1.1 antibody, Hyb 127.5.7.3.1 antibody, Hyb 127.8.2.2.2 antibody, Hyb 1.6.1 antibody, Hyb 1.11.1 antibody, Hyb 1.17.1 antibody, Hyb 1.18.1 antibody, Hyb 1.19.1 antibody, Hyb 1.23.1 antibody, Hyb 1.24 antibody, Hyb 1.25 antibody, Hyb 1.29 antibody, Hyb 1.33 antibody, Hyb 1.38 antibody, Hyb 1.39 antibody, Hyb 1.40 antibody, Hyb 1.45 antibody, Hyb 1.46 antibody, Hyb 1.48 antibody, Hyb 1.49 antibody, Hyb 1.51 antibody, Hyb 6.4.1 antibody, F3 antibody, Humanized F3 antibody, C1 antibody, Humanized C1 antibody, 6.4 antibody, anti-mPDGF-C goat IgG antibody, C3.1 antibody, PDGFR-B1 monoclonal antibody, PDGFR-B2 monoclonal antibody, 6D11 monoclonal antibody, Sis 1 monoclonal antibody, PR7212 monoclonal antibody, PR292 monoclonal antibody, HYB 9610 monoclonal antibody, HYB 9611 monoclonal antibody, HYB 9612 monoclonal antibody, HYB 9613 monoclonal antibody, human antibody g162. ranibizumab, bevacizumab, KH902, DC101, Mab25, Mab73, 4A5, 4E10, 5F12, VA01, BL2, G6-31 antibody, or anti-mPDGF-C goat IgG antibody, the invention also relates to antibody fragments. Unless specified otherwise, the term antibody refers only to whole antibodies.

The antagonist antibodies of the invention include monoclonal inhibitory antibodies. Monoclonal antibodies, or fragments thereof, encompass all immunoglobulin classes such as IgM, IgG, IgD, IgE, IgA, or their subclasses, such as the IgG subclasses or mixtures thereof. IgG and its subclasses are useful, such as IgG₁, IgG₂, IgG₂ₐ, IgG_{2b}, IgG₃ or IgG_{M}. The IgG subtypes IgG_{1/kappa} and IgG_{2b/kapp} are included as useful embodiments. Fragments of the invention are truncated or modified antibody fragments with an antigen-complementary binding site. In some embodiments, an antibody fragment is formed by light and heavy chains, such as Fv, Fab or F(ab')₂ fragments, or single-stranded fragments.

The invention further includes derivatives of antibodies of the present invention which retain their antagonist activity while altering one or more other properties related to their use as a pharmaceutical agent, *e.g.,* serum stability or efficiency of production. Examples of such antibody derivatives include peptides, peptidomimetics derived from the antigen-binding regions of the antibodies, and antibodies, antibody fragments or peptides bound to solid or liquid carriers such as polyethylene glycol, glass, synthetic polymers such as polyacrylamide, polystyrene, polypropylene, polyethylene or natural polymers such as cellulose, sepharose or agarose, or conjugates with enzymes, toxins or radioactive or nonradioactive markers such as ³H, ¹²³I, ¹²⁵I, ¹³¹I, ³²P, ³⁵ S, ¹⁴C, ⁵¹Cr, ³⁶Cl, ⁵⁷Co, ⁵⁵Fe, ⁵⁹Fe, ⁹⁰Y, ^{99m}Tc, ⁷⁵Se, or antibodies, fragments, or peptides covalently bonded to fluorescent/chemiluminescent labels such as rhodamine, fluorescein, isothiocyanate, phycoerythrin, phycocyanin, fluorescamine, metal chelates, avidin, streptavidin or biotin.

In some embodiments, a monoclonal antibody of the present invention can be modified by splicing a variable (including hypervariable) domain of the antibody with a constant domain (e.g., "humanized" antibodies), or a light chain with a heavy chain, or a chain from one species with a chain from another species, or fusions with heterologous proteins, regardless of species of origin or immunoglobulin class or subclass designation, as well as antibody fragments, so long as they exhibit the desired biological activity. See, for example, U.S. Patent No. 4,816,567 and Mage & Lamoyi, in Monoclonal Antibody Production Techniques and Applications, pp.79-97 (Marcel Dekker, Inc.), New York (1987). Methods for humanizing non-human antibodies are well known in the art.

### 5.5 Treatment or Prevention of an Ophthalmological disease

In some embodiments of the invention, the ophthalmological disease is a neovascular disorder. In other embodiments of the invention, the ophthalmological disease results in retinal edema. Illustrative ophthalmological disease are listed below.

### 5.5.1 Treatment or Prevention of age-related macular degeneration

In one embodiment, the ophthalmological disease is age-related macular degeneration. Examples of age-related macular degeneration are nonneovascular (also known as "Dry") and neovascular (also known as "Wet") macular degeneration. In one embodiment the dry age-related macular degeneration is associated with the formation of drusen. Treating or preventing dry macular degeneration also encompasses treating or preventing an abnormality of the retinal pigment epithelium. Examples of abnormalities of the retinal pigment epithelium include geographic atrophy, non-geographic atrophy, focal hypopigmentation, and focal hyperpigmentation. Treating or preventing wet age-related macular degeneration also encompasses treating or preventing choroidal neovascularization or pigment epithelial detachment.

### 5.5.2 Treatment or Prevention of polypoidal choroidal vasculopathy

In one embodiment, the ophthalmological disease is polypoidal choroidal vasculopathy. Polypoidal choroidal vasculopathy is characterized by a lesion from an inner choroidal vascular network of vessels ending in an aneurysmal bulge or outward projection (Ciardella et al. (2004) Surv Ophthalmol. 49:25-37).

### 5.5.3 Treatment or Prevention of a condition associated with choroidal neovascularization

In one embodiment, the ophthalmological disease is a condition associated with choroidal neovascularization. Examples of conditions associated with choroidal neovascularization include a degenerative, inflammatory, traumatic or idiopathic condition. Treating or preventing a degenerative disorder associated with choroidal neovascularization also encompasses treating or preventing a heredodegerative disorder. Examples of heredodegerative disorders include vitelliform macular dystrophy, fundus flavimaculatus and optic nerve head drusen. Examples of degenerative conditions associated with choroidal neovascularization include myopic degeneration or angioid streaks. Treating or preventing an inflammatory disorder associated with choroidal neovascularization also encompasses treating or preventing ocular histoplasmosis syndrome, multifocal choroiditis, serpininous choroiditis, toxoplasmosis, toxocariasis, rubella, Vogt-Koyanagi-Harada syndrome, Behcet syndrome or sympathetic ophthalmia. Treating or preventing a traumatic disorder associated with choroidal neovascularization also encompasses treating or preventing choroidal rupture or a traumatic condition caused by intense photocoagulation.

### 5.5.4 Treatment or Prevention of hypertensive retinopathy

In one embodiment, the ophthalmological disease is hypertensive retinopathy.

### 5.5.5 Treatment or Prevention of diabetic retinopathy

In one embodiment, the ophthalmological disease is diabetic retinopathy. Diabetic retinopathy can be nonproliferative or proliferative diabetic retinopathy. Examples of nonproliferative diabetic retinopathy include macular edema and macular ischemia.

### 5.5.6 Treatment or Prevention of sickle cell retinopathy

In one embodiment, the ophthalmological disease is sickle cell retinopathy.

### 5.5.7 Treatment or Prevention of a condition associated with peripheral retinal neovascularization

In one embodiment, the ophthalmological disease is a condition associated with peripheral retinal neovascularization. Examples of conditions associated with peripheral retinal neovascularization include ischemic vascular disease, inflammatory disease with possible ischemia, incontinentia pigmenti, retinitis pigmentosa, retinoschisis or chronic retinal detachment.

Examples of ischemic vascular disease include proliferative diabetic retinopathy, branch retinal vein occlusion, branch retinal arteriolar occlusion, carotid cavernous fistula, sickling hemoglobinopathy, non-sickling hemoglobinopathy, IRVAN syndrome (retinal vasculitic disorder characterized by idiopathic retinal vasculitis, an aneurysm, and neuroretinitis), retinal embolization, retinopathy of prematurity, familial exudative vitreoretinopathy, hyperviscosity syndrome, aortic arch syndrome or Eales disease. Examples of sickling hemoglobinopathy include SS hemoglobinopathy and SC hemoglobinopathy. Examples of non-sickling hemoglobinopathy include AC hemoglobinopathy and AS hemoglobinopathy. Examples of hyperviscosity syndrome include leukemia, Waldenstrom macroglobulinemia, multiple myeloma, polycythemia or myeloproliferative disorder.

Treating or preventing an inflammatory disease with possible ischemia also encompasses treating or preventing retinal vasculitis associated with systemic disease, retinal vasculitis associated with an infectious agent, uveitis or birdshot retinopathy. Examples of systemic diseases include systemic lupus erythematosis, Behcet's disease, inflammatory bowel disease, sarcoidosis, multiple sclerosis, Wegener's granulomatosis and polyarteritis nodosa. Examples of infectious agents include a bacterial agent that is the causative agent for syphilis, tuberculosis, Lyme disease or cat-scratch disease, a virus such as herpesvirus, or a parasite such as Toxocara canis or Toxoplasma gondii. Examples of uveitis include pars planitis or Fuchs uveitis syndrome.

### 5.5.8 Treatment or Prevention of retinopathy of prematurity

In one embodiment, the ophthalmological disease is retinopathy of prematurity. Retinopathy of prematurity can result from abnormal growth of blood vessels in the vascular bed supporting the developing retina (Pollan C (2009) Neonatal Netw. 28:93-101).

### 5.5.9 Treatment or Prevention of venous occlusive disease

In one embodiment, the ophthalmological disease is venous occlusive disease. Examples of venous occlusive disease include branch retinal vein occlusion and central retinal vein occlusion. A branch retinal vein occlusion can be a blockage of the portion of the circulation that drains the retina of blood. The blockage can cause back-up pressure in the capillaries, which can lead to hemorrhages and also to leakage of fluid and other constituents of blood.

### 5.5.10 Treatment or Prevention of arterial occlusive disease

In one embodiment, the ophthalmological disease is arterial occlusive disease. Examples of arterial occlusive disease include branch retinal artery occlusion, central retinal artery occlusion or ocular ischemic syndrome. A branch retinal artery occlusion (BRAO) can occur when one of the branches of the arterial supply to the retina becomes occluded.

### 5.5.11 Treatment or Prevention of central serous chorioretinopathy

In one embodiment, the ophthalmological disease is central serous chorioretinopathy (CSC). In one embodiment, CSC is characterized by leakage of fluid in the central macula.

### 5.5.12 Treatment or Prevention of cystoid macular edema

In one embodiment, the ophthalmological disease is cystoid macular edema (CME). In one embodiment, CME affects the central retina or macula. In another embodiment, CME occurs after cataract surgery.

### 5.5.13 Treatment or Prevention of retinal telangiectasia

In one embodiment, the ophthalmological disease is retinal telangiectasia. In one embodiment, retinal telangiectasia is characterized by dilation and tortuosity of retinal vessels and formation of multiple aneurysms. Idiopathic JXT, Leber's miliary aneurysms, and Coats' disease are three types of retinal telangiectasias.

### 5.5.14 Treatment or Prevention of arterial macroaneurysm

In one embodiment, the ophthalmological disease is arterial macroaneurysm.

### 5.5.15 Treatment or Prevention of retinal angiomatosis

In one embodiment, the ophthalmological disease is retinal angiomatosis. In one embodiment, retinal angiomatosis occurs when the ocular vessels form multiple angiomas.

### 5.5.16 Treatment or Prevention of radiation-induced retinopathy

In one embodiment, the ophthalmological disease is radiation-induced retinopathy (RIRP). In one embodiment, RIRP may display symptoms such as macular edema and nonproliferative and proliferative retinopathy.

### 5.5.17 Treatment or Prevention of Rubeosis iridis

In one embodiment, the ophthalmological disease is rubeosis iridis. In another embodiment, rubeosis iridis results in the formation of neovascular glaucoma. In another embodiment, rubeosis iridis is caused by diabetic retinopathy, central retinal vein occlusion, ocular ischemic syndrome, or chronic retinal detachment.

### 5.5.16 Treatment or Prevention of a neoplasm

In one embodiment, the ophthalmological disease is a neoplasm. Examples of neoplams include an eyelid tumor, a conjunctival tumor, a choroidal tumor, an iris tumor, an optic nerve tumor, a retinal tumor, an infiltrative intraocular tumor or an orbital tumor. Examples of an eyelid tumor include basal cell carcinoma, squamous carcinoma, sebaceous carcinoma, malignant melanoma, capillary hemangioma, hydrocystoma, nevus or seborrheic keratosis. Examples of a conjunctival tumor include conjunctival Kaposi's sarcoma, squamous carcinoma, intraepithelial neoplasia of the conjunctiva, epibular dermoid, lymphoma of the conjunctiva, melanoma, pingueculum, or pterygium. Examples of a choroidal tumor include choroidal nevus, choroidal hemangioma, metastatic choroidal tumor, choroidal osteoma, choroidal melanoma, ciliary body melanoma or nevus of Ota. Examples of an iris tumor include anterior uveal metastasis, iris cyst, iris melanocytoma, iris melanoma, or pearl cyst of the iris. Examples of an optic nerve tumor include optic nerve melanocytoma, optic nerve sheath meningioma, choroidal melanoma affecting the optic nerve, or circumpapillary metastasis with optic neuropathy. Examples of a retinal tumor include retinal pigment epithelial (RPE) hypertrophy, RPE adenoma, RPE carcinoma, retinoblastoma, hamartoma of the RPE, or von IIippel angioma. Examples of an infiltrative intraocular tumor include chronic lymphocytic leukemia, infiltrative choroidopathy, or intraocular lymphoma. Examples of an orbital tumor include adenoid cystic carcinoma of the lacrimal gland, cavernous hemangioma of the orbit, lymphangioma of the orbit, orbital mucocele, orbital pseudotumor, orbital rhabdomyosarcoma, periocular hemangioma of childhood, or sclerosing orbital psuedotumor.

### 5.6 Compositions for Therapeutic or Prophylactic Administration

The PDGF- antagonist or VEGF. antagonist of Table 1 or 2 can be administered as a component of a composition that further comprises a pharmaceutically acceptable carrier or vehicle. In one embodiment, a composition of the invention comprises an effective amount of a PDGF antagonist, a VEGF antagonist of Table 1 or 2 and a pharmaceutically accetable carrier or vehicle. In another embodiment, a composition comprising a PDGF antagonist and another composition comprising a VEGF antagonist are administered.

Administration of each antagonist may be by any suitable means that results in an amount of PDGF antagonist and VEGF antagonist of Table 1 or 2 that is effective for the treatment or prevention of an ophthalmological disease. Each antagonist, for example, can be admixed with a suitable carrier substance, and is generally present in an amount of 1-95% by weight of the total weight of the composition. The composition may be provided in a dosage form that is suitable for ophthalmic, oral, parenteral (*e.g*., intravenous, intramuscular, subcutaneous), rectal, transdermal, nasal, or inhalant administration. In one embodiment, the composition is in a form that is suitable for injection directly in the eye. The composition may be in form of, *e.g.,* tablets, capsules, pills, powders, granulates, suspensions, emulsions, solutions, gels including hydrogels, pastes, ointments, creams, plasters, delivery devices, suppositories, enemas, injeclables, implants, sprays, drops or aerosols. The compositions comprising one or more antagonists can be formulated according to conventional pharmaceutical practice (see, *e.g.,* Remington: The Science and Practice of Pharmacy, (20th ed.) ed. A. R. Gennaro, 2000, Lippincott Williams & Wilkins, Philadelphia, PA and Encyclopedia of Pharmaceutical Technology, eds., J. Swarbrick and J. C. Boylan, 1988-2002, Marcel Dekker, New York).

The compositions are, in one useful aspect, administered parenterally (*e.g*., by intramuscular, intraperitoneal, intravenous, intraocular, intravitreal, retro-bulbar, subconjunctival, subtenon or subcutaneous injection or implant) or systemically. Formulations for parenteral or systemic administration include sterile aqueous or non-aqueous solutions, suspensions, or emulsions. A variety of aqueous carriers can be used, *e*.*g*., water, buffered water, saline, and the like. Examples of other suitable vehicles include polypropylene glycol, polyethylene glycol, vegetable oils, gelatin, hydrogels, hydrogenated naphalenes, and injectable organic esters, such as ethyl oleate. Such formulations may also contain auxiliary substances, such as preserving, wetting, buffering, emulsifying, and/or dispersing agents. Biocompatible, biodegradable lactide polymer, lactide/glycolide copolymer, or polyoxyethylene-polyoxypropylene copolymers may be used to control the release of the active ingredients.

Alternatively, the compositions can be administered by oral ingestion. Compositions intended for oral use can be prepared in solid or liquid forms, according to any method known to the art for the manufacture of pharmaceutical compositions.

Solid dosage forms for oral administration include capsules, tablets, pills, powders, and granules. Generally, these pharmaceutical preparations contain active ingredients admixed with non-toxic pharmaceutically acceptable excipients. These include, for example, inert diluents, such as calcium carbonate, sodium carbonate, lactose, sucrose, glucose, mannitol, cellulose, starch, calcium phosphate, sodium phosphate, kaolin and the like. Binding agents, buffering agents, and/or lubricating agents (e.g., magnesium stearate) may also be used. Tablets and pills can additionally be prepared with enteric coatings. The compositions may optionally contain sweetening, flavoring, coloring, perfuming, and preserving agents in order to provide a more palatable preparation.

For example, compositions of the present invention may be administered intraocularly by intravitreal injection into the eye as well as by subconjunctival and subtenon injections. Other routes of administration include transcleral, retrobulbar, intraperitoneal, intramuscular, and intravenous. Alternatively, compositions can be administered using a drug delivery device or an intraocular implant (see below).

Liquid dosage forms for oral administration can include pharmaceutically acceptable emulsions, solutions, suspensions, syrups, and soft gelatin capsules. These forms can contain inert diluents commonly used in the art, such as water or an oil medium, and can also include adjuvants, such as wetting agents, emulsifying agents, and suspending agents.

In some instances, the compositions can also be administered topically, for example, by patch or by direct application to a region, such as the epidermis or the eye, susceptible to or affected by a neovascular disorder, or by iontophoresis.

Compositions useful for ophthalmic use include tablets comprising one or more antagonists in admixture with a pharmaceutically acceptable excipient. These excipients may be, for example, inert diluents or fillers (*e.g.,* sucrose and sorbitol), lubricating agents, glidants, and antiadhesives (*e.g*., magnesium stearate, zinc stearate, stearic acid, silicas, hydrogenated vegetable oils, or talc).

The antagonists of the present invention may be admixed in a tablet or other vehicle, or may be partitioned. In one example, one antagonist is contained on the inside of the tablet, and the other antagonist is on the outside, such that a substantial portion of the other antagonist is released prior to the release of the contained antagonist. If desired, antagonists in a tablet form may be administered using a drug delivery device (see below).

In one embodiment, compositions that comprise a PDGF antagonist can comprise one or more pharmaceutically acceptable excipients. In one embodiment, excipients for compositions that comprise a PDGF antagonist include, but are not limited to, buffering agents, nonionic surfactants, preservatives, tonicity agents, amino acids, and pH-adjusting agents. Suitable buffering agents include, but are not limited to, monobasic sodium phosphate, dibasic sodium phosphate, and sodium acetate. Suitable nonionic surfactants include, but are not limited to, polyoxyethylene sorbitan fatty acid esters such as polysorbate 20 and polysorbate 80. Suitable preservatives include, but are not limited to, benzyl alcohol. Suitable tonicity agents include, but are not limited to sodium chloride, mannitol, and sorbitol. Suitable amino acids include, but are not limited to glycine and histidine. Suitable pH-adjusting agents include, but are not limited to, hydrochloric acid, acetic acid, and sodium hydroxide. In one embodiment, the pH-adjusting agent or agents are present in an amount effective to provide a pH of about 3 to about 8, about 4 to about 7, about 5 to about 6, about 6 to about 7, or about 7 to about 7.5. In one embodiment, a composition comprising a PDGF antagonist does not comprise a preservative. In another embodiment, a composition comprising a PDGF antagonist does not comprise an antimicrobial agent. In another embodiment, a composition comprising a PDGF antagonist does not comprise a bacteriostat.

In one embodiment, a composition comprising a PDGF antagonist is in the form of an aqueous solution that is suitable for injection. In one embodiment, a composition comprises a PDGF antagonist, a buffering agent, a pH-adjusting agent, and water for injection. In another embodiment, a composition comprises a PDGF antagonist, monobasic sodium phosphate, dibasic sodium phosphate, sodium chloride, hydrochloride acid, and sodium hydroxide. In one embodiment, the PDGF antagonist is a pegylated anti-PDGF aptamer. In another embodiment, the pegylated anti-PDGF aptamer is ARC-127. In another embodiment, the pegylated anti-PDCF antagonist is a compound of Formula A. In another embodiment, the pegylated anti-PDGF antagonist is Antagonist A. In another embodiment, the pegylated anti-PDGF antagonist is a compound of Formula B. In another embodiment, the pegylated anti-PDGF antagonist is Antagonist B. In another embodiment, the pegylated anti-PDGF antagonist is a compound of Formula C. In another embodiment, the pegylated anti-PDGF antagonist is a compound of Formula D. In another embodiment, the PDGF antagonist is a non-pegylated anti-PDGF aptamer. In another embodiment, the non-pegylated aptamer is Antagonist C. In another embodiment, the non-pegylated aptamer is Antagonist D.

In one embodiment, compositions that comprise a VEGF antagonist can comprise one or more pharmaceutically acceptable excipients. In one embodiment, excipients for compositions that comprise a VEGF antagonist include, but are not limited to, buffering agents, nonionic surfactants, preservatives, tonicity agents, sugars, amino acids, and pH-adjusting agents. Suitable buffering agents include, but are not limited to, monobasic sodium phosphate, dibasic sodium phosphate, and sodium acetate. Suitable nonionic surfactants include, but are not limited to, polyoxyethylene sorbitan fatty acid esters such as polysorbate 20 and polysorbate 80. Suitable preservatives include, but are not limited to, benzyl alcohol. Suitable tonicity agents include, but are not limited to sodium chloride, mannitol, and sorbitol. Suitable sugars include, but are not limited to, α,α-trehalose. Suitable amino acids include, but are not limited to, glycine and histidine. Suitable pH-adjusting agents include, but are not limited to, hydrochloric acid, acetic acid, and sodium hydroxide. In one embodiment, the pH-adjusting agent or agents are present in an amount effective to provide a pH of about 3 to about 8, about 4 to about 7, about 5 to about 6, about 6 to about 7, or about 7 to about 7.5. In one embodiment, a composition comprising a VEGF antagonist does not comprise a preservative. Suitable excipients for the VEGF antagonist also include those described in U.S. Patent No. 7,365,166, the contents of which are herein incorporated by reference in their entirety.

In one embodiment, the composition is in the form of an aqueous solution that is suitable for injection. In one embodiment, the composition comprises a VEGF antagonist, a buffering agent, a sugar, a nonionic surfactant, and water for injection. In another embodiment, the composition comprises a VEGF antagonist, monobasic sodium phosphate, dibasic sodium phosphate, α,α-trehalose dehydrate, and polysorbate 20. In one embodiment, the composition comprises a VEGF antagonist, a buffering agent, a pH-adjusting agent, a tonicity agent, and water that is suitable for injection. In another embodiment, the composition comprises a VEGF antagonist, monobasic sodium phosphate, dibasic sodium phosphate, sodium chloride, hydrochloric acid, and sodium hydroxide. In one embodiment, the VEGF- antagonist is a pegylated anti-VEGF aptamer.

In another embodiment, the VEGF antagonist is ranibizumab or bevacizumab. This invention includes the pharmaceutically acceptable salts of the antagonists of Table 1 or 2. An antagonist of the present invention can possess a sufficiently basic functional group, which can react with any of a number of inorganic and organic acids, to form a pharmaceutically acceptable salt. A pharmaceutically-acceptable acid addition salt is formed from a pharmaceutically-acceptable acid, as is well known in the art. Such salts include the pharmaceutically acceptable salts listed in Journal of Pharmaceutical Science, 66, 2-19 (1977) and The Handbook of Pharmaceutical Salts; Properties, Selection, and Use. P. H. Stahl and C. G. Wermuth (ED.s), Verlag, Zurich (Switzerland) 2002, which are hereby incorporated by reference in their entirety.

Pharmaceutically acceptable salts include sulfate, citrate, acetate, oxalate, chloride, bromide, iodide, nitrate, bisulfate, phosphate, acid phosphate, isonicotinate, lactate, salicylate, acid citrate, tartrate, oleate, tannate, pantothenate, bitartrate, ascorbate, succinate, maleate, gentisinate, fumarate, gluconate, glucaronate, saccharate, formate, benzoate, glutamate, methanesulfonate, ethanesulfonate, benzenesulfonate, p-toluenesulfonate, camphorsulfonate, pamoate, phenylacetate, trifluoroacetate, acrylate, chlorobenzoate, dinitrobenzoate, hydroxybenzoate, methoxybenzoate, methylbenzoate, o-acetoxybenzoate, naphthalene-2-benzoate, isobutyrate, phenylbutyrate, .alpha.-hydroxybutyrate, butyne-1,4-dicarboxylate, hexyne-1,4-dicarboxylate, caprate, caprylate, cinnamate, glycollate, heptanoate, hippurate, malate, hydroxymaleate, malonate, mandelate, mesylate, nicotinate, phthalate, teraphthalate, propiolatc, propionate, phenylpropionate, sebacate, suberatc, p-bromobenzenesulfonate, chlorobenzenesulfonate, ethylsulfonate, 2-hydroxyethylsulfonate, methylsulfonate, naphthalene-1-sulfonate, naphthalene-2-sulfonate, naphthalene-1,5-sulfonate, xylenesulfonate, and tartarate salts. The term "pharmaceutically acceptable salt" also refers to a salt of an antagonists of the present invention having an acidic functional group, such as a carboxylic acid functional group, and a base. Suitable bases include, but are not limited to, hydroxides of alkali metals such as sodium, potassium, and lithium; hydroxides of alkaline earth metal such as calcium and magnesium; hydroxides of other metals, such as aluminum and zinc; ammonia, and organic amines, such as unsubstituted or hydroxy-substituted mono-, di-, or tri-alkylamines, dicyclohexylamine; tributyl amine; pyridine; N-methyl, N-ethylamine; diethylamine; triethylamine; mono-, bis-, or tris-(2-OH-lower alkylamines), such as mono-; bis-, or tris-(2-hydroxyethyl)amine, 2-hydroxy-tert-butylamine, or tris-(hydroxymethyl)methylamine, N,N-di-lower alkyl-N-(hydroxyl-lower alkyl)-amities, such as N,N-dimethyl-N-(2-hydroxyethyl)amine or tri-(2-hydroxyethyl)amine: N-methyl-D-glucamine; and amino acids such as arginine, lysine, and the like. The term "pharmaceutically acceptable salt" also includes a hydrate of a compound of the invention.

In one embodiment, each of the PDGF and VEGF antagonists of Table 1 or 2 is administered in an amount effective to treat or prevent an ophthalmological disease. The amount of antagonist that is admixed with the carrier materials to produce a single dosage can vary depending upon the mammal being treated and the particular mode of administration.

The dosage of each antagonist can depend on several factors including the severity of the condition, whether the condition is to be treated or prevented, and the age, weight, and health of the person to be treated. Additionally, pharmacogenomic (the effect of genotype on the pharmacokinetic, pharmacodynamic or efficacy profile of a therapeutic) information about a particular patient may affect dosage used. Furthermore, the exact individual dosages can be adjusted somewhat depending on a variety of factors, including the specific combination of antagonists being administered, the time of administration, the route of administration, the nature of the formulation, the rate of excretion, the particular ophthalmological disease being treated, the severity of the disorder, and the anatomical location of the neovascular disorder. Some variations in the dosage can be expected.

Generally, when orally administered to a mammal, the dosage of an antagonist of the present invention is normally 0.001 mg/kg/day to 100 mg/kg/day, 0.01 mg/kg/day to 50 mg/kg/day, or 0.1 mg/kg/day to 10 mg/kg/day. Generally, when orally administered to a human, the dosage of an antagonist of the present invention is normally 0.001 mg to 300 mg per day, 1 mg to 200 mg per day, or 5 mg to 50 mg per day. Dosages up to 200 mg per day may be necessary. For administration of an antagonist of the present invention by parenteral injection, the dosage is normally 0.1 mg to 250 mg per day, 1 mg to 20 mg per day, or 3 mg to 5 mg per day. Injections may be given up to four times daily. Generally, when orally or parenterally administered, the dosage of a PDGF or VEGF antagonist of Table 1 or 2 for use in the present invention is normally 0.1 mg to 1500 mg per day, or 0.5 mg to 10 mg per day, or 0.5 mg to 5 mg per day. A dosage of up to 3000 mg per day can be administered.

When ophthalmologically administered to a human, for example intravitreally, the dosage of an antagonist of Table 1 or 2 is normally 0.003 mg to 5.0 mg per eye per administration, or 0.03 mg to 3.0 mg per eye per administration, or 0.1 mg to 1.0 mg per eye per administration. In one embodiment, the dosage of PDGF antagonist of Table 1 or 2 is 0.03 mg, 0.3 mg, 1.5 mg or 3.0 mg per eye. In another ambodiment, the dosage of VEGF antagonist of Table 1 or 2 is 0.5 mg per eye. The dosage can range from 0.01 mL to 0.2 mL administered per eye, or 0.03 mL to 0.15 mL administered per eye, or 0.05 mL to 0.10 mL administered per eye.

For example, the PDGF aptamer Antagonist A, Antagonist B or Antagonist C or a pharmaceutically acceptable salt thereof can be delivered intravitreally at up to 30 mg/ml with injection volumes up to 100 µL.

Administration of each antagonist of Table 1 or 2 can, independently, be one to four times daily or one to four times per month or one to six times per year or once every two, three, four or five years. Administration can be for the duration of one day or one month, two months, three months, six months, one year, two years, three years, and may even be for the life of the patient. In one embodiment, the administration is performed once a month for three months. Chronic, long-term administration will be indicated in many cases. The dosage may be administered as a single dose or divided into multiple doses. In general, the desired dosage should be administered at set intervals for a prolonged period, usually at least over several weeks or months, although longer periods of administration of several months or years or more may be needed.

In addition to treating pre-existing ophthalmological diseases, the compositions can be administered prophylactically in order to prevent or slow the onset of these disorders. In prophylactic applications, the composition can be administered to a patient susceptible to or otherwise at risk of a particular ophthalmological disease.

In one embodiment, the PDGF antagonist and the VEGF antagonist of Table 1 or 2 are administered to a mammal in need of treatment therewith, typically in the form of an injectable pharmaceutical composition. The PDGF antagonist and VEGF antagonist of Table 1 or 2 can be administered either in separate compositions or in a pharmaceutical composition comprising both the PDGF antagonist and VEGF antagonist. The administration can be by injection, for example by intraocular injection, or by using a drug delivery device. Parenteral, systemic, or transdermal administration is also within the scope of the invention.

The administration of the PDGF antagonist and the VEGF antagonist of Table 1 or 2 can be sequential in time or concurrent. When administered sequentially, the administration of each can be by the same or different route. In one embodiment, a PDGF antagonist of Table 1 or 2 is administered within 90 days, 30 days, 10 days, 5 days, 24 hours , 1 hour, 30 minutes, 10 minutes, 5 minutes or one minute of administration of a VEGF antagonist of Table 1 or 2. Where the PDGF antagonist is administered prior to the VEGF antagonist, the VEGF antagnoist is administered within a time and in an amount such that the total amount of PDGF antagonist and VEGF antagonist is effective to treat or prevent an ophthalmological disease. Where the VEGF antagonist is administered prior to the PDGF antagonist, the PDGF antagnoist is administered within a time and in an amount such that the total amount of PDGF antagonist and VEGF antagonist is effective to treat or prevent an ophthalmological disease.

Pharmaceutical compositions according to the invention may be formulated to release a PDGF or VEGF antagonist of Table 1 or 2 substantially immediately upon administration or at any predetermined time period after administration, using controlled release formulations. For example, a pharmaceutical composition can be provided in sustained-release form. The use of immediate or sustained release compositions depends on the nature of the condition being treated. If the condition consists of an acute disorder, treatment with an immediate release form can be utilized over a prolonged release composition. For certain preventative or long-term treatments, a sustained released composition can also be appropriate.

Administration of one or both of the antagonists of Table 1 or 2 in controlled release formulations can be useful where the antagonist, either alone or in combination, has (i) a narrow therapeutic index (e.g., the difference between the plasma concentration leading to harmful side effects or toxic reactions and the plasma concentration leading to a therapeutic effect is small; generally, the therapeutic index, TI, is defined as the ratio of median lethal dose (LD₅₀) to median effective dose (ED₅₀)); (ii) a narrow absorption window in the gastro-intestinal tract; or (iii) a short biological half-life, so that frequent dosing during a day is required in order to sustain the plasma level at a therapeutic level.

Many strategies can be pursued to obtain controlled release in which the rate of release outweighs the rate of degradation or metabolism of the therapeutic antagonist. For example, controlled release can be obtained by the appropriate selection of formulation parameters and ingredients, including, *e*.*g*., appropriate controlled release compositions and coatings. Examples include single or multiple unit tablet or capsule compositions, oil solutions, suspensions, emulsions, microcapsules, microspheres, nanoparticles, patches, and liposomes. Methods for preparing such sustained or controlled release formulations are well known in the art.

The PDGF antagonist or VEGF antagonist can also be delivered using a drug-delivery device such as an implant. Such implants can be biodegradable and/or biocompatible, or can be non-biodegradable. The implants can be permeable to the PDGF antagonist or VEGF antagonist. Ophthalmic drug delivery devices can be inserted into a chamber of the eye, such as the anterior or posterior chamber or can be implanted in or on the sclera, choroidal space, or an avascularized region exterior to the vitreous. In one embodiment, the implant can be positioned over an avascular region, such as on the sclera, so as to allow for transcleral diffusion of the PDGF antagonist or VEGF antagonist to the desired site of treatment, *e.g.,* the intraocular space and macula of the eye. Furthermore, the site of transcleral diffusion can be proximal to a site of neovascularization such as a site proximal to the macula. Suitable drug delivery devices are described, for example, in U.S. Publication Nos. 2008/0286334; 2008/0145406; 2007/0184089; 2006/0233860; 2005/0244500; 2005/0244471; and 2005/0244462, and U.S. Patent Nos. 6,808,719 and 5,322,691, the contents of each of which is herein incorporated by reference in its entirety.

In one embodiment, the implant comprises a PDGF antagonist and/or VEGF antagonist dispersed in a biodegradable polymer matrix. The matrix can comprise PLGA (polylactic acid-polyglycolic acid copolymer), an ester-end capped polymer, an acid end-capped polymer, or a mixture thereof. In another embodiment, the implant comprises a PDGF antagonist and/or a VEGF antagonist, a surfactant, and lipophilic compound. The lipophilic compound can be present in an amount of about 80-99% by weight of the implant. Suitable lipophilic compounds include, but are not limited to, glyceryl palmitostearate, dicthylene glycol monostearate, propylene glycol monostearate, glyceryl monostearate, glyceryl monolinoleate, glyceryl monooleate, glyceryl monopalmitate, glyceryl monolaurate, glyceryl dilaurate, glyceryl monomyristate, glyceryl dimyristate, glyceryl monopalmitate, glyceryl dipalmitate, glyceryl monostearate, glyceryl distearate, glyceryl monooleate, glyceryl dioleate, glyceryl monolinoleate, glyceryl dilinoleate, glyceryl monoarachidate, glyceryl diarachidate, glyceryl monobehenate, glyceryl dibehenate, and mixtures thereof. In another embodiment, the implant comprises a PDGF antagonist and/or a VEGF antagonist housed within a hollow sleeve. The PDGF antagonist or VEGF antagonist, or both, are delivered to the eye by inserting the sleeve into the eye, releasing the implant from the sleeve into the eye, and then removing the sleeve from the eye. An example of this delivery device is described in U.S. Publication No. 2005/0244462, which is hereby incorporated by reference in its entirety.

In one embodiment, the implant is a flexible ocular insert device adapted for the controlled sustained release of a PDGF antagonist and/or a VEGF antagonist into the eye. In one embodiment, the device includes an elongated body of a polymeric material in the form of a rod or tube containing a PDGF antagonist, VEGF antagonist or both, and with at least two anchoring protrusions extending radially outwardly from the body. The device may have a length of at least 8 mm and the diameter of its body portion including the protrusions does not exceed 1.9 mm. The sustained release mechanism can, for example, be by diffusion or by osmosis or bioerosion. The insert device can be inserted into the upper or lower fornix of the eye so as to be independent of movement of the eye by virtue of the fornix anatomy. The protrusions can be of various shapes such as, for example, ribs, screw threads, dimples or bumps, truncated cone-shaped segments or winding braid segments. In a further embodiment, the polymeric material for the body is selected as one which swells in a liquid environment. Thus a device of smaller initial size can be employed. The insert device can be of a size and configuration such that, upon insertion into the upper or lower fornix, the device remains out of the field of vision so as to be well retained in place and imperceptible by a recipient over a prolonged period of use. The device can be retained in the upper or lower fornix for 7 to 14 days or longer. An example of this device is described in U.S. Patent No. 5,322,691, which is hereby incorporated by reference in its entirety.

The invention relates to kits comprising one or more pharmaceutical compositions and instructions for use. At least two antagonists of Table 1 or 2 can be formulated together or in separate compositions and in individual dosage amounts. The antagonists of Table 1 or 2 are also useful when formulated as pharmaceutically acceptable salts. In one embodiment, the kits comprise a composition comprising a PDGF antagonist and a pharmaceutically acceptable carrier or vehicle and another composition comprising a VEGF antagonist and a pharmaceutically acceptable carrier or vehicle. In another embodiment, the kits comprise a composition comprising a VEGF antagonist, a PDGF antagonist and a pharmaceutically acceptable carrier or vehicle. Each of the kits' compositions can be contained in a container.

The kits can comprise (1) an amount of a PDGF antagonist of Table 1 or 2 and a pharmaceutically acceptable carrier, vehicle, or diluent in a first unit dosage form; (2) an amount of a VEGF antagonist of Table 1 or 2 and a pharmaceutically acceptable carrier, vehicle, or diluent in a second unit dosage form; and (3) a container. The container can be used to separate components and include, for example, a divided bottle or a divided foil packet. The separate antagonist compositions may also, if desired, be contained within a single, undivided container. The kits can also comprise directions for the administration of the antagonists. The kits are particularly advantageous when the separate components are administered in different dosage forms, are administered at different dosage levels, or when titration of the individual antagonists is desired.

### 7. EXAMPLES

### Example 1: Corneal Neovascularization (Corneal NV)

Corneal Neovascularization is a widely used animal model that allows clear visualization of abnormal vascular growth in the eye. The vessels that grow into the normally avascular cornea, can become well established, making this an attractive model to study vessel regression. To induce experimental corneal NV, male C57BL/6 mice (18-20 g; Charles River, Wilmington, MA) are anesthetized with intramuscular ketamine hydrochloride (25 mg/kg) and xylazine (10 mg/kg). NaOH (2 ul of 0.2 mM) is applied topically. The corneal and limbal epithelia are removed by applying a rotary motion parallel to the limbus using #21 blade (Feather, Osaka, Japan). After 7 days, mice are treated with intra-peritoneal injections of 2.0 mg/ml of Antagonist A, an anti-PDGF aptamer, agent twice a day or by intra-peritoneal injections of 2.0 mg/mL of ranibizumab (as the commercially available composition Lucentis^{®}, an anti-VEGF antibody agent, twice a day or both for 7 days. At day 14 following corneal NV induction, mice receive 20 ug/g of fluorescein-isothiocyanate coupled concanavalin A lectin (Vector Laboratories, Burlingame, CA) intravenously while deeply anesthetized with xylazine hydrochloride and ketamine hydrochloride. Thirty minutes later, mice eyes are enucleated, and the corneas flat-mounted. Corneal NV is visualized using fluorescence microscopy and quantified using Openlab software. The percent of cornea covered by vessels is calculated as a percentage of total corneal area.

The effects of the administration of Antagonist A and ranibizumab are measured for decrease in vessel growth and pictures of the fluorescent microscopic image are taken.

### Example 2: Administration of Antagonist A and Ranibizumab

The objectives of this study were to assess safety of Antagonist A, an intravitreal anti-PDGF aptamer targeting pericytes, in combination with ranibizumab in subjects with neovascular age-related macular degeneration (NV-AMD).

Dose-escalating, uncontrolled, single- and multiple-dose, multicenter phase 1 study. Included were subjects with predominantly or minimally classic subfoveal NV-AMD ≤ 5 disc areas in total lesion size. Subjects were enrolled in a dose escalation scheme to a single injection of 0.03 mg/eye and 3 monthly injections of ranibizumab (as the commercially available composition Lucentis^{®}) 0.5 mg/eye (n=3), or to three monthly injections of one of 4 different doses of Antagonist A (0.03, 0.3, 1.5, 3.0 mg/eye) and ranibizumab (as the commercially available composition Lucentis^{®}) (0.5mg/eye) (n=3-8/dose), administered as separate injections. Assessments included vital signs and clinical lab tests, complete ocular examination with intraocular pressure, standardized ETDRS visual acuity, color fundus photos and fluorescein angiography, and optical coherence tomography.

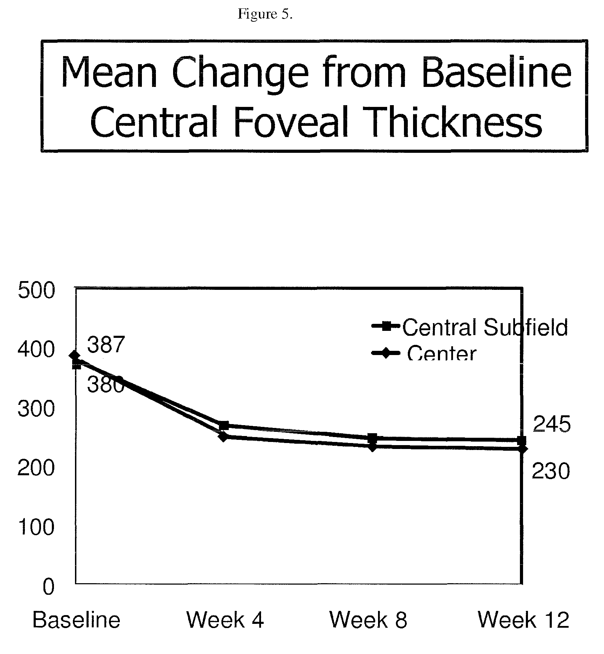

No evidence of drug related adverse events were detected. All of the ocular adverse events were associated with the intravitreal injection. In the combined analysis of 22 subjects over 12 weeks, 36%, 45% and 59% of the subjects gained ≥ 15 letters at weeks 4, 8, and 12 respectively. The mean change in visual acuity was +11.2, +12.3 and + 14.0 ETDRS letters at weeks 4 (n=22), 8 (n=22), and 12 (n=22). The mean center point retinal thickness was 387 µm at baseline and 230 µm at week 12 (see Figure 5). Analysis of FA by independent readers revealed a mean decrease in CNV area of 86% at Week 12 compared to baseline.

These results suggest potential bioactivity associated with regression of the neovascular membrane.

### Example 3: Patterns of choroidal neovascularization (CNV) Fluorescein and Indocyanine Green Angiographic Regression Responses After Ranibizumab Monotherapy or Ranibizumab and Antagonist A Combotherapy

Anti-VEGF monotherapy for NV-AMD can cause stabilization of CNV lesion size and leakage. The fluorescein angiographic (FA) and dynamic indocyanine green angiographic (ICGA) patterns of CNV regression responses in eyes receiving either ranibizumab only or ranibizumab and Antagonist A were compared.

A retrospective review was performed of 20 cases of NV-AMD in which 2-3 doses of intravitreal ranibizumab (as the commercially available composition Lucentis^{®}) monotherapy successfully induced anatomic improvement by OCT. These eyes were compared with 13 eyes of patients in a study of monthly intravitreal) ranibizumab (as the commercially available composition Lucentis^{®}) (up to 3 doses) plus intravitreal Antagonist A (at least one but up to 3 doses). Eyes were imaged by FA pretreatment and at various times post treatment. Eyes could also be imaged by dynamic ICGA (Spectralis, Heidelberg). Angiograms were evaluated to assess the changes in lesion size and vascular perfusion.

Three angiographic patterns of "OCT successful" responses to treatment were observed. (1) Stable inactivity was characterized by FA with stable lesion size and uniform low grade fluorescein hyperfluorescence (staining) of the CNV. ICGA typically demonstrated persistence of feeder arteries with branching arterioles. (2) Vascular regression demonstrated FA with stable CNV area but shrinkage of area of fluorescein staining. ICGA demonstrated disappearance of homogenous capillaries and small branching arterioles. (3) Lesion regression was characterized by partial to nearly complete disappearance of both the CNV lesion and hyperfluorescent staining. Persistent hypofluorescence in the bed of the CNV was often present. ICGA revealed significant disappearance of most vascular components. Partial or extensive lesion regression occurred in 85% (11 of 13 eyes) treated with ranibizumab and Antagonist A, compared with only 20% (4 of 20 eyes) treated with ranibizumab monotherapy. In contrast, stable inactivity was observed in only 15% (2 of 13 eyes) treated with ranibizumab and Antagonist A versus 55% (11 of 20 eyes) treated with ranibizumab monotherapy.

### Example 4: Synthesis of Antagonist A

The iterative chemical synthesis of the 32-mer oligonucleotide of Antagonist A was performed on a solid phase inverted deoxyribothymidine controlled pore glass (CPG) support using a flow through reactor design. The oligonucleotide synthesis process was comprised of four chemical reactions carried out in the following sequence:
(a) deblocking of the dimethyoxytrityl (DMT) protected nucleoside or nascent oligonucleotide (detritylation); (b) activation and coupling of the incoming phosphoramidite (amidite); (c) oxidation of the resultant phosphite triester to the pentavalent phosphate linkage; and (d) capping of oligonucleotide chains that failed to successfully couple.

Starting with an inverted thymidine CPG support (3'-DMT-5'-dT-CPG) the four steps above were repeated to add phosphoramidites in the order of the sequence until the desired oligonucleotide, terminating in the hexylamino linker, was synthesized. The internal hexaethylene glycol spacers were coupled in the same manner as the other phosphoramidites.

The first step in the cycle involved removal of the dimethyoxytrityl protecting group on the terminal hydroxyl group of the nascent oligonucleotide chain. This was achieved by treating the DMT protected oligonucleotide on CPG with a solution of dichloroacetic acid in dichloromethane. This reaction produced the unprotected terminal hydroxyl group. The cleaved DMT group was removed with the dichloroacetic acid/dichloromethane (DCA/DCM) solvent. The CPG was then washed with acetonitrile (ACN).

The second step involved activation of the incoming phosphoramidite with ethylthiotetrazole (ETT) to produce a species that would quickly couple with the terminal hydroxyl group produced in the previous step. The resultant phosphite triester was washed with ACN to remove activator and unreacted phosphoramidite.

The third step is oxidation of the newly formed phosphite triester to the pentavalent phosphate. This was accomplished by reacting the phosphite triester with a mixture of iodine and pyridine in water. Unused oxidant was washed from the CPG with ACN.

The fourth step involved capping of any unreacted hydroxyls that had failed to couple. The CPG was treated with a mixture of CAP NMI (N-methylimidazole in ACN) and CAP ALA (acetic anhydride, 2,6-lutidine, ACN). These reagents were washed from the CPG with ACN.

This cycle of four reactions was repeated until an oligonucleotide of the correct length and sequence was assembled on the solid support. The last phosphoramidite (hexylamino linker at the 5' terminus of the oligonucleotide) was reacted in the same fashion as the other phosphoramidites used in the synthesis; however, this linker was not capped.

The oligonucleotide was deprotected and cleaved by treating the solid support, containing the crude synthesized oligonucleotide, with a t-butyl amine/ammonium hydroxide solution. The CPG was separated from the deprotected and cleaved oligonucleotide. The purity of the crude fully deprotected oligonucleotide was determined by analytical anion exchange chromatography and met a specification of greater than 50%.

The resultant oligonucleotide from Stage 1 was filtered, diluted and purified by preparative anion exchange chromatography (AX HPLC). Fractions were analyzed for product purity by analytical anion exchange HPLC. Individual fractions with a purity greater than 70% unpegylated aptamer, defined as the full length oligonucleotide that contains the 5'-hexylamino linker, were combined. In preparation for pegylation, the resultant fraction pool was first desalted and then concentrated using ultrafiltration. In some instances, the anion exchange chromatography step was replaced by a step in which diafiltration against sodium chloride was used to remove amine salts prior to Stage3.

In forming a covalent bond between the primary amine on the 5' end of the oligonucleotide and the pegylation reagent (mPEG2- NHS ester), the reaction was conducted at pH 9 in sodium borate buffer. The reaction has been demonstrated to be site specific to the hexylamino linker at the 5' end of the oligonucleotide using the pegylation conditions described.

The pegylated oligonucleotide was purified from unconjugated PEG reagent, unpegylated aptamer, and other by-products by the same preparative AX HPLC method described above for Stage 2. The individual fractions were analyzed by analytical AX HPLC. Fractions with greater than 85% full length pegylated oligonucleotide were pooled and the resultant pool was desalted, concentrated, and filtered.

The resultant drug substance was vacuum freeze dried to reduce the water content.

### Example 5: Choroidal Neovascularization (CNV)

Experimental CNV is useful as a model for Age-related Macular degeneration (AMD). In CNV, vessels of the choroid grow through breaks in Bruch's membrane and into the retina, similar to what is observed in AMD patients. To induce experimental CNV, male C57BL/6 mice (18-20 g; Charles River, Wilmington, MA) are anesthetized with intramuscular ketamine hydrochloride (25 mg/kg) and xylazine (10 mg/kg) and the mice pupils are dilated with 1% tropicamide. Four burns are generated using diode laser photocoagulation (75-µm spot size, 0.1-second duration, 90 mW, Oculight SL laser, IRIDEX, Mountain View,CA) and a hand-held cover slide as a contact lens. Burns are localized to the 3, 6, 9 and 12 o'clock positions of the posterior pole of the retina. Production of a bubble in the choroid at the time of laser photocoagulation, which indicates rupture of Bruch's membrane, is an important factor in obtaining choroidal neovascularization, so only mice in which a bubble is produced for all four burns are included in the study. After 7 days, mice are treated with (a) an intra-peritoneal injection of 2.0 mg/ml of Antagonist A twice a day for seven days; (b) an intra-peritoneal injection of 2.0 mg/mL of ranibizumab (as the commercially available composition Lucentis^{®}) twice a day for 7 days; or (c) an intra-peritoneal injection of 2.0 mg/ml of Antagonist A and an intra-peritoneal injection of 2.0 mg/mL of ranibizumab (as the commercially available composition Lucentis^{®}), both being administered twice a day for 7 days. The area of choroidal NV lesions is measured in flat-mounted choroid stained with platelet endothelial cell adhesion molecule (PECAM) antibody. Flat-mounts are examined by fluorescence microscopy and quantified using Openlab software.

The effects of the administration of one or more of (a), (b) and (c) are measured for decrease in CNV area compared to untreated controls.

### 8. INCORPORATION BY REFERENCE

All publications and patent applications disclosed in this specification are herein incorporated by reference to the same extent as if each individual publication or patent application was specifically and individually indicated to be incorporated by reference.

### FURTHER EMBODIMENTS:

**1.** A method for treating or preventing an ophthalmological disease, comprising administering to a mammal in need thereof an effective amount of:
   (a) Antagonist A or a pharmaceutically acceptable salt thereof; and
   (b) ranibizumab, bevacizumab, aflibercept, KH902 VEGF receptor-Fc fusion protein, 2C3 antibody, ORA102, pegaptanib, bevasiranib, SIRNA-027, decursin, decursinol, picropodophyllin, guggulsterone, PLG101, eicosanoid LXA4, PTK787, pazopanib, axitinib, CDDO-Me, CDDO-Imm, shikonin, beta-hydroxyisovalerylshikonin, or ganglioside GM3, DC101 antibody, Mab25 antibody, Mab73 antibody, 4A5 antibody, 4E10 antibody, 5F12 antibody, VA01 antibody, BL2 antibody, VEGF-related protein, sFLT01, sFLT02, Peptide B3, TG100801, sorafenib, or G6-31 antibody, or a pharmaceutically acceptable salt thereof.
**2.** A method for treating or preventing an ophthalmological disease, comprising administering to a mammal in need thereof an effective amount of:
   (a) a compound of Formula B or a pharmaceutically acceptable salt thereof; and
   (b) ranibizumab, bevacizumab, aflibercept, KH902 VEGF receptor-Fc fusion protein, 2C3 antibody, ORA102, pegaptanib, bevasiranib, SIRNA-027, decursin, decursinol, picropodophyllin, guggulsterone, PLG101, eicosanoid LXA4, PTK787, pazopanib, axitinib, CDDO-Me, CDDO-Imm, shikonin, beta-hydroxyisovalerylshikonin, or ganglioside GM3, DC101 antibody, Mab25 antibody, Mab73 antibody, 4A5 antibody, 4E10 antibody, 5F12 antibody, VA01 antibody, BL2 antibody, VEGF-related protein, sFLT01, sFLT02, Peptide B3, TG100801, sorafenib, or G6-31 antibody, or a pharmaceutically acceptable salt thereof.
**3.** A method for treating or preventing an ophthalmological disease, comprising administering to a mammal in need thereof an effective amount of:
   (a) a compound of Formula C or a pharmaceutically acceptable salt thereof; and
   (b) ranibizumab, bevacizumab, aflibercept, KH902 VEGP receptor-Fc fusion protein, 2C3 antibody, ORA102, pegaptanib, bevasiranib, SIRNA-027, decursin, decursinol, picropodophyllin, guggulsterone, PLG101, eicosanoid LXA4, PTK787, pazopanib, axitinib, CDDO-Me, CDDO-Imm, shikonin, beta-hydroxyisovalerylshikonin, or ganglioside GM3, DC101 antibody, Mab25 antibody, Mab73 antibody, 4A5 antibody, 4E10 antibody, 5F12 antibody, VA01 antibody, BL2 antibody, VEGF-related protein, sFLT01, sFLT02, Peptide B3, TG100801, sorafenib, or G6-31 antibody, or a pharmaceutically acceptable salt thereof.
**4.** A method for treating or preventing an ophthalmological disease, comprising administering to a mammal in need thereof an effective amount of:
   (a) Antagonist D or a pharmaceutically acceptable salt thereof; and
   (b) ranibizumab, bevacizumab, aflibercept, KH902 VEGF receptor-Fc fusion protein, 2C3 antibody, ORA102, pegaptanib, bevasiranib, SIRNA-027, decursin, decursinol, picropodophyllin, guggulsterone, PLG101, eicosanoid LXA4, PTK787, pazopanib, axitinib, eDDO-Me, CDDO-Imm, shikonin, beta-hydroxyisovalerylshikonin, or ganglioside GM3, DC101 antibody, Mab25 antibody, Mab73 antibody, 4A5 antibody, 4E10 antibody, 5F12 antibody, VA01 antibody, BL2 antibody, VEGF-related protein, sFLT01, sFLT02, Peptide B3, TG100801, sorafenib, or G6-31 antibody, or a pharmaceutically acceptable salt thereof.
**5.** A method for treating or preventing an ophthalmological disease, comprising administering to a mammal in need thereof an effective amount of:
   (a) a compound of Formula E or a pharmaceutically acceptable salt thereof; and
   (b) ranibizumab, bevacizumab, aflibercept, KH902 VEGF receptor-Fc fusion protein, 2C3 antibody, ORA102, pegaptanib, bevasiranib, SIRNA-027, decursin, decursinol, picropodophyllin, guggulsterone, PLG101, eicosanoid LXA4, PTK787, pazopanib, axitinib, CDDO-Me, CDDO-Imm, shikonin, beta-hydroxyisovalerylshikonin, or ganglioside GM3, DC101 antibody, Mab25 antibody, Mab73 antibody, 4A5 antibody, 4E10 antibody, 5F12 antibody, VA01 antibody, BL2 antibody, VEGF-related protein, sFLT01, sFLT02, Peptide B3, TG100801, sorafenib, or G6-31 antibody, or a pharmaceutically acceptable salt thereof.
**6.** The method of item 1, wherein the ophthalmological disease is age-related macular degeneration, polypoidal choroidal vasculopathy, condition associated with choroidal neovascularization, hypertensive retinopathy, diabetic retinopathy, sickle cell retinopathy, condition associated with peripheral retinal neovascularization, retinopathy of prematurity, venous occlusive disease, arterial occlusive disease, central serous chorioretinopathy, cystoid macular edema, retinal telangiectasia, arterial macroaneurysm, retinal angiomatosis, radiation-induced retinopathy, rubeosis iridis, or a neoplasm.
**7.** The method of item 2, wherein the ophthalmological disease is age-related macular degeneration, polypoidal choroidal vasculopathy, condition associated with choroidal neovascularization, hypertensive retinopathy, diabetic retinopathy, sickle cell retinopathy, condition associated with peripheral retinal neovascularization, retinopathy of prematurity, venous occlusive disease, arterial occlusive disease, central serous chorioretinopathy, cystoid macular edema, retinal telangiectasia, arterial macroaneurysm, retinal angiomatosis, radiation-induced retinopathy, rubeosis iridis, or a neoplasm.
**8.** The method of item 3, wherein the ophthalmological disease is age-related macular degeneration, polypoidal choroidal vasculopathy, condition associated with choroidal neovascularization, hypertensive retinopathy, diabetic retinopathy, sickle cell retinopathy, condition associated with peripheral retinal neovascularization, retinopathy of prematurity, venous occlusive disease, arterial occlusive disease, central serous chorioretinopathy, cystoid macular edema, retinal telangiectasia, arterial macroaneurysm, retinal angiomatosis, radiation-induced retinopathy, rubeosis iridis, or a neoplasm.
**9.** The method of item 4, wherein the ophthalmological disease is age-related macular degeneration, polypoidal choroidal vasculopathy, condition associated with choroidal neovascularization, hypertensive retinopathy, diabetic retinopathy, sickle cell retinopathy, condition associated with peripheral retinal neovascularization, retinopathy of prematurity, venous occlusive disease, arterial occlusive disease, central serous chorioretinopathy, cystoid macular edema, retinal telangiectasia, arterial macroaneurysm, retinal angiomatosis, radiation-induced retinopathy, rubeosis iridis, or a neoplasm.
**10.** The method of item 5, wherein the ophthalmological disease is age-related macular degeneration, polypoidal choroidal vasculopathy, condition associated with choroidal neovascularization, hypertensive retinopathy, diabetic retinopathy, sickle cell retinopathy, condition associated with peripheral retinal neovascularization, retinopathy of prematurity, venous occlusive disease, arterial occlusive disease, central serous chorioretinopathy, cystoid macular edema, retinal telangiectasia, arterial macroaneurysm, retinal angiomatosis, radiation-induced retinopathy, rubeosis iridis, or a neoplasm.
**11.** The method of item 1, wherein (a) and (b) are administered within 24 hours of each other.
**12.** The method of item 2, wherein (a) and (b) are administered within 24 hours of each other.
**13.** The method of item 3, wherein (a) and (b) are administered within 24 hours of each other.
**14.** The method of item 4, wherein (a) and (b) are administered within 24 hours of each other.
**15.** The method of item 5, wherein (a) and (b) are administered within 24 hours of each other.
**16.** The method of item 1, wherein (a) and (b) are administered within 60 minutes of each other.
**17.** The method of item 2, wherein (a) and (b) are administered within 60 minutes of each other.
**18.** The method of item 3, wherein (a) and (b) are administered within 60 minutes of each other.
**19.** The method of item 4, wherein (a) and (b) are administered within 60 minutes of each other.
**20.** The method of item 5, wherein (a) and (b) are administered within 60 minutes of each other.
**21.** The method of item 1, wherein (a) and (b) are administered concurrently.
**22.** The method of item 2, wherein (a) and (b) are administered concurrently.
**23.** The method of item 3, wherein (a) and (b) are administered concurrently.
**24.** The method of item 4, wherein (a) and (b) are administered concurrently.
**25.** The method of item 5, wherein (a) and (b) are administered concurrently.
**26.** The method of item 1, wherein (a) and (b) are present in the same composition.
**27.** The method of item 2, wherein (a) and (b) are present in the same composition.
**28.** The method of item 3, wherein (a) and (b) are present in the same composition.
**29.** The method of item 4, wherein (a) and (b) are present in the same composition.
**30.** The method of item 5, wherein (a) and (b) are present in the same composition.
**31.** The method of item 1, further comprising administering an effective amount of another agent useful for treating or preventing an ophthalmological disease.
**32.** The method of item 2, further comprising administering an effective amount of another agent useful for treating or preventing an ophthalmological disease.
**33.** The method of item 3, further comprising administering an effective amount of another agent useful for treating or preventing an ophthalmological disease.
**34.** The method of item 4, further comprising administering an effective amount of another agent useful for treating or preventing an ophthalmological disease.
**35.** The method of item 5, further comprising administering an effective amount of another agent useful for treating or preventing an ophthalmological disease.
**36.** The method of item 1, wherein (a) or (b) are present in a drug-delivery device.
**37.** The method of item 2, wherein (a) or (b) are present in a drug-delivery device.
**38.** The method of item 3, wherein (a) or (b) are present in a drug-delivery device.
**39.** The method of item 4, wherein (a) or (b) are present in a drug-delivery device.
**40.** The method of item 5, wherein (a) or (b) are present in a drug-delivery device.
**41.** The method of item 1, wherein (a) and (b) are present in a drug-delivery device.
**42.** The method of item 2, wherein (a) and (b) are present in a drug-delivery device.
**43.** The method of item 3, wherein (a) and (b) are present in a drug-delivery device.
**44.** The method of item 4, wherein (a) and (b) are present in a drug-delivery device.
**45.** The method of item 5, wherein (a) and (b) are present in a drug-delivery device.
**46.** The method of item 1, wherein (a) and (b) are present in the same drug-delivery device.
**47.** The method of item 2, wherein (a) and (b) are present in the same drug-delivery device.
**48.** The method of item 3, wherein (a) and (b) are present in the same drug-delivery device.
**49.** The method of item 4, wherein (a) and (b) are present in the same drug-delivery device.
**50.** The method of item 5, wherein (a) and (b) are present in the same drug-delivery device.
**51.** The method of item 1, wherein (a) or (b) are administered intraocularly.
**52.** The method of item 2, wherein (a) or (b) are administered intraocularly.
**53.** The method of item 3, wherein (a) or (b) are administered intraocularly.
**54.** The method of item 4, wherein (a) or (b) are administered intraocularly.
**55.** The method of item 5, wherein (a) or (b) are administered intraocularly.
**56.** The method of item 1, wherein (a) and (b) are administered intraocularly.
**57.** The method of item 2, wherein (a) and (b) are administered intraocularly.
**58.** The method of item 3, wherein (a) and (b) are administered intraocularly.
**59.** The method of item 4, wherein (a) and (b) are administered intraocularly.
**60.** The method of item 5, wherein (a) and (b) are administered intraocularly.
**61.** The method of item 51, wherein intraocular administration is by intravitreal administration or anterior chamber administration.
**62.** The method of item 52, wherein intraocular administration is by intravitreal administration or anterior chamber administration.
**63.** The method of item 53, wherein intraocular administration is by intravitreal administration or anterior chamber administration.
**64.** The method of item 54, wherein intraocular administration is by intravitreal administration or anterior chamber administration.
**65.** The method of item 55, wherein intraocular administration is by intravitreal administration or anterior chamber administration.
**66.** The method of item 56, wherein intraocular administration is by intravitreal administration or anterior chamber administration.
**67.** The method of item 57, wherein intraocular administration is by intravitreal administration or anterior chamber administration.
**68.** The method of item 58, wherein intraocular administration is by intravitreal administration or anterior chamber administration.
**69.** The method of item 59, wherein intraocular administration is by intravitreal administration or anterior chamber administration.
**70.** The method of item 60, wherein intraocular administration is by intravitreal administration or anterior chamber administration.
**71.** The method of item 1, wherein the mammal is a human.
**72.** The method of item 2, wherein the mammal is a human.
**73.** The method of item 3, wherein the mammal is a human.
**74.** The method of item 4. wherein the mammal is a human.
**75.** The method o1 item 5, wherein the mammal is a human.
**76.** The method of item 1, wherein the (a) is administered at a dosage of at least 0.003 mg.
**77.** The method of item 2, wherein the (a) is administered at a dosage of at least 0.003 mg.
**78.** The method of item 3, wherein the (a) is administered at a dosage of at least 0.003 mg.
**79.** The method of item 4, wherein the (a) is administered at a dosage of at least 0.003 mg.
**80.** The method of item 5, wherein the (a) is administered at a dosage of at least 0.003 mg.
**81.** The method of item 1, wherein the (b) is administered at a dosage of at least 0.003 mg.
**82.** The method of item 2, wherein the (b) is administered at a dosage of at least 0.003 mg.
**83.** The method of item 3, wherein the (b) is administered at a dosage of at least 0.003 mg.
**84.** The method of item 4, wherein the (b) is administered at a dosage of at least 0.003 mg.
**85.** The method of item 5, wherein the (b) is administered at a dosage of at least 0.003 mg.
**86.** The method of item 1, wherein (b) is ranibizumab or a pharmaceutically acceptable salt thereof.
**87.** The method of item 1, wherein (b) is bevacizumab or a pharmaceutically acceptable salt thereof.
**88.** The method of item 1, wherein (b) is aflibercept or a pharmaceutically acceptable salt thereof.
**89.** The method of item 2, wherein the compound of Formula B is Antagonist B or a pharmaceutically acceptable salt thereof.
**90.** The method of item 72, wherein (b) is ranibizumab or a pharmaceutically acceptable salt thereof.
**91.** The method of item 72, wherein (b) is bevacizumab or a pharmaceutically acceptable salt thereof.
**92.** The method of item 72, wherein (b) is aflibercept or a pharmaceutically acceptable salt thereof.
**93.** The method of item 3, wherein the compound of Formula C is Antagonist C or a pharmaceutically acceptable salt thereof.
**94.** The method of item 93, wherein (b) is ranibizumab or a pharmaceutically acceptable salt thereof.
**95.** The method of item 93, wherein (b) is bevacizumab or a pharmaccutically acceptable salt thereof.
**96.** The method of item 93, wherein (b) is aflibercept or a pharmaceutically acceptable salt thereof.
**97.** The method of item 4, wherein (b) is ranibizumab or a pharmaceutically acceptable salt thereof.
**98.** The method of item 4, wherein (b) is bevacizumab or a pharmaceutically acceptable salt thereof.
**99.** The method of item 4, wherein (b) is aflibercept or a pharmaceutically acceptable salt thereof.
**100.** A composition comprising an effective amount of:
   (a) Antagonist A, a compound of Formula B, a compound of Formula C, Antagonist D, a compound of Formula E or a pharmaceutically acceptable salt thereof;
   (b) ranibizumab, bevacizumab, aflibercept, KH902 VEGF receptor-Fc fusion protein, 2C3 antibody, ORA102, pegaptanib, bevasiranib, SIRNA-027, decursin, decursinol, picropodophyllin, guggulsterone, PLG101, eicosanoid LXA4, PTK787, pazopanib, axitinib, CDDO-Me, CDDO-lmm, shikonin, beta-hydroxyisovalerylshikonin, or ganglioside GM3, DC101 antibody, Mab25 antibody, Mab73 antibody, 4A5 antibody, 4E10 antibody, 5F12 antibody, VA01 antibody, BL2 antibody, VEGF-related protein, sFLT01, sFLT02, Peptide B3, TG100801, sorafenib, or G6-31 antibody; and
   (c) a pharmaceutically acceptable carrier or vehicle.
**101.**The composition of item 100, further comprising an effective amount of another agent useful for treating or preventing an ophthalmological disease.
**102.**The composition of item 100, wherein (a) is Antagonist A or a phamaceutically acceptable salt thereof.
**103.**The composition of item 102, wherein (b) is ranibizumab or a pharmaceutically acceptable salt thereof.
**104.**The composition of item 102, wherein (b) is bevacizumab or a pharmaceutically acceptable salt thereof.
**105.**The composition of item 102, wherein (b) is aflibercept or a pharmaceutically acceptable salt thereof.
**106.**The composition of item 100, wherein the compound of Formula B is Antagonist B or a pharmaceutically acceptable salt thereof.
**107.**The composition of item 106, wherein (b) is ranibizumab or a pharmaceutically acceptable salt thereof.
**108.**The composition of item 106, wherein (b) is bevacizumab or a pharmaceutically acceptable salt thereof.
**109.**The composition of item 106, wherein (b) is aflibercept or a pharmaceutically acceptable salt thereof.
**110.**The composition of item 100, wherein the compound of Formula C is Antagonist C or a pharmaceutically acceptable salt thereof.
**111.**The composition of item 110, wherein (b) is ranibizumab or a pharmaceutically acceptable salt thereof.
**112.**The composition of item 110, wherein (b) is bevacizumab or a pharmaceutically acceptable salt thereof.
**113.**The composition of item 110, wherein (b) is aflibercept or a pharmaceutically acceptable salt thereof.
**114.**The composition of item 100, wherein (a) is Antagonist D or a pharmaceutically acceptable salt thereof.
**115.**The composition of item 114, wherein (b) is ranibizumab or a pharmaceutically acceptable salt thereof.
**116.**The composition of item 114, wherein (b) is bevacizumab or a pharmaceutically acceptable salt thereof.
**117.**The composition of item 114, wherein (b) is aflibercept or a pharmaceutically acceptable salt thereof.
**118.**The composition of item 100, wherein (a) is a compound of Formula E or a pharmaceutically acceptable salt thereof.
**119.**The composition of item 118, wherein (b) is ranibizumab or a pharmaceutically acceptable salt thereof.
**120.**The composition of item 118, wherein (b) is bevacizumab or a pharmaceutically acceptable salt thereof.
**121.** The composition of item 118, wherein (b) is aflibercept or a pharmaceutically acceptable salt thereof.
**122.** Antagonist A or a pharmaceutically acceptable salt thereof.
**123.** A composition comprising: (a) an effective amount of Antagonist A or a pharmaceutically acceptable salt thereof; and (b) a pharmaceutically acceptable carrier or vehicle.
**124.** A compound of Formula B or a pharmaceutically acceptable salt thereof.
**125.** A composition comprising: (a) an effective amount of a compound of Formula B or a pharmaceutically acceptable salt thereof; and (b) a pharmaceutically acceptable carrier or vehicle.
**126.** The compound of item 124, wherein the compound is Antagonist B.
**127.** The composition of item 125, wherein the compound is Antagonist B.
**128.**A compound of Formula C or a pharmaceutically acceptable salt thereof.
**129.** A composition comprising: (a) an effective amount of a compound of Formula C or a pharmaceutically acceptable salt thereof; and (b) a pharmaceutically acceptable carrier or vehicle.
**130.** The compound of item 128, wherein the compound is Antagonist C.
**131.** The composition of item 129, wherein the compound is Antagonist C.
**132.**A compound of Formula E or a pharmaceutically acceptable salt thereof.
**133.** A composition comprising: (a) an effective amount of a compound of Formula E or a pharmaceutically acceptable salt thereof; and (b) a pharmaceutically acceptable carrier or vehicle.
**134.** The compound of item 132, wherein the compound is Antagonist D.
**135.** The composition of item 133, wherein the compound is Antagonist D.

## Claims

1. A combination of (a) a PDGF antagonist and (b) a VEGF antagonist for use in treating or preventing wet age-related macular degeneration, wherein
(a) the PDGF antagonist is Antagonist A having the formula 5'-[mPEG2 40 kD]-[HN-(CH₂)₆O]CAGGCU_{f}AC_{f}Gₘ[PO₃(CH₂CH₂O)₆] CGTAGₘAGₘCAU_{f}C_{f}Aₘ[PO₃(CH₂CH₂O)₆]TGATC_{f}C_{f}U_{f}Gₘ-iT-3'
wherein
[mPEG2 40 kD] represents two about 20 kD polyethylene glycol polymer chains which are covalently linked to the two amino groups of a lysine residue via carbamate linkages, which lysine residue is in turn linked with the oligonucleotide via an amide bond to [HN-(CH₂)₆O];
[HN-(CH₂)₆O] is attached to the oligonucleotide via a phosphodiester linkage; C_{f} and U_{f} represent the 2'-fluoro substituted forms of cytosine and uridine, respectively;
Gₘ and Aₘ represent 2'-methoxy substituted forms of guanosine and adenosine, respectively;
or a pharmaceutically acceptable salt thereof; and
(b) the VEGF antagonist is aflibercept or a pharmaceutically acceptable salt thereof, and wherein the PDGF antagonist (a) and the VEGF antagonist (b) are administered sequentially or concurrently.

2. The combination for use of claim 1, wherein the Antagonist A has the formula 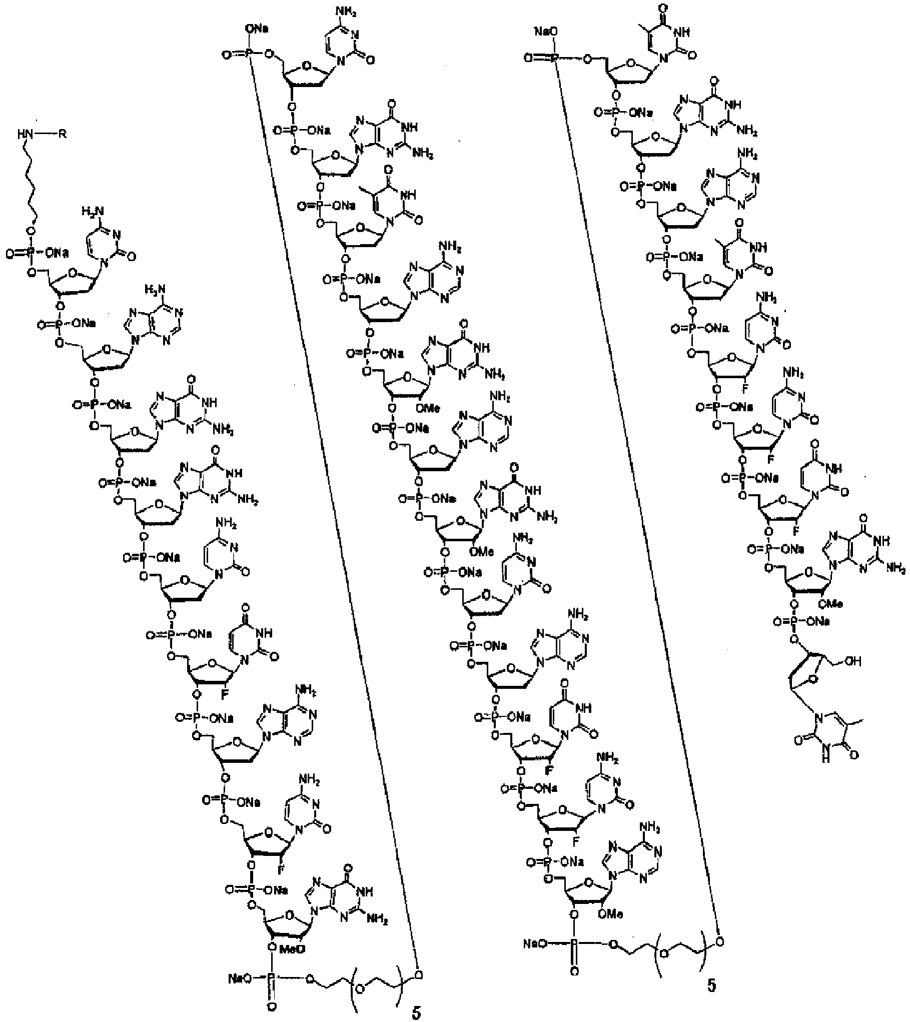 or another pharmaceutically acceptable salt thereof,
wherein R represents the following structure 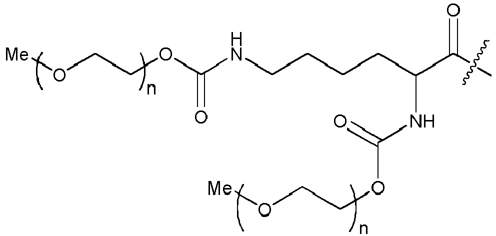 and n is about 450.

3. The combination for use of any one of the preceding claims, wherein (a) and (b) are administered within 24 hours of each other.

4. The combination for use of any one of the preceding claims, wherein (a) and (b) are administered within 60 minutes of each other.

5. The combination for use of any one of the preceding claims, wherein (a) and (b) are administered concurrently.

6. The combination for use of any one of the preceding claims, wherein (a) and (b) are present in separate compositions.

7. The combination for use of any one of the preceding claims, wherein the use further comprises administering an effective amount of another agent useful for treating or preventing an ophthalmological disease.

8. The combination for use of any one of the preceding claims, wherein (a) and/or (b) is/are present in a drug-delivery device.

9. The combination for use of claim 8, wherein (a) and (b) are present in the same drug-delivery device.

10. The combination for use of any one of the preceding claims, wherein (a) and/or (b) is/are administered intraocularly.

11. The combination for use of claim 10, wherein (a) and/or (b) is administered by intravitreal administration or anterior chamber administration.

12. A composition for use in treating or preventing wet age-related macular degeneration comprising an effective amount of:
(a) Antagonist A having the formula 
5′-[mPEG2 40 kD]-[HN-(CH2)6O]CAGGCUfACfGm[PO3(CH2CH2O)6]
CGTAGmAGmCAUfCfAm[PO3(CH2CH2O)6]TGATCfCfUfGm-iT-3′
 wherein
[mPEG2 40 kD] represents two about 20 kD polyethylene glycol polymer chains which are covalently linked to the two amino groups of a lysine residue via carbamate linkages, which lysine residue is in turn linked with the oligonucleotide via an amide bond to [HN-(CH₂)₆O];
[HN-(CH₂)₆O] is attached to the oligonucleotide via a phosphodiester linkage; C_{f} and U_{f} represent the 2'-fluoro substituted forms of cytosine and uridine, respectively;
Gₘ and Aₘ represent 2'-methoxy substituted forms of guanosine and adenosine, respectively;
or a pharmaceutically acceptable salt thereof;
(b) aflibercept or a pharmaceutically acceptable salt thereof; and
(c) a pharmaceutically acceptable carrier or vehicle.

13. The composition for use of claim 12, wherein the Antagonist A has the formula 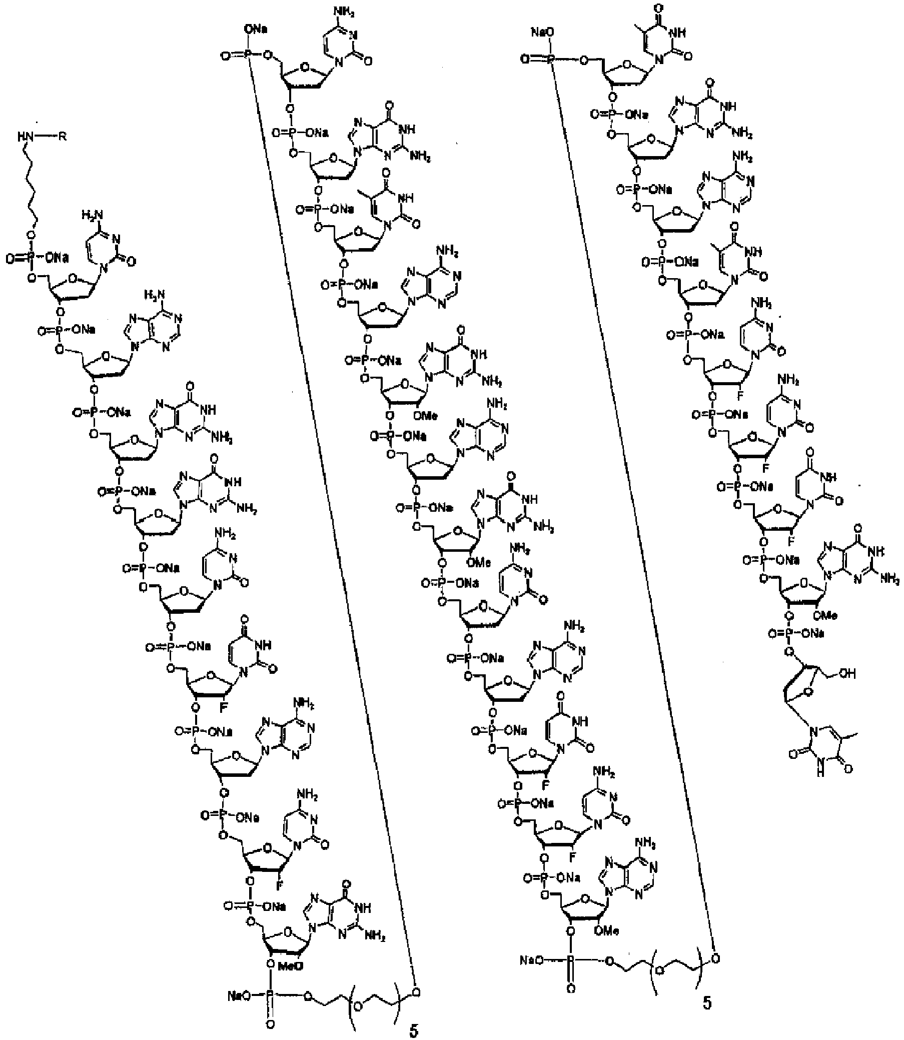 or another pharmaceutically acceptable salt thereof,
wherein R represents the following structure 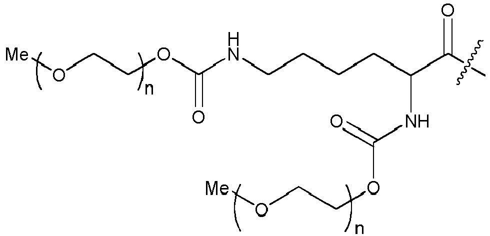 and n is about 450.

14. The composition for use of claim 12 or 13, further comprising an effective amount of another agent useful for treating or preventing an ophthalmological disease.

15. A kit for use in treating or preventing wet age-related macular degeneration comprising:
(a) a composition comprising Antagonist A having the formula 
5´-[mPEG2 40 kD]-[HN-(CH2)6O]CAGGCUfACfGm[PO3(CH2CH2O)6]
CGTAGmAGmCAUfCfAm[PO3(CH2CH2O)6]TGATCfCfUfGm-iT-3´
 wherein
[mPEG2 40 kD] represents two about 20 kD polyethylene glycol polymer chains which are covalently linked to the two amino groups of a lysine residue via carbamate linkages, which lysine residue is in turn linked with the oligonucleotide via an amide bond to [HN-(CH₂)₆O];
[HN-(CH₂)₆O] is attached to the oligonucleotide via a phosphodiester linkage;
C_{f} and U_{f} represent the 2'-fluoro substituted forms of cytosine and uridine, respectively;
Gₘ and Aₘ represent 2'-methoxy substituted forms of guanosine and adenosine, respectively;
or a pharmaceutically acceptable salt thereof, and a pharmaceutically acceptable carrier or vehicle; and
(b) a composition comprising aflibercept or a pharmaceutically acceptable salt thereof, and a pharmaceutically acceptable carrier or vehicle.

16. The kit for use of claim 15, wherein the Antagonist A has the formula 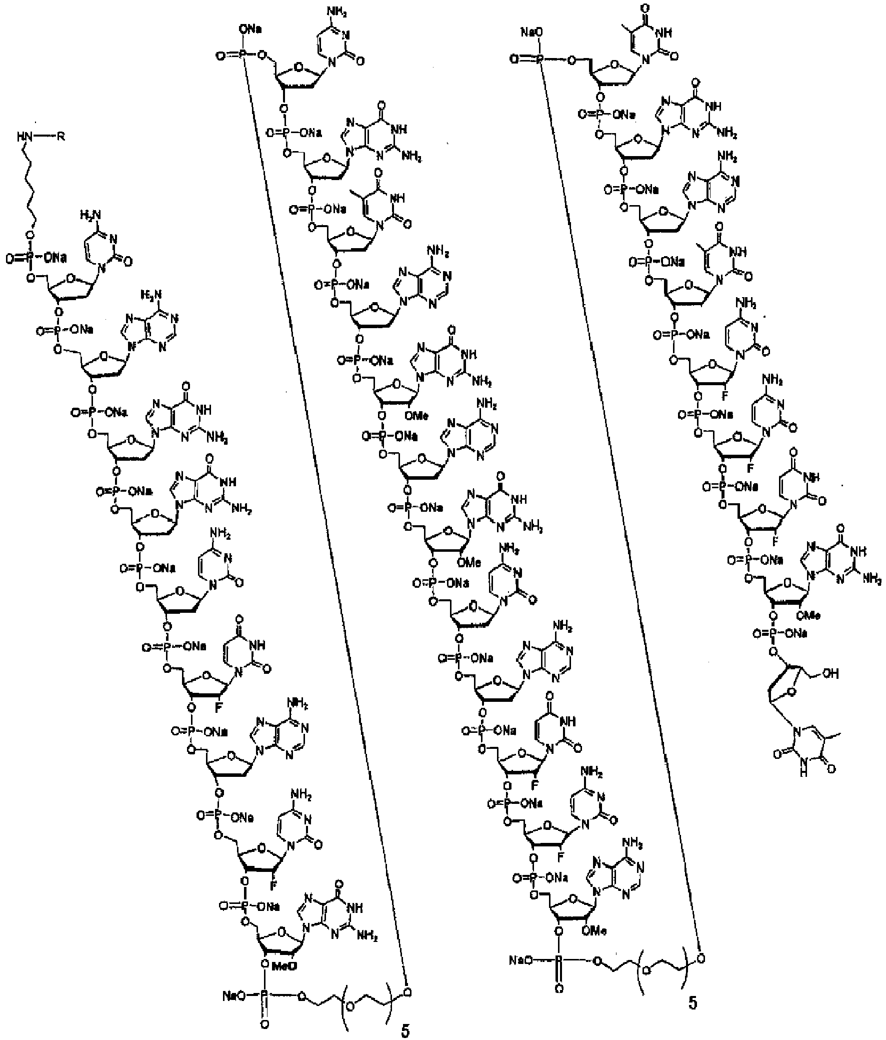 or another pharmaceutically acceptable salt thereof,
wherein R represents the following structure 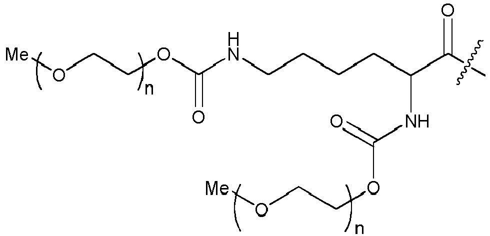 and n is about 450.


SEQUENCE LISTING

<110>  Ophthotech Corporation

<120>  METHODS FOR TREATING OR PREVENTING OPHTHALMOLOGICAL DISEASES

<130>  O8103-DIV1

<150>  EP 10770279.7
<151>  2010-04-28

<150>  61/174,746
<151>  2009-05-01

<150>  61/178,010
<151>  2009-05-13

<150>  61/245,784
<151>  2009-09-25

<160>  27

<170>  PatentIn version 3.5

<210>  1
<211>  2137
<212>  DNA
<213>  Homo sapiens

<400>  1
ccctgcctgc ctccctgcgc acccgcagcc tcccccgctg cctccctagg gctcccctcc      60

ggccgccagc gcccattttt cattccctag atagagatac tttgcgcgca cacacataca     120

tacgcgcgca aaaaggaaaa aaaaaaaaaa aagcccaccc tccagcctcg ctgcaaagag     180

aaaaccggag cagccgcagc tcgcagctcg cagcccgcag cccgcagagg acgcccagag     240

cggcgagcgg gcgggcagac ggaccgacgg actcgcgccg cgtccacctg tcggccgggc     300

ccagccgagc gcgcagcggg cacgccgcgc gcgcggagca gccgtgcccg ccgcccgggc     360

ccgccgccag ggcgcacacg ctcccgcccc cctacccggc ccgggcggga gtttgcacct     420

ctccctgccc gggtgctcga gctgccgttg caaagccaac tttggaaaaa gtttttgggg     480

ggagacttgg gccttgaggt gcccagctcc gcgctttccg attttggggg cctttccaga     540

aaatgttgca aaaaagctaa gccggcgggc agaggaaaac gcctgtagcc ggcgagtgaa     600

gacgaaccat cgactgccgt gttccttttc ctcttggagg ttggagtccc ctgggcgccc     660

ccacacggct agacgcctcg gctggttcgc gacgcagccc cccggccgtg gatgctgcac     720

tcgggctcgg gatccgccca ggtagcggcc tcggacccag gtcctgcgcc caggtcctcc     780

cctgcccccc agcgacggag ccggggccgg gggcggcggc gccgggggca tgcgggtgag     840

ccgcggctgc agaggcctga gcgcctgatc gccgcggacc cgagccgagc ccacccccct     900

ccccagcccc ccaccctggc cgcgggggcg gcgcgctcga tctacgcgtt cggggccccg     960

cggggccggg cccggagtcg gcatgaatcg ctgctgggcg ctcttcctgt ctctctgctg    1020

ctacctgcgt ctggtcagcg ccgaggggga ccccattccc gaggagcttt atgagatgct 1080

gagtgaccac tcgatccgct cctttgatga tctccaacgc ctgctgcacg gagaccccgg 1140

agaggaagat ggggccgagt tggacctgaa catgacccgc tcccactctg gaggcgagct 1200

ggagagcttg gctcgtggaa gaaggagcct gggttccctg accattgctg agccggccat 1260

gatcgccgag tgcaagacgc gcaccgaggt gttcgagatc tcccggcgcc tcatagaccg 1320

caccaacgcc aacttcctgg tgtggccgcc ctgtgtggag gtgcagcgct gctccggctg 1380

ctgcaacaac cgcaacgtgc agtgccgccc cacccaggtg cagctgcgac ctgtccaggt 1440

gagaaagatc gagattgtgc ggaagaagcc aatctttaag aaggccacgg tgacgctgga 1500

agaccacctg gcatgcaagt gtgagacagt ggcagctgca cggcctgtga cccgaagccc 1560

ggggggttcc caggagcagc gagccaaaac gccccaaact cgggtgacca ttcggacggt 1620

gcgagtccgc cggcccccca agggcaagca ccggaaattc aagcacacgc atgacaagac 1680

ggcactgaag gagacccttg gagcctaggg gcatcggcag gagagtgtgt gggcagggtt 1740

atttaatatg gtatttgctg tattgccccc atggggcctt ggagtagata atattgtttc 1800

cctcgtccgt ctgtctcgat gcctgattcg gacggccaat ggtgcctccc ccacccctcc 1860

acgtgtccgt ccacccttcc atcagcgggt ctcctcccag cggcctccgg ctcttgccca 1920

gcagctcaag aagaaaaaga aggactgaac tccatcgcca tcttcttccc ttaactccaa 1980

gaacttggga taagagtgtg agagagactg atggggtcgc tctttggggg aaacgggttc 2040

cttcccctgc acctggcctg ggccacacct gagcgctgtg gactgtcctg aggagccctg 2100

aggacctctc agcatagcct gcctgatccc tgaaccc 2137

<210> 2
<211> 241
<212> PRT
<213> Homo sapiens

<400> 2

<210>  3
<211>  1723
<212>  DNA
<213>  Homo sapiens

<400>  3
tcgcggaggc ttggggcagc cgggtagctc ggaggtcgtg gcgctggggg ctagcaccag      60

cgctctgtcg ggaggcgcag cggttaggtg gaccggtcag cggactcacc ggccagggcg     120

ctcggtgctg gaatttgata ttcattgatc cgggttttat ccctcttctt ttttcttaaa     180

catttttttt taaaactgta ttgtttctcg ttttaattta tttttgcttg ccattcccca     240

cttgaatcgg gccgacggct tggggagatt gctctacttc cccaaatcac tgtggatttt     300

ggaaaccagc agaaagagga aagaggtagc aagagctcca gagagaagtc gaggaagaga 360

gagacggggt cagagagagc gcgcgggcgt gcgagcagcg aaagcgacag gggcaaagtg 420

agtgacctgc ttttgggggt gaccgccgga gcgcggcgtg agccctcccc cttgggatcc 480

cgcagctgac cagtcgcgct gacggacaga cagacagaca ccgcccccag ccccagctac 540

cacctcctcc ccggccggcg gcggacagtg gacgcggcgg cgagccgcgg gcaggggccg 600

gagcccgcgc ccggaggcgg ggtggagggg gtcggggctc gcggcgtcgc actgaaactt 660

ttcgtccaac ttctgggctg ttctcgcttc ggaggagccg tggtccgcgc gggggaagcc 720

gagccgagcg gagccgcgag aagtgctagc tcgggccggg aggagccgca gccggaggag 780

ggggaggagg aagaagagaa ggaagaggag agggggccgc agtggcgact cggcgctcgg 840

aagccgggct catggacggg tgaggcggcg gtgtgcgcag acagtgctcc agccgcgcgc 900

gctccccagg ccctggcccg ggcctcgggc cggggaggaa gagtagctcg ccgaggcgcc 960

gaggagagcg ggccgcccca cagcccgagc cggagaggga gcgcgagccg cgccggcccc 1020

ggtcgggcct ccgaaaccat gaactttctg ctgtcttggg tgcattggag ccttgccttg 1080

ctgctctacc tccaccatgc caagtggtcc caggctgcac ccatggcaga aggaggaggg 1140

cagaatcatc acgaagtggt gaagttcatg gatgtctatc agcgcagcta ctgccatcca 1200

atcgagaccc tggtggacat cttccaggag taccctgatg agatcgagta catcttcaag 1260

ccatcctgtg tgcccctgat gcgatgcggg ggctgctgca atgacgaggg cctggagtgt 1320

gtgcccactg aggagtccaa catcaccatg cagattatgc ggatcaaacc tcaccaaggc 1380

cagcacatag gagagatgag cttcctacag cacaacaaat gtgaatgcag accaaagaaa 1440

gatagagcaa gacaagaaaa aaaatcagtt cgaggaaagg gaaaggggca aaaacgaaag 1500

cgcaagaaat cccggtataa gtcctggagc gttccctgtg ggccttgctc agagcggaga 1560

aagcatttgt ttgtacaaga tccgcagacg tgtaaatgtt cctgcaaaaa cacagactcg 1620

cgttgcaagg cgaggcagct tgagttaaac gaacgtactt gcagatgtga caagccgagg 1680

cggtgagccg ggcaggagga aggagcctcc ctcagggttt cgg 1723

<210> 4
<211> 215
<212> PRT
<213> Homo sapiens

<400> 4

Met Asn Phe Leu Leu Ser Trp Val His Trp Ser Leu Ala Leu Leu Leu
1               5                   10                  15

Tyr Leu His His Ala Lys Trp Ser Gln Ala Ala Pro Met Ala Glu Gly
            20                  25                  30

Gly Gly Gln Asn His His Glu Val Val Lys Phe Met Asp Val Tyr Gln
        35                  40                  45

Arg Ser Tyr Cys His Pro Ile Glu Thr Leu Val Asp Ile Phe Gln Glu
    50                  55                  60

Tyr Pro Asp Glu Ile Glu Tyr Ile Phe Lys Pro Ser Cys Val Pro Leu
65                  70                  75                  80

Met Arg Cys Gly Gly Cys Cys Asn Asp Glu Gly Leu Glu Cys Val Pro
                85                  90                  95

Thr Glu Glu Ser Asn Ile Thr Met Gln Ile Met Arg Ile Lys Pro His
            100                 105                 110

Gln Gly Gln His Ile Gly Glu Met Ser Phe Leu Gln His Asn Lys Cys
        115                 120                 125

Glu Cys Arg Pro Lys Lys Asp Arg Ala Arg Gln Glu Lys Lys Ser Val
    130                 135                 140

Arg Gly Lys Gly Lys Gly Gln Lys Arg Lys Arg Lys Lys Ser Arg Tyr
145                 150                 155                 160

Lys Ser Trp Ser Val Pro Cys Gly Pro Cys Ser Glu Arg Arg Lys His
                165                 170                 175

Leu Phe Val Gln Asp Pro Gln Thr Cys Lys Cys Ser Cys Lys Asn Thr
            180                 185                 190

Asp Ser Arg Cys Lys Ala Arg Gln Leu Glu Leu Asn Glu Arg Thr Cys
        195                 200                 205

Arg Cys Asp Lys Pro Arg Arg
    210                 215

<210>  5
<211>  5598
<212>  DNA
<213>  Homo sapiens

<400>  5
ggcccctcag ccctgctgcc cagcacgagc ctgtgctcgc cctgcccaac gcagacagcc      60

agacccaggg cggcccctct ggcggctctg ctcctcccga aggatgcttg gggagtgagg     120

cgaagctggg cgctcctctc ccctacagca gcccccttcc tccatccctc tgttctcctg      180

agccttcagg agcctgcacc agtcctgcct gtccttctac tcagctgtta cccactctgg     240

gaccagcagt ctttctgata actgggagag ggcagtaagg aggacttcct ggagggggtg 300

actgtccaga gcctggaact gtgcccacac cagaagccat cagcagcaag gacaccatgc 360

ggcttccggg tgcgatgcca gctctggccc tcaaaggcga gctgctgttg ctgtctctcc 420

tgttacttct ggaaccacag atctctcagg gcctggtcgt cacacccccg gggccagagc 480

ttgtcctcaa tgtctccagc accttcgttc tgacctgctc gggttcagct ccggtggtgt 540

gggaacggat gtcccaggag cccccacagg aaatggccaa ggcccaggat ggcaccttct 600

ccagcgtgct cacactgacc aacctcactg ggctagacac gggagaatac ttttgcaccc 660

acaatgactc ccgtggactg gagaccgatg agcggaaacg gctctacatc tttgtgccag 720

atcccaccgt gggcttcctc cctaatgatg ccgaggaact attcatcttt ctcacggaaa 780

taactgagat caccattcca tgccgagtaa cagacccaca gctggtggtg acactgcacg 840

agaagaaagg ggacgttgca ctgcctgtcc cctatgatca ccaacgtggc ttttctggta 900

tctttgagga cagaagctac atctgcaaaa ccaccattgg ggacagggag gtggattctg 960

atgcctacta tgtctacaga ctccaggtgt catccatcaa cgtctctgtg aacgcagtgc 1020

agactgtggt ccgccagggt gagaacatca ccctcatgtg cattgtgatc gggaatgagg 1080

tggtcaactt cgagtggaca tacccccgca aagaaagtgg gcggctggtg gagccggtga 1140

ctgacttcct cttggatatg ccttaccaca tccgctccat cctgcacatc cccagtgccg 1200

agttagaaga ctcggggacc tacacctgca atgtgacgga gagtgtgaat gaccatcagg 1260

atgaaaaggc catcaacatc accgtggttg agagcggcta cgtgcggctc ctgggagagg 1320

tgggcacact acaatttgct gagctgcatc ggagccggac actgcaggta gtgttcgagg 1380

cctacccacc gcccactgtc ctgtggttca aagacaaccg caccctgggc gactccagcg 1440

ctggcgaaat cgccctgtcc acgcgcaacg tgtcggagac ccggtatgtg tcagagctga 1500

cactggttcg cgtgaaggtg gcagaggctg gccactacac catgcgggcc ttccatgagg 1560

atgctgaggt ccagctctcc ttccagctac agatcaatgt ccctgtccga gtgctggagc 1620

taagtgagag ccaccctgac agtggggaac agacagtccg ctgtcgtggc cggggcatgc 1680

cccagccgaa catcatctgg tctgcctgca gagacctcaa aaggtgtcca cgtgagctgc 1740

cgcccacgct gctggggaac agttccgaag aggagagcca gctggagact aacgtgacgt 1800

actgggagga ggagcaggag tttgaggtgg tgagcacact gcgtctgcag cacgtggatc 1860

ggccactgtc ggtgcgctgc acgctgcgca acgctgtggg ccaggacacg caggaggtca 1920

tcgtggtgcc acactccttg ccctttaagg tggtggtgat ctcagccatc ctggccctgg 1980

tggtgctcac catcatctcc cttatcatcc tcatcatgct ttggcagaag aagccacgtt 2040

acgagatccg atggaaggtg attgagtctg tgagctctga cggccatgag tacatctacg 2100

tggaccccat gcagctgccc tatgactcca cgtgggagct gccgcgggac cagcttgtgc 2160

tgggacgcac cctcggctct ggggcctttg ggcaggtggt ggaggccacg gctcatggcc 2220

tgagccattc tcaggccacg atgaaagtgg ccgtcaagat gcttaaatcc acagcccgca 2280

gcagtgagaa gcaagccctt atgtcggagc tgaagatcat gagtcacctt gggccccacc 2340

tgaacgtggt caacctgttg ggggcctgca ccaaaggagg acccatctat atcatcactg 2400

agtactgccg ctacggagac ctggtggact acctgcaccg caacaaacac accttcctgc 2460

agcaccactc cgacaagcgc cgcccgccca gcgcggagct ctacagcaat gctctgcccg 2520

ttgggctccc cctgcccagc catgtgtcct tgaccgggga gagcgacggt ggctacatgg 2580

acatgagcaa ggacgagtcg gtggactatg tgcccatgct ggacatgaaa ggagacgtca 2640

aatatgcaga catcgagtcc tccaactaca tggcccctta cgataactac gttccctctg 2700

cccctgagag gacctgccga gcaactttga tcaacgagtc tccagtgcta agctacatgg 2760

acctcgtggg cttcagctac caggtggcca atggcatgga gtttctggcc tccaagaact 2820

gcgtccacag agacctggcg gctaggaacg tgctcatctg tgaaggcaag ctggtcaaga 2880

tctgtgactt tggcctggct cgagacatca tgcgggactc gaattacatc tccaaaggca 2940

gcaccttttt gcctttaaag tggatggctc cggagagcat cttcaacagc ctctacacca 3000

ccctgagcga cgtgtggtcc ttcgggatcc tgctctggga gatcttcacc ttgggtggca 3060

ccccttaccc agagctgccc atgaacgagc agttctacaa tgccatcaaa cggggttacc 3120

gcatggccca gcctgcccat gcctccgacg agatctatga gatcatgcag aagtgctggg 3180

aagagaagtt tgagattcgg ccccccttct cccagctggt gctgcttctc gagagactgt 3240

tgggcgaagg ttacaaaaag aagtaccagc aggtggatga ggagtttctg aggagtgacc 3300

acccagccat ccttcggtcc caggcccgct tgcctgggtt ccatggcctc cgatctcccc 3360

tggacaccag ctccgtcctc tatactgccg tgcagcccaa tgagggtgac aacgactata 3420

tcatccccct gcctgacccc aaacccgagg ttgctgacga gggcccactg gagggttccc 3480

ccagcctagc cagctccacc ctgaatgaag tcaacacctc ctcaaccatc tcctgtgaca 3540

gccccctgga gccccaggac gaaccagagc cagagcccca gcttgagctc caggtggagc 3600

cggagccaga gctggaacag ttgccggatt cggggtgccc tgcgcctcgg gcggaagcag 3660

aggatagctt cctgtagggg gctggcccct accctgccct gcctgaagct ccccccctgc 3720

cagcacccag catctcctgg cctggcctga ccgggcttcc tgtcagccag gctgccctta 3780

tcagctgtcc ccttctggaa gctttctgct cctgacgtgt tgtgccccaa accctggggc 3840

tggcttagga ggcaagaaaa ctgcaggggc cgtgaccagc cctctgcctc cagggaggcc 3900

aactgactct gagccagggt tcccccaggg aactcagttt tcccatatgt aagatgggaa 3960

agttaggctt gatgacccag aatctaggat tctctccctg gctgacaggt ggggagaccg 4020

aatccctccc tgggaagatt cttggagtta ctgaggtggt aaattaactt ttttctgttc 4080

agccagctac ccctcaagga atcatagctc tctcctcgca ctttttatcc acccaggagc 4140

tagggaagag accctagcct ccctggctgc tggctgagct agggcctagc cttgagcagt 4200

gttgcctcat ccagaagaaa gccagtctcc tccctatgat gccagtccct gcgttccctg 4260

gcccgagctg gtctggggcc attaggcagc ctaattaatg ctggaggctg agccaagtac 4320

aggacacccc cagcctgcag cccttgccca gggcacttgg agcacacgca gccatagcaa 4380

gtgcctgtgt ccctgtcctt caggcccatc agtcctgggg ctttttcttt atcaccctca 4440

gtcttaatcc atccaccaga gtctagaagg ccagacgggc cccgcatctg tgatgagaat 4500

gtaaatgtgc cagtgtggag tggccacgtg tgtgtgccag tatatggccc tggctctgca 4560

ttggacctgc tatgaggctt tggaggaatc cctcaccctc tctgggcctc agtttcccct 4620

tcaaaaaatg aataagtcgg acttattaac tctgagtgcc ttgccagcac taacattcta 4680

gagtattcca ggtggttgca catttgtcca gatgaagcaa ggccatatac cctaaacttc 4740

catcctgggg gtcagctggg ctcctgggag attccagatc acacatcaca ctctggggac 4800

tcaggaacca tgccccttcc ccaggccccc agcaagtctc aagaacacag ctgcacaggc 4860

cttgacttag agtgacagcc ggtgtcctgg aaagccccaa gcagctgccc cagggacatg 4920

ggaagaccac gggacctctt tcactaccca cgatgacctc cgggggtatc ctgggcaaaa 4980

gggacaaaga gggcaaatga gatcacctcc tgcagcccac cactccagca cctgtgccga 5040

ggtctgcgtc gaagacagaa tggacagtga ggacagttat gtcttgtaaa agacaagaag 5100

cttcagatgg taccccaaga aggatgtgag aggtggccgc ttggagtttg cccctcaccc 5160

accagctgcc ccatccctga ggcagcgctc catgggggta tggttttgtc actgcccaga 5220

cctagcagtg acatctcatt gtccccagcc cagtgggcat tggaggtgcc aggggagtca 5280

gggttgtagc caagacgccc ccgcacgggg agggttggga agggggtgca ggaagctcaa 5340

cccctctggg caccaaccct gcattgcagg ttggcacctt acttccctgg gatccccaga 5400

gttggtccaa ggagggagag tgggttctca atacggtacc aaagatataa tcacctaggt 5460

ttacaaatat ttttaggact cacgttaact cacatttata cagcagaaat gctattttgt 5520

atgctgttaa gtttttctat ctgtgtactt ttttttaagg gaaagatttt aatattaaac 5580

ctggtgcttc tcactcac 5598

<210> 6
<211> 1106
<212> PRT
<213> Homo sapiens

<400> 6

<210> 7
<211> 4017
<212> DNA
<213> Homo sapiens

<400> 7
atggtcagct actgggacac cggggtcctg ctgtgcgcgc tgctcagctg tctgcttctc 60

acaggatcta gttcaggttc aaaattaaaa gatcctgaac tgagtttaaa aggcacccag 120

cacatcatgc aagcaggcca gacactgcat ctccaatgca ggggggaagc agcccataaa 180

tggtctttgc ctgaaatggt gagtaaggaa agcgaaaggc tgagcataac taaatctgcc 240

tgtggaagaa atggcaaaca attctgcagt actttaacct tgaacacagc tcaagcaaac 300

cacactggct tctacagctg caaatatcta gctgtaccta cttcaaagaa gaaggaaaca 360

gaatctgcaa tctatatatt tattagtgat acaggtagac ctttcgtaga gatgtacagt 420

gaaatccccg aaattataca catgactgaa ggaagggagc tcgtcattcc ctgccgggtt 480

acgtcaccta acatcactgt tactttaaaa aagtttccac ttgacacttt gatccctgat 540

ggaaaacgca taatctggga cagtagaaag ggcttcatca tatcaaatgc aacgtacaaa 600

gaaatagggc ttctgacctg tgaagcaaca gtcaatgggc atttgtataa gacaaactat 660

ctcacacatc gacaaaccaa tacaatcata gatgtccaaa taagcacacc acgcccagtc 720

aaattactta gaggccatac tcttgtcctc aattgtactg ctaccactcc cttgaacacg 780

agagttcaaa tgacctggag ttaccctgat gaaaaaaata agagagcttc cgtaaggcga 840

cgaattgacc aaagcaattc ccatgccaac atattctaca gtgttcttac tattgacaaa 900

atgcagaaca aagacaaagg actttatact tgtcgtgtaa ggagtggacc atcattcaaa 960

tctgttaaca cctcagtgca tatatatgat aaagcattca tcactgtgaa acatcgaaaa 1020

cagcaggtgc ttgaaaccgt agctggcaag cggtcttacc ggctctctat gaaagtgaag 1080

gcatttccct cgccggaagt tgtatggtta aaagatgggt tacctgcgac tgagaaatct 1140

gctcgctatt tgactcgtgg ctactcgtta attatcaagg acgtaactga agaggatgca 1200

gggaattata caatcttgct gagcataaaa cagtcaaatg tgtttaaaaa cctcactgcc 1260

actctaattg tcaatgtgaa accccagatt tacgaaaagg ccgtgtcatc gtttccagac 1320

ccggctctct acccactggg cagcagacaa atcctgactt gtaccgcata tggtatccct 1380

caacctacaa tcaagtggtt ctggcacccc tgtaaccata atcattccga agcaaggtgt 1440

gacttttgtt ccaataatga agagtccttt atcctggatg ctgacagcaa catgggaaac 1500

agaattgaga gcatcactca gcgcatggca ataatagaag gaaagaataa gatggctagc 1560

accttggttg tggctgactc tagaatttct ggaatctaca tttgcatagc ttccaataaa 1620

gttgggactg tgggaagaaa cataagcttt tatatcacag atgtgccaaa tgggtttcat 1680

gttaacttgg aaaaaatgcc gacggaagga gaggacctga aactgtcttg cacagttaac 1740

aagttcttat acagagacgt tacttggatt ttactgcgga cagttaataa cagaacaatg 1800

cactacagta ttagcaagca aaaaatggcc atcactaagg agcactccat cactcttaat 1860

cttaccatca tgaatgtttc cctgcaagat tcaggcacct atgcctgcag agccaggaat 1920

gtatacacag gggaagaaat cctccagaag aaagaaatta caatcagaga tcaggaagca 1980

ccatacctcc tgcgaaacct cagtgatcac acagtggcca tcagcagttc caccacttta 2040

gactgtcatg ctaatggtgt ccccgagcct cagatcactt ggtttaaaaa caaccacaaa 2100

atacaacaag agcctggaat tattttagga ccaggaagca gcacgctgtt tattgaaaga 2160

gtcacagaag aggatgaagg tgtctatcac tgcaaagcca ccaaccagaa gggctctgtg 2220

gaaagttcag catacctcac tgttcaagga acctcggaca agtctaatct ggagctgatc 2280

actctaacat gcacctgtgt ggctgcgact ctcttctggc tcctattaac cctctttatc 2340

cgaaaaatga aaaggtcttc ttctgaaata aagactgact acctatcaat tataatggac 2400

ccagatgaag ttcctttgga tgagcagtgt gagcggctcc cttatgatgc cagcaagtgg 2460

gagtttgccc gggagagact taaactgggc aaatcacttg gaagaggggc ttttggaaaa 2520

gtggttcaag catcagcatt tggcattaag aaatcaccta cgtgccggac tgtggctgtg 2580

aaaatgctga aagagggggc cacggccagc gagtacaaag ctctgatgac tgagctaaaa 2640

atcttgaccc acattggcca ccatctgaac gtggttaacc tgctgggagc ctgcaccaag 2700

caaggagggc ctctgatggt gattgttgaa tactgcaaat atggaaatct ctccaactac 2760

ctcaagagca aacgtgactt attttttctc aacaaggatg cagcactaca catggagcct 2820

aagaaagaaa aaatggagcc aggcctggaa caaggcaaga aaccaagact agatagcgtc 2880

accagcagcg aaagctttgc gagctccggc tttcaggaag ataaaagtct gagtgatgtt 2940

gaggaagagg aggattctga cggtttctac aaggagccca tcactatgga agatctgatt 3000

tcttacagtt ttcaagtggc cagaggcatg gagttcctgt cttccagaaa gtgcattcat 3060

cgggacctgg cagcgagaaa cattctttta tctgagaaca acgtggtgaa gatttgtgat 3120

tttggccttg cccgggatat ttataagaac cccgattatg tgagaaaagg agatactcga 3180

cttcctctga aatggatggc tcctgaatct atctttgaca aaatctacag caccaagagc 3240

gacgtgtggt cttacggagt attgctgtgg gaaatcttct ccttaggtgg gtctccatac 3300

ccaggagtac aaatggatga ggacttttgc agtcgcctga gggaaggcat gaggatgaga 3360

gctcctgagt actctactcc tgaaatctat cagatcatgc tggactgctg gcacagagac 3420

ccaaaagaaa ggccaagatt tgcagaactt gtggaaaaac taggtgattt gcttcaagca 3480

aatgtacaac aggatggtaa agactacatc ccaatcaatg ccatactgac aggaaatagt 3540

gggtttacat actcaactcc tgccttctct gaggacttct tcaaggaaag tatttcagct 3600

ccgaagttta attcaggaag ctctgatgat gtcagatatg taaatgcttt caagttcatg 3660

agcctggaaa gaatcaaaac ctttgaagaa cttttaccga atgccacctc catgtttgat 3720

gactaccagg gcgacagcag cactctgttg gcctctccca tgctgaagcg cttcacctgg 3780

actgacagca aacccaaggc ctcgctcaag attgacttga gagtaaccag taaaagtaag 3840

gagtcggggc tgtctgatgt cagcaggccc agtttctgcc attccagctg tgggcacgtc 3900

agcgaaggca agcgcaggtt cacctacgac cacgctgagc tggaaaggaa aatcgcgtgc 3960

tgctccccgc ccccagacta caactcggtg gtcctgtact ccaccccacc catctag 4017

<210> 8
<211> 1338
<212> PRT
<213> Homo sapiens

<400> 8

Met Val Ser Tyr Trp Asp Thr Gly Val Leu Leu Cys Ala Leu Leu Ser
1               5                   10                  15

Cys Leu Leu Leu Thr Gly Ser Ser Ser Gly Ser Lys Leu Lys Asp Pro
            20                  25                  30

Glu Leu Ser Leu Lys Gly Thr Gln His Ile Met Gln Ala Gly Gln Thr
        35                  40                  45

Leu His Leu Gln Cys Arg Gly Glu Ala Ala His Lys Trp Ser Leu Pro

50                  55                  60

Glu Met Val Ser Lys Glu Ser Glu Arg Leu Ser Ile Thr Lys Ser Ala
65                  70                  75                  80

Cys Gly Arg Asn Gly Lys Gln Phe Cys Ser Thr Leu Thr Leu Asn Thr
                85                  90                  95

Ala Gln Ala Asn His Thr Gly Phe Tyr Ser Cys Lys Tyr Leu Ala Val
            100                 105                 110

Pro Thr Ser Lys Lys Lys Glu Thr Glu Ser Ala Ile Tyr Ile Phe Ile
        115                 120                 125

Ser Asp Thr Gly Arg Pro Phe Val Glu Met Tyr Ser Glu Ile Pro Glu
    130                 135                 140

Ile Ile His Met Thr Glu Gly Arg Glu Leu Val Ile Pro Cys Arg Val
145                 150                 155                 160

Thr Ser Pro Asn Ile Thr Val Thr Leu Lys Lys Phe Pro Leu Asp Thr
                165                 170                 175

Leu Ile Pro Asp Gly Lys Arg Ile Ile Trp Asp Ser Arg Lys Gly Phe
            180                 185                 190

Ile Ile Ser Asn Ala Thr Tyr Lys Glu Ile Gly Leu Leu Thr Cys Glu
        195                 200                 205

Ala Thr Val Asn Gly His Leu Tyr Lys Thr Asn Tyr Leu Thr His Arg
    210                 215                 220

Gln Thr Asn Thr Ile Ile Asp Val Gln Ile Ser Thr Pro Arg Pro Val
225                 230                 235                 240

Lys Leu Leu Arg Gly His Thr Leu Val Leu Asn Cys Thr Ala Thr Thr
                245                 250                 255

Pro Leu Asn Thr Arg Val Gln Met Thr Trp Ser Tyr Pro Asp Glu Lys
            260                 265                 270

Asn Lys Arg Ala Ser Val Arg Arg Arg Ile Asp Gln Ser Asn Ser His
        275                 280                 285

Ala Asn Ile Phe Tyr Ser Val Leu Thr Ile Asp Lys Met Gln Asn Lys
    290                 295                 300

Asp Lys Gly Leu Tyr Thr Cys Arg Val Arg Ser Gly Pro Ser Phe Lys
305                 310                 315                 320

Ser Val Asn Thr Ser Val His Ile Tyr Asp Lys Ala Phe Ile Thr Val
                325                 330                 335

Lys His Arg Lys Gln Gln Val Leu Glu Thr Val Ala Gly Lys Arg Ser
            340                 345                 350

Tyr Arg Leu Ser Met Lys Val Lys Ala Phe Pro Ser Pro Glu Val Val
        355                 360                 365

Trp Leu Lys Asp Gly Leu Pro Ala Thr Glu Lys Ser Ala Arg Tyr Leu
    370                 375                 380

Thr Arg Gly Tyr Ser Leu Ile Ile Lys Asp Val Thr Glu Glu Asp Ala
385                 390                 395                 400

Gly Asn Tyr Thr Ile Leu Leu Ser Ile Lys Gln Ser Asn Val Phe Lys
                405                 410                 415

Asn Leu Thr Ala Thr Leu Ile Val Asn Val Lys Pro Gln Ile Tyr Glu
            420                 425                 430

Lys Ala Val Ser Ser Phe Pro Asp Pro Ala Leu Tyr Pro Leu Gly Ser
        435                 440                 445

Arg Gln Ile Leu Thr Cys Thr Ala Tyr Gly Ile Pro Gln Pro Thr Ile
    450                 455                 460

Lys Trp Phe Trp His Pro Cys Asn His Asn His Ser Glu Ala Arg Cys
465                 470                 475                 480

Asp Phe Cys Ser Asn Asn Glu Glu Ser Phe Ile Leu Asp Ala Asp Ser
                485                 490                 495

Asn Met Gly Asn Arg Ile Glu Ser Ile Thr Gln Arg Met Ala Ile Ile
            500                 505                 510

Glu Gly Lys Asn Lys Met Ala Ser Thr Leu Val Val Ala Asp Ser Arg
        515                 520                 525

Ile Ser Gly Ile Tyr Ile Cys Ile Ala Ser Asn Lys Val Gly Thr Val
    530                 535                 540

Gly Arg Asn Ile Ser Phe Tyr Ile Thr Asp Val Pro Asn Gly Phe His
545                 550                 555                 560

Val Asn Leu Glu Lys Met Pro Thr Glu Gly Glu Asp Leu Lys Leu Ser
                565                 570                 575

Cys Thr Val Asn Lys Phe Leu Tyr Arg Asp Val Thr Trp Ile Leu Leu
            580                 585                 590

Arg Thr Val Asn Asn Arg Thr Met His Tyr Ser Ile Ser Lys Gln Lys
        595                 600                 605

Met Ala Ile Thr Lys Glu His Ser Ile Thr Leu Asn Leu Thr Ile Met
    610                 615                 620

Asn Val Ser Leu Gln Asp Ser Gly Thr Tyr Ala Cys Arg Ala Arg Asn
625                 630                 635                 640

Val Tyr Thr Gly Glu Glu Ile Leu Gln Lys Lys Glu Ile Thr Ile Arg
                645                 650                 655

Asp Gln Glu Ala Pro Tyr Leu Leu Arg Asn Leu Ser Asp His Thr Val
            660                 665                 670

Ala Ile Ser Ser Ser Thr Thr Leu Asp Cys His Ala Asn Gly Val Pro
        675                 680                 685

Glu Pro Gln Ile Thr Trp Phe Lys Asn Asn His Lys Ile Gln Gln Glu
    690                 695                 700

Pro Gly Ile Ile Leu Gly Pro Gly Ser Ser Thr Leu Phe Ile Glu Arg
705                 710                 715                 720

Val Thr Glu Glu Asp Glu Gly Val Tyr His Cys Lys Ala Thr Asn Gln
                725                 730                 735

Lys Gly Ser Val Glu Ser Ser Ala Tyr Leu Thr Val Gln Gly Thr Ser
            740                 745                 750

Asp Lys Ser Asn Leu Glu Leu Ile Thr Leu Thr Cys Thr Cys Val Ala
        755                 760                 765

Ala Thr Leu Phe Trp Leu Leu Leu Thr Leu Phe Ile Arg Lys Met Lys
    770                 775                 780

Arg Ser Ser Ser Glu Ile Lys Thr Asp Tyr Leu Ser Ile Ile Met Asp
785                 790                 795                 800

Pro Asp Glu Val Pro Leu Asp Glu Gln Cys Glu Arg Leu Pro Tyr Asp
                805                 810                 815

Ala Ser Lys Trp Glu Phe Ala Arg Glu Arg Leu Lys Leu Gly Lys Ser
            820                 825                 830

Leu Gly Arg Gly Ala Phe Gly Lys Val Val Gln Ala Ser Ala Phe Gly
        835                 840                 845

Ile Lys Lys Ser Pro Thr Cys Arg Thr Val Ala Val Lys Met Leu Lys
    850                 855                 860

Glu Gly Ala Thr Ala Ser Glu Tyr Lys Ala Leu Met Thr Glu Leu Lys
865                 870                 875                 880

Ile Leu Thr His Ile Gly His His Leu Asn Val Val Asn Leu Leu Gly
                885                 890                 895

Ala Cys Thr Lys Gln Gly Gly Pro Leu Met Val Ile Val Glu Tyr Cys
            900                 905                 910

Lys Tyr Gly Asn Leu Ser Asn Tyr Leu Lys Ser Lys Arg Asp Leu Phe
        915                 920                 925

Phe Leu Asn Lys Asp Ala Ala Leu His Met Glu Pro Lys Lys Glu Lys
    930                 935                 940

Met Glu Pro Gly Leu Glu Gln Gly Lys Lys Pro Arg Leu Asp Ser Val
945                 950                 955                 960

Thr Ser Ser Glu Ser Phe Ala Ser Ser Gly Phe Gln Glu Asp Lys Ser
                965                 970                 975

Leu Ser Asp Val Glu Glu Glu Glu Asp Ser Asp Gly Phe Tyr Lys Glu
            980                 985                 990

Pro Ile Thr Met Glu Asp Leu Ile  Ser Tyr Ser Phe Gln  Val Ala Arg
        995                 1000                 1005

Gly Met  Glu Phe Leu Ser Ser  Arg Lys Cys Ile His  Arg Asp Leu
    1010                 1015                 1020

Ala Ala  Arg Asn Ile Leu Leu  Ser Glu Asn Asn Val  Val Lys Ile
    1025                 1030                 1035

Cys Asp  Phe Gly Leu Ala Arg  Asp Ile Tyr Lys Asn  Pro Asp Tyr
    1040                 1045                 1050

Val Arg  Lys Gly Asp Thr Arg  Leu Pro Leu Lys Trp  Met Ala Pro

1055                1060                1065

Glu Ser  Ile Phe Asp Lys Ile  Tyr Ser Thr Lys Ser  Asp Val Trp
    1070                1075                1080

Ser Tyr  Gly Val Leu Leu Trp  Glu Ile Phe Ser Leu  Gly Gly Ser
    1085                1090                1095

Pro Tyr  Pro Gly Val Gln Met  Asp Glu Asp Phe Cys  Ser Arg Leu
    1100                1105                1110

Arg Glu  Gly Met Arg Met Arg  Ala Pro Glu Tyr Ser  Thr Pro Glu
    1115                1120                1125

Ile Tyr  Gln Ile Met Leu Asp  Cys Trp His Arg Asp  Pro Lys Glu
    1130                1135                1140

Arg Pro  Arg Phe Ala Glu Leu  Val Glu Lys Leu Gly  Asp Leu Leu
    1145                1150                1155

Gln Ala  Asn Val Gln Gln Asp  Gly Lys Asp Tyr Ile  Pro Ile Asn
    1160                1165                1170

Ala Ile  Leu Thr Gly Asn Ser  Gly Phe Thr Tyr Ser  Thr Pro Ala
    1175                1180                1185

Phe Ser  Glu Asp Phe Phe Lys  Glu Ser Ile Ser Ala  Pro Lys Phe
    1190                1195                1200

Asn Ser  Gly Ser Ser Asp Asp  Val Arg Tyr Val Asn  Ala Phe Lys
    1205                1210                1215

Phe Met  Ser Leu Glu Arg Ile  Lys Thr Phe Glu Glu  Leu Leu Pro
    1220                1225                1230

Asn Ala  Thr Ser Met Phe Asp  Asp Tyr Gln Gly Asp  Ser Ser Thr
    1235                1240                1245

Leu Leu  Ala Ser Pro Met Leu  Lys Arg Phe Thr Trp  Thr Asp Ser
    1250                1255                1260

Lys Pro  Lys Ala Ser Leu Lys  Ile Asp Leu Arg Val  Thr Ser Lys
    1265                1270                1275

Ser Lys  Glu Ser Gly Leu Ser  Asp Val Ser Arg Pro  Ser Phe Cys
    1280                1285                1290

His Ser Ser Cys Gly His Val Ser Glu Gly Lys Arg Arg Phe Thr
    1295                1300                1305

Tyr Asp His Ala Glu Leu Glu Arg Lys Ile Ala Cys Cys Ser Pro
    1310                1315                1320

Pro Pro Asp Tyr Asn Ser Val Val Leu Tyr Ser Thr Pro Pro Ile
    1325                1330                1335

<210> 9
<211> 5830
<212> DNA
<213> Homo sapiens

<400> 9
actgagtccc gggaccccgg gagagcggtc agtgtgtggt cgctgcgttt cctctgcctg 60

cgccgggcat cacttgcgcg ccgcagaaag tccgtctggc agcctggata tcctctccta 120

ccggcacccg cagacgcccc tgcagccgcc ggtcggcgcc cgggctccct agccctgtgc 180

gctcaactgt cctgcgctgc ggggtgccgc gagttccacc tccgcgcctc cttctctaga 240

caggcgctgg gagaaagaac cggctcccga gttctgggca tttcgcccgg ctcgaggtgc 300

aggatgcaga gcaaggtgct gctggccgtc gccctgtggc tctgcgtgga gacccgggcc 360

gcctctgtgg gtttgcctag tgtttctctt gatctgccca ggctcagcat acaaaaagac 420

atacttacaa ttaaggctaa tacaactctt caaattactt gcaggggaca gagggacttg 480

gactggcttt ggcccaataa tcagagtggc agtgagcaaa gggtggaggt gactgagtgc 540

agcgatggcc tcttctgtaa gacactcaca attccaaaag tgatcggaaa tgacactgga 600

gcctacaagt gcttctaccg ggaaactgac ttggcctcgg tcatttatgt ctatgttcaa 660

gattacagat ctccatttat tgcttctgtt agtgaccaac atggagtcgt gtacattact 720

gagaacaaaa acaaaactgt ggtgattcca tgtctcgggt ccatttcaaa tctcaacgtg 780

tcactttgtg caagataccc agaaaagaga tttgttcctg atggtaacag aatttcctgg 840

gacagcaaga agggctttac tattcccagc tacatgatca gctatgctgg catggtcttc 900

tgtgaagcaa aaattaatga tgaaagttac cagtctatta tgtacatagt tgtcgttgta 960

gggtatagga tttatgatgt ggttctgagt ccgtctcatg gaattgaact atctgttgga 1020

gaaaagcttg tcttaaattg tacagcaaga actgaactaa atgtggggat tgacttcaac 1080

tgggaatacc cttcttcgaa gcatcagcat aagaaacttg taaaccgaga cctaaaaacc 1140

cagtctggga gtgagatgaa gaaatttttg agcaccttaa ctatagatgg tgtaacccgg 1200

agtgaccaag gattgtacac ctgtgcagca tccagtgggc tgatgaccaa gaagaacagc 1260

acatttgtca gggtccatga aaaacctttt gttgcttttg gaagtggcat ggaatctctg 1320

gtggaagcca cggtggggga gcgtgtcaga atccctgcga agtaccttgg ttacccaccc 1380

ccagaaataa aatggtataa aaatggaata ccccttgagt ccaatcacac aattaaagcg 1440

gggcatgtac tgacgattat ggaagtgagt gaaagagaca caggaaatta cactgtcatc 1500

cttaccaatc ccatttcaaa ggagaagcag agccatgtgg tctctctggt tgtgtatgtc 1560

ccaccccaga ttggtgagaa atctctaatc tctcctgtgg attcctacca gtacggcacc 1620

actcaaacgc tgacatgtac ggtctatgcc attcctcccc cgcatcacat ccactggtat 1680

tggcagttgg aggaagagtg cgccaacgag cccagccaag ctgtctcagt gacaaaccca 1740

tacccttgtg aagaatggag aagtgtggag gacttccagg gaggaaataa aattgaagtt 1800

aataaaaatc aatttgctct aattgaagga aaaaacaaaa ctgtaagtac ccttgttatc 1860

caagcggcaa atgtgtcagc tttgtacaaa tgtgaagcgg tcaacaaagt cgggagagga 1920

gagagggtga tctccttcca cgtgaccagg ggtcctgaaa ttactttgca acctgacatg 1980

cagcccactg agcaggagag cgtgtctttg tggtgcactg cagacagatc tacgtttgag 2040

aacctcacat ggtacaagct tggcccacag cctctgccaa tccatgtggg agagttgccc 2100

acacctgttt gcaagaactt ggatactctt tggaaattga atgccaccat gttctctaat 2160

agcacaaatg acattttgat catggagctt aagaatgcat ccttgcagga ccaaggagac 2220

tatgtctgcc ttgctcaaga caggaagacc aagaaaagac attgcgtggt caggcagctc 2280

acagtcctag agcgtgtggc acccacgatc acaggaaacc tggagaatca gacgacaagt 2340

attggggaaa gcatcgaagt ctcatgcacg gcatctggga atccccctcc acagatcatg 2400

tggtttaaag ataatgagac ccttgtagaa gactcaggca ttgtattgaa ggatgggaac 2460

cggaacctca ctatccgcag agtgaggaag gaggacgaag gcctctacac ctgccaggca 2520

tgcagtgttc ttggctgtgc aaaagtggag gcatttttca taatagaagg tgcccaggaa 2580

aagacgaact tggaaatcat tattctagta ggcacggcgg tgattgccat gttcttctgg 2640

ctacttcttg tcatcatcct acggaccgtt aagcgggcca atggagggga actgaagaca 2700

ggctacttgt ccatcgtcat ggatccagat gaactcccat tggatgaaca ttgtgaacga 2760

ctgccttatg atgccagcaa atgggaattc cccagagacc ggctgaagct aggtaagcct 2820

cttggccgtg gtgcctttgg ccaagtgatt gaagcagatg cctttggaat tgacaagaca 2880

gcaacttgca ggacagtagc agtcaaaatg ttgaaagaag gagcaacaca cagtgagcat 2940

cgagctctca tgtctgaact caagatcctc attcatattg gtcaccatct caatgtggtc 3000

aaccttctag gtgcctgtac caagccagga gggccactca tggtgattgt ggaattctgc 3060

aaatttggaa acctgtccac ttacctgagg agcaagagaa atgaatttgt cccctacaag 3120

accaaagggg cacgattccg tcaagggaaa gactacgttg gagcaatccc tgtggatctg 3180

aaacggcgct tggacagcat caccagtagc cagagctcag ccagctctgg atttgtggag 3240

gagaagtccc tcagtgatgt agaagaagag gaagctcctg aagatctgta taaggacttc 3300

ctgaccttgg agcatctcat ctgttacagc ttccaagtgg ctaagggcat ggagttcttg 3360

gcatcgcgaa agtgtatcca cagggacctg gcggcacgaa atatcctctt atcggagaag 3420

aacgtggtta aaatctgtga ctttggcttg gcccgggata tttataaaga tccagattat 3480

gtcagaaaag gagatgctcg cctccctttg aaatggatgg ccccagaaac aatttttgac 3540

agagtgtaca caatccagag tgacgtctgg tcttttggtg ttttgctgtg ggaaatattt 3600

tccttaggtg cttctccata tcctggggta aagattgatg aagaattttg taggcgattg 3660

aaagaaggaa ctagaatgag ggcccctgat tatactacac cagaaatgta ccagaccatg 3720

ctggactgct ggcacgggga gcccagtcag agacccacgt tttcagagtt ggtggaacat 3780

ttgggaaatc tcttgcaagc taatgctcag caggatggca aagactacat tgttcttccg 3840

atatcagaga ctttgagcat ggaagaggat tctggactct ctctgcctac ctcacctgtt 3900

tcctgtatgg aggaggagga agtatgtgac cccaaattcc attatgacaa cacagcagga 3960

atcagtcagt atctgcagaa cagtaagcga aagagccggc ctgtgagtgt aaaaacattt 4020

gaagatatcc cgttagaaga accagaagta aaagtaatcc cagatgacaa ccagacggac 4080

agtggtatgg ttcttgcctc agaagagctg aaaactttgg aagacagaac caaattatct 4140

ccatcttttg gtggaatggt gcccagcaaa agcagggagt ctgtggcatc tgaaggctca 4200

aaccagacaa gcggctacca gtccggatat cactccgatg acacagacac caccgtgtac 4260

tccagtgagg aagcagaact tttaaagctg atagagattg gagtgcaaac cggtagcaca 4320

gcccagattc tccagcctga ctcggggacc acactgagct ctcctcctgt ttaaaaggaa 4380

gcatccacac cccaactccc ggacatcaca tgagaggtct gctcagattt tgaagtgttg 4440

ttctttccac cagcaggaag tagccgcatt tgattttcat ttcgacaaca gaaaaaggac 4500

ctcggactgc agggagccag tcttctaggc atatcctgga agaggcttgt gacccaagaa 4560

tgtgtctgtg tcttctccca gtgttgacct gatcctcttt tttcattcat ttaaaaagca 4620

ttatcatgcc cctgctgcgg gtctcaccat gggtttagaa caaagagctt caagcaatgg 4680

ccccatcctc aaagaagtag cagtacctgg ggagctgaca cttctgtaaa actagaagat 4740

aaaccaggca acgtaagtgt tcgaggtgtt gaagatggga aggatttgca gggctgagtc 4800

tatccaagag gctttgttta ggacgtgggt cccaagccaa gccttaagtg tggaattcgg 4860

attgatagaa aggaagacta acgttacctt gctttggaga gtactggagc ctgcaaatgc 4920

attgtgtttg ctctggtgga ggtgggcatg gggtctgttc tgaaatgtaa agggttcaga 4980

cggggtttct ggttttagaa ggttgcgtgt tcttcgagtt gggctaaagt agagttcgtt 5040

gtgctgtttc tgactcctaa tgagagttcc ttccagaccg ttagctgtct ccttgccaag 5100

ccccaggaag aaaatgatgc agctctggct ccttgtctcc caggctgatc ctttattcag 5160

aataccacaa agaaaggaca ttcagctcaa ggctccctgc cgtgttgaag agttctgact 5220

gcacaaacca gcttctggtt tcttctggaa tgaataccct catatctgtc ctgatgtgat 5280

atgtctgaga ctgaatgcgg gaggttcaat gtgaagctgt gtgtggtgtc aaagtttcag 5340

gaaggatttt acccttttgt tcttccccct gtccccaacc cactctcacc ccgcaaccca 5400

tcagtatttt agttatttgg cctctactcc agtaaacctg attgggtttg ttcactctct 5460

gaatgattat tagccagact tcaaaattat tttatagccc aaattataac atctattgta 5520

ttatttagac ttttaacata tagagctatt tctactgatt tttgcccttg ttctgtcctt 5580

tttttcaaaa aagaaaatgt gttttttgtt tggtaccata gtgtgaaatg ctgggaacaa 5640

tgactataag acatgctatg gcacatatat ttatagtctg tttatgtaga aacaaatgta 5700

atatattaaa gccttatata taatgaactt tgtactattc acattttgta tcagtattat 5760

gtagcataac aaaggtcata atgctttcag caattgatgt cattttatta aagaacattg 5820

aaaaacttga 5830

<210> 10
<211> 1356
<212> PRT
<213> Homo sapiens

<400> 10

<210>  11
<211>  2305
<212>  DNA
<213>  Homo sapiens

<400>  11
   ttcttggggc tgatgtccgc aaatatgcag aattaccggc cgggtcgctc ctgaagccag      60
   cgcggggagc gagcgcggcg gcggccagca ccgggaacgc accgaggaag aagcccagcc     120
   cccgccctcc gccccttccg tccccacccc ctacccggcg gcccaggagg ctccccggct     180
   gcggcgcgca ctccctgttt ctcctcctcc tggctggcgc tgcctgcctc tccgcactca     240
   ctgctcgccg ggcgccgtcc gccagctccg tgctccccgc gccaccctcc tccgggccgc     300
   gctccctaag ggatggtact gaatttcgcc gccacaggag accggctgga gcgcccgccc     360
   cgcgcctcgc ctctcctccg agcagccagc gcctcgggac gcgatgagga ccttggcttg     420
   cctgctgctc ctcggctgcg gatacctcgc ccatgttctg gccgaggaag ccgagatccc     480
   ccgcgaggtg atcgagaggc tggcccgcag tcagatccac agcatccggg acctccagcg     540
   actcctggag atagactccg tagggagtga ggattctttg gacaccagcc tgagagctca     600
   cggggtccac gccactaagc atgtgcccga gaagcggccc ctgcccattc ggaggaagag     660
   aagcatcgag gaagctgtcc ccgctgtctg caagaccagg acggtcattt acgagattcc     720
   tcggagtcag gtcgacccca cgtccgccaa cttcctgatc tggcccccgt gcgtggaggt     780
   gaaacgctgc accggctgct gcaacacgag cagtgtcaag tgccagccct cccgcgtcca     840
   ccaccgcagc gtcaaggtgg ccaaggtgga atacgtcagg aagaagccaa aattaaaaga     900
   agtccaggtg aggttagagg agcatttgga gtgcgcctgc gcgaccacaa gcctgaatcc     960
   ggattatcgg gaagaggaca cggatgtgag gtgaggatga gccgcagccc tttcctggga    1020
   catggatgta catggcgtgt tacattcctg aacctactat gtacggtgct ttattgccag    1080
   tgtgcggtct ttgttctcct ccgtgaaaaa ctgtgtccga gaacactcgg gagaacaaag    1140
   agacagtgca catttgttta atgtgacatc aaagcaagta ttgtagcact cggtgaagca    1200
   gtaagaagct tccttgtcaa aaagagagag agagagagag agagagaaaa caaaaccaca    1260
   aatgacaaaa acaaaacgga ctcacaaaaa tatctaaact cgatgagatg gagggtcgcc    1320
   ccgtgggatg gaagtgcaga ggtctcagca gactggattt ctgtccgggt ggtcacaggt    1380
   gcttttttgc cgaggatgca gagcctgctt tgggaacgac tccagagggg tgctggtggg    1440
   ctctgcaggg cccgcaggaa gcaggaatgt cttggaaacc gccacgcgaa ctttagaaac    1500
   cacacctcct cgctgtagta tttaagccca tacagaaacc ttcctgagag ccttaagtgg    1560
   tttttttttt tgtttttgtt ttgttttttt tttttttgtt tttttttttt tttttttttt    1620
   ttacaccata aagtgattat taagcttcct tttactcttt ggctagcttt tttttttttt 1680
   tttttttttt ttttttttaa ttatctcttg gatgacattt acaccgataa cacacaggct 1740
   gctgtaactg tcaggacagt gcgacggtat ttttcctagc aagatgcaaa ctaatgagat 1800
   gtattaaaat aaacatggta tacctaccta tgcatcattt cctaaatgtt tctggctttg 1860
   tgtttctccc ttaccctgct ttatttgtta atttaagcca ttttgaaaga actatgcgtc 1920
   aaccaatcgt acgccgtccc tgcggcacct gccccagagc ccgtttgtgg ctgagtgaca 1980
   acttgttccc cgcagtgcac acctagaatg ctgtgttccc acgcggcacg tgagatgcat 2040
   tgccgcttct gtctgtgttg ttggtgtgcc ctggtgccgt ggtggcggtc actccctctg 2100
   ctgccagtgt ttggacagaa cccaaattct ttatttttgg taagatattg tgctttacct 2160
   gtattaacag aaatgtgtgt gtgtggtttg tttttttgta aaggtgaagt ttgtatgttt 2220
   acctaatatt acctgttttg tatacctgag agcctgctat gttcttcttt tgttgatcca 2280
   aaattaaaaa aaaaatacca ccaac 2305

<210> 12
<211> 196
<212> PRT
<213> Homo sapiens

<400> 12

Met Arg Thr Leu Ala Cys Leu Leu Leu Leu Gly Cys Gly Tyr Leu Ala
1               5                   10                  15

His Val Leu Ala Glu Glu Ala Glu Ile Pro Arg Glu Val Ile Glu Arg
            20                  25                  30

Leu Ala Arg Ser Gln Ile His Ser Ile Arg Asp Leu Gln Arg Leu Leu
        35                  40                  45

Glu Ile Asp Ser Val Gly Ser Glu Asp Ser Leu Asp Thr Ser Leu Arg
    50                  55                  60

Ala His Gly Val His Ala Thr Lys His Val Pro Glu Lys Arg Pro Leu
65                  70                  75                  80

Pro Ile Arg Arg Lys Arg Ser Ile Glu Glu Ala Val Pro Ala Val Cys
                85                  90                  95

Lys Thr Arg Thr Val Ile Tyr Glu Ile Pro Arg Ser Gln Val Asp Pro
            100                 105                 110

Thr Ser Ala Asn Phe Leu Ile Trp Pro Pro Cys Val Glu Val Lys Arg
        115                 120                 125

Cys Thr Gly Cys Cys Asn Thr Ser Ser Val Lys Cys Gln Pro Ser Arg
    130                 135                 140

Val His His Arg Ser Val Lys Val Ala Lys Val Glu Tyr Val Arg Lys
145                 150                 155                 160

Lys Pro Lys Leu Lys Glu Val Gln Val Arg Leu Glu Glu His Leu Glu
                165                 170                 175

Cys Ala Cys Ala Thr Thr Ser Leu Asn Pro Asp Tyr Arg Glu Glu Asp
            180                 185                 190

Thr Asp Val Arg
        195

<210>  13
<211>  6633
<212>  DNA
<213>  Homo sapiens

<400>  13
   ttctccccgc cccccagttg ttgtcgaagt ctgggggttg ggactggacc ccctgattgc      60
   gtaagagcaa aaagcgaagg cgcaatctgg acactgggag attcggagcg cagggagttt     120
   gagagaaact tttattttga agagaccaag gttgaggggg ggcttatttc ctgacagcta     180
   tttacttaga gcaaatgatt agttttagaa ggatggacta taacattgaa tcaattacaa     240
   aacgcggttt ttgagcccat tactgttgga gctacaggga gagaaacagg aggagactgc     300
   aagagatcat ttgggaaggc cgtgggcacg ctctttactc catgtgtggg acattcattg     360
   cggaataaca tcggaggaga agtttcccag agctatgggg acttcccatc cggcgttcct     420
   ggtcttaggc tgtcttctca cagggctgag cctaatcctc tgccagcttt cattaccctc     480
   tatccttcca aatgaaaatg aaaaggttgt gcagctgaat tcatcctttt ctctgagatg     540
   ctttggggag agtgaagtga gctggcagta ccccatgtct gaagaagaga gctccgatgt     600
   ggaaatcaga aatgaagaaa acaacagcgg cctttttgtg acggtcttgg aagtgagcag     660
   tgcctcggcg gcccacacag ggttgtacac ttgctattac aaccacactc agacagaaga     720
   gaatgagctt gaaggcaggc acatttacat ctatgtgcca gacccagatg tagcctttgt     780
   acctctagga atgacggatt atttagtcat cgtggaggat gatgattctg ccattatacc     840
   ttgtcgcaca actgatcccg agactcctgt aaccttacac aacagtgagg gggtggtacc     900
   tgcctcctac gacagcagac agggctttaa tgggaccttc actgtagggc cctatatctg     960
   tgaggccacc gtcaaaggaa agaagttcca gaccatccca tttaatgttt atgctttaaa    1020
   agcaacatca gagctggatc tagaaatgga agctcttaaa accgtgtata agtcagggga    1080
   aacgattgtg gtcacctgtg ctgtttttaa caatgaggtg gttgaccttc aatggactta 1140
   ccctggagaa gtgaaaggca aaggcatcac aatgctggaa gaaatcaaag tcccatccat 1200
   caaattggtg tacactttga cggtccccga ggccacggtg aaagacagtg gagattacga 1260
   atgtgctgcc cgccaggcta ccagggaggt caaagaaatg aagaaagtca ctatttctgt 1320
   ccatgagaaa ggtttcattg aaatcaaacc caccttcagc cagttggaag ctgtcaacct 1380
   gcatgaagtc aaacattttg ttgtagaggt gcgggcctac ccacctccca ggatatcctg 1440
   gctgaaaaac aatctgactc tgattgaaaa tctcactgag atcaccactg atgtggaaaa 1500
   gattcaggaa ataaggtatc gaagcaaatt aaagctgatc cgtgctaagg aagaagacag 1560
   tggccattat actattgtag ctcaaaatga agatgctgtg aagagctata cttttgaact 1620
   gttaactcaa gttccttcat ccattctgga cttggtcgat gatcaccatg gctcaactgg 1680
   gggacagacg gtgaggtgca cagctgaagg cacgccgctt cctgatattg agtggatgat 1740
   atgcaaagat attaagaaat gtaataatga aacttcctgg actattttgg ccaacaatgt 1800
   ctcaaacatc atcacggaga tccactcccg agacaggagt accgtggagg gccgtgtgac 1860
   tttcgccaaa gtggaggaga ccatcgccgt gcgatgcctg gctaagaatc tccttggagc 1920
   tgagaaccga gagctgaagc tggtggctcc caccctgcgt tctgaactca cggtggctgc 1980
   tgcagtcctg gtgctgttgg tgattgtgat catctcactt attgtcctgg ttgtcatttg 2040
   gaaacagaaa ccgaggtatg aaattcgctg gagggtcatt gaatcaatca gcccggatgg 2100
   acatgaatat atttatgtgg acccgatgca gctgccttat gactcaagat gggagtttcc 2160
   aagagatgga ctagtgcttg gtcgggtctt ggggtctgga gcgtttggga aggtggttga 2220
   aggaacagcc tatggattaa gccggtccca acctgtcatg aaagttgcag tgaagatgct 2280
   aaaacccacg gccagatcca gtgaaaaaca agctctcatg tctgaactga agataatgac 2340
   tcacctgggg ccacatttga acattgtaaa cttgctggga gcctgcacca agtcaggccc 2400
   catttacatc atcacagagt attgcttcta tggagatttg gtcaactatt tgcataagaa 2460
   tagggatagc ttcctgagcc accacccaga gaagccaaag aaagagctgg atatctttgg 2520
   attgaaccct gctgatgaaa gcacacggag ctatgttatt ttatcttttg aaaacaatgg 2580
   tgactacatg gacatgaagc aggctgatac tacacagtat gtccccatgc tagaaaggaa 2640
   agaggtttct aaatattccg acatccagag atcactctat gatcgtccag cctcatataa 2700
   gaagaaatct atgttagact cagaagtcaa aaacctcctt tcagatgata actcagaagg 2760
   ccttacttta ttggatttgt tgagcttcac ctatcaagtt gcccgaggaa tggagttttt 2820
   ggcttcaaaa aattgtgtcc accgtgatct ggctgctcgc aacgtcctcc tggcacaagg 2880
   aaaaattgtg aagatctgtg actttggcct ggccagagac atcatgcatg attcgaacta 2940
   tgtgtcgaaa ggcagtacct ttctgcccgt gaagtggatg gctcctgaga gcatctttga 3000
   caacctctac accacactga gtgatgtctg gtcttatggc attctgctct gggagatctt 3060
   ttcccttggt ggcacccctt accccggcat gatggtggat tctactttct acaataagat 3120
   caagagtggg taccggatgg ccaagcctga ccacgctacc agtgaagtct acgagatcat 3180
   ggtgaaatgc tggaacagtg agccggagaa gagaccctcc ttttaccacc tgagtgagat 3240
   tgtggagaat ctgctgcctg gacaatataa aaagagttat gaaaaaattc acctggactt 3300
   cctgaagagt gaccatcctg ctgtggcacg catgcgtgtg gactcagaca atgcatacat 3360
   tggtgtcacc tacaaaaacg aggaagacaa gctgaaggac tgggagggtg gtctggatga 3420
   gcagagactg agcgctgaca gtggctacat cattcctctg cctgacattg accctgtccc 3480
   tgaggaggag gacctgggca agaggaacag acacagctcg cagacctctg aagagagtgc 3540
   cattgagacg ggttccagca gttccacctt catcaagaga gaggacgaga ccattgaaga 3600
   catcgacatg atggacgaca tcggcataga ctcttcagac ctggtggaag acagcttcct 3660
   gtaactggcg gattcgaggg gttccttcca cttctggggc cacctctgga tcccgttcag 3720
   aaaaccactt tattgcaatg cggaggttga gaggaggact tggttgatgt ttaaagagaa 3780
   gttcccagcc aagggcctcg gggagcgttc taaatatgaa tgaatgggat attttgaaat 3840
   gaactttgtc agtgttgcct ctcgcaatgc ctcagtagca tctcagtggt gtgtgaagtt 3900
   tggagataga tggataaggg aataataggc cacagaaggt gaactttgtg cttcaaggac 3960
   attggtgaga gtccaacaga cacaatttat actgcgacag aacttcagca ttgtaattat 4020
   gtaaataact ctaaccaagg ctgtgtttag attgtattaa ctatcttctt tggacttctg 4080
   aagagaccac tcaatccatc catgtacttc cctcttgaaa cctgatgtca gctgctgttg 4140
   aactttttaa agaagtgcat gaaaaaccat ttttgaacct taaaaggtac tggtactata 4200
   gcattttgct atctttttta gtgttaagag ataaagaata ataattaacc aaccttgttt 4260
   aatagatttg ggtcatttag aagcctgaca actcattttc atattgtaat ctatgtttat 4320
   aatactacta ctgttatcag taatgctaaa tgtgtaataa tgtaacatga tttccctcca 4380
   gagaaagcac aatttaaaac aatccttact aagtaggtga tgagtttgac agtttttgac 4440
   atttatatta aataacatgt ttctctataa agtatggtaa tagctttagt gaattaaatt 4500
   tagttgagca tagagaacaa agtaaaagta gtgttgtcca ggaagtcaga atttttaact 4560
   gtactgaata ggttccccaa tccatcgtat taaaaaacaa ttaactgccc tctgaaataa 4620
   tgggattaga aacaaacaaa actcttaagt cctaaaagtt ctcaatgtag aggcataaac 4680
   ctgtgctgaa cataacttct catgtatatt acccaatgga aaatataatg atcagcaaaa 4740
   agactggatt tgcagaagtt tttttttttt ttcttcatgc ctgatgaaag ctttggcaac 4800
   cccaatatat gtattttttg aatctatgaa cctgaaaagg gtcagaagga tgcccagaca 4860
   tcagcctcct tctttcaccc cttaccccaa agagaaagag tttgaaactc gagaccataa 4920
   agatattctt tagtggaggc tggatgtgca ttagcctgga tcctcagttc tcaaatgtgt 4980
   gtggcagcca ggatgactag atcctgggtt tccatccttg agattctgaa gtatgaagtc 5040
   tgagggaaac cagagtctgt atttttctaa actccctggc tgttctgatc ggccagtttt 5100
   cggaaacact gacttaggtt tcaggaagtt gccatgggaa acaaataatt tgaactttgg 5160
   aacagggttg gaattcaacc acgcaggaag cctactattt aaatccttgg cttcaggtta 5220
   gtgacattta atgccatcta gctagcaatt gcgaccttaa tttaactttc cagtcttagc 5280
   tgaggctgag aaagctaaag tttggttttg acaggttttc caaaagtaaa gatgctactt 5340
   cccactgtat gggggagatt gaactttccc cgtctcccgt cttctgcctc ccactccata 5400
   ccccgccaag gaaaggcatg tacaaaaatt atgcaattca gtgttccaag tctctgtgta 5460
   accagctcag tgttttggtg gaaaaaacat tttaagtttt actgataatt tgaggttaga 5520
   tgggaggatg aattgtcaca tctatccaca ctgtcaaaca ggttggtgtg ggttcattgg 5580
   cattctttgc aatactgctt aattgctgat accatatgaa tgaaacatgg gctgtgatta 5640
   ctgcaatcac tgtgctatcg gcagatgatg ctttggaaga tgcagaagca ataataaagt 5700
   acttgactac ctactggtgt aatctcaatg caagccccaa ctttcttatc caactttttc 5760
   atagtaagtg cgaagactga gccagattgg ccaattaaaa acgaaaacct gactaggttc 5820
   tgtagagcca attagacttg aaatacgttt gtgtttctag aatcacagct caagcattct 5880
   gtttatcgct cactctccct tgtacagcct tattttgttg gtgctttgca ttttgatatt 5940
   gctgtgagcc ttgcatgaca tcatgaggcc ggatgaaact tctcagtcca gcagtttcca 6000
   gtcctaacaa atgctcccac ctgaatttgt atatgactgc atttgtgggt gtgtgtgtgt 6060
   tttcagcaaa ttccagattt gtttcctttt ggcctcctgc aaagtctcca gaagaaaatt 6120
   tgccaatctt tcctactttc tatttttatg atgacaatca aagccggcct gagaaacact 6180
   atttgtgact ttttaaacga ttagtgatgt ccttaaaatg tggtctgcca atctgtacaa 6240
   aatggtccta tttttgtgaa gagggacata agataaaatg atgttataca tcaatatgta 6300
   tatatgtatt tctatataga cttggagaat actgccaaaa catttatgac aagctgtatc 6360
   actgccttcg tttatatttt tttaactgtg ataatcccca caggcacatt aactgttgca 6420
   cttttgaatg tccaaaattt atattttaga aataataaaa agaaagatac ttacatgttc 6480
   ccaaaacaat ggtgtggtga atgtgtgaga aaaactaact tgatagggtc taccaataca 6540
   aaatgtatta cgaatgcccc tgttcatgtt tttgttttaa aacgtgtaaa tgaagatctt 6600
   tatatttcaa taaatgatat ataatttaaa gtt 6633

<210> 14
<211> 1089

<212>  PRT
<213>  Homo sapiens

<400>  14

<210>  15
<211>  10
<212>  RNA
<213>  Artificial Sequence

<220>
<223>  Synthetic anti-VEGF aptamer

<400>  15
gaagaauugg                                                            10

<210>  16
<211>  8
<212>  RNA
<213>  Artificial Sequence

<220>
<223>  Synthetic anti-VEGF aptamer

<400>  16
uuggacgc                                                              8

<210>  17
<211>  8
<212>  RNA
<213>  Artificial Sequence

<220>
<223>  Synthetic anti-VEGF aptamer

<400>  17
gugaaugc                                                              8

<210>  18
<211>  27
<212>  RNA
<213>  Artificial Sequence

<220>
<223>  Synthetic anti-VEGF aptamer

<400>  18
cggaaucagu gaaugcuuau acauccg                                          27


<210>  19
<211>  27
<212>  RNA
<213>  Artificial Sequence

<220>
<223>  Synthetic anti-VEGF aptamer

<400>  19
cggaaucagu gaaugcuuau acauccg                                          27


<210>  20
<211>  32
<212>  DNA
<213>  Artificial Sequence

<220>
<223>  Synthetic anti-PDGF aptamer


<220>
<221>  modified_base
<222>  (10)..(10)
<223>  n=hexaethylene-glycol phosphoramidite

<220>
<221>  misc_feature
<222>  (10)..(10)
<223>  n is a, c, g, t or u

<220>
<221>  modified_base
<222>  (23)..(23)
<223>  n=hexaethylene-glycol phosphoramidite

<220>
<221>  misc_feature
<222>  (23)..(23)
<223>  n is a, c, g, t or u

<400>  20
caggcuacgn cgtagagcau cantgatccu gt                                    32


<210>  21
<211>  30
<212>  DNA
<213>  Artificial Sequence

<220>
<223>  Synthetic anti-PDGF aptamer

<400>  21
caggctacgc gtagagcatc atgatcctgt                                        30


<210>  22
<211>  32
<212>  DNA
<213>  Artificial Sequence

<220>
<223>  Synthetic anti-PDGF aptamer


<220>
<221>  misc_feature
<222>  (10)..(10)
<223>  n is a, c, g, t or u

<220>
<221>  misc_feature
<222>  (23)..(23)
<223>  n is a, c, g, t or u

<400>  22
caggcuacgn cgtagagcau cantgatccu gt                                     32


<210>  23
<211>  32
<212>  DNA
<213>  Artificial Sequence

<220>
<223>  Synthetic anti-PDGF aptamer


<220>
<221>  misc_feature
<222>  (1)..(32)
<223>  U at positions 6, 20 and 30 is 2'-fluoro-2'-deoxyuridine

<220>
<221>  misc_feature
<222>  (1)..(32)
<223>  C at positions 8, 21, 28 and 29 is 2'-fluoro-2'-deoxycytidine

<220>
<221>  misc_feature
<222>  (1)..(32)
<223>  G at positions 9, 15, 17 and 31 is 2'-O-Methyl-2'-deoxyguanosine

<220>
<221>  misc_feature
<222>  (1)..(32)
<223>  A at position 22 is 2'-O-Methyl-2'-deoxyadenosine

<220>
<221>  misc_feature
<222>  (1)..(32)

<223>  N at positions 10 and 23 is from a hexaethylene glycol
       phosphoramidite

<220>
<221>  misc_feature
<222>  (32)..(32)
<223>  Nucleotide 32 is an inverted orientation T (3'-3'-linked)

<400>  23
caggcuacgn cgtagagcau cantgatccu gt                                    32


<210>  24
<211>  118
<212>  PRT
<213>  Artificial Sequence

<220>
<223>  Synthetic anti-VEGF antibody

<400>  24

Glu Val Gln Leu Val Glu Ser Gly Gly Gly Leu Val Gln Pro Gly Gly
1               5                   10                  15


Ser Leu Arg Leu Ser Cys Ala Ala Ser Gly Tyr Asp Phe Thr His Tyr
            20                  25                  30


Gly Met Asn Trp Val Arg Gln Ala Pro Gly Lys Gly Leu Glu Trp Val
        35                  40                  45


Gly Trp Ile Asn Thr Tyr Thr Gly Glu Pro Thr Tyr Ala Ala Asp Phe
    50                  55                  60


Lys Arg Arg Phe Thr Phe Ser Leu Asp Thr Ser Lys Ser Thr Ala Tyr
65                  70                  75                  80


Leu Gln Met Asn Ser Leu Arg Ala Glu Asp Thr Ala Val Tyr Tyr Cys
                85                  90                  95


Ala Lys Tyr Pro Tyr Tyr Tyr Gly Thr Ser His Trp Tyr Phe Asp Val
            100                 105                 110


Trp Gly Gln Gly Thr Leu
        115


<210>  25
<211>  110
<212>  PRT
<213>  Artificial Sequence

<220>
<223>  Synthetic anti-VEGF antibody

<400>  25

Asp Ile Gln Leu Thr Gln Ser Pro Ser Ser Leu Ser Ala Ser Val Gly
1               5                   10                  15

Asp Arg Val Thr Ile Thr Cys Ser Ala Ser Gln Asp Ile Ser Asn Tyr
            20                  25                  30

Leu Asn Trp Tyr Gln Gln Lys Pro Gly Lys Ala Pro Lys Val Leu Ile
        35                  40                  45

Tyr Phe Thr Ser Ser Leu His Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Ser Thr Val Pro Trp
                85                  90                  95

Thr Phe Gly Gln Gly Thr Lys Val Glu Ile Lys Arg Thr Val
            100                 105                 110

<210>  26
<211>  118
<212>  PRT
<213>  Artificial Sequence

<220>
<223>  Synthetic anti-VEGF antibody

<400>  26

Glu Val Gln Leu Val Glu Ser Gly Gly Gly Leu Val Gln Pro Gly Gly
1               5                   10                  15

Ser Leu Arg Leu Ser Cys Ala Ala Ser Gly Tyr Thr Phe Thr Asn Tyr
            20                  25                  30

Gly Met Asn Trp Val Arg Gln Ala Pro Gly Lys Gly Leu Glu Trp Val
        35                  40                  45

Gly Trp Ile Asn Thr Tyr Thr Gly Glu Pro Thr Tyr Ala Ala Asp Phe
    50                  55                  60

Lys Arg Arg Phe Thr Phe Ser Leu Asp Thr Ser Lys Ser Thr Ala Tyr
65                  70                  75                  80

Leu Gln Met Asn Ser Leu Arg Ala Glu Asp Thr Ala Val Tyr Tyr Cys
                85                  90                  95

Ala Lys Tyr Pro His Tyr Tyr Gly Ser Ser His Trp Tyr Phe Asp Val
            100                 105                 110

Trp Gly Gln Gly Thr Leu
        115

<210>  27
<211>  110
<212>  PRT
<213>  Artificial Sequence

<220>
<223>  Synthetic anti-VEGF antibody

<400>  27

Asp Ile Gln Met Thr Gln Ser Pro Ser Ser Leu Ser Ala Ser Val Gly
1               5                   10                  15

Asp Arg Val Thr Ile Thr Cys Ser Ala Ser Gln Asp Ile Ser Asn Tyr
            20                  25                  30

Leu Asn Trp Tyr Gln Gln Lys Pro Gly Lys Ala Pro Lys Val Leu Ile
        35                  40                  45

Tyr Phe Thr Ser Ser Leu His Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Ser Thr Val Pro Trp
                85                  90                  95

Thr Phe Gly Gln Gly Thr Lys Val Glu Ile Lys Arg Thr Val
            100                 105                 110